# EUROPÄISCHE PATENTSCHRIFT

(11) **EP 1 515 983 B1**
(45) Veröffentlichungstag und Bekanntmachung des Hinweises auf die Patenterteilung: **21.02.2007**
(21) Anmeldenummer: 03738012.8
(22) Anmeldetag: 10.06.2003
(51) Int. Cl.: C07K 5/00

(54) **ANTIBAKTERIELLE AMID-MAKROZYKLEN**
ANTIBACTERIAL AMIDE MACROCYCLES
MACROCYCLES D'AMIDE ANTIBACTERIENS

(30) Priorität: 17.06.2002 DE 10226921
(43) Veröffentlichungstag der Anmeldung: 23.03.2005
(73) Patentinhaber: AiCuris GmbH & Co. KG, 42117 Wuppertal (DE)
(72) Erfinder: LAMPE, Thomas, 40223 Düsseldorf (DE); ADELT, Isabelle, 40225 Düsseldorf (DE); BEYER, Dieter, 42289 Wuppertal (DE); BRUNNER, Nina, 45147 Essen (DE); ENDERMANN, Rainer, 42113 Wuppertal (DE); EHLERT, Kerstin, 42115 Wuppertal (DE); KROLL, Hein-Peter, 42115 Wuppertal (DE); VON NUSSBAUM, Franz, 40219 Düsseldorf (DE); RADDATZ, Siegfried, 51065 Köln (DE); RUDOLPH, Joachim, Guilford, CT 06437 (US); SCHIFFER, Guido, 42111 Wuppertal (DE); SCHUMACHER, Andreas, 79588 Efringen-Kirchen (DE); CANCHO-GRANDE, Yolanda, 40723 Hilden (DE); MICHELS, Martin, 42653 Solingen (DE); WEIGAND, Stefan, 42115 Wuppertal (DE)
(74) Vertreter: Findeisen, Marco
(86) Internationale Anmeldenummer: PCT/EP2003/006078
(87) Internationale Veröffentlichungsnummer: WO 2003/106480

(56) Entgegenhaltungen:
- US-A- 5 840 682
- DATABASE CAPLUS [Online] CHEMICAL ABSTRACTS SERVICE, COLUMBUS, OHIO, US; Database accession no. 1993:60099 XP002258609 & U. SCHMIDT ET AL.: "Amino acids and peptides. 84. Synthesis of biologically active cyclopeptides. 24. Total sysnthesis of the biphenomycins. III. Synthesis of biphenomycin B" SYNTHESIS., Bd. 10, 1992, Seiten 1025-1030, THIEME VERLAG, STUTTGART., DE ISSN: 0039-7881 in der Anmeldung erwähnt
- DATABASE CAPLUS [Online] CHEMICAL ABSTRACTS SERVICE, COLUMBUS, OHIO, US; Database accession no. 1987:637278 XP002258610 & K. RAJAMOORTHI ET AL.: "Stereochenistry of the cyclic tripeptide antibiotic WS-43708A" JOURNAL OF ORGANIC CHEMISTRY., Bd. 52, Nr. 24, 1987, Seiten 5435-5437, AMERICAN CHEMICAL SOCIETY, WASHINGTON, DC., US ISSN: 0022-3263 in der Anmeldung erwähnt
- DATABASE CAPLUS [Online] CHEMICAL ABSTRACTS SERVICE, COLUMBUS, OHIO, US; Database accession no. 1986:84995 XP002258611 & I. UCHIDA ET AL.: "Biphenomycins A and B, novel peptide antibiotics. II. Structural elucidation of biphenomycins A and B" JOURNAL OF ANTIBIOTICS., Bd. 38, Nr. 11, 1985, Seiten 1462-1468, JAPAN ANTIBIOTICS RESEARCH ASSOCIATION, TOKYO., JP ISSN: 0021-8820 in der Anmeldung erwähnt
- DATABASE CAPLUS [Online] CHEMICAL ABSTRACTS SERVICE, COLUMBUS, OHIO, US; Database accession no. 1985:167162 XP002258612 & I. UTCHIDA ET AL.: "Structure of WS-43708A, a novel cyclic peptide antibiotic" JOURNAL OF ORGANIC CHEMISTRY., Bd. 50, Nr. 8, 1985, Seiten 1341-1342, AMERICAN CHEMICAL SOCIETY, WASHINGTON, DC., US ISSN: 0022-3263 in der Anmeldung erwähnt

## Beschreibung

Die Erfindung betrifft antibakterielle Amid-Makrozyklen und Verfahren zu ihrer Herstellung sowie ihre Verwendung zur Herstellung von Arzneimitteln zur Behandlung und/oder Prophylaxe von Krankheiten, insbesondere von bakteriellen Infektionen.

In US 3,452,136, Dissertation R. U. Meyer, Universität Stuttgart, Deutschland 1991, Dissertation V. Leitenberger, Universität Stuttgart, Deutschland 1991, Synthesis (1992), (10), 1025-30, J. Chem. Soc., Perkin Trans. 1 (1992), (1), 123-30, J. Chem. Soc., Chem. Commun. (1991), (10), 744, Synthesis (1991), (5), 409-13, J. Chem. Soc., Chem. Commun. (1991), (5), 275-7, J. Antibiot. (1985), 38(11), 1462-8, J. Antibiot. (1985), 38(11), 1453-61, wird der Naturstoff Biphenomycin B als antibakteriell wirksam beschrieben. Die Struktur von Biphenomycin B entspricht nachfolgender Formel (I), wobei R¹, R², R^{3'}, R⁴, R⁷, R⁸ und R⁹ gleich Wasserstoff sind, R³ gleich 3-Amino-2-hydroxy-prop-1-yl ist und C(O)NR⁵R⁶ durch Carboxyl (COOH) ersetzt ist. Teilschritte der Synthese von Biphenomycin B werden in Synlett (2003), 4, 522-525 beschrieben.

Chirality (1995), 7(4), 181-92, J. Antibiot. (1991), 44(6), 674-7, J. Am. Chem. Soc. (1989), 111(19), 7323-7, J. Am. Chem. Soc. (1989), 111(19), 7328-33, J. Org. Chem. (1987), 52(24), 5435-7, Anal. Biochem. (1987), 165(1), 108-13, J. Org. Chem. (1985), 50(8), 1341-2, J. Antibiot. (1993), 46(3), C-2, J. Antibiot. (1993), 46(1), 135-40, Synthesis (1992), (12), 1248-54, Appl. Environ. Microbiol. (1992), 58(12), 3879-8, J. Chem. Soc., Chem. Commun. (1992), (13), 951-3 beschreiben einen strukturell verwandten Naturstoff, Biphenomycin A, der am Makrozyklus eine weitere Substitution mit einer Hydroxygruppe aufweist.

Die Naturstoffe entsprechen hinsichtlich ihrer Eigenschaften nicht den Anforderungen, die an antibakterielle Arzneimittel gestellt werden. Auf dem Markt sind zwar strukturell andersartige antibakteriell wirkende Mittel vorhanden, es kann aber regelmäßig zu einer Resistenzentwicklung kommen. Neue Mittel für eine gute und wirksamere Therapie sind daher wünschenswert.

Eine Aufgabe der vorliegenden Erfindung ist es daher, neue und alternative Verbindungen mit gleicher oder verbesserter antibakterieller Wirkung zur Behandlung von bakteriellen Erkrankungen bei Menschen und Tieren zur Verfügung zu stellen.

Überraschenderweise wurde gefunden, dass Derivate dieser Naturstoffe, worin die Carboxylgruppe des Naturstoffs gegen eine Amidgruppe ausgetauscht wird, antibakteriell wirksam sind.

Gegenstand der Erfindung sind Verbindungen der Formel 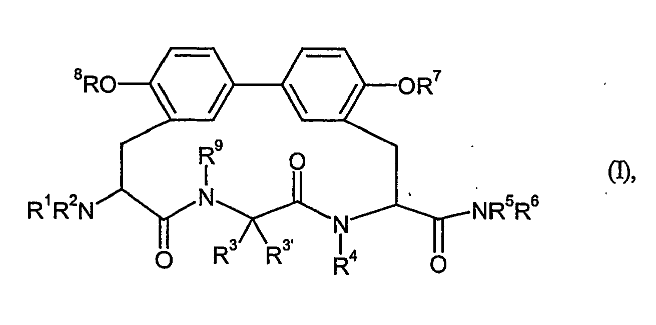 worin
- R¹: gleich Wasserstoff, Alkyl, Aryl, Heteroaryl, Heterocyclyl, Alkylcarbonyl, Arylcarbonyl, Heterocyclylcarbonyl, Heteroarylcarbonyl, Alkoxycarbonyl, Aminocarbonyl, Alkylaminocarbonyl, Dialkylaminocarbonyl, Alkylsulfonyl, Arylsulfonyl, Heterocyclylsulfonyl, Heteroarylsulfonyl oder ein carbonylgebundener Aminosäurerest ist,
wobei R¹ außer Wasserstoff substituiert sein kann mit 0, 1, 2 oder 3 Substituenten R¹⁻¹, wobei die Substituenten R¹⁻¹ unabhängig voneinander ausgewählt werden aus der Gruppe bestehend aus Halogen, Alkyl, Trifluormethyl, Trifluormethoxy, Nitro, Cyano, Amino, Alkylamino, Dialkylamino, Cycloalkyl, Aryl, Heteroaryl, Heterocyclyl, Hydroxy, Alkoxy und Carboxyl,
- R²: gleich Wasserstoff oder Alkyl ist,
wobei R² außer Wasserstoff substituiert sein kann mit 0, 1, 2 oder 3 Substituenten R²⁻¹, wobei die Substituenten R²⁻¹ unabhängig voneinander ausgewählt werden aus der Gruppe bestehend aus Halogen, Amino, Alkylamino und Dialkylamino,
oder
- R¹ und R²: zusammen mit dem Stickstoffatom, an das sie gebunden sind, einen Heterocyclus bilden, der substituiert sein kann mit 0, 1 oder 2 Substituenten R¹⁻², wobei die Substituenten R¹⁻² unabhängig voneinander ausgewählt werden aus der Gruppe bestehend aus Halogen, Trifluormethyl, Amino, Alkylamino, Dialkylamino, Cycloalkyl, Aryl, Heteroaryl, Heterocyclyl, Hydroxy, Alkoxy, Carboxyl, Alkoxycarbonyl und Aminocarbonyl,
- R³: gleich Wasserstoff, Alkyl oder die Seitengruppe einer Aminosäure ist, worin Alkyl substituiert sein kann mit 0, 1, 2 oder 3 Substituenten R³⁻¹, wobei die Substituenten R³⁻¹ unabhängig voneinander ausgewählt werden aus der Gruppe bestehend aus Trifluormethyl, Nitro, Amino, Alkylamino, Dialkylamino, Cycloalkyl, Aryl, Heteroaryl, Heterocyclyl, Hydroxy, Alkoxy, Carboxyl, Alkoxycarbonyl, Aminocarbonyl, Alkylaminocarbonyl, Dialkylaminocarbonyl, Guanidino und Amidino,
worin Cycloalkyl, Aryl, Heteroaryl und Heterocyclyl substituiert sein können mit 0, 1 oder 2 Substituenten R³⁻², wobei die Substituenten R³⁻² unabhängig voneinander ausgewählt werden aus der Gruppe bestehend aus Halogen, Alkyl, Trifluormethyl und Amino,
und worin freie Aminogruppen in der Seitengruppe der Aminosäure mit Alkyl, Alkenyl, Alkinyl, Cycloalkyl, Aryl, Heteroaryl, Heterocyclyl, Alkyl-carbonyl, Arylcarbonyl, Heteroarylcarbonyl, Heterocyclylcarbonyl, Alkoxy-carbonyl, Aminocarbonyl, Alkylaminocarbonyl, Dialkylamino-carbonyl, Arylaminocarbonyl, Alkylsulfonyl, Arylsulfonyl, Heterocyclyl-sulfonyl oder Heteroarylsulfonyl substituiert sein können,
- R^{3'}: gleich Wasserstoff, C₁-C₆-Alkyl oder C₃-C₈-Cycloalkyl ist,
- R⁴: gleich Wasserstoff, C₁-C₆-Alkyl oder C₃-C₈-Cycloalkyl ist,
- R⁵: gleich Wasserstoff, Alkyl, Alkenyl, Cycloalkyl, Aryl, Heteroaryl, Hetero-cyclyl oder ein amingebundener Aminosäurerest ist,
wobei R⁵ substituiert sein kann mit 0, 1, 2 oder 3 Substituenten R⁵⁻¹, wobei die Substituenten R⁵⁻¹ unabhängig voneinander ausgewählt werden aus der Gruppe bestehend aus Halogen, Alkyl, Trifluormethyl, Trifluormethoxy, Nitro, Cyano, Amino, Alkylamino, Dialkylamino, Cycloalkyl, Aryl, Heteroaryl, Heterocyclyl, Hydroxy, Alkoxy, Carboxyl, Alkoxycarbonyl, Aminocarbonyl, Alkylaminocarbonyl, Dialkylaminocarbonyl, Aminosulfonyl, Alkylaminosulfonyl, Dialkylaminosulfonyl, Arylaminosulfonyl, Heterocyclyl-aminosulfonyl, Heteroarylaminosulfonyl, Aminocarbonylamino, Hydroxy-carbonylamino und Alkoxycarbonylamino,
worin Alkyl, Alkylamino, Dialkylamino, Cycloalkyl, Aryl, Heteroaryl und Heterocyclyl substituiert sein können mit 0, 1, 2 oder 3 Substituenten R⁵⁻², wobei die Substituenten R⁵⁻² unabhängig voneinander ausgewählt werden aus der Gruppe bestehend aus Hydroxy, Amino, Carboxyl und Aminocarbonyl,
- R⁶: gleich Wasserstoff, Alkyl oder Cycloalkyl ist,
oder
- R⁵ und R⁶: zusammen mit dem Stickstoffatom, an das sie gebunden sind, einen Heterocyclus bilden, der substituiert sein kann mit 0, 1, 2 oder 3 Substituenten R⁵⁻⁶, wobei die Substituenten R⁵⁻⁶ unabhängig voneinander ausgewählt werden aus der Gruppe bestehend aus Halogen, Alkyl, Trifluor-methyl, Nitro, Amino, Alkylamino, Dialkylamino, Cycloalkyl, Aryl, halogeniertes Aryl, Heteroaryl, Heterocyclyl, Hydroxy, Alkoxy, Carboxyl, Alkylcarbonyl, Alkoxycarbonyl, Aminocarbonyl, Alkylaminocarbonyl und Dialkylaminocarbonyl,
- R⁷: gleich Wasserstoff, C₁-C₆-Alkyl, Alkylcarbonyl oder C₃-C₈-Cycloalkyl ist,
- R⁸: gleich Wasserstoff oder C₁-C₆-Alkyl ist und
- R⁹: gleich Wasserstoff oder C₁-C₆-Alkyl ist,
und ihre Salze, ihre Solvate und die Solvate ihrer Salze.

Erfindungsgemäße Verbindungen sind die Verbindungen der Formel (I) und deren Salze, Solvate und Solvate der Salze, die von Formel (I) umfassten Verbindungen der nachfolgend genannten Formel (I') und deren Salze, Solvate und Solvate der Salze sowie die von Formel (I) und/oder (I') umfassten, nachfolgend als Ausführungsbeispiel(e) genannten Verbindungen und deren Salze, Solvate und Solvate der Salze, soweit es sich bei den von Formel (I) und/oder (I') umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich durch bekannte Verfahren wie Chromatographie an chiraler Phase oder Kristallisation mit chiralen Aminen oder chiralen Säuren die stereoisomer einheitlichen Bestandteile in bekannter Weise isolieren.

Die Erfindung betrifft in Abhängigkeit von der Struktur der Verbindungen auch Tautomere der Verbindungen.

Als Salze sind im Rahmen der Erfindung physiologisch unbedenkliche Salze der erfindungsgemäßen Verbindungen bevorzugt.

Physiologisch unbedenkliche Salze der Verbindungen (I) umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure, Trifluoressigsäure und Benzoesäure.

Physiologisch unbedenkliche Salze der Verbindungen (I) umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Dihydroabietylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:
Alkyl sowie die Alkylteile in Substituenten wie Alkoxy, Mono- und Dialkylamino, Alkylsulfonyl umfassen lineares und verzweigtes Alkyl, z.B. C₁-C₁₂-, insbesondere C₁-C₆- und C₁-C₄-Alkyl.
C₁-C₆-Alkyl umfasst Methyl, Ethyl, n- und i-Propyl, n-, i-, sek.- und *tert*.-Butyl, n-Pentyl, Isopentyl, Neopentyl, Hexyl,
C₁-C₄-Alkyl umfasst Methyl, Ethyl, n- und i-Propyl, n-, i-, sek.- und *tert*.-Butyl,
Alkylcarbonyl steht im Rahmen der Erfindung vorzugsweise für einen geradkettigen oder verzweigten Alkylrest mit 1 bis 6 bzw. 1 bis 4 Kohlenstoffatomen. Beispielhaft und vorzugsweise seien genannt: Methylcarbonyl, Ethylcarbonyl, n-Propylcarbonyl, Isopropylcarbonyl und t-Butylcarbonyl.
Alkenyl umfasst lineares und verzweigtes C₂-C₁₂-, insbesondere C₂-C₆- und C₂-C₄-Alkenyl, wie z.B. Vinyl, Allyl, Prop-1-en-1-yl, Isopropenyl, But-1-enyl, But-2-enyl, Buta-1.2-dienyl, Buta-1.3-dienyl.
Alkinyl umfasst lineares und verzweigtes C₂-C₁₂-, insbesondere C₂-C₆- und C₂-C₄-Alkinyl, wie z.B. Ethinyl, Propargyl (2-Propinyl), 1-Propinyl, But-1-inyl, But-2-inyl.
Cycloalkyl umfasst polycyclische gesättigte Kohlenwasserstoffreste mit bis zu 14 Kohlenstoffatomen, nämlich monocyclisches C₃-C₁₂-, vorzugsweise C₃-C₈-Alkyl, insbesondere C₃-C₆-Alkyl wie z.B. Cyclopropyl, Cyclobutyl, Cyclopentyl, Cyclohexyl, Cycloheptyl, Cyclooctyl, Cyclononyl, und polycyclisches Alkyl, d.h. vorzugsweise bicyclisches und tricyclisches, gegebenenfalls spirocyclisches C₇-C₁₄-Alkyl, wie z.B. Bicyclo[2.2.1]-hept-1-yl, Bicyclo[2.2.1]-hept-2-yl, Bicyclo[2.2.1]-hept-7-yl, Bicyclo[2.2.2]-oct-2-yl, Bicyclo[3.2.1]-oct-2-yl, Bicyclo[3.2.2]-non-2-yl und Adamantyl.
Aryl steht im Rahmen der Erfindung für einen aromatischen Rest mit vorzugsweise 6 bis 10 Kohlenstoffatomen. Bevorzugte Arylreste sind Phenyl und Naphthyl.
Alkoxy steht im Rahmen der Erfindung vorzugsweise für einen geradkettigen oder verzweigten Alkoxyrest insbesondere mit 1 bis 6, 1 bis 4 bzw. 1 bis 3 Kohlenstoffatomen. Bevorzugt ist ein geradkettiger oder verzweigter Alkoxyrest mit 1 bis 3 Kohlenstoffatomen. Beispielhaft und vorzugsweise seien genannt: Methoxy, Ethoxy, n-Propoxy, Isopropoxy, t-Butoxy, n-Pentoxy und n-Hexoxy.
Alkoxycarbonyl steht im Rahmen der Erfindung vorzugsweise für einen geradkettigen oder verzweigten Alkoxyrest mit 1 bis 6 bzw. 1 bis 4 Kohlenstoffatomen, der über eine Carbonylgruppe verknüpft ist. Bevorzugt ist ein geradkettiger oder verzweigter Alkoxycarbonylrest mit 1 bis 4 Kohlenstoffatomen. Beispielhaft und vorzugsweise seien genannt: Methoxycarbonyl, Ethoxycarbonyl, n-Propoxycarbonyl, Isopropoxycarbonyl und t-Butoxycarbonyl.
Monoalkylamino (Alkylamino) steht im Rahmen der Erfindung für eine Amino-Gruppe mit einem geradkettigen oder verzweigten Alkylsubstituenten, der vorzugsweise 1 bis 6, 1 bis 4 bzw. 1 oder 2 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethylamino, n-Propylamino, Isopropylamino, t-Butylamino, n-Pentylamino und n-Hexylamino.
Dialkylamino steht im Rahmen der Erfindung für eine Amino-Gruppe mit zwei gleichen oder verschiedenen geradkettigen oder verzweigten Alkylsubstituenten, die vorzugsweise jeweils 1 bis 6, 1 bis 4 bzw. 1 oder 2 Kohlenstoffatome aufweisen. Bevorzugt sind geradkettige oder verzweigte Dialkylamino-Reste mit jeweils 1, 2, 3 oder 4 Kohlenstoffatomen pro Alkylsubstituent. Beispielhaft und vorzugsweise seien genannt: *N,N*-Dimethylamino, *N,N*-Diethylamino, *N*-Ethyl-*N*-methylamino, *N*-Methyl-*N*-n-propylamino, *N*-Isopropyl-*N*-n-propylamino, *N*-t-Butyl-*N*-methylamino, N-Ethyl-*N*-n-pentylamino und *N*-n-Hexyl-*N*-methylamino.
Monoalkylaminocarbonyl (Akylaminocarbonyl) oder Dialkylaminocarbonyl steht im Rahmen der Erfindung für eine Amino-Gruppe, die über eine Carbonylgruppe verknüpft ist und die einen geradkettigen oder verzweigten bzw. zwei gleiche oder verschiedene geradkettige oder verzweigte Alkylsubstituenten mit vorzugsweise jeweils 1 bis 4 bzw. 1 oder 2 Kohlenstoffatomen aufweist. Beispielhaft und vorzugsweise seien genannt: Methylaminocarbonyl, Ethylaminocarbonyl, Isopropylaminocarbonyl, t-Butylaminocarbonyl, *N,N-*Dimethylaminocarbonyl, *N,N-*Diethylaminocarbonyl, *N*-Ethyl-*N*-methylaminocarbonyl und *N*-t-Butyl-*N*-methylaminocarbonyl.
Arylaminocarbonyl steht im Rahmen der Erfindung für einen aromatischen Rest mit vorzugsweise 6 bis 10 Kohlenstoffatomen, der über eine Aminocarbonyl-Gruppe verknüpft ist. Bevorzugte Reste sind Phenylaminocarbonyl und Naphthylaminocarbonyl.
Alkylcarbonylamino (Acylamino) steht im Rahmen der Erfindung für eine Amino-Gruppe mit einem geradkettigen oder verzweigten Alkanoylsubstituenten, der vorzugsweise 1 bis 6, 1 bis 4 bzw. 1 oder 2 Kohlenstoffatome aufweist und über die Carbonylgruppe verknüpft ist. Bevorzugt ist ein Monoacylamino-Rest mit 1 oder 2 Kohlenstoffatomen. Beispielhaft und vorzugsweise seien genannt: Formamido, Acetamido, Propionamido, n-Butyramido und Pivaloylamido.
Alkoxycarbonylamino steht im Rahmen der Erfindung für eine Amino-Gruppe mit einem geradkettigen oder verzweigten Alkoxycarbonylsubstituenten, der vorzugsweise im Alkoxyrest 1 bis 6 bzw. 1 bis 4 Kohlenstoffatome aufweist und über die Carbonylgruppe verknüpft ist. Bevorzugt ist ein Alkoxycarbonylamino-Rest mit 1 bis 4 Kohlenstoffatomen. Beispielhaft und vorzugsweise seien genannt: Methoxycarbonylamino, Ethoxycarbonylamino, n-Propoxycarbonylamino und t-Butoxycarbonylamino.
Heterocyclyl (Heterocyclus) steht für einen mono- oder polycyclischen, heterocyclischen Rest mit 4 bis 10 Ringatomen und bis zu 3, vorzugsweise 1 Heteroatomen bzw. Heterogruppen aus der Reihe N, O, S, SO, SO₂. 4- bis 8-gliedriges, insbesondere 5- bis 6-gliedriges Heterocyclyl ist bevorzugt. Mono- oder bicyclisches Heterocyclyl ist bevorzugt. Besonders bevorzugt ist monocyclisches Heterocyclyl. Als Heteroatome sind N und O bevorzugt. Die Heterocyclyl-Reste können gesättigt oder teilweise ungesättigt sein. Gesättigte Heterocyclyl-Reste sind bevorzugt. Die Heterocyclylreste können über ein Kohlenstoffatom oder ein Heteroatom gebunden sein. Besonders bevorzugt sind 5- bis 6-gliedrige, monocyclische gesättigte Heterocyclylreste mit bis zu zwei Heteroatomen aus der Reihe O, N und S. Beispielsweise und vorzugsweise seien genannt: Oxetan-3-yl, Pyrrolidin-2-yl, Pyrrolidin-3-yl, Pyrrolinyl, Tetrahydrofuranyl, Tetrahydrothienyl, Pyranyl, Piperidin-1-yl, Piperidin-2-yl, Piperidin-3-yl, Piperidin-4-yl, Thiopyranyl, Morpholin-1-yl, Morpholin-2-yl, Morpholin-3-yl, Perhydroazepinyl, Piperazin-1-yl, Piperazin-2-yl. Ein Stickstoff-Heterocyclylring ist dabei ein Heterocyclus, der als Heteroatome nur Stickstoffatome aufweist.
Heteroaryl steht für einen aromatischen, mono- oder bicyclischen Rest mit 5 bis 10 Ringatomen und bis zu 5 Heteroatomen aus der Reihe S, O und/oder N. Bevorzugt sind 5- bis 6-gliedrige Heteroaryle mit bis zu 4 Heteroatomen. Der Heteroarylrest kann über ein Kohlenstoff- oder Heteroatom gebunden sein. Beispielsweise und vorzugsweise seien genannt: Thienyl, Furyl, Pyrrolyl, Thiazolyl, Oxazolyl, Imidazolyl, Pyridyl, Pyrimidyl, Pyridazinyl, Indolyl, Indazolyl, Benzofuranyl, Benzothiophenyl, Chinolinyl, Isochinolinyl.
Carbonyl steht für eine -C(O)-Gruppe. Dementsprechend sind Arylcarbonyl, Heterocyclylcarbonyl und Heteroarylcarbonyl an der Carbonylgruppe mit den entsprechenden Resten substituiert, d.h. Aryl, Heterocyclyl etc.
Sulfonyl steht für eine -S(O)₂-Gruppe. Dementsprechend sind Alkylsulfonyl, Arylsulfonyl, Heterocyclylsulfonyl und Heteroarylsulfonyl an der Sulfonylgruppe mit den entsprechenden Resten substituiert, d.h. Alkyl, Aryl etc.
Aminosulfonyl steht für eine -S(O)₂NH₂-Gruppe. Dementsprechend sind Alkylaminosulfonyl, Dialkylaminosulfonyl, Arylaminosulfonyl, Heterocyclylaminosulfonyl und Heteroarylaminosulfonyl an der Aminogruppe mit den entsprechenden Resten substituiert, d.h. Alkyl, Aryl etc.
Halogen schließt im Rahmen der Erfindung Fluor, Chlor, Brom und Iod ein. Bevorzugt sind Fluor oder Chlor.
Unter der Seitengruppe einer Aminosäure wird im Rahmen der Erfindung derjenige organische Rest eines α-Aminosäuremoleküls verstanden, der an das α-Kohlenstoffatom der Aminosäure gebunden ist. Bevorzugt sind dabei die Reste natürlich vorkommender α-Aminosäuren in der L- oder in der D-Konfiguration, insbesondere natürlich vorkommende α-Aminosäuren in der natürlichen L-Konfiguration.
Hierzu zählen beispielsweise Wasserstoff (Glycin), Methyl (Alanin), Prop-2-yl (Valin), 2-Methyl-prop-1-yl (Leucin), 1-Methyl-prop-1-yl (Isoleucin), eine (3-Indolyl)-methylgruppe (Tryptophan), eine Benzylgruppe (Phenylalanin), eine Methylthioethylgruppe (Methionin), Hydroxymethyl (Serin), p-Hydroxybenzyl (Tyrosin), 1-Hydroxy-eth-1-yl (Threonin), Mercaptomethyl (Cystein), Carbamoylmethyl (Asparagin), Carbamoylethyl (Glutamin), Carboxymethyl (Asparaginsäure), Carboxyethyl (Glutaminsäure), 4-Aminobut-1-yl (Lysin), 3-Guanidinoprop-1-yl (Arginin), Imidazol-4-ylmethyl (Histidin), 3-Ureidoprop-1-yl (Citrullin), Mercaptoethyl (Homocystein), Hydroxyethyl (Homoserin), 4-Amino-3-hydroxybut-1-yl (Hydroxylysin), 3-Amino-prop-1-yl (Ornithin), 2-Hydroxy-3-amino-prop-1-yl (Hydroxyornithin).
Carbonylgebundener Aminosäurerest steht für einen Aminosäurerest, der über die Carbonylgruppe der Aminosäure-Säurefunktion gebunden ist. Bevorzugt sind dabei α-Aminosäuren in der L- oder in der D-Konfiguration, insbesondere natürlich vorkommende α-Aminosäuren in der natürlichen L-Konfiguration, z.B. Glycin, L-Alanin und L-Prolin.
Amingebundener Aminosäurerest steht für einen Aminosäurerest, der über die Aminogruppe der Aminosäure gebunden ist. Bevorzugt sind dabei α-Aminosäuren oder β-Aminosäuren. Besonders bevorzugt sind dabei α-Aminosäuren in der L- oder in der D-Konfiguration, insbesondere natürlich vorkommende α-Aminosäuren in der natürlichen L-Konfiguration, z.B. Glycin (R⁵ gleich Carboxylmethyl), Alanin (R⁵ gleich 1-Carboxyleth-1-yl). Die Säurefunktion der Aminosäure kann auch als Ester, z. B. Methyl-, Ethyl-, tert-Butylester, oder als Amid, z. B. Aminocarbonyl-, Alkylaminocarbonyl-, Dialkylaminocarbonyl-, Benzylaminocarbonyl-Gruppe, vorliegen.
Unter Aminoschutzgruppen werden im Rahmen der vorliegenden Erfindung solche organischen Reste verstanden, mit denen Aminogruppen vorübergehend gegen den Angriff von Reagenzien geschützt werden können, so dass Reaktionen wie Oxidation, Reduktion, Substitution und Kondensation nur an den gewünschten (ungeschützten) Stellen stattfinden. Sie sind für die Dauer des Schutzes unter allen Bedingungen der durchzuführenden Reaktionen und Reinigungsoperationen stabil und wieder unter milden Bedingungen selektiv und mit hoher Ausbeute abspaltbar (Römpp Lexikon Chemie - Version 2.0, Stuttgart/New York: Georg Thieme Verlag 1999; T. W. Greene, P. G. Wuts, Protective Groups in Organic Synthesis, 3^{rd} ed., John Wiley, New York, 1999)

Bevorzugt sind hierbei Oxycarbonylderivative wie Carbamate und insbesondere die folgenden Gruppen: Benzyloxycarbonyl, 4-Brom-benzyloxycarbonyl, 2-Chlor-benzyloxycarbonyl, 3-Chlor-benzyloxycarbonyl, Dichlorbenzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, 3,4,5-Trimethoxybenzyloxycarbonyl, Methoxycarbonyl, Ethoxycarbonyl, Propoxycarbonyl, Isopropoxycarbonyl, Butoxycarbonyl, Isobutoxycarbonyl, *tert*-Butoxycarbonyl, Pentoxycarbonyl, Isopentoxycarbonyl, Hexoxycarbonyl, Cyclohexoxycarbonyl, Octoxycarbonyl, 2-Ethylhexoxycarbonyl, 2-Iodhexoxycarbonyl, 2-Bromethoxycarbonyl, 2-Chlorethoxycarbonyl, 2,2,2-Trichlorethoxycarbonyl, 2,2,2-Trichlor-tert-butoxycarbonyl, Benzhydryloxycarbonyl, Bis-(4-methoxyphenyl)methoxycarbonyl, Phenacyloxycarbonyl, 2-Trimethylsilylethoxycarbonyl, Phenacyloxycarbonyl, 2-Trimethylsilylethoxycarbonyl, 2-(Di-n-butyl-methyl-silyl)ethoxycarbonyl, 2-Triphenylsilylethoxycarbonyl, 2-(Dimethyl-tert-butylsilyl)ethoxycarbonyl, Methyloxycarbonyl, Vniyloxycarbonyl, Allyloxycarbonyl, Phenoxycarbonyl, Tolyloxycarbonyl, 2,4-Dinitrophenoxycarbonyl, 4-Nitrophenoxycarbonyl, 2,4,5-Trichlorphenoxycarbonyl, Naphthyloxycarboyl, Fluorenyl-9-methoxycarbonyl, Valeroyl, Isovaleroyl, Butyryl, Ethylthiocarbonyl, Methylthiocarbonyl, Butylthiocarboyl, tert-Butylthiocarbonyl, Phenylthiocarbonyl, Benzylthiocarbonyl, Methylaminocarbonyl, Ethylaminocarbonyl, Propylaminocarbonyl, iso-Propylaminocarbonyl, Formyl, Acetyl, Propionyl, Pivaloyl, 2-Chloracetyl, 2-Bromacetyl, 2-Iodacetyl, 2,2,2-Trifluoracetyl, 2,2,2-Trichloracetyl, Benzoyl, 4-Chlorbenzoyl, 4-Methoxybenzoyl, 4-Nitrobenzyl, 4-Nitrobenzoyl, Naphthylcarbonyl, Phenoxyacetyl, Adamantylcarbonyl, Dicyclohexylphosphoryl, Diphenylphosphoryl, Dibenzylphosphoryl, Di-(4-nitrobenzyl)-phophoryl, Phenoxyphenylphosphoryl, Diethylphosphinyl, Diphenylphosphinyl, Phthaloyl, Phthalimido oder Benzyloxymethylen.

Besonders bevorzugt sind *tert*-Butyloxycarbonyl (Boc), 9-Fluorenylmethyloxycarbonyl (FMOC), Benzyloxycarbonyl (Cbz- / Z-) und Allyloxycarbonyl (Aloc).

Ein Symbol * an einer Bindung bedeutet die Verknüpfungsstelle im Molekül.

Bevorzugt im Rahmen der vorliegenden Erfindung sind Verbindungen, welche der Formel 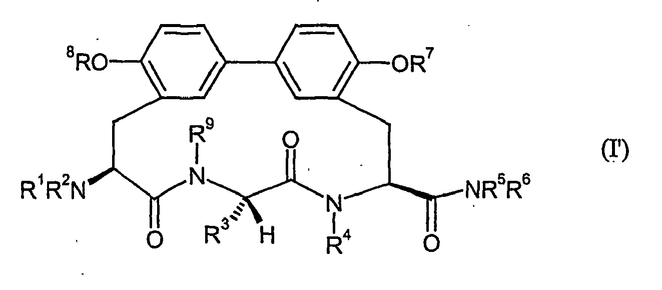 entsprechen, worin R¹ bis R⁹ die gleiche Bedeutung wie in Formel (I) haben,
und ihre Salze, ihre Solvate und die Solvate ihrer Salze.

Bevorzugt im Rahmen der vorliegenden Erfindung sind erfindungsgemäße Verbindungen, bei denen
- R¹: gleich Wasserstoff, Alkyl, Aryl, Heteroaryl, Heterocyclyl, Alkylcarbonyl, Arylcarbonyl, Heterocyclylcarbonyl, Heteroarylcarbonyl, Alkoxycarbonyl, Aminocarbonyl, Alkylaminocarbonyl, Dialkylaminocarbonyl, Alkylsulfonyl, Arylsulfonyl, Heterocyclylsulfonyl, Heteroarylsulfonyl oder ein carbonylgebundener Aminosäurerest ist,
wobei R¹ außer Wasserstoff substituiert sein kann mit 0, 1, 2 oder 3 Substituenten R¹⁻¹, wobei die Substituenten R¹⁻¹ unabhängig voneinander ausgewählt werden aus der Gruppe bestehend aus Halogen, Alkyl, Trifluormethyl, Trifluormethoxy, Nitro, Cyano, Amino, Alkylamino, Dialkylamino, Cycloalkyl, Aryl, Heteroaryl, Heterocyclyl, Hydroxy, Alkoxy und Carboxyl,
- R²: gleich Wasserstoff oder Alkyl ist,
wobei R² außer Wasserstoff substituiert sein kann mit 0, 1, 2 oder 3 Substituenten R²⁻¹, wobei die Substituenten R²⁻¹ unabhängig voneinander ausgewählt werden aus der Gruppe bestehend aus Halogen, Amino, Alkylamino und Dialkylamino,
oder
- R¹ und R²: zusammen mit dem Stickstoffatom, an das sie gebunden sind, einen Heterocyclus bilden, der substituiert sein kann mit 0, 1 oder 2 Substituenten R¹⁻², wobei die Substituenten R¹⁻² unabhängig voneinander ausgewählt werden aus der Gruppe bestehend aus Halogen, Trifluormethyl, Amino, Alkylamino, Dialkylamino, Cycloalkyl, Aryl, Heteroaryl, Heterocyclyl, Hydroxy, Alkoxy, Carboxyl, Alkoxycarbonyl und Aminocarbonyl,
- R³: gleich Wasserstoff, Alkyl oder die Seitengruppe einer Aminosäure ist, worin Alkyl substituiert sein kann mit 0, 1, 2 oder 3 Substituenten R³⁻¹, wobei die Substituenten R³⁻¹ unabhängig voneinander ausgewählt werden aus der Gruppe bestehend aus Trifluormethyl, Nitro, Amino, Alkylamino, Dialkylamino, Cycloalkyl, Aryl, Heteroaryl, Heterocyclyl, Hydroxy, Alkoxy, Carboxyl, Alkoxycarbonyl, Aminocarbonyl, Alkylaminocarbonyl und Dialkylaminocarbonyl,
worin Cycloalkyl, Aryl, Heteroaryl und Heterocyclyl substituiert sein können mit 0, 1 oder 2 Substituenten R³⁻², wobei die Substituenten R³⁻² unabhängig voneinander ausgewählt werden aus der Gruppe bestehend aus Halogen, Alkyl, Trifluormethyl und Amino,
und worin freie Aminogruppen in der Seitengruppe der Aminosäure mit Alkyl, Alkenyl, Cycloalkyl, Alkylcarbonyl, Arylcarbonyl, Heteroarylcarbonyl, Heterocyclylcarbonyl, Alkoxycarbonyl, Aminocarbonyl, Alkylaminocarbonyl, Dialkylaminocarbonyl, Arylaminocarbonyl, Alkylsulfonyl, Arylsulfonyl, Heterocyclylsulfonyl oder Heteroarylsulfonyl substituiert sein können,
- R^{3'}: gleich Wasserstoff oder C₁-C₆-Alkyl ist,
- R⁴: gleich Wasserstoff, C₁-C₆-Alkyl oder C₃-C₈-Cycloalkyl ist,
- R⁵: gleich Wasserstoff, Alkyl, Alkenyl, Cycloalkyl, Aryl, Heteroaryl, Heterocyclyl oder ein amingebundener Aminosäurerest ist,
wobei R⁵ substituiert sein kann mit 0, 1, 2 oder 3 Substituenten R⁵⁻¹, wobei die Substituenten R⁵⁻¹ unabhängig voneinander ausgewählt werden aus der Gruppe bestehend aus Halogen, Alkyl, Trifluormethyl, Trifluormethoxy, Cyano, Amino, Alkylamino, Dialkylamino, Cycloalkyl, Aryl, Heteroaryl, Heterocyclyl, Hydroxy, Alkoxy, Carboxyl, Alkoxycarbonyl, Aminocarbonyl, Alkylaminocarbonyl und Dialkylaminocarbonyl,
- R⁶: gleich Wasserstoff, Alkyl oder Cycloalkyl ist,
oder
- R⁵ und R⁶: zusammen mit dem Stickstoffatom, an das sie gebunden sind, einen Heterocyclus bilden, der substituiert sein kann mit 0, 1, 2 oder 3 Substituenten R⁵⁻⁶, wobei die Substituenten R⁵⁻⁶ unabhängig voneinander ausgewählt werden aus der Gruppe bestehend aus Halogen, Alkyl, Trifluormethyl, Nitro, Amino, Alkylamino, Dialkylamino, Cycloalkyl, Aryl, halogeniertes Aryl, Heteroaryl, Heterocyclyl, Hydroxy, Alkoxy, Carboxyl, Alkylcarbonyl, Alkoxycarbonyl, Aminocarbonyl, Alkylaminocarbonyl und Dialkylaminocarbonyl,
- R⁷: gleich Wasserstoff oder C₁-C₆-Alkyl ist,
- R⁸: gleich Wasserstoff oder C₁-C₆-Alkyl ist,
und
- R⁹: gleich Wasserstoff oder C₁-C₆-Alkyl ist.

Bevorzugt im Rahmen der vorliegenden Erfindung sind auch erfindungsgemäße Verbindungen, bei denen
- R¹: gleich Wasserstoff, Alkyl, Alkylcarbonyl, Arylcarbonyl, Heterocyclylcarbonyl, Heteroarylcarbonyl, Alkoxycarbonyl oder ein carbonylgebundener Aminosäurerest ist,
wobei R¹ außer Wasserstoff substituiert sein kann mit 0, 1 oder 2 Substituenten R¹⁻¹, wobei die Substituenten R¹⁻¹ unabhängig voneinander ausgewählt werden aus der Gruppe bestehend aus Halogen, Trifluormethyl, Amino, Alkylamino, Dialkylamino, Phenyl, 5- bis 6-gliedriges Heteroaryl, 5-bis 6-gliedriges Heterocyclyl, Hydroxy und Alkoxy,
- R²: gleich Wasserstoff oder Methyl ist,
- R³: gleich Aminocarbonylmethyl, 3-Aminopropyl, 2 -Hydroxy-3-aminopropyl, 3-Guanidinopropyl, 2-Aminocarbonylethyl, 2-Hydroxycarbonylethyl, 4-Aminobutyl, Hydroxymethyl oder 2-Hydroxyethyl, 4-Amino-3-hydroxybutan-1-yl ist,
und worin freie Aminogruppen in der Seitengruppe der Aminosäure mit Alkyl, Alkenyl, C₃-C₆-Cycloalkyl, Alkylcarbonyl, Phenylcarbonyl, 5- bis 6-gliedriges Heteroarylcarbonyl, 5- bis 6-gliedriges Heterocyclylcarbonyl, Alkoxycarbonyl, Aminocarbonyl, Alkylaminocarbonyl, Dialkylaminocarbonyl, Phenylaminocarbonyl, Alkylsulfonyl, Arylsulfonyl, 5- bis 6-gliedriges Heterocyclylsulfonyl oder 5- bis 6-gliedriges Heteroarylsulfonyl substituiert sein können,
- R^{3'}: gleich Wasserstoff ist,
- R⁴: gleich Wasserstoff oder Methyl ist,
- R⁵: gleich Wasserstoff, Alkyl, C₃-C₆-Cycloalkyl, Phenyl, 5- bis 6-gliedriges Heteroaryl, 5- bis 6-gliedriges Heterocyclyl oder ein amingebundener Aminosäurerest ist,
wobei für den Fall, dass R⁵ gleich Alkyl, C₃-C₆-Cycloalkyl oder 5- bis 6-gliedriges Heterocyclyl ist, dieses substituiert sein kann mit 0, 1 oder 2 Substituenten R⁵⁻², wobei die Substituenten R⁵⁻² unabhängig voneinander ausgewählt werden aus der Gruppe bestehend aus Alkyl, Trifluormethyl, Amino, Alkylamino, Dialkylamino, C₃-C₆-Cycloalkyl, Phenyl, 5- bis 6-gliedriges Heteroaryl, 5- bis 6-gliedriges Heterocyclyl, Hydroxy, Alkoxy, Carboxyl, Alkoxycarbonyl, Aminocarbonyl, Alkylaminocarbonyl und Dialkylaminocarbonyl,
und
wobei für den Fall, dass R⁵ gleich Phenyl oder 5- bis 6-gliedriges Heteroaryl ist, dieses substituiert sein kann mit 0, 1 oder 2 Substituenten R⁵⁻³, wobei die Substituenten R⁵⁻³ unabhängig voneinander ausgewählt werden aus der Gruppe bestehend aus Halogen, Trifluormethyl, Trifluormethoxy, Amino, Alkylamino, Dialkylamino, C₃-C₆-Cycloalkyl, 5- bis 6-gliedriges Heteroaryl, 5- bis 6-gliedriges Heterocyclyl, Hydroxy, Alkoxy, Carboxyl, Alkoxycarbonyl, Aminocarbonyl, Alkylaminocarbonyl und Dialkylaminocarbonyl,
und
wobei für den Fall, dass R⁵ gleich amingebundener Aminosäurerest, dieser substituiert sein kann mit 0, 1 oder 2 Substituenten R⁵⁻⁴, wobei die Substituenten R⁵⁻⁴ unabhängig voneinander ausgewählt werden aus der Gruppe bestehend aus Halogen, Trifluormethyl, Trifluormethoxy, Amino, Alkylamino, Dialkylamino, C₃-C₆-Cycloalkyl, Phenyl, 5- bis 6-gliedriges Heteroaryl, 5- bis 6-gliedriges Heterocyclyl, Hydroxy, Alkoxy, Carboxyl, Alkoxycarbonyl, Aminocarbonyl, Alkylaminocarbonyl und Dialkylaminocarbonyl,
- R⁶: gleich Wasserstoff, Alkyl oder C₃-C₆-Cycloalkyl ist,
oder
- R⁵ und R⁶: zusammen mit dem Stickstoffatom, an das sie gebunden sind, einen 5- bis 6-gliedrigen Heterocyclus bilden, der substituiert sein kann mit 0, 1 oder 2 Substituenten R⁵⁻⁶, wobei die Substituenten R⁵⁻⁶ unabhängig voneinander ausgewählt werden aus der Gruppe bestehend aus Amino, Alkylamino, Dialkylamino, C₃-C₆-Cycloalkyl, Phenyl, halogeniertes Phenyl, 5- bis 6-gliedriges Heteroaryl, Hydroxy, Alkoxy, Carboxyl und Aminocarbonyl,
- R⁷: gleich Wasserstoff ist,
- R⁸: gleich Wasserstoff ist, und
- R⁹: gleich Wasserstoff oder Methyl ist.

Bevorzugt im Rahmen der vorliegenden Erfindung sind auch erfindungsgemäße Verbindungen, bei denen
- R¹: gleich Wasserstoff, Alkyl oder Alkylcarbonyl ist,
- R²: gleich Wasserstoff ist,
- R³: gleich Alkyl oder die Seitengruppe einer Aminosäure ist, worin Alkyl substituiert sein kann mit 0, 1, 2 oder 3 Substituenten R³⁻¹, wobei die Substituenten R³⁻¹ unabhängig voneinander ausgewählt werden aus der Gruppe bestehend aus Trifluormethyl, Nitro, Amino, Alkylamino, Dialkylamino, Cycloalkyl, Aryl, Heteroaryl, Heterocyclyl, Hydroxy, Alkoxy, Carboxyl, Alkoxycarbonyl, Aminocarbonyl, Alkylaminocarbonyl, Dialkylaminocarbonyl, Guanidino und Amidino,
worin Cycloalkyl, Aryl, Heteroaryl und Heterocyclyl substituiert sein können mit 0, 1 oder 2 Substituenten R³⁻², wobei die Substituenten R³² unabhängig voneinander ausgewählt werden aus der Gruppe bestehend aus Halogen, Alkyl, Trifluormethyl und Amino,
und worin freie Aminogruppen in der Seitengruppe der Aminosäure mit Alkyl substituiert sein können,
- R^{3'}: gleich Wasserstoff, C₁-C₆-Alkyl oder C₃-C₈-Cycloalkyl ist,
- R⁴: gleich Wasserstoff, C₁-C₆-Alkyl oder C₃-C₈-Cycloalkyl ist,
- R⁵: gleich Wasserstoff, Alkyl, Alkenyl, Cycloalkyl, Aryl, Heteroaryl, Heterocyclyl oder ein amingebundener Aminosäurerest ist,
wobei Alkyl, Alkenyl, Cycloalkyl, Aryl, Heteroaryl und Heterocyclyl substituiert sein können mit 0, 1, 2 oder 3 Substituenten R⁵⁻¹, wobei die Substituenten R⁵⁻¹ unabhängig voneinander ausgewählt werden aus der Gruppe bestehend aus Halogen, Alkyl, Trifluormethyl, Trifluormethoxy, Nitro, Cyano, Amino, Alkylamino, Dialkylamino, Cycloalkyl, Aryl, Heteroaryl, Heterocyclyl, Hydroxy, Alkoxy, Carboxyl, Alkoxycarbonyl, Aminocarbonyl, Alkylaminocarbonyl und Dialkylaminocarbonyl,
worin Alkyl, Alkylamino, Dialkylamino, Cycloalkyl, Aryl, Heteroaryl und Heterocyclyl substituiert sein können mit 0, 1, 2 oder 3 Substituenten R⁵⁻², wobei die Substituenten R⁵⁻² unabhängig voneinander ausgewählt werden aus der Gruppe bestehend aus Hydroxy, Amino, Carboxyl und Aminocarbonyl,
- R⁶: gleich Wasserstoff, Alkyl oder Cycloalkyl ist,
oder
- R⁵ und R⁶: zusammen mit dem Stickstoffatom, an das sie gebunden sind, einen Heterocyclus bilden, der substituiert sein kann mit 0, 1, 2 oder 3 Substituenten R⁵⁻⁶, wobei die Substituenten R⁵⁻⁶ unabhängig voneinander ausgewählt werden aus der Gruppe bestehend aus Halogen, Alkyl, Amino, Alkylamino, Dialkylamino, Hydroxy, Alkoxy, Carboxyl, Alkoxycarbonyl, Aminocarbonyl, Alkylaminocarbonyl und Dialkylaminocarbonyl,
- R⁷: gleich Wasserstoff, C₁-C₆-Alkyl, Alkylcarbonyl oder C₃-C₈-Cycloalkyl ist,
- R⁸: gleich Wasserstoff ist,
und
- R⁹: gleich Wasserstoff ist.

Bevorzugt im Rahmen der vorliegenden Erfindung sind auch erfindungsgemäße Verbindungen, bei denen
- R¹: gleich Wasserstoff ist,
- R²: gleich Wasserstoff ist,
- R³: gleich Alkyl oder die Seitengruppe einer Aminosäure ist, worin Alkyl substituiert sein kann mit 0, 1, 2 oder 3 Substituenten R³⁻¹, wobei die Substituenten R³⁻¹ unabhängig voneinander ausgewählt werden aus der Gruppe bestehend aus Amino, Alkylamino, Dialkylamino, Cycloalkyl, Heteroaryl, Heterocyclyl, Hydroxy, Alkoxy, Carboxyl, Alkoxycarbonyl, Aminocarbonyl, Alkylaminocarbonyl, Dialkylaminocarbonyl, Guanidino und Amidino,
worin Cycloalkyl, Heteroaryl und Heterocyclyl substituiert sein können mit 0, 1 oder 2 Substituenten R³⁻², wobei die Substituenten R³² unabhängig voneinander ausgewählt werden aus der Gruppe bestehend aus Alkyl und Amino,
- R^{3'}: gleich Wasserstoff ist,
- R⁴: gleich Wasserstoff, C₁-C₆-Alkyl oder C₃-C₈-Cycloalkyl ist,
- R⁵: gleich Wasserstoff, Alkyl, Cycloalkyl, Aryl, Heteroaryl, Heterocyclyl oder ein amingebundener Aminosäurerest ist,
wobei Alkyl, Cycloalkyl, Aryl, Heteroaryl und Heterocyclyl substituiert sein können mit 0, 1, 2 oder 3 Substituenten R⁵⁻¹, wobei die Substituenten R⁵⁻¹ unabhängig voneinander ausgewählt werden aus der Gruppe bestehend aus Halogen, Alkyl, Trifluormethyl, Trifluormethoxy, Nitro, Cyano, Amino, Alkylamino, Dialkylamino, Cycloalkyl, Aryl, Heteroaryl, Heterocyclyl, Hydroxy, Alkoxy, Carboxyl, Alkoxycarbonyl, Aminocarbonyl, Alkylaminocarbonyl und Dialkylaminocarbonyl,
worin Alkyl, Alkylamino, Dialkylamino, Cycloalkyl, Aryl, Heteroaryl und Heterocyclyl substituiert sein können mit 0, 1, 2 oder 3 Substituenten R⁵⁻², wobei die Substituenten R⁵⁻² unabhängig voneinander ausgewählt werden aus der Gruppe bestehend aus Hydroxy, Amino, Carboxyl und Aminocarbonyl,
- R⁶: gleich Wasserstoff, Alkyl oder C₃-C₈-Cycloalkyl ist,
oder
- R⁵ und R⁶: zusammen mit dem Stickstoffatom, an das sie gebunden sind, ein Piperidinyl, Morpholinyl, Piperazinyl oder Pyrrolidinyl bilden, wobei Piperidinyl, Morpholinyl, Piperazinyl und Pyrrolidinyl substitiuiert sein können mit 0, 1, 2 oder 3 Substituenten, wobei die Substituenten unabhängig voneinander ausgewählt werden aus der Gruppe bestehend aus Alkyl, Amino, Alkylamino, Dialkylamino, Hydroxy, Alkoxy, Carboxyl, Alkoxycarbonyl und Aminocarbonyl,
- R⁷: gleich Wasserstoff ist,
- R⁸: gleich Wasserstoff ist,
und
- R⁹: gleich Wasserstoff ist.

Bevorzugt im Rahmen der vorliegenden Erfindung sind auch erfindungsgemäße Verbindungen, bei denen
- R¹: gleich Wasserstoff ist,
- R²: gleich Wasserstoff ist,
- R³: gleich Aminocarbonylmethyl, 3-Aminoprop-1-yl, 2-Hydroxy-3-aminoprop-1-yl, 1-Hydroxy-3-aminoprop-1-yl, 3-Guanidinoprop-1-yl, 2-Aminocarbonylethyl, 2-Hydroxycarbonylethyl, 4-Aminobut-1-yl, Hydroxymethyl, 2-Hydroxyethyl, 2-Aminoethyl, 4-Amino-3-hydroxybut-1-yl oder (1-Piperidin-3-yl)-methyl ist,
- R^{3'}: gleich Wasserstoff ist,
- R⁴: gleich Wasserstoff, Methyl, Ethyl, iso-Propyl oder Cyclopropyl ist,
- R⁵: gleich Wasserstoff, C₁-C₆-Alkyl oder C₃-C₈-Cycloalkyl ist,
wobei Alkyl und Cycloalkyl substituiert sein können mit 0, 1, 2 oder 3 Substituenten R⁵⁻¹, wobei die Substituenten R⁵⁻¹ unabhängig voneinander ausgewählt werden aus der Gruppe bestehend aus Halogen, C₁-C₆-Alkyl, Trifluormethyl, Trifluormethoxy, Amino, C₁-C₆-Alkylamino, C₁-C₆-Dialkylamino, C₃-C₈-Cycloalkyl, C₆-C₁₀-Aryl, 5- bis 10-gliedriges Heteroaryl, 5- bis 7-gliedriges Heterocyclyl, Hydroxy, Alkoxy, Carboxyl, C₁-C₆-Alkoxycarbonyl, Amino-carbonyl, C₁-C₆-Alkylaminocarbonyl und C₁-C₆-Dialkylaminocarbonyl,
- R⁶: gleich Wasserstoff oder Methyl ist,
oder
- R⁵ und R⁶: zusammen mit dem Stickstoffatom, an das sie gebunden sind, ein Piperidinyl oder Morpholinyl bilden,
- R⁷: gleich Wasserstoff ist,
- R⁸: gleich Wasserstoff ist,
und
- R⁹: gleich Wasserstoff ist.

Besonders bevorzugt im Rahmen der vorliegenden Erfindung sind erfindungsgemäße Verbindungen, bei denen
- R¹: gleich Wasserstoff ist,
- R²: gleich Wasserstoff ist,
- R³: gleich 3-Aminoprop-1-yl oder 2-Hydroxy-3-aminoprop-1-yl ist,
- R^{3'}: gleich Wasserstoff ist,
- R⁴: gleich Wasserstoff oder Methyl ist,
- R⁵: gleich Wasserstoff, C₁-C₆-Alkyl oder Cyclopropyl ist,
wobei Alkyl substituiert sein kann mit 0, 1, 2 oder 3 Substituenten R⁵⁻¹, wobei die Substituenten R⁵⁻¹ unabhängig voneinander ausgewählt werden aus der Gruppe bestehend aus Trifluormethyl, Amino, Hydroxy, Carboxyl, Aminocarbonyl und Phenyl,
- R⁶: gleich Wasserstoff oder Methyl ist,
- R⁷: gleich Wasserstoff ist,
- R⁸: gleich Wasserstoff ist,
und
- R⁹: gleich Wasserstoff ist.

Bevorzugt im Rahmen der vorliegenden Erfindung sind auch erfindungsgemäße Verbindungen, bei denen R¹ gleich Wasserstoff ist.

Bevorzugt im Rahmen der vorliegenden Erfindung sind auch erfindungsgemäße Verbindungen, bei denen R² gleich Wasserstoff ist.

Bevorzugt im Rahmen der vorliegenden Erfindung sind auch erfindungsgemäße Verbindungen, bei denen R³ gleich 3-Aminoprop-1-yl oder 2-Hydroxy-3-aminoprop-1-yl ist.

Bevorzugt im Rahmen der vorliegenden Erfindung sind auch erfmdungsgemäße Verbindungen, bei denen R^{3'} gleich Wasserstoff ist.

Bevorzugt im Rahmen der vorliegenden Erfindung sind auch erfindungsgemäße Verbindungen, bei denen R⁴ gleich Wasserstoff oder Methyl ist.

Bevorzugt im Rahmen der vorliegenden Erfindung sind auch erfindungsgemäße Verbindungen, bei denen
- R⁵: gleich Wasserstoff, C₁-C₆-Alkyl oder Cyclopropyl ist,
wobei Alkyl substituiert sein kann mit 0, 1, 2 oder 3 Substituenten R⁵⁻¹, wobei die Substituenten R⁵⁻¹ unabhängig voneinander ausgewählt werden aus der Gruppe bestehend aus Trifluormethyl, Amino, Hydroxy, Carboxyl, Aminocarbonyl und Phenyl,

Bevorzugt im Rahmen der vorliegenden Erfindung sind auch erfindungsgemäße Verbindungen, bei denen R⁶ gleich Wasserstoff oder Methyl ist.

Bevorzugt im Rahmen der vorliegenden Erfindung sind auch erfindungsgemäße Verbindungen, bei denen R⁵ und R⁶ zusammen mit dem Stickstoffatom, an das sie gebunden sind, ein Piperidinyl oder Morpholinyl bilden.

Bevorzugt im Rahmen der vorliegenden Erfindung sind auch erfindungsgemäße Verbindungen, bei denen R⁷ gleich Wasserstoff ist.

Bevorzugt im Rahmen der vorliegenden Erfindung sind aucherfindungsgemäße Verbindungen, bei denen R⁸ gleich Wasserstoff ist.

Bevorzugt im Rahmen der vorliegenden Erfindung sind auch erfindungsgemäße Verbindungen, bei denen R⁹ gleich Wasserstoff ist.

Gegenstand der Erfindung ist weiterhin ein Verfahren zur Herstellung der Verbindungen der Formel (I), wobei Verbindungen der Formel 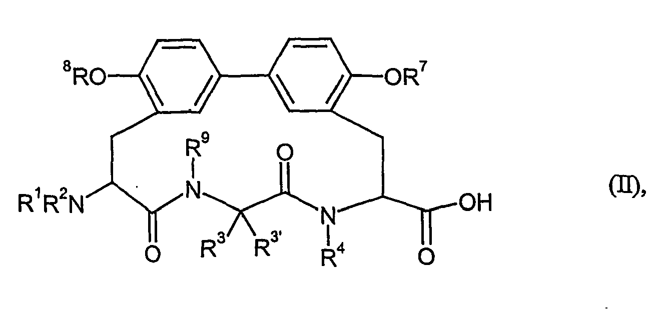 worin R¹ bis R⁴ und R⁷ bis R⁹ die oben angegebene Bedeutung haben, wobei die Verbindungen (II) gegebenenfalls in aktivierter Form (Acyldonor) vorliegen können,
mit Verbindungen der Formel

H-NR⁵R⁶ (III),

worin R⁵ und R⁶ die oben angegebene Bedeutung haben, umgesetzt werden.

Gegebenenfalls wird vor der Umsetzung von Verbindungen der Formel (II), mit Verbindungen der Formel (III) die Blockierung reaktiver Funktionalitäten (z.B. freie Aminofunktionen) in Verbindungen der Formel (II) vorgenommen. Dies geschieht nach Standardverfahren der Schutzgruppenchemie. Bevorzugt sind säurelabile Schutzgruppen an R¹ (oder R²), oder als Substituenten in den Resten R³ und R^{3'}, insbesondere bevorzugt ist Boc. Reaktive Funktionalitäten in den Resten R⁵ und R⁶ von Verbindungen der Formel (III) werden bereits geschützt mit in die Synthese eingebracht, bevorzugt sind säurelabile Schutzgruppen (z.B. Boc). Nach erfolgter Umsetzung zu Verbindungen der Formel (I) können die Schutzgruppen durch Entschützungsreaktion abgespalten werden. Dies geschieht nach Standardverfahren der Schutzgruppenchemie. Bevorzugt sind Entschützungsreaktionen unter sauren Bedingungen.

Stellt z.B. R² in Verbindungen der Formel (I) eine selektiv abspaltbare Schutzgruppe dar, kann nach Entschützung (z.B. nach Hydrogenolyse im Fall R² = Z) die freigelegte Aminofunktion (R² = H) mit dem gewünschten Substituenten R² funktionalisiert werden.

Zur Überführung der Verbindungen (II) in die aktivierte Form (Acyldonor) sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid Hydrochlorid (EDC) (gegebenenfalls in Gegenwart von Pentafluorphenol (PFP)), N-Cyclohexylcarbodiimid-N-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoroborat (TPTU) oder O-(7-Azabenzotriazol-1-yl)-N,N,N',N'-tetramethyl-uroniumhexafluorophosphat (HATU), oder Benzotriazol-1-yloxytris(dimethylamino)-phosphoniumhexafluorophosphat (BOP), oder Mischungen aus diesen mit Basen, gegebenenfalls in Gegenwart von Kupplungsadditiven wie 1-Hydroxybenzotriazol (HOBt), geeignet.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4-Dimethylaminopyridin oder Diisopropylethylamin.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoffe wie Benzol, Toluol, Tetrahydrofuran, Dioxan, Acetonitril oder Dimethylformamid. Ebenso ist es möglich, Gemische der Lösemittel einzusetzen. Besonders bevorzugt sind wasserfreies Dichlormethan und Dimethylformamid.

Bevorzugt ist die Aktivierung mit O-(7-Azabenzotriazol-1-yl)-N,N,N',N'-tetramethyl-uroniumhexafluorophosphat (HATU) in Dimethylformamid.

Die Verbindungen der Formel (III) sind bekannt oder können analog bekannten Verfahren hergestellt werden.

Die Verbindungen der Formel (II) sind bekannt oder können hergestellt werden, indem in Verbindungen der Formel 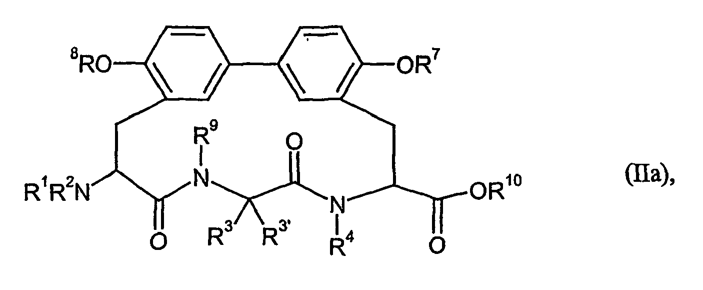 worin
- R¹ bis R⁴ und R⁷ bis R⁹: die oben angegebene Bedeutung haben und
- R¹⁰: gleich Benzyl (alternativ für Alkyl, z.B. Methyl oder Ethyl) ist,
der Ester gespalten wird. Diese Esterspaltung erfolgt im Fall von R¹⁰ gleich Benzyl vorzugsweise mit Wasserstoff in Gegenwart von Palladium auf Kohle. Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoff wie Tetrahydrofuran, Dioxan, Dimethylformamid oder Alkohole (bevorzugt sind Methanol, Ethanol und Isopropanol), gegebenenfalls in Gegenwart von Säure mit einem oder mehreren Säureäquivalenten. Ebenso ist es möglich, Gemische der Lösemittel einzusetzen. Besonders bevorzugt sind Ameisensäure in Ethanol, wässrige Essigsäure und THF.

Alternativ können die Ester (R¹⁰ = Benzyl, Alkyl) auch durch basische Hydrolyse in die entsprechenden Carbonsäuren gespalten werden. Als Basen werden bevorzugt wässriges Lithium- oder Natriumhydroxid eingesetzt. Als Lösemittel eignen sich hierbei organische Lösemittel, die teilweise oder unbegrenzt mit Wasser mischbar sind. Hierzu gehören Alkohole (bevorzugt sind Methanol und Ethanol), Tetrahydrofuran, Dioxan und Dimethylformamid. Ebenso ist es möglich, Gemische der Lösemittel einzusetzen. Besonders bevorzugt sind Methanol, Tetrahydrofuran und Dimethylformamid.

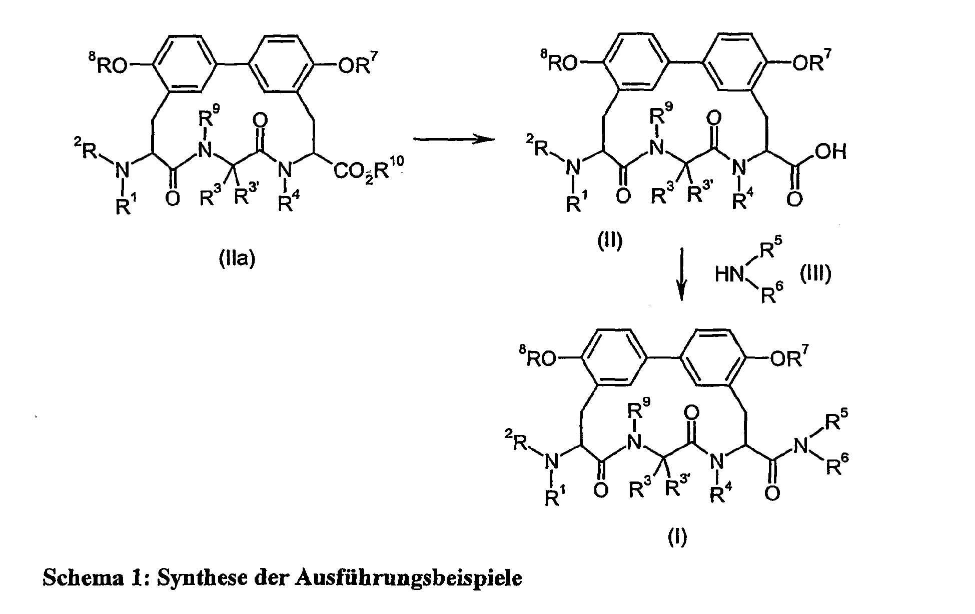

Die Verbindungen der Formel (IIa) können hergestellt werden, indem Verbindungen der Formel 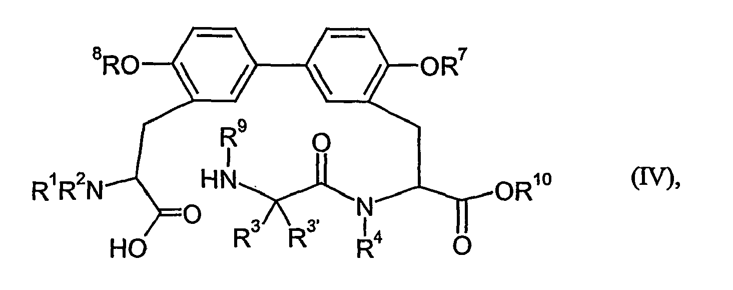 worin
- R¹ bis R⁴ und R⁷ bis R¹⁰: die oben angegebene Bedeutung haben,
wobei diese Verbindungen gegebenenfalls in aktivierter Form vorliegen, unter Peptidkupplung zyklisiert werden. Alternativ kann ein mehrstufiger Prozess erfolgen, bei dem Verbindungen der Formel 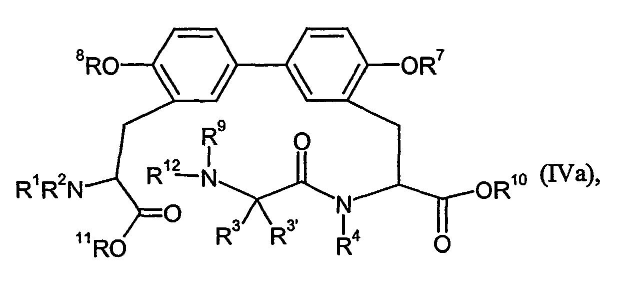 worin
- R¹ bis R⁴ und R⁷ bis R¹⁰: die oben angegebene Bedeutung haben,
- R¹¹: nach Aktivierung gleich Pentafluorphenol ist und
- R¹²: gleich eine Aminschutzgruppe (bevorzugt Boc) ist,
durch Schutzgruppenabspaltung der Aminschutzgruppe (zu R¹² gleich Wasserstoff) und anschließende Zyklisierung unter basischen Bedingungen zu Verbindungen der Formel (IIa) umgesetzt werden.

Zur Überführung der Verbindungen in die aktivierte Form sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC) (gegebenenfalls in Gegenwart von Pentafluorphenol (PFP)), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetramethyluronium-hexafluorophosphat (HBTU), 2-(2-Oxo-1-(2H)-pyridyl)-1,1,3,3-tetramethyluroniumtetrafluoroborat (TPTU) oder O-(7-Azabenzotriazol-1-yl)-N,N,N',N'-tetramethyluroniumhexafluorophosphat (HATU), oder Benzotriazol-1-yloxytris(dimethylamino)-phosphoniumhexafluorophosphat (BOP), oder Mischungen aus diesen mit Basen, gegebenenfalls in Gegenwart von 1-Hydroxybenztriazol (HOBt), geeignet.

Basen sind beispielsweise Alkalicarbonate, wie z.B. Natrium- oder Kaliumcarbonat, oder -hydrogencarbonat, oder bevorzugt organische Basen wie Trialkylamine z.B. Triethylamin, N-Methylmorpholin, N-Methylpiperidin, 4-Dimethylaminopyridin oder Diisopropylethylamin.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoffe wie Benzol, Toluol, Tetrahydrofuran, Dioxan, Dimethylformamid oder Acetonitril. Ebenso ist es möglich, Gemische der Lösemittel einzusetzen. Besonders bevorzugt sind Dichlormethan und Dimethylformamid.

Besonders bevorzugt ist die Aktivierung in Form eines Pentafluorphenylesters (R¹¹ = C₆F₅) und anschließendem basenkatalysierten Ringschluss.

Die Verbindungen der Formel (IV) sind bekannt, können analog bekannten Verfahren hergestellt werden oder indem Verbindungen der Formel 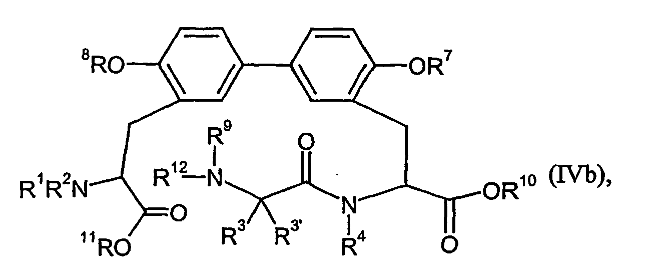 worin
- R¹ bis R⁴ und R⁷ bis R¹⁰ und R¹²: die oben angegebene Bedeutung haben und
- R¹¹: gleich eine Silylschutzgruppe, insbesondere 2-(Trimethylsilyl)-ethyl, ist,
nach Abspaltung der Schutzgruppe an R¹², mit Fluorid, insbesondere mit Tetrabutylammoniumfluorid, umgesetzt werden.

Als Lösemittel eignen sich hierbei inerte organische Lösemittel, die sich unter den Reaktionsbedingungen nicht verändern. Hierzu gehören Halogenkohlenwasserstoffe wie Dichlormethan, Kohlenwasserstoffe wie Benzol, Toluol, Tetrahydrofuran, Dioxan und Dimethylformamid. Ebenso ist es möglich, Gemische der Lösemittel einzusetzen. Bevorzugte Lösungsmittel sind Tetrahydrofuran und Dimethylformamid.

Die Verbindungen der Formel (IVb) sind bekannt, können analog bekannten Verfahren hergestellt werden oder indem Verbindungen der Formel 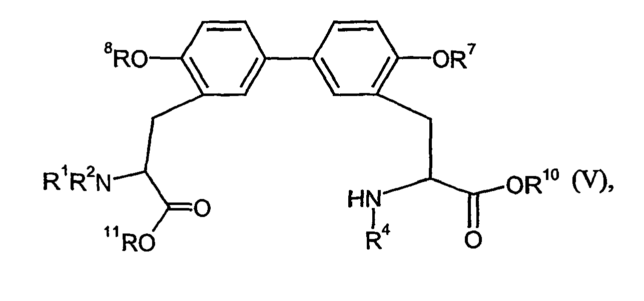 worin
- R¹, R², R⁴, R⁷, R⁸ und R¹⁰: die oben angegebene Bedeutung haben,
- R¹¹: gleich eine Silylschutzgruppe ist,
mit Verbindungen der Formel 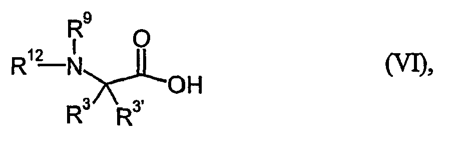 worin
- R³, R^{3'}, R⁹ und R¹²: die oben angegebene Bedeutung haben und
wobei die Verbindungen gegebenenfalls in aktivierter Form vorliegen können, umgesetzt werden.

Zur Überführung der Verbindungen in die aktivierte Form sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid Hydrochlorid (EDC) (gegebenenfalls in Gegenwart von Pentafluorphenol (PFP)), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluroniumhexafluorophosphat (HBTU), 2-(2-Oxo-1-(2H)-pyridyl)-1,1,3,3-tetramethyluroniumtetrafluoroborat (TPTU) oder O-(7-Azabenzotriazol-1-yl)-N,N,N',N'-tetramethyluroniumhexafluorophosphat (HATU), oder Benzotriazol-1-yloxytris(dimethylamino)-phosphoniumhexafluorophosphat (BOP), oder Mischungen aus diesen mit Basen, gegebenenfalls unter Zusatz von Kupplungsadditiven wie 1-Hydroxybenzotriazol (HOBt), geeignet.

Basen sind beispielsweise Alkalicarbonate, wie z.B. Natrium- oder Kaliumcarbonat, oder -hydrogencarbonat, oder bevorzugt organische Basen wie Trialkylamine z.B. Triethylamin, N-Methylmorpholin, N-Methylpiperidin, 4-Dimethylaminopyridin oder Diisopropylethylamin.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoffe wie Benzol, Toluol, Acetonitril, Tetrahydrofuran, Dioxan oder Dimethylformamid. Ebenso ist es möglich, Gemische der Lösemittel einzusetzen. Besonders bevorzugt sind wasserfreies Dichlormethan und Dimethylformamid.

Besonders bevorzugt ist die Umsetzung in Gegenwart von HATU und *N,N*-Diisopropylethylamin.

Die Verbindungen der Formel (VI) sind bekannt oder können analog bekannten Verfahren hergestellt werden.

Die Verbindungen der Formel (V), beziehungsweise ihre Salze (z.B. Hydrochloride), sind bekannt, können analog bekannten Verfahren hergestellt werden oder indem Verbindungen der Formel 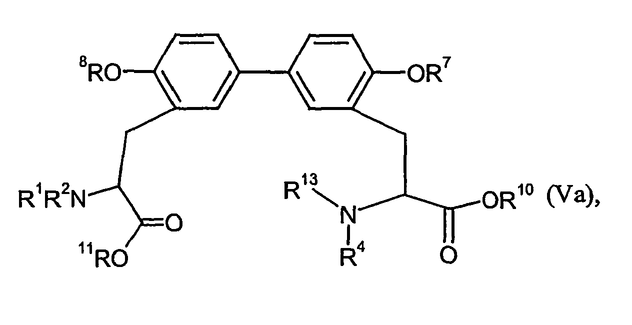 worin
- R¹, R², R⁴, R⁷, R⁸ und R¹⁰: die oben angegebene Bedeutung haben,
- R¹¹: gleich eine Silylschutzgruppe ist, und
- R¹³: gleich eine Aminschutzgruppe, insbesondere Boc, ist,
durch Entschützung an R¹³ hergestellt werden. Dies geschieht nach Standardverfahren der Schutzgruppenchemie, im Falle von R¹³ gleich Boc bevorzugt mit Chlorwasserstoff in Dioxan.

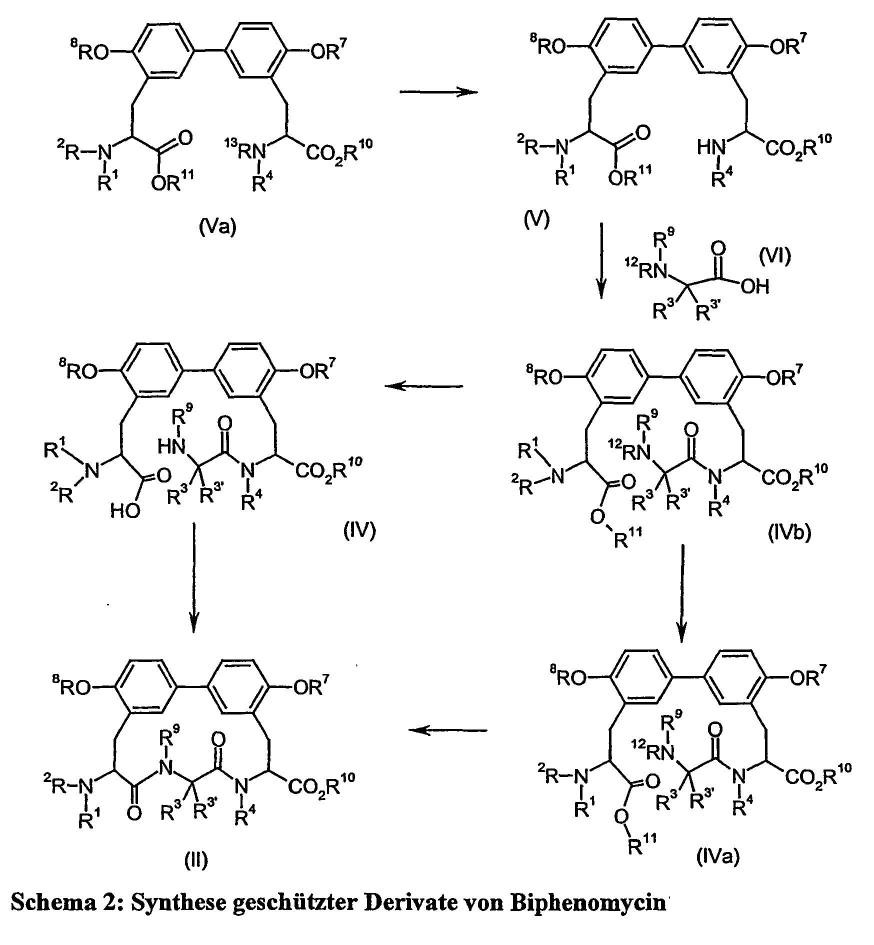

Die Verbindungen der Formel (Va) sind bekannt, können analog bekannten Verfahren hergestellt werden oder indem Verbindungen der Formel 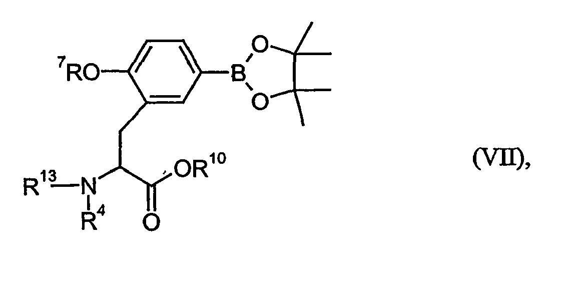 worin
- R⁴ und R⁷: die oben angegebene Bedeutung haben,
- R¹⁰: gleich Benzyl oder Alkyl ist und
- R¹³: gleich eine Aminoschutzgruppe (bevorzugt Boc) ist,
mit Verbindungen der Formel 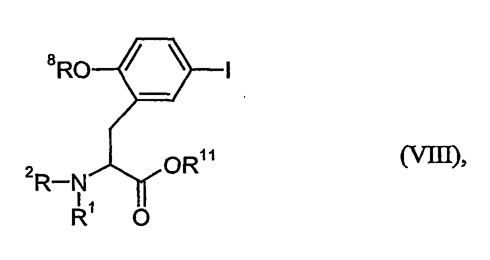 worin
- R¹, R² und R⁸: die oben angegebene Bedeutung haben und
- R¹¹: gleich eine Silylschutzgruppe, insbesondere 2-(Trimethylsilyl)-ethyl, ist,
umgesetzt werden. Die Umsetzung, bekannt als Suzuki-Reaktion (*Synlett* 1992, 207-210; *Chem. Rev.* 1995, 95, 2457-2483), erfolgt in Gegenwart von Palladium-Katalysatoren und einer Base, bevorzugt in Gegenwart von Bis(diphenylphosphino)-ferrocen-palladium(II)chlorid und Caesiumcarbonat.

Als Lösemittel eignen sich hierbei inerte organische Lösemittel, die sich unter den Reaktionsbedingungen nicht verändern. Hierzu gehören Kohlenwasserstoffe wie Benzol, Toluol, Tetrahydrofuran, Dioxan, Dimethylformamid und Dimethylsulfoxid.

Ebenso ist es möglich, Gemische der Lösemittel einzusetzen. Besonders bevorzugt sind Dimethylformamid und Dimethylsulfoxid.

Die Verbindungen der Formel (VII) sind bekannt, können analog bekannten Verfahren hergestellt werden oder indem Verbindungen der Formel 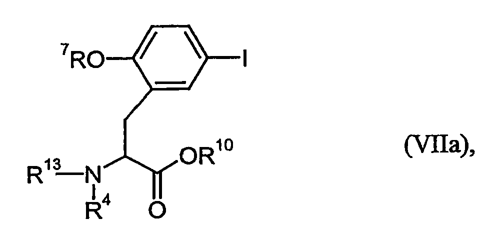 worin
- R⁴ und R⁷: die oben angegebene Bedeutung haben,
- R¹⁰: gleich Benzyl oder Alkyl ist und
- R¹³: gleich eine Aminoschutzgruppe (bevorzugt Boc) ist,
mit Bis(pinacolato)diboron umgesetzt werden. Diese Umsetzung, bekannt als spezielle Variante der Suzuki-Reaktion (*J. Org. Chem*. 1995, 7508-7510; *Tetrahedron Lett*., 1997, 3841-3844), erfolgt in Gegenwart von Palladium-Katalysatoren und einer Base, bevorzugt in Gegenwart von Bis(diphenylphosphino)-ferrocen-palladium(II)chlorid und von Kaliumacetat.

Als Lösemittel eignen sich hierbei inerte organische Lösemittel, die sich unter den Reaktionsbedingungen nicht verändern. Hierzu gehören Kohlenwasserstoffe wie Benzol, Toluol, Tetrahydrofuran, Dioxan, Dimethylformamid und Dimethylsulfoxid. Ebenso ist es möglich, Gemische der Lösemittel einzusetzen. Besonders bevorzugt sind Dimethylformamid und Dimethylsulfoxid.

Die Verbindungen der Formel (VIIa) sind bekannt, können analog bekannten Verfahren hergestellt werden oder indem Verbindungen der Formel 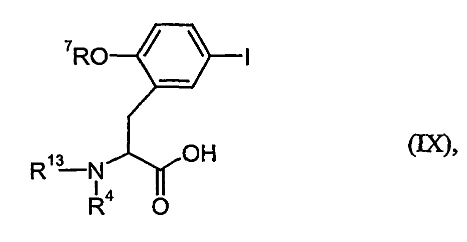 worin
- R⁴ und R⁷: die oben angegebene Bedeutung haben und
- R¹³: gleich eine Aminoschutzgruppe (bevorzugt Boc) ist,
nach Aktivierung der freien Carboxylatfunktion mit ¹⁰R-OH (bevorzugt Benzylalkohol, Allylalkohol und niedere aliphatische Alkohole) in Gegenwart von 4-Dimethylaminopyridin umgesetzt werden.

Zur Überführung der Carbonsäuren in die aktivierte Form sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC) N-Cyclohexylcarbodiimid-N'-propyloxymethyl-Polystyrol (PS-Carbodiimid) oder Carbonylverbindungen wie Carbonyldiimidazol geeignet.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoffe wie Benzol, Toluol, Acetonitril, Tetrahydrofuran, Dioxan oder Dimethylformamid. Ebenso ist es möglich, Gemische der Lösemittel einzusetzen. Besonders bevorzugt sind wasserfreies Dichlormethan und Acetonitril.

Bevorzugt sind Umsetzungen mit Aktivierung durch EDC oder DIC in absolutem Acetonitril oder Dichlormethan bei tiefer Temperatur (-10°C).

Die Verbindungen der Formel (VIII) sind bekannt, können analog bekannten Verfahren hergestellt werden oder indem Verbindungen der Formel 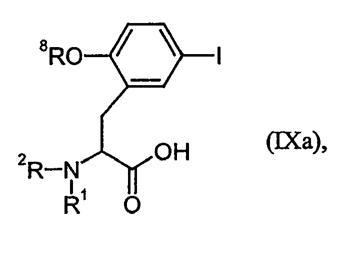 worin
R¹, R² und R⁸ die oben angegebene Bedeutung haben
nach Aktivierung der freien Carboxylatfunktion mit ¹¹R-OH (bevorzugt 2-Trimethylsilylethanol) in Gegenwart von 4-Dimethylaminopyridin umgesetzt werden.

Zur Überführung der Carbonsäuren in die aktivierte Form sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC) N-Cyclohexylcarbodiimid-N'-propyloxymethyl-Polystyrol (PS-Carbodiimid) oder Carbonylverbindungen wie Carbonyldiimidazol geeignet.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoffe wie Benzol, Toluol, Acetonitril, Tetrahydrofuran, Dioxan oder Dimethylformamid. Ebenso ist es möglich, Gemische der Lösemittel einzusetzen. Besonders bevorzugt sind wasserfreies Dichlormethan und Acetonitril.

Bevorzugt sind Umsetzungen mit Aktivierung durch EDC oder DIC in absolutem Acetonitril oder Dichlormethan bei tiefer Temperatur (-10°C).

Die Carbonsäuren der Formel (IXa) sind bekannt, können analog bekannten Verfahren hergestellt werden oder indem Verbindungen der Formel 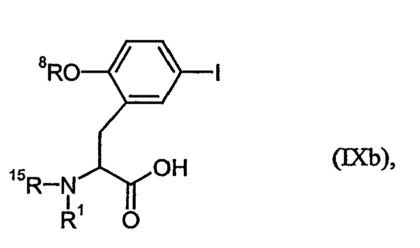 worin
- R¹ und R⁸: die oben angegebene Bedeutung haben und
- R¹⁵: gleich eine Aminoschutzgruppe, insbesondere Boc, ist,
in der ersten Stufe an R¹⁵ entschützt werden. Dies geschieht nach Standardverfahren der Schutzgruppenchemie, im Falle von R¹⁵ gleich Boc bevorzugterweise mit Chlorwasserstoff in Dioxan oder mit Trifluoressigsäure in Dichlormethan in Gegenwart geringer Mengen Wasser. Das erhaltene freie Amin 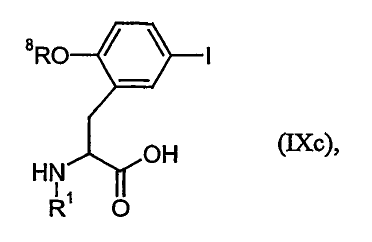 worin
- R¹ und R⁸: die oben angegebene Bedeutung haben,
wobei das Amin gegebenenfalls in Form eines Salzes, vorzugsweise Hydrochlorid oder Trifluoracetat, vorliegen kann,
wird in der zweiten Stufe mit R²-X, worin R² die oben angegebene Bedeutung hat und X für eine Abgangsgruppe steht, in Gegenwart einer Base in inerten Lösungsmitteln umgesetzt, gegebenenfalls in Gegenwart von Kaliumiodid, bevorzugt in einem Temperaturbereich von 0°C über Raumtemperatur bis zum Rückfluss der Lösungsmittel bei Normaldruck um. Bevorzugt für X sind Mesylat, Tosylat, Succinat oder Halogen, wobei für Halogen Chlor, Brom oder Iod bevorzugt ist.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylpiperidin, 4-Dimethylaminopyridin oder Diisopropylethylamin.

Als Lösemittel eignen sich hierbei inerte organische Lösemittel, die sich unter den Reaktionsbedingungen nicht verändern. Hierzu gehören Halogenkohlenwasserstoffe wie Dichlormethan oder Trichlormethan, Kohlenwasserstoffe wie Benzol, Toluol, Acetonitril, Tetrahydrofuran, Dioxan, Aceton oder Dimethylformamid. Ebenso ist es möglich, Gemische der Lösemittel einzusetzen. Besonders bevorzugt sind Dimethylformamid und Dichlormethan. 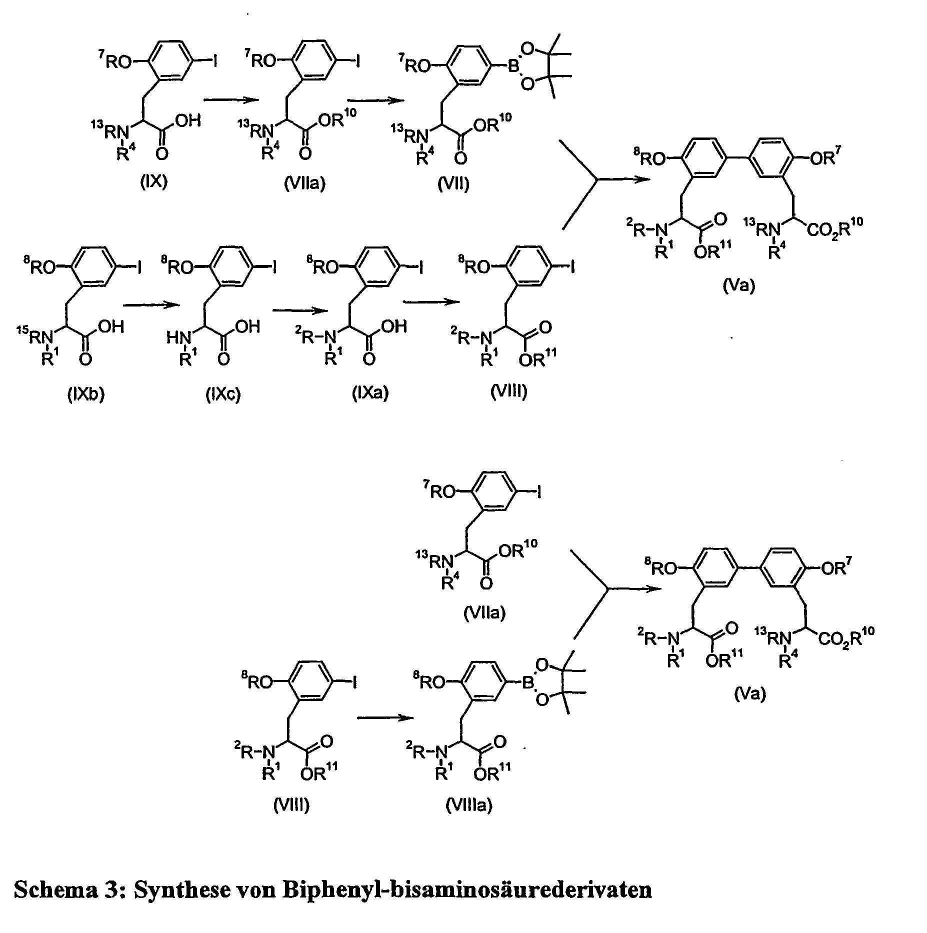 R² kann optional eine Schutzgruppe (z.B. Z, d.h. Benzyloxycarbonyl) darstellen.

In einem Alternativverfahren können die Verbindungen der Formel (Va) hergestellt werden, indem Verbindungen der Formel 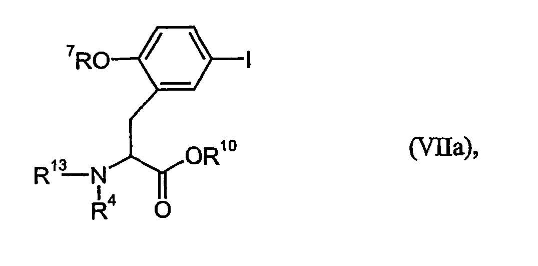 worin
- R⁴ und R⁷: die oben angegebene Bedeutung haben,
- R¹⁰: gleich Benzyl oder Alkyl ist und
- R¹³: gleich eine Aminoschutzgruppe (bevorzugt Boc) ist,
mit Verbindungen der Formel 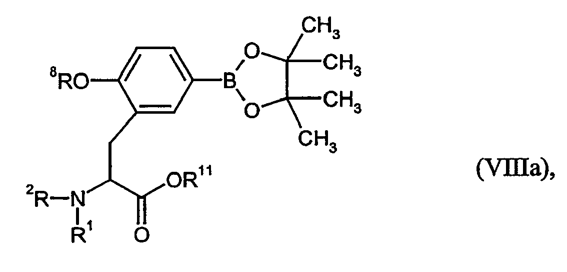 worin
- R¹, R² und R⁸: die oben angegebene Bedeutung haben und
- R¹¹: gleich eine Silylschutzgruppe, insbesondere 2-(Trimethylsilyl)-ethyl, ist,
umgesetzt werden. Die Umsetzung, bekannt als Suzuki Reaktion (*Synlett* 1992, 207-210; *C*h*em. Rev.* 1995, 95, 2457-2483), erfolgt in Gegenwart von Palladium-Katalysatoren und einer Base, bevorzugt in Gegenwart von Bis(diphenylphosphino)-ferrocen-palladium(II)chlorid und Caesiumcarbonat.

Als Lösemittel eignen sich hierbei inerte organische Lösemittel, die sich unter den Reaktionsbedingungen nicht verändern. Hierzu gehören Kohlenwasserstoffe wie Benzol, Toluol, Tetrahydrofuran, Dioxan, Dimethylformamid und Dimethylsulfoxid. Ebenso ist es möglich, Gemische der Lösemittel einzusetzen. Besonders bevorzugt sind Dimethylformamid und Dimethylsulfoxid.

Die Verbindungen der Formel (VIIIa) können aus den Verbindungen der Formel (VIII) nach dem für die Verbindungen (VII) beschriebenen Verfahren hergestellt werden.

Die enantiomerenreinen Verbindungen der Formeln (IX) und (IXb) sind bekannt oder können aus racemischen Vorläufern nach bekannten Verfahren, wie z.B. Kristallisation mit chiralen Aminbasen oder durch Chromatograpie an chiralen, stationären Phasen, erhalten werden.

Die Verbindungen der Formeln (IX) und (IXb) sind bekannt, können analog bekannten Verfahren hergestellt werden oder indem Verbindungen der Formeln 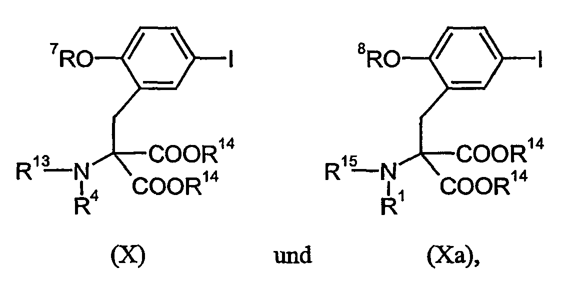 worin
- R⁴ und R⁷ und R¹ und R⁸: die oben angegebene Bedeutung haben,
- R¹³ und R¹⁵: gleich eine Aminoschutzgruppe sind und
- R¹⁴: gleich Alkyl (besonders bevorzugt Ethyl) ist,
decarboxyliert werden. Diese Reaktion findet bevorzugt in basischem Medium in einem Wasser-Ethanol-Gemisch statt.

Die Verbindungen der Formeln (X) und (Xa) sind bekannt, können analog bekannten Verfahren hergestellt werden oder indem Verbindungen der Formeln 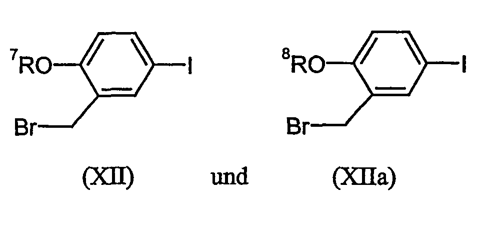 worin
R⁷ und R⁸ die oben angegebene Bedeutung haben,
mit Verbindungen der Formeln 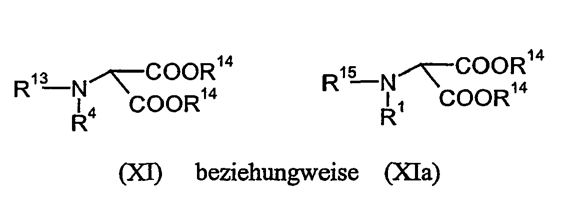 worin
- R⁴ und R¹: die oben angegebene Bedeutung haben,
- R¹³ und R¹⁵: gleich eine Aminoschutzgruppe sind und
- R¹⁴: gleich Alkyl (bevorzugt Ethyl) ist,
umgesetzt werden. Diese Reaktion findet bevorzugt mit Alkalialkoholat in Alkohol, besonders mit Natriumethylat in Ethanol statt.

Die Verbindungen der Formeln (XII) und (XIIa) sind bekannt, können analog bekannten Verfahren hergestellt werden oder indem Verbindungen der Formeln 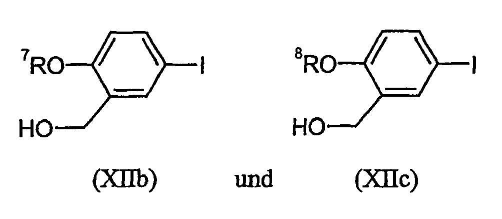 worin
- R⁷ und R⁸: die oben angegebene Bedeutung haben,
mit Phosphortribromid umgesetzt werden. Bevorzugt findet die Reaktion in Toluol statt.

Die Verbindungen der Formeln (XIIb) und (XIIc) sind bekannt, können analog bekannten Verfahren hergestellt werden oder indem Verbindungen der Formel 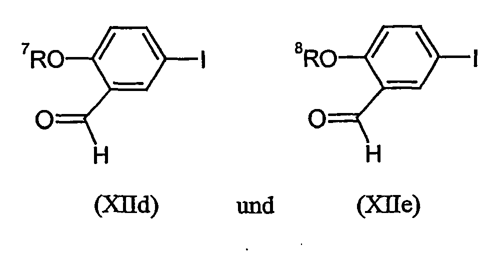 worin
- R⁷ und R⁸: die oben angegebene Bedeutung haben,
reduziert werden. Die Reduktion findet bevorzugt mit Diisobutylaluminiumhydrid-Lösung in Dichlormethan unter nachfolgender Zugabe einer gesättigten Kaliumnatriumtarirat-Lösung statt.

Die Verbindungen der Formeln (XIId) und (XIIe) sind bekannt, können analog bekannten Verfahren hergestellt werden oder indem 2-Hydroxy-5-iod-benzaldehyd mit Verbindungen der Formeln 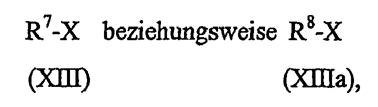 worin
- R⁷ und R⁸: die oben angegebene Bedeutung haben und
- X: für eine Abgangsgruppe steht, in inerten Lösungsmitteln umgesetzt werden,
gegebenenfalls in Gegenwart einer Base, gegebenenfalls in Gegenwart von Kaliumiodid, bevorzugt in einem Temperaturbereich von Raumtemperatur bis zum Rückfluss der Lösungsmittel bei Normaldruck um. Bevorzugt für X sind Mesylat, Tosylat oder Halogen, wobei für Halogen Chlor, Brom oder Iod bevorzugt sind.

Inerte Lösungsmittel sind beispielsweise Halogenkohlenwasserstoffe wie Methylenchlorid, Trichlormethan oder 1,2-Dichlorethan, Ether wie Dioxan, Tetrahydrofuran oder 1,2-Dimethoxyethan, oder andere Lösemittel wie Aceton, Dimethylformamid, Dimethylacetamid, 2-Butanon oder Acetonitril, bevorzugt Tetrahydrofuran, Methylenchlorid, Aceton, 2-Butanon, Acetonitril, Dimethylformamid oder 1,2-Dimethoxyethan. Bevorzugt ist Dimethylformamid.

Basen sind beispielsweise Alkalicarbonate wie Cäsiumcarbonat, Natrium- oder Kaliumcarbonat, oder Natrium- oder Kaliummethanolat, oder Natrium- oder Kaliumethanolat oder Kalium-*tert*.-butylat, oder Amide wie Natriumamid, Lithium-bis-(trimethylsilyl)amid oder Lithiumdiisopropylamid, oder metallorganische Verbindungen wie Butyllithium oder Phenyllithium, tertiäre Aminbasen wie Triethylamin oder Diisopropylethylamin, oder andere Basen wie Natriumhydrid, DBU, bevorzugt Kalium-*tert*.-butylat, Cäsiumcarbonat, DBU, Natriumhydrid, Kaliumcarbonat oder Natriumcarbonat. Bevorzugt ist Kaliumcarbonat.

Die Verbindungen der Formeln (XIII) und (XIIIa) sind bekannt oder können analog bekannten Verfahren hergestellt werden.

Die Herstellung der erfindungsgemäßen Verbindungen kann durch folgendes Syntheseschema verdeutlicht werden. Hierbei ist der besseren Übersichtlichkeit halber die in der Beschreibung verwendete lateinische Nummerierung beibehalten, das Schema zeigt jedoch teilweise spezielle Ausführungsformen, insbesondere R¹⁴ in (XI) und (XIa) gleich Ethyl und R¹³ und R¹⁵ gleich Boc.

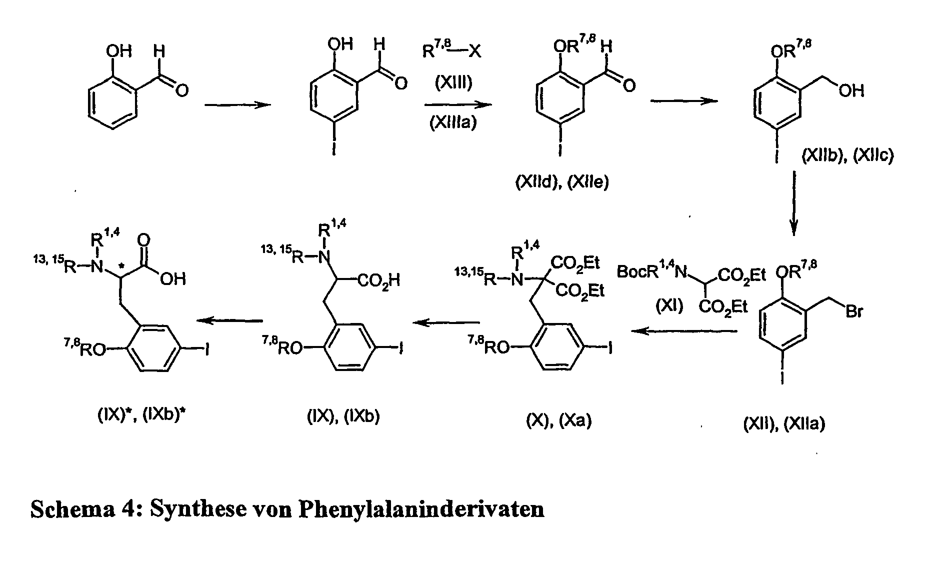

In einem alternativen Verfahren können die Substituenten R⁵ und R⁶ auch über die Verbindungen der Formel (VII) bzw. (VIIa) in die Synthese eingebracht werden. Dazu wird die Säurefunktion der Verbindungen der Formel (VII) bzw. (VIIa) nach dem Fachmann bekannten Bedingungen freigesetzt und mit Verbindungen der Formel (III) nach dem Fachmann bekannten Bedingungen umgesetzt.

Die erfindungsgemäßen Verbindungen zeigen ein nicht vorhersehbares, wertvolles pharmakologisches und pharmakokinetisches Wirkspektrum.

Sie eignen sich daher zur Verwendung als Arzneimittel zur Behandlung und/oder Prophylaxe von Krankheiten bei Menschen und Tieren.

Die erfindungsgemäßen Verbindungen können aufgrund ihrer pharmakologischen Eigenschaften allein oder in Kombination mit anderen Wirkstoffen zur Behandlung und/oder Prävention von Infektionskrankheiten, insbesondere von bakteriellen Infektionen, eingesetzt werden.

Beispielsweise können lokale und/oder systemische Erkrankungen behandelt und/oder verhindert werden, die durch die folgenden Erreger oder durch Mischungen der folgenden Erreger verursacht werden:

Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytocy), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen topisch anwendbaren Zubereitungen verhindert, gebessert oder geheilt werden können, seien beispielsweise genannt:

Infektionskrankheiten beim Menschen wie z. B. septische Infektionen, Knochen- und Gelenkinfektionen, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, septische Arthritis, Mastitis, Tonsillitis, Genital-Infektionen und Augeninfektionen.

Außer beim Menschen können bakterielle Infektionen auch bei anderen Spezies behandelt werden. Beispielhaft seien genannt:
Schwein: Coli-diarrhoe, Enterotoxamie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie z.B. Pasteurella, Brucella, Campylobacter, Listeria, Erysipelothris, Corynebakterien, Borellia, Treponema, Nocardia, Rikettsie, Yersinia, erweitert.

Die vorliegende Erfindung betrifft weiterhin Verbindungen der Formel (I) zur Bekämpfung von Erkrankungen, insbesondere bakterieller Erkrankungen, Arzneimittel, enthaltend Verbindungen der Formel (I) und Hilfsstoffe sowie die Verwendung von Verbindungen der Formel (I) zur Herstellung eines Arzneimittels zur Behandlung von bakteriellen Erkrankungen.

Weiterer Gegenstand der vorliegenden Erfindung sind Arzneimittel, die mindestens eine erfindungsgemäße Verbindung, vorzugsweise zusammen mit einem oder mehreren pharmakologisch unbedenklichen Hilfs- oder Trägerstoffen enthalten, sowie deren Verwendung zu den zuvor genannten Zwecken.

Der Wirkstoff kann systemisch und/oder lokal wirken. Zu diesem Zweck kann er auf geeignete Weise appliziert werden, wie z.B. oral, parenteral, pulmonal, nasal, sublingual, lingual, buccal, rectal, transdermal, conjunctival, otisch oder als Implantat.

Für diese Applikationswege kann der Wirkstoff in geeigneten Applikationsformen verabreicht werden.

Für die orale Applikation eignen sich bekannte, den Wirkstoff schnell und/oder modifiziert abgebende Applikationsformen, wie z.B. Tabletten (nicht überzogene sowie überzogene Tabletten, z.B. mit magensaftresistenten Überzüge versehene Tabletten oder Filmtabletten), Kapseln, Dragees, Granulate, Pellets, Pulver, Emulsionen, Suspensionen, Lösungen und Aerosole.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Für die sonstigen Applikationswege eignen sich z.B. Inhalationsarzneiformen (u.a. Pulverinhalatoren, Nebulizer), Nasentropfen/-lösungen, Sprays; lingual, sublingual oder buccal zu applizierende Tabletten oder Kapseln, Suppositorien, Ohren- und Augen-präparationen, Vaginalkapseln, wässrige Suspensionen (Lotionen, Schüttelmixturen), lipophile Suspensionen, Salben, Cremes, Milch, Pasten, Streupuder oder Implantate.

Die Wirkstoffe können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und/oder Geruchskorrigentien.

Im Allgemeinen hat es sich als vorteilhaft erwiesen, bei parenteraler Applikation Mengen von etwa 5 bis 250 mg/kg Körpergewicht je 24 h zur Erzielung wirksamer Ergebnisse zu verabreichen. Bei oraler Applikation beträgt die Menge etwa 5 bis 100 mg/kg Körpergewicht je 24 h.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Verwendete Abkürzungen:

- Aloc: Allyloxycarbonyl
- aq.: Wässrig
- Äquiv.: Äquivalent
- Bn: Benzyl
- Boc: *tert*.-Butoxycarbonyl
- CDCl₃: Chloroform
- CH: Cyclohexan
- D: dublett (im ¹H-NMR)
- Dd: Dublett von dublett
- DCM: Dichlormethan
- DCC: Dicyclohexylcarbodiimid
- DIC: Diisopropylcarbodiimid
- DIPEA: Diisopropylethylamin
- DMSO: Dimethylsulfoxid
- DMAP: 4-*N*,*N*-Dimethylaminopyridin
- DMF: Dimethylformamid
- d. Th.: der Theorie
- EDC: *N'*-(3-Dimethylaminopropyl)-*N*-ethylcarbodiimid x HCl
- EE: Ethylacetat (Essigsäureethylester)
- ESI: Elektrospray-Ionisation (bei MS)
- ges.: gesättigt
- HATU: *O*-(7-Azabenzotriazol-1-yl)-*N,N,N',N'*-tetramethyluroniumhexafluorphosphat
- HBTU: *O*-(Benzotriazol-1-yl)-*N,N,N',N'*-tetramethyluroniumhexafluorphosphat
- HOBt: 1-Hydroxy-1H-benzotriazol x H₂O
- H: Stunde(n)
- HPLC: Hochdruck-, Hochleistungsflüssigchromatographie
- LC-MS: Flüssigchromatographie-gekoppelte Massenspektroskopie
- M: multiplett (im ¹H-NMR)
- Min: Minuten
- MS: Massenspektroskopie
- MeOH: Methanol
- NMR: Kernresonanzspektroskopie
- MTBE: Methyl-tert. -butylether
- Pd/C: Palladium/Kohle
- proz.: Prozent
- Q: quartett (im ¹H-NMR)
- R_{f}: Retentionsindex (bei DC)
- RT: Raumtemperatur
- Rₜ: Retentionszeit (bei HPLC)
- S: singulett (im ¹H-NMR)
- T: triplett (im ¹H-NMR)
- TBS: *tert*.-Butyldimethylsilyl
- THF: Tetrahydrofuran
- TMSE: 2-(Trimethylsilyl)-ethyl
- TPTU: 2-(2-Oxo-1(2H)-pyridyl)-1,1,3,3-tetramethyluroniumtetrafluoroborat
- Z: Benzyloxycarbonyl

### Allgemeine Methoden LC-MS und HPLC

**Präparative RP-HPLC:** Säule: YMC-Gel; Eluent: Acetonitril/Wasser (Gradient); Fluß: 50 ml/min; Temp.: 25°C; Detektion UV 210 nm.

**Methode 1 (HPLC):** Säule: Kromasil C18, L-R Temperatur: 30°C; Fluss: 0.75 ml/min; Eluent A: 0.01 M HClO₄, Eluent B: Acetonitril, Gradient: → 0.5 min 98%A → 4.5 min 10%A → 6.5 min 10%A.

**Methode 2 (HPLC):** Säule: Kromasil C18 60*2 mm, L-R Temperatur: 30°C; Fluss: 0.75 ml/min, Eluent A: 0.01 M H₃PO₄, Eluent B: Acetonitril, Gradient: → 0.5 min 90%A → 4.5 min 10%A → 6.5 min 10%A.

**Methode 3 (HPLC):** Säule: Kromasil C18 60*2 mm, L-R Temperatur: 30°C; Fluss: 0.75 ml/min; Eluent A: 0.005 M HClO₄, Eluent B: Acetonitril, Gradient: → 0.5 min 98%A → 4.5 min 10%A → 6.5 min 10%A.

**Methode 4 (HPLC):** Säule: Symmetry C18 2.1x150 mm; Säulenofen: 50°C; Fluss: 0.6 ml/min; Eluent A: 0.6 g 30%ige Salzsäure/ 1 Wasser, Eluent B: Acetonitril, Gradient: 0.0 min 90%A → 4.0 min 10%A → 9 min 10%A.

**Methode 5 (LC-MS):** Instrument Micromass Quattro LCZ; Säule Symmetry C18, 50 mm x 2.1 mm, 3.5 µm; Temperatur: 40°C; Fluss: 0.5 ml/min; Eluent A: Acetonitril + 0.1 % Ameisensäure, Eluent B: Wasser + 0.1% Ameisensäure, Gradient: 0.0 min 10%A → 4 min 90%A → 6 min 90%A

**Methode 6 (LC-MS):** Instrument Micromass Platform LCZ; Säule Symmetry C18, 50 mm x 2.1 mm, 3.5 µm; Temperatur: 40°C; Fluss: 0.5 ml/min; Eluent A: Acetonitril + 0.1% Ameisensäure, Eluent B: Wasser + 0.1% Ameisensäure, Gradient: 0.0 min 10%A → 4 min 90%A → 6 min 90%A.

**Methode 7 (LC-MS):** Instrument Micromass Quattro LCZ; Säule Symmetry C18, 50 mm x 2.1 mm, 3.5 µm; Temperatur: 40°C; Fluss: 0.5 ml/min; Eluent A: Acetonitril + 0.1% Ameisensäure, Eluent B: Wasser + 0.1% Ameisensäure, Gradient: 0.0 min 5%A → 1 min 5%A → 5 min 90%A → 6 min 90%A

**Methode 8 (HPLC):** Säule: 250*4 mm, Kromasil 100, C-18, 5 µm; Temperatur: 40°C; Fluss: 1 ml/min; Eluent: Acetonitril 15% und 0.2 %ige Perchlorsäure 85%; UV-Detektion: 210 nm.

**Methode 9 (LC-MS):** Instrument: Waters Alliance 2790 LC; Säule: Symmetry C18, 50 mm x 2.1 mm, 3.5 µm; Eluent A: Wasser + 0.1% Ameisensäure, Eluent B: Acetonitril + 0.1% Ameisensäure; Gradient: 0.0 min 5%B → 5.0 min 10%B → 6.0 min 10%B; Temperatur: 50°C; Fluss: 1.0 ml/min; UV-Detektion: 210 nm.

**Methode 10 (LC-MS):** ZMD Waters; Säule: Inertsil ODS3 50 mm x 2.1 mm, 3 µm; Temperatur: 40°C; Fluss: 0.5 ml/min; Eluent A: Wasser + 0.05 % Ameisensäure, Eluent B: Acetonitril + 0.05 % Ameisensäure, Gradient: 0.0 min 5%B → 12 min → 100 %B → 15 min 100%B.

**Methode 11 (LC-MS):** MAT 900, Finnigan MAT, Bremen; Säule: X-terra 50mm x 2.1 mm, 2.5 µm; Temperatur: 25°C; Fluss: 0.5 ml/min; Eluent A: Wasser + 0.01 % Ameisensäure, Eluent B: Acetonitril + 0.01 % Ameisensäure, Gradient: 0.0 min 10 %B → 15 min → 90 %B → 30 min 90%B.

**Methode 12 (LC-MS):** TSQ 7000, Finnigan MAT, Bremen; Säule: Inertsil ODS3 50 mm x 2.1 mm, 3 µm; Temperatur: 25°C; Fluss: 0.5 ml/min; Eluent A: Wasser + 0.05 % Ameisensäure, Eluent B: Acetonitril + 0.05 % Ameisensäure, Gradient: 0.0 min 15%B → 15 min → 100%B → 30 min 100%B.

**Methode 13 (LC-MS):** 7 Tesla Apex II mit externer Elektrospray-Ionenquelle, Bruker Daltronics; Säule: X-terra C18 50 mm x 2.1 mm, 2.5 µm; Temperatur: 25°C; Fluss: 0.5 ml/min; Eluent A: Wasser + 0.1 % Ameisensäure, Eluent B: Acetonitril + 0.1 % Ameisensäure, Gradient: 0.0 min 5%B → 13 min → 100%B → 15 min 100%B.

**Methode 14 (HPLC):** Säule: X-Terra^{™} der Firma Waters, RP₈, 5 µm, 3.9×150 mm; Start: 95%A, 5%B; 12min: 5%A, 95%B. Eluent A: Wasser +0.01% Trifluoressigsäure; Eluent B: Acetonitril +0.01 % Trifluoressigsäure; Fluss: 1.2 ml/min.

**Methode 15 (LC-MS):** Gerätetyp MS: Micromass ZQ; Gerätetyp HPLC: Waters Alliance 2795; Säule: Merck Chromolith SpeedROD RP-18e 50x4.6mm; Eluent A: Wasser + 500 µl 50%ige Ameisensäure / 1; Eluent B: Acetonitril + 500 µl 50%ige Ameisensäure / 1; Gradient: 0.0 min 10%B→ 3.0 min 95%B→ 4.0 min 95%B; Ofen: 35 °C; Fluss: 0.0 min 1.0 ml/min→ 3.0 min 3.0 ml/min→ 4.0 min 3.0 ml/min; UV-Detektion: 210 nm.

**Methode 16 (LC-MS):** Gerätetyp MS: Micromass ZQ; Gerätetyp HPLC: Waters Alliance 2795; Säule: Merck Chromolith SpeedROD RP-18e 50x4.6mm; Eluent A: Wasser + 500 µl 50%ige Ameisensäure / 1; Eluent B: Acetonitril + 500 µl 50%ige Ameisensäure /1; Gradient: 0.0 min 10%B→ 2.0 min 95%B→ 4.0 min 95%B; Ofen: 35 °C; Fluss: 0.0 min 1.0 ml/min→ 2.0 min 3.0 ml/min→ 4.0 min 3.0 ml/min; UV-Detektion: 210 nm.

**Methode 17 (LC-MS):** Instrument: Micromass Platform LCZ mit HPLC Agilent Serie 1100; Säule: Grom-SIL120 ODS-4 HE, 50 mm x 2.0 mm, 3 µm; Eluent A: 1 1 Wasser + 1 ml 50%ige Ameisensäure, Eluent B: 1 1 Acetonitril + 1 ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 210 nm.

**Methode 18 (LC-MS):** Gerätetyp MS: Micromass ZQ; Gerätetyp HPLC: Waters Alliance 2795; Säule: Merck Chromolith SpeedROD RP-18e 50x4.6mm; Eluent A: Wasser + 500 µl 50%ige Ameisensäure / 1; Eluent B: Acetonitril + 500 µl 50%ige Ameisensäure / 1; Gradient: 0.0 min 10%B→ 3.0 min 95%B→ 4.0 min 95%B; Ofen: 35 °C; Fluss: 0.0 min 1.0 ml/min→ 3.0 min 3.0 ml/min→ 4.0 min 3.0 ml/min; UV-Detektion: 210 nm.

**Methode 19 (LC-MS):** Gerätetyp MS: Micromass ZQ; Gerätetyp HPLC: Waters Alliance 2790; Säule: Grom-Sil 120 ODS-4 HE 50 mm x 2 mm, 3.0 µm; Eluent B: Acetonitril + 0.05% Ameisensäure, Eluent A: Wasser + 0.05% Ameisensäure; Gradient: 0.0 min 5%B → 2.0 min 40%B → 4.5 min 90%B→ 5.5 min 90%B; Ofen: 45 °C; Fluss: 0.0 min 0.75 ml/min → 4.5 min 0.75 ml/min→ 5.5 min 1.25 ml/min; UV-Detektion: 210 nm.

**Methode 20 (LC-MS):** Gerätetyp MS: Micromass ZQ; Gerätetyp HPLC: Waters Alliance 2790; Säule: Uptisphere C 18, 50 mm x 2.0 mm, 3.0 µm; Eluent B: Acetonitril + 0.05% Ameisensäure, Eluent A: Wasser + 0.05% Ameisensäure; Gradient: 0.0 min 5%B → 2.0 min 40%B → 4.5 min 90%B→ 5.5 min 90%B; Ofen: 45 °C; Fluss: 0.0 min 0.75 ml/min → 4.5 min 0.75 ml/min→ 5.5 min 1.25 ml/min; UV-Detektion: 210 nm.

**Methode 21 (LC-MS):** Instrument: Micromass Quattro LCZ mit HPLC Agilent Serie 1100 ; Säule: UPTISPHERE HDO, 50 mm x 2.0 mm, 3 µm; Eluent A: 1 1 Wasser + 1 ml 50%ige Ameisensäure, Eluent B: 1 1 Acetonitril + 1 ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 208-400 nm.

**Methode 22 (LC-MS):** Gerätetyp MS: Micromass ZQ; Gerätetyp HPLC: HP 1100 Series; UV DAD; Säule: Grom-Sil 120 ODS-4 HE 50x2 mm, 3.0 µm; Eluent A: Wasser + 500 µl 50%ige Ameisensäure / 1, Eluent B: Acetonitril + 500 µl 50%ige Ameisensäure / 1; Gradient: 0.0 min 0%B → 2.9 min 70%B → 3.1 min 90%B → 4.5 min 90%B; Ofen: 50 °C; Fluss: 0.8 ml/min; UV-Detektion: 210 nm.

**Methode 23 (LC-MS):** Gerätetyp MS: Micromass ZQ; Gerätetyp HPLC: Waters Alliance 2795; Säule: Phenomenex Synergi 2µ Hydro- RP Mercury 20x4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A (Fluss: 1 ml/min) → 2.5 min 30%A (Fluss: 2 ml/min)→ 3.0 min 5%A (Fluss: 2 ml/min) → 4.5 min 5%A (Fluss: 2 ml/min); Ofen: 50°C; UV-Detektion: 210 nm.

**Methode 24 (LC-MS):** Gerätetyp MS: Micromass ZQ; Gerätetyp HPLC: HP 1100 Series; UV DAD; Säule: Phenomenex Synergi 2µ Hydro- RP Mercury 20x4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A (Fluss: 1 ml/min) → 2.5 min 30%A (Fluss: 2 ml/min) → 3.0 min 5%A (Fluss: 2 ml/min) → 4.5 min 5%A (Fluss: 2 ml/min); Ofen: 50°C; UV-Detektion: 210 nm.

**Methode 25 (LC-MS):** Gerätetyp MS: Micromass ZQ; Gerätetyp HPLC: HP 1100 Series; UV DAD; Säule: Grom-Sil 120 ODS-4 HE 50x2 mm, 3.0 µm; Eluent A: Wasser + 500 µl 50%ige Ameisensäure / 1, Eluent B: Acetonitril + 500 µl 50%ige Ameisensäure / 1; Gradient: 0.0 min 70%B → 4.5 min 90%B; Ofen: 50 °C, Fluss: 0.8 ml/min, UV-Detektion: 210 nm.

**Methode 26 (LC-MS):** Instrument: Micromass Quattro LCZ, mit HPLC Agilent Serie 1100; Säule: Grom-SIL120 ODS-4 HE, 50 mm x 2.0 mm, 3 µm; Eluent A: 1 1 Wasser + 1 ml 50%ige Ameisensäure, Eluent B: 1 1 Acetonitril + 1 ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 208-400 nm.

### Chemische Synthese der Beispiele

### Synthese der Ausgangsverbindungen:

Synthese von substituierten Phenylalaninderivaten am Beispiel von (-)-3-(2-Benzyloxy-5-iodophenyl)-2(*S*)-*tert*-butoxycarbonylamino-propionsäure [(-)-6A]

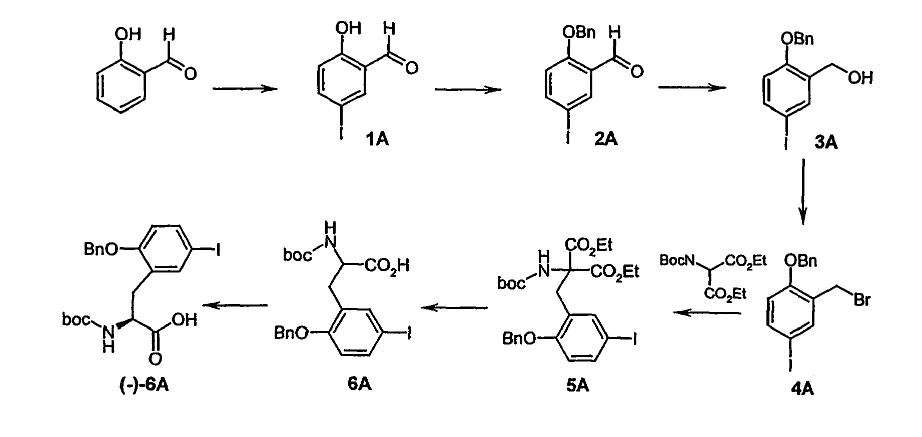

Synthese von geschützten Biphenyl-bisaminosäuren am Beispiel von 2(*S*)-Benzyloxycarbonylamino-3-[4,4'-bis-benzyloxy-3'-(2(*S*)-benzyloxycarbonyl-2(*S*)-*tert*-butoxycarbonyl-amino-ethyl)-biphenyl-3-yl]-propionsäure-2(*S*)-trimethylsilanyl-ethylester (12A)

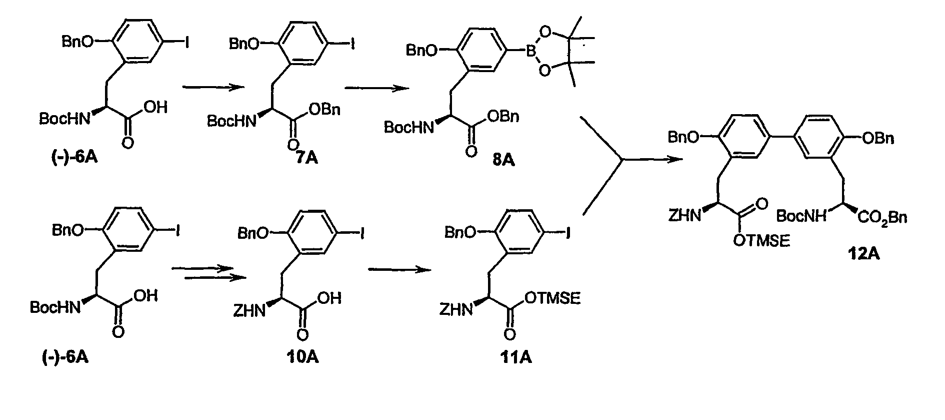

Synthese geschützter Hydroxyornithinderivate am Beispiel von 5-Benzyloxycarbonylamino-2(*S*)-*tert*-butoxycarbonylamino-4(*R*)-(*tert*-butyldimethylsilyloxy)-pentansäure (14A)

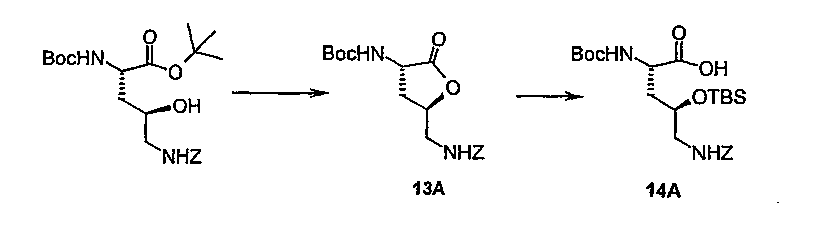

Synthese geschützter Biphenomycin-Derivate am Beispiel von (8*S*,11*S*,14*S*)-14-[(*tert*-Butoxycarbonyl)amino]-11-{(2R)-3-[(*tert*-butoxycarbonyl)amino]-2-hydroxypropyl}-5,17-dihydroxy-10,13-dioxo-9,12-diazatricyclo[14.3.1.1^{2,6}]-henicosa-1(20),2(21),3,5,16,18-hexaen-8-carbonsäure (21A)

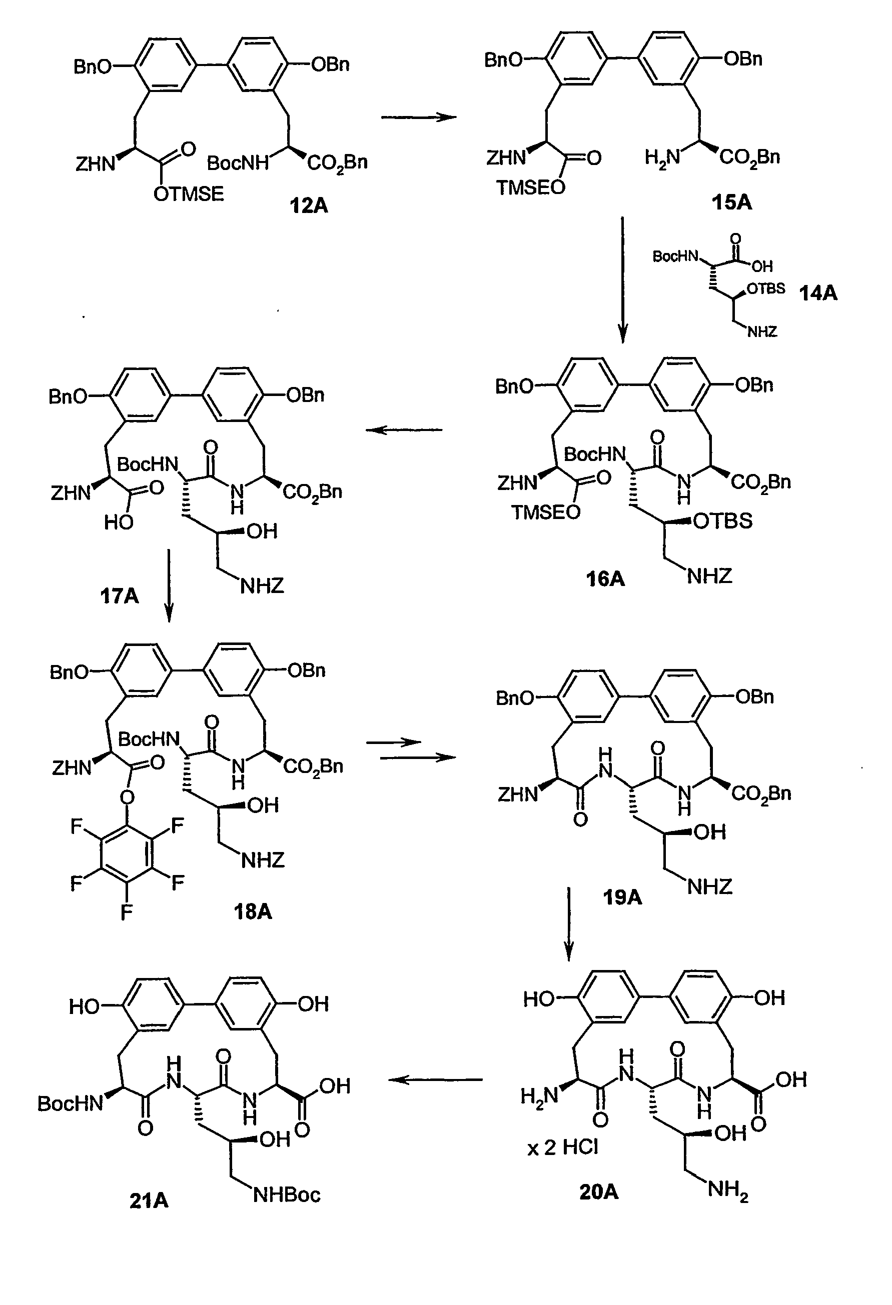

### Ausgangsverbindungen

### Beispiel 1A

### 2-Hydroxy-5-iod-benzaldehyd

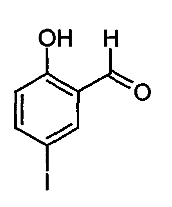

Zu einer Lösung von 188 g (1.54 mol) Salicylaldehyd in 1 1 wasserfreiem Dichlormethan in einem ausgeheizten Kolben wird eine Lösung von 250 g (1.54 mol) Iodchlorid in 600 ml wasserfreiem Dichlormethan unter Argon über 2 h zugetropft. Nach 3 Tagen Rühren bei RT wird eine gesättigte wässrige Natriumsulfit-Lösung unter kräftigem Rühren hinzugegeben. Die organische Phase wird abgetrennt, einmal mit Wasser und gesättigter wässriger Natriumchlorid-Lösung gewaschen und über Natriumsulfat getrocknet. Das Lösungsmittel wird eingedampft und der Rückstand aus Essigsäureethylester umkristallisiert. Man erhält 216 g (57% d. Th.) des Produktes.
LC-MS (ESI, Methode 10): m/z = 246 (M-H)⁻.
¹H-NMR (400 MHz, CDCl₃): δ = 6.7 (d, 1H), 7.77 (dd, 1H), 7.85 (d, 1H), 9.83 (s, 1H), 10.95 (s, 1H).

### Beispiel 2A

### 2-Benzyloxy-5-iodbenzaldehyd

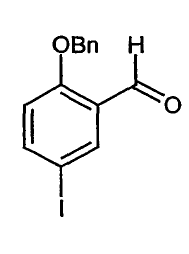

Zu einer Lösung von 100 g (0.40 mol) 2-Hydroxy-5-iodbenzaldehyd (Beispiel 1A) in 1.5 1 Dimethylformamid werden 67.2 g (0.48 mol) Kaliumcarbonat und nach wenigen Minuten 51 ml (0.44 mol) Benzylchlorid hinzugegeben. Das Reaktionsgemisch wird 24 h bei 120°C unter Rückfluss gerührt. Nach weiteren 24 h Rühren bei RT und Zugabe von 1.51 Wasser kristallisiert ein Feststoff aus. Der Niederschlag wird abgesaugt, zweimal mit Wasser gewaschen und im Vakuum getrocknet. Der Feststoff wird aus 230 ml Ethanol umkristallisiert. Man erhält 122.9 g (90% d. Th.) des Produktes.
LC-MS (ESI, Methode 10): m/z = 338 (M+H)⁺.
¹H-NMR (400 MHz, CDCl₃): δ = 5.18 (s, 2H), 6.84 (d, 1H), 7.33-7.45 (m, 5H), 7.78 (dd, 1H), 8.12 (d, 1H), 10.4 (s, 1H).

### Beispiel 3A

### (2-Benzyloxy-5-iod-phenyl)-methanol

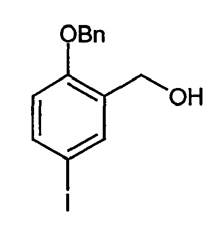

Zu einer auf 0°C gekühlten Lösung von 33.98 g (100.5 mmol) 2-Benzyloxy-5-iodbenzaldehyd (Beispiel 2A) in 200 ml Dichlormethan werden 100 ml einer 1 M Diisobutylaluminiumhydrid-Lösung in Dichlormethan zugegeben. Nach 2 h Rühren bei 0°C wird unter Kühlung eine gesättigte Kaliumnatriumtartrat-Lösung hinzugegeben (stark exotherme Reaktion) und das Reaktionsgemisch 2 h weiter gerührt. Nach Abtrennung der Phasen wird die organische Phase zweimal mit Wasser und einmal mit gesättigter wässriger Natriumchlorid-Lösung gewaschen und über Natriumsulfat getrocknet. Das Lösungsmittel wird im Vakuum abgedampft. Man erhält 31.8 g (93% d. Th.) des Produktes.
¹H-NMR (400 MHz, CDCl₃): δ = 2.17 (t, 1H), 4.68 (d, 2H), 5.1 (s, 2H), 6.72 (d, 1H), 7.32-7.42 (m, 5H), 7.54 (dd, 1H), 7.63 (d, 1H).

### Beispiel 4A

### 1-Benzyloxy-2-brommethyl-4-iodbenzol

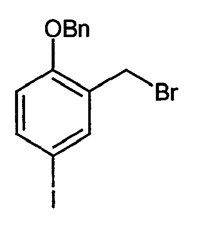

Zu einer Lösung von 35 g (103 mmol) (2-Benzyloxy-5-iod-phenyl)-methanol (Beispiel 3A) in 350 ml Toluol werden bei 40°C 3.3 ml (35 mmol) Phosphortribromid hinzugetropft. Innerhalb von 15 min wird die Temperatur des Reaktionsgemisches auf 100°C erhöht und weitere 10 min bei dieser Temperatur gerührt. Nach Abkühlung werden die beiden Phasen getrennt. Die organische Phase wird zweimal mit destilliertem Wasser und einmal mit gesättigter wässriger Natriumchlorid-Lösung gewaschen. Die organische Phase wird über Natriumsulfat getrocknet und eingedampft. Die Ausbeute beträgt 41 g (99% d. Th.).
¹H-NMR (300 MHz, CDCl₃): δ = 4.45 (s, 2H), 5.06 (s, 2H), 7.30 (m, 8H).

### Beispiel 5A

### 2-(2-Benzyloxy-5-iod-benzyl)-2-tert-butoxycarbonylamino-malonsäurediethylester

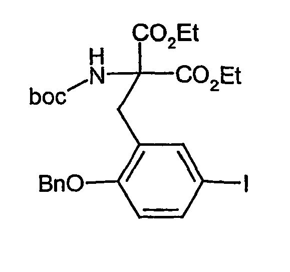

Zu einer Lösung von 28 g (101.7 mmol) 2-[*N*-(*tert*-Butoxycarbonyl)amino]malonsäure-diethylester und 7.9 ml (101.7 mmol) Natriumethylat in 300 ml Ethanol werden 41 g (101.7 mmol) von 1-Benzyloxy-2-brommethyl-4-iodbenzol (Beispiel 4A) hinzugegeben. Nach 3 h Rühren bei RT saugt man das ausgefallene Produkt ab. Nach Trocknung im Vakuum werden 55 g (90% d. Th.) Produkt isoliert.
1H-NMR (400 MHz, CDCl₃): δ = 1.12 (t, 6 H), 1.46 (s, 9H), 3.68 (s, 2H), 3.8-3.9 (m, 2H), 4.15-4.25 (m, 2H), 5.0 (s, 2H), 5.7 (s, 1H), 6.58 (d, 1H), 7.28-7.4 (m, 6H), 7.4 (dd, 1H).

### Beispiel 6A

### (+/-)-3-(2-Benzyloxy-5-iod-phenyl)-2-tert-butoxycarbonylamino-propionsäure

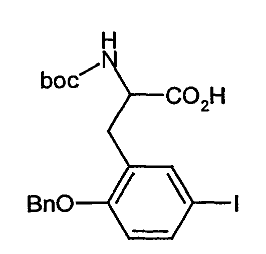

Zu einer Suspension von 58 g (97 mmol) 2-(2-Benzyloxy-5-iod-benzyl)-2-*tert-*butoxycarbonylamino-malonsäurediethylester (Beispiel 5A) in 800 ml eines Gemisches von Ethanol und Wasser (7:3) werden 400 ml 1 N Natronlauge hinzugegeben. Nach 3 h unter Rückfluss wird der pH-Wert der Reaktionsmischung nach Abkühlung auf Raumtemperatur mit konz. Salzsäure auf ca. pH 2 eingestellt. Die Reaktionsmischung wird eingedampft. Der Rückstand wird in MTBE und Wasser aufgenommen. Die wässrige Phase wird dreimal mit MTBE extrahiert. Die vereinigten organischen Phasen werden über Natriumsulfat getrocknet, filtriert und eingeengt. Nach Trocknung im Vakuum erhält man 47 g (97% d. Th.) des Produkts.
¹H-NMR (400 MHz, DMSO): δ = 1.32 (s, 9H), 2.68 (dd, 1H), 3.18 (dd, 1H), 4.25 (m, 1H), 5.15 (s, 2H), 6.88 (d, 1 H), 7.08 (d, 1H), 7.30-7.40 (m, 3 H), 7.45-7.55 (m, 3 H).

### Beispiel (-)-6A

### 3-(2-Benzyloxy-5-iod-phenyl)-2(S)-tert-butoxycarbonylamino-propionsäure

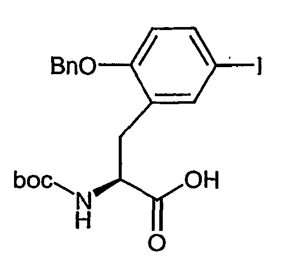

Das Racemat aus Beispiel 6A [(+/-)-3-(2-Benzyloxy-5-iod-phenyl)-2(*S*)-*tert*-butoxycarbonylamino-propionsäure] wird an einer chiralen stationären Kieselgelphase, basierend auf dem Selektor aus Poly(*N*-Methacryloyl-L-Leucin-dicyclopropylmethylamid), mit einem Gemisch aus *i*-Hexan/Ethylacetat als Elutionsmittel getrennt. Das zuerst eluierte Enantiomer (98.9% ee) ist in Dichlormethan rechtsdrehend ([α] _{D}²¹: + 3.0°, c = 0.54, Dichlormethan) und entspricht dem (R)-Enantiomer Beispiel (+)-6A, wie durch Einkristallröntgenstrukturanalyse bestimmt wurde. Die Reinheit des zweiten, linksdrehenden Enantiomers Beispiel (-)-6A, d.h. des (*S*)-Enantiomers, beträgt > 99% ee.

### Beispiel 7A

### 3-(2-Benzyloxy-5-iod-phenyl)-2(S)-tert-butoxycarbonylamino-propionsäure benzylester

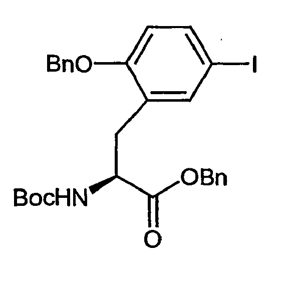

Unter Argon werden 10 g (20.11 mmol) (-)-3-(2-Benzyloxy-5-iod-phenyl)-2(*S*)-*tert-*butoxycarbonylamino-propionsäure [Beispiel (-)-6A] in 200 ml Acetonitril gelöst. Dazu werden 246 mg (2.01 mmol) 4-Dimethylaminopyridin und 4.16 ml (40.22 mmol) Benzylalkohol hinzugefügt. Die Mischung wird auf -10°C abgekühlt und mit 4.63 g (24.13 mmol) EDC versetzt. Man lässt alles langsam auf RT kommen und rührt über Nacht. Nach ca. 16 h wird das Gemisch im Vakuum einrotiert und der Rückstand säulenchromatographisch an Silicagel (Laufmittel: Dichlormethan) gereinigt. Ausbeute: 10.65 g (88% d. Th.).
HPLC (Methode 3): Rₜ = 6.03 min; LC-MS (Methode 9): Rₜ = 4.70 min
MS (DCI): m/z = 605 (M+NH₄)⁺.
¹H-NMR (200 MHz, CDCl₃): δ = 1.38 (s, 9H), 2.97 (dd, 1H), 3.12 (dd, 1H), 4.50-4.70 (m, 1H), 5.00-5.10 (m, 4H), 5.22 (d, 1H), 6.64 (d, 1H), 7.28-7.36 (m, 7H), 7.37-7.52 (m, 5H).

### Beispiel 8A

### 3-[2-Benzyloxy-5-(4,4,5,5-tetramethyl-[1,3,2]dioxaborolan-2-yl)-phenyl]-2(S)-tert-butoxycarbonylamino-propionsäurebenzylester

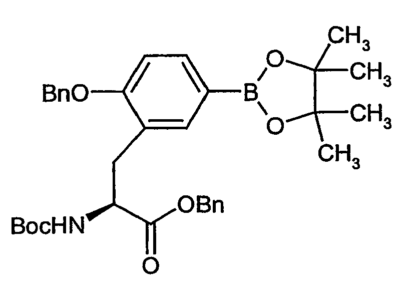

Zu einer Lösung von 10.30 g (17.53 mol) 3-(2-Benzyloxy-5-iod-phenyl)-2(*S*)-*tert-*butoxycarbonylamino-propionsäurebenzylester (Beispiel 7A) in 70 ml DMSO werden 5.15 g (52.60 mmol) Kaliumacetat zugegeben. Die Mischung wird deoxygeniert, indem durch die kräftig gerührte Lösung 15 min lang Argon durchgeleitet wird. Dann werden 5.17 g (20.16 mmol) Bis(pinacolato)diboran und 515 mg (0.70 mmol) Bis(diphenylphosphino)ferrocenpalladium(II)chlorid zugegeben. Unter leichtem Argonstrom wird nun auf 80°C erhitzt und nach 6 h wieder abgekühlt. Die Mischung wird säulenchromatographisch an Silicagel (Laufmittel: Dichlormethan) gereinigt. Vorhandene Reste an DMSO werden per Kugelrohrdestillation abgetrennt.
Der Rückstand wird erneut säulenchromatographisch an Silicagel (Laufmittel: Cyclohexan:Ethylacetat 4:1) gereinigt.
Ausbeute: 8.15 g (79% d. Th.).
HPLC (Methode 3): Rₜ = 6.26 min.
LC-MS (Methode 6): Rₜ = 5.93 und 6.09 min.
MS (EI): m/z = 588 (M+H)⁺.
¹H-NMR (200 MHz, CDCl₃): δ = 1.26 (s, 6H), 1.33 (s, 9H), 1.36 (s, 6H), 2.91-3.10 (m, 1H), 3.12-3.28 (m, 1H), 4.49-4.68 (m, 1H), 5.05 (dd, 2H), 5.11 (dd, 2H), 5.30 (d, 1H), 6.90 (d, 1H), 7.27-7.37 (m, 7H), 7.38-7.42 (m, 3H), 7.55-7.62 (m, 1H), 7.67 (dd, 1H).

### Beispiel 9A

### 2(S)-Amino-3-(2-benzyloxy-5-iod-phenyl)-propionsäure Hydrochlorid

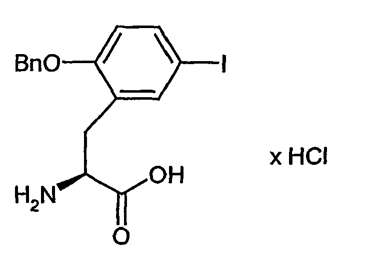

12 g (24.13 mmol) 3-(2-Benzyloxy-5-iod-phenyl)-2(*S*)-*tert*-butoxycarbonylaminopropionsäure [Beispiel (-)-6A] werden unter Argon in 60 ml 4 M Salzsäure-Lösung in Dioxan gegeben und 2 h bei RT gerührt. Die Reaktionslösung wird eingeengt und im Hochvakuum getrocknet.
Ausbeute: 10.47 g (100 % d. Th.).
HPLC (Methode 3): Rₜ = 4.10 min.
MS (EI): m/z = 398 (M+H-HCl)⁺.
¹H-NMR (200 MHz, CDCl₃): δ = 3.17-3.31 (m, 1H), 3.33-3.47 (m, 1H), 4.22 (t, 1H), 5.13 (s, 2H), 6.69 (d, 1 H), 7.24-7.40 (m, 2H), 7.41-7.45 (m, 2H), 7.48 (d, 1H), 7.52 (d, 1H), 7.60 (d, 1H), 8.66 (br.s, 2H).

### Beispiel 10A

### 2(S)-Benzyloxycarbonylamino-3-(2-benzyloxy-5-iod-phenyl)-propionsäure

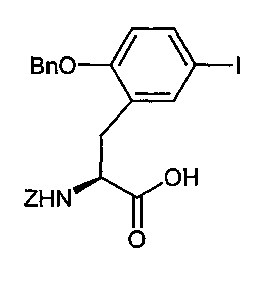

Eine Lösung aus 10.46 g (24.13 mmol) 2(*S*)-Amino-3-(2-benzyloxy-5-iodphenyl)-propionsäure Hydrochlorid (Beispiel 9A) in DMF wird mit 9.25 ml (53.09 mol) *N,N*-Diisopropylethylamin versetzt. Dazu gibt man 6.615 g (26.54 mmol) *N*-(Benzyloxycarbonyl)succinimid (Z-OSuc) zu. Die resultierende Lösung wird über Nacht gerührt und dann im Vakuum einrotiert. Der Rückstand wird in Dichlormethan aufgenommen und jeweils zweimal mit 0.1 N Salzsäurelösung und gesättigter wässriger Natriumchlorid-Lösung ausgeschüttelt. Die organische Phase wird getrocknet, filtriert und eingeengt. Die Mischung wird durch Säulenchromatographie an Silicagel (Laufmittel: Cyclohexan/Diethylether 9:1 bis 8:2) gereinigt.
Ausbeute: 8.30 g (65% d. Th.).
HPLC (Methode 3): Rₜ = 5.01 min.
MS (EI): m/z = 532 (M+H)⁺.
¹H-NMR (200 MHz, DMSO): δ = 3.14-3.3 (m, 2 H), 4.25-4.45 (m, 1H), 4.97 (s, 2H), 5.14 (s, 2H), 6.88 (d, 1 H), 7.20-7.56 (m, 12 H), 7.62 (d, 1 H), 12.73 (br.s, 1H).

### Beispiel 11A

### 2(S)-Benzyloxycarbonylamino-3-(2-benzyloxy-5-iod-phenyl)-propionsäure-(2-trimethylsilyl)-ethylester

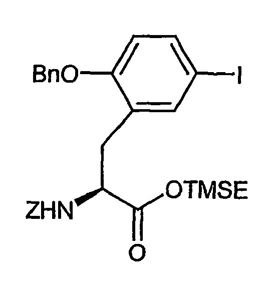

8.35 g (15.7 mmol) 2(*S*)-Benzyloxycarbonylamino-3-(2-benzyloxy-5-iod-phenyl)-propionsäure (Beispiel 10A) werden in 150 ml THF vorgelegt und mit 2.14 g (18.07 mmol) 2-Trimethylsilylethanol und 250 mg (2.04 mmol) 4-Dimethylaminopyridin versetzt. Die Mischung wird auf 0° abgekühlt und mit 2.38 g (2.95 ml, 18.86 mmol) *N*,*N*'-Diisopropylcarboddiimid, gelöst in 40 ml THF, versetzt. Es wird über Nacht bei RT gerührt und zur Aufarbeitung im Vakuum einrotiert. Der Rückstand wird in Dichlormethan aufgenommen und jeweils zweimal mit 0.1 N Salzsäurelösung und gesättigter wässriger Natriumchlorid-Lösung ausgeschüttelt. Die organische Phase wird getrocknet, filtriert und eingeengt. Die Mischung wird säulenchromatographisch (Silicagel, Laufmittel: Cyclohexan/Diethylether 9:1 bis 8:2) gereinigt.
Ausbeute: 8.2 g (83% d. Th.).
HPLC (Methode 3): Rₜ = 6.42 min
MS (EI): m/z = 532 (M+H)⁺.
¹H-NMR (300 MHz, CDCl₃): δ = 0.01 (s, 9H), 0.88 (t, 2H), 2.96 (dd, 1H), 3.13 (dd, 1H), 4.04-4.17 (m, 2H), 4.51-4.62 (m, 1H), 4.95-5.05 (m, 4H), 5.44 (d, 1H), 6.64 (d, 1H), 7.25-7.33 (m, 7 H), 7.37 (dd, 4H), 7.45 (dd, 1H).

### Beispiel 12A

### 2(S)-Benzyloxycarbonylamino-3-[4,4'-bis-benzyloxy-3'-(2(S)-benzyloxycarbonyl-2-tert-butoxycarbonylamino-ethyl)-biphenyl-3-yl]-propionsäure-2-(trimethylsilyl)-ethylester

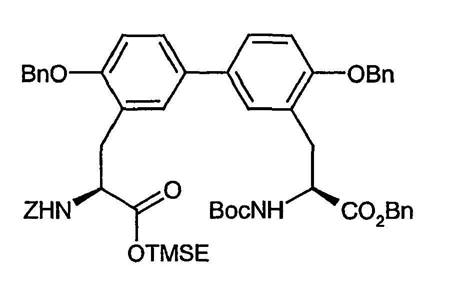

### Methode A:

Zu einer Lösung von 0.316 g (0.5 mmol) 2(*S*)-Benzyloxycarbonylamino-3-(2-benzyloxy-5-iod-phenyl)-propionsäure-(2-trimethylsilyl)-ethylester (Beispiel 11A) in 2.5 ml entgastem DMF werden unter Argon bei RT 45.8 mg (0.05 mmol) Bis(diphenylphosphino)ferrocen-palladium(II)chlorid (PdCl₂(dppf)) und 0.325 g (1.0 mmol) Cäsiumcarbonat hinzugegeben. Das Reaktionsgemisch wird auf 40°C erhitzt. Innerhalb von 30 min wird eine Lösung von 0.294 g (0.5 mmol) 3-[2-Benzyloxy-5-(4,4,5,5-tetramethyl-[1,3,2]dioxaborolan-2-yl)-phenyl]-2(*S*)-*tert*-butoxycarbonylamino-propionsäurebenzylester (Beispiel 8A) in 2.5 ml entgastem DMF zugetropft. Das Reaktionsgemisch wird 4 h bei 40°C und weitere 2 h bei 50°C gerührt. Das Lösungsmittel wird eingedampft und der Rückstand in Essigsäureethylester aufgenommen. Die organische Phase wird zweimal mit Wasser ausgeschüttelt, über Natriumsulfat getrocknet und eingeengt. Das Rohprodukt wird durch Kieselgelchromatographie mit Dichlormethan/Essigsäureethylester (30/1) gereinigt. Man erhält 0.320 g (66% d. Th.) des Produktes.

### Methode B:

Eine Lösung von 6.99 g (11.06 mmol) 2(*S*)-Benzyloxycarbonylamino-3-(2-benzyloxy-5-iod-phenyl)-propionsäure-(2-trimethylsilyl)-ethylester (Beispiel 11A) und 6.50 g (11.06 mmol) 3-[2-Benzyloxy-5-(4,4,5,5-tetramethyl-[1,3,2]dioxaborolan-2-yl)-phenyl]-2(*S*)-*tert*-butoxycarbonylamino-propionsäurebenzylester (Beispiel 8A) in 40 ml DMF wird entgast, indem Argon durchgeleitet wird (ca. 30 min). Anschließend gibt man 812 mg (1.11 mmol) Bis(diphenylphosphino)ferrocenpalladium(II)chlorid (PdCl₂(dppf)) und 7.21 g (22.13 mmol) Cäsiumcarbonat dazu. Das Reaktionsgemisch wird mit Argon leicht überströmt und für 2.5 h auf 80°C erhitzt. Die Mischung wird abgekühlt und säulenchromatographisch an Silicagel (Laufmittel: Cyclohexan/Ethylacetat 7:3) gereinigt. Vor der kompletten Einengung zur Trockne wird die Mischung mit Diisopropylether versetzt. Die entstandenen Kristalle werden abgesaugt und im Hochvakuum getrocknet.
Ausbeute: 6.54 g (61 % d. Th.).
HPLC (Methode 3): Rₜ = 7.65 min
MS (EI): m/z = 987 (M+Na), 965 (M+H)⁺.
¹H-NMR (200 MHz, CDCl₃): δ = 0.00 (s, 9H), 0.90 (t, 2H), 1.37 (s, 9H), 3.02-3.35 (m, 4H) 4.06-4.25 (m, 2H), 4.55-4.73 (m, 2H), 4.98-5.18 (m, 8H), 5.40 (d, 1H), 5.63 (d, 1H), 6.88-7.00 (m, 2H), 7.19-7.39 (m, 20H), 7.42-7.53 (m, 4H).

### Beispiel 13A

### N^{a}-(tert-Butoxycarbonyl)-N^{ε}(benzyloxycarbonyl)-(2S,4R)-hydroxyornithinlacton

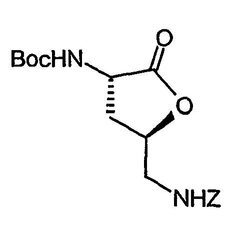

Eine Lösung von 7.60 g (17.3 mmol) 5-Benzyloxycarbonylamino-2(*S*)-*tert*-butoxycarbonylamino-4(*R*)-hydroxy-pentansäure-*tert*-butylester (Darstellung beschrieben in *Org. Lett*., 2001, 3, 20, 3153-3155) in 516 ml Dichlormethan und 516 ml Trifluoressigsäure wird 2 h bei RT gerührt. Das Lösungsmittel wird eingedampft.

Das zurückbleibende Rohprodukt wird in 2.6 l wasserfreiem Methanol gelöst und unter Rühren bei 0°C werden 6.3 g (28.8 mmol) Di-*tert*-Butyldicarbonat und 7.3 ml (52.43 mmol) Triethylamin hinzugegeben. Nach 15 h wird die Reaktionslösung eingedampft und der Rückstand in 1 l Essigsäureethylester aufgenommen. Nach Trennung der Phasen wird die organische Phase zweimal mit einer 5%-igen Zitronensäure-Lösung, zweimal mit Wasser und einmal mit gesättigter wässriger Natriumchlorid-Lösung ausgeschüttelt, über Natriumsulfat getrocknet und eingeengt. Das Rohprodukt wird durch Kieselgelchromatographie mit Toluol/Aceton (5/1) gereinigt. Man erhält 4.92 g (78% d. Th.) des Produktes.
LC-HR-FT-ICR-MS (Methode13): ber. für C₁₈H₂₈N₃O₆ (M+NH₄)⁺ 382.19726 gef. 382.19703.
¹H-NMR (400 MHz, CDCl₃): δ = 1.45 (s, 9H), 2.3-2.4 (m, 1H), 2.45-2.55 (m, 1H), 3.3-3.4 (m, 1H), 3.5-3.6 (m, 1H), 4.17-4.28 (m, 1H), 4.7-4.8 (m, 1H), 5.0-5.15 (m, 4H), 7.3-7.4 (m, 5H).

### Beispiel 14A

### 5-Benzyloxycarbonylamino-2(S)-tert-butoxycarbonylamino-4(R)-(tert-butyldimethyl-silanyloxy)-pentansäure

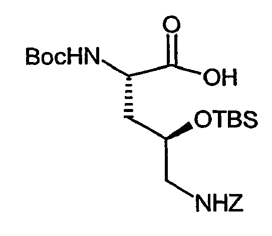

### Methode A:

Zu einer Lösung von 0.73 g (2 mmol) *N*^{a}-(*tert*-Butoxycarbonyl)-*N*^{ε}(benzyloxycarbonyl)-(2*S*,4*R*)-hydroxyornithinlacton (13A) in 50 ml 1,4-Dioxan werden bei 0°C 2 ml 1 M Natronlauge hinzugegeben. Die Reaktionslösung wird 2 h gerührt und dann eingedampft. Der Rückstand wird in 50 ml Dichlormethan aufgenommen. Zu dieser Lösung werden 1.12 ml (8 mmol) Triethylamin hinzugegeben und nach einer kurzen Zeit 1.38 ml (6 mmol) Trifluormethansulfonsäure-*tert*-butyl-dimethylsilylester zugetropft. Nach 3 h Rühren bei RT wird das Reaktionsgemisch mit Dichlormethan verdünnt. Die organische Phase wird mit 1 N Natriumbicarbonat-Lösung gewaschen, über Natriumsulfat getrocknet und eingedampft. Das Rohprodukt wird in 7.4 ml 1,4-Dioxan gelöst und mit 36.2 ml 0.1 N Natronlauge versetzt. Nach 3 h Rühren bei RT wird die Reaktionslösung eingedampft und der Rückstand in Wasser und Essigsäureethylester aufgenommen. Die organische Phase wird dreimal mit Essigsäureethylester extrahiert. Die vereinigten organischen Phasen werden über Natriumsulfat getrocknet und eingedampft. Man erhält 0.90 g (90% d. Th.) des Produktes.

### Methode B:

Eine Lösung von 14.0 g (38 mmol) 2(*S*)-tert-Butoxycarbonylamino-4(*R*)-hydroxy-5-nitro-pentansäure-benzylester in 840 ml Ethanol/Wasser 9/1 wird mit 1.96 g Palladium auf Kohle (10%ig) versetzt und unter Normaldruck 24 h bei RT hydriert. Es wird über Kieselgur filtriert, und das Filtrat wird mit 14.7 g (114 mmol) Diisopropylethylamin versetzt. Anschließend werden 11.4 g (45.6 mmol) N-(Benzyloxycarbonyloxy)-succinimid hinzugegeben, und es wird 4 h bei RT gerührt. Die Lösung wird eingeengt, der Rückstand in Dichlormethan aufgenommen und zweimal mit 0.1 N Salzsäure ausgeschüttelt. Die organische Phase wird abgetrennt und mit 14.7 g (114 mmol) Diisopropylamin alkalisch gestellt. Die Lösung wird auf 0°C gekühlt, mit 30.1 g (114 mmol) Trifluormethansulfonsäure-dimethyl-tert-butylsilylester versetzt und bei RT 2.5 h gerührt. Die organische Phase wird mit gesättigter Natriumhydrogencarbonat-Lösung gewaschen, über Natriumsulfat getrocknet und einrotiert. Der Rückstand wird in 50 ml Dioxan gelöst, mit 200 ml 0.1N Natronlauge versetzt und 3 h bei RT gerührt. Es wird mehrmals mit Essigsäureethylester extrahiert, die gesammelten organischen Phasen werden über Natriumsulfat getrocknet und im Vakuum eingeengt. Der Rückstand wird an Kieselgel chromatographiert (Laufmittel: Dichlormethan/Ethanol 20/1, 9/1). Man erhält 8.11 g (43% d. Th.) des Produkts.
MS (ESI): m/z = 497 (M+H)⁺.
¹H-NMR (300 MHz, d₆-DMSO): δ = 0.00 (s, 6H), 0.99 (s, 9H), 1.33 (s, 9H), 1.59 (m, 1H), 1.80 (m, 1H), 2.75-3.15 (m, 2H), 3.81 (m, 1H), 3.98 (m, 1H), 4.96 (m, 2H), 7.04 (d, 1H), 7.19 (m, 1H), 7.30 (m, 5H), 12.37 (br. s, 1H).

### Beispiel 15A

### 3-[3'-(2(S)-Amino-2-benzyloxycarbonyl-ethyl)-4,4'-bis-benzylozy-biphenyl-3-yl]-2(S)-benzyloxycarbonylamino-propionsäure-2-(trimethylsilyl)-ethylester Hydrochlorid

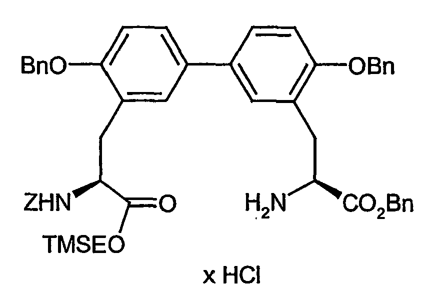

Zu einer auf 0°C gekühlten Lösung von 2.65 g (2.75 mmol) 2(*S*)-Benzyloxycarbonylamino-3-[4,4'-bis-benzyloxy-3'-(2(*S*)-benzyloxycarbonyl-2-*tert*-butoxycarbonylamino-ethyl)-biphenyl-3-yl]-propionsäure-2-(trimethylsilyl)-ethylester (Beispiel 12A) in 50 ml wasserfreiem Dioxan werden 50 ml einer 4 M Salzsäure-Dioxan-Lösung über ca. 20 min hinzugegeben. Nach 3 h Rühren wird die Reaktionslösung eingedampft und im Hochvakuum getrocknet.
Ausbeute: 100% d. Th.
HPLC (Methode 3): Rₜ = 5.96 min.
MS (EI): m/z = 865 (M+H)⁺.

### Beispiel 16A

### 2(S)-[5-Benzyloxycarbonylamino-2(S)-tert-butoxycarbonylamino-4(R)-(tert-butyldimethylsilyloxy)-pentanoylamino]-3-{4,4'-bis-benzyloxy-3'-[2(S)-benzyloxycarbonylamino-2-(2-trimethylsilyl-ethoxycarbonyl)-ethyl]-biphenyl-3-yl}-propionsäurebenzylester

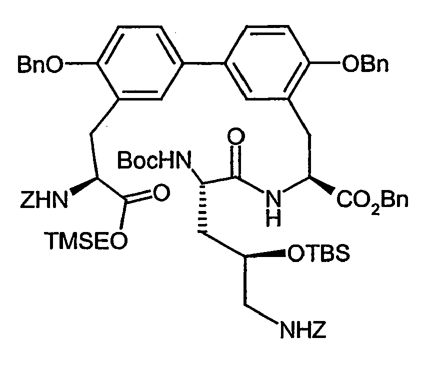

Zu einer auf 0°C gekühlten Lösung von 0.520 g (0.58 mmol) 3-[3'-(2(*S*)-Amino-2-benzyloxycarbonyl-ethyl)-4,4'-bis-benzyloxy-biphenyl-3-yl]-2(*S*)-benzyloxycarbonylamino-propionsäure-(2-trimethylsilyl)-ethylester Hydrochlorid (Beispiel 15A) und 0.287 g (0.58 mmol) 5-Benzyloxycarbonylamino-2(*S*)-*tert*-butoxycarbonylamino-4(*R*)-(*tert*-butyldimethylsilyloxy)-pentansäure (Beispiel 14A) in 7.3 ml wasserfreiem DMF werden 0.219 g (0.58 mmol) HATU und 0.082 g (0.63 mmol) *N,N*-Diisopropylethylamin hinzugegeben. Nach 30 min Rühren bei 0°C werden zusätzliche 0.164 g (1.26 mmol) *N,N*-Diisopropylethylamin hinzugegeben. Das Reaktionsgemisch wird 15 h bei RT gerührt. Das Lösungsmittel wird dann eingedampft und der Rückstand in Essigsäureethylester aufgenommen. Die organische Phase wird dreimal mit Wasser und einmal mit gesättigter wässriger Natriumchlorid-Lösung gewaschen, über Natriumsulfat getrocknet und eingeengt. Das Rohprodukt wird durch Kieselgelchromatographie mit Dichlormethan/Essigsäureethylester (Gradient 30/1→20/1→10/1) gereinigt. Man erhält 533 mg (66% d. Th.) des Produktes.
LC-MS (ESI, Methode 12): m/z =1342 (M+H)⁺, 1365 (M+Na)⁺.

### Beispiel 17A

### 2(S)-Benzyloxycarbonylamino-3-{4,4'-bis-benzyloxy-3'-[2(S)-benzyloxycarbonyl-2-(5-benzyloxycarbonylamino-2(S)-tert-butoxycarbonylamino-4(R)-hydroxy-pentanoylamino)-ethyl]-biphenyl-3-yl}-propionsäure

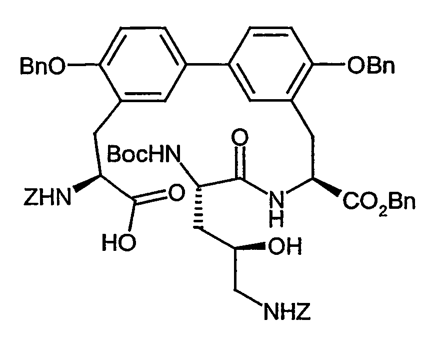

### Methode A:

Zu einer Lösung von 0.360 g (0.27 mmol) 2(*S*)-[5-Benzyloxycarbonylamino-2(*S*)-*tert*-butoxycarbonylamino-4(*R*)-(*tert*-butyldimethylsilyloxy)-pentanoylamino]-3-{4,4'-bis-benzyloxy-3'-[2(*S*)-benzyloxycarbonylamino-2-(2-trimethylsilyl-ethoxycarbonyl)-ethyl]-biphenyl-3-yl}-propionsäurebenzylester (Beispiel 16A) in 22.5 ml wasserfreiem DMF werden 0.80 ml einer 1.0 M Lösung von Tetrabutylammoniumfluorid in THF hinzugegeben. Nach 1 h Rühren bei RT wird das Reaktionsgemisch auf 0°C gekühlt und mit Wasser versetzt. Nach Zugabe von Essigsäureethylester werden die Phasen getrennt. Die organische Phase wird mit einer 1.0 M Lösung Kaliumhydrogensulfat gewaschen, über Natriumsulfat getrocknet und eingedampft. Man erhält 0.331 g des Rohproduktes. Das Rohprodukt wird ohne weitere Reinigung umgesetzt.
LC-MS (ESI, Methode 10): m/z = 1129 (M+H)⁺.
LC-HR-FT-ICR-MS: ber. für C₆₅H₆₉N₄O₁₄ (M+H)⁺ 1129.48048 gef. 1129.48123.

### Methode B:

Zu einer Lösung von 800 mg (0.6 mmol) 2(*S*)-[5-Benzyloxycarbonylamino-2(*S*)-*tert-*butoxycarbonylamino-4(*R*)-(*tert*-butyldimethylsilyloxy)-pentanoylamino]-3-{4,4'-bis-benzyloxy-3'-[2(*S*)-benzyloxycarbonylamino-2-(2-trimethylsilyl-ethoxycarbonyl)-ethyl]-biphenyl-3-yl}-propionsäurebenzylester (Beispiel 16A) in 26 ml absolutem DMF werden bei RT tropfenweise 1.8 ml 1N Tetrabutylammoniumfluorid in THF hinzugegeben. Nach 25 min bei RT wird auf 0°C gekühlt und mit viel Eiswasser versetzt. Es wird sofort mit Ethylacetat und etwas 1N Salzsäure-Lösung versetzt. Die organische Phase mit Magnesiumsulfat getrocknet, eingeengt und 1 h im Hochvakuum getrocknet. Das Rohprodukt wird ohne weitere Reinigung umgesetzt.

### Beispiel 18A

### 2(S)-(5-Benzyloxycarbonylamino-2(S)-tert-butoxycarbonylamino-4(R)-hydroxypentanoylamino)-3-[4,4'-bis-benzyloxy-3'-(2(S)-benzyloxycarbonylamino-2-pentafluorphenyloxycarbonyl-ethyl)-biphenyl-3-yl]-propionsäurebenzylester

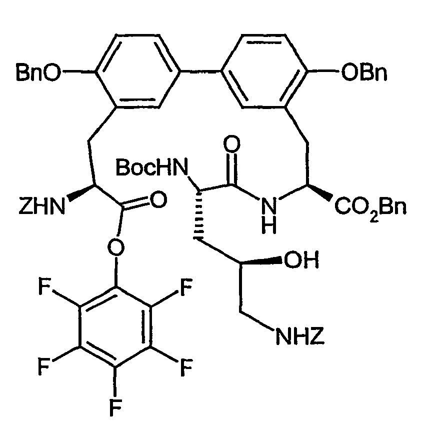

### Methode A:

Zu einer auf -25°C gekühlten Lösung von 104 mg (92 µmol) 2(*S*)-Benzyloxycarbonylamino-3-{4,4'-bis-benzyloxy-3'-[2(*S*)-benzyloxycarbonyl-2-(5-benzyloxycarbonylamino-2(*S*)-*tert*-butoxycarbonylamino-4(*R*)-hydroxy-pentanoylamino)-ethyl]-biphenyl-3-yl}-propionsäure (Beispiel 17A) in 3 ml Dichlormethan werden unter Argon 90 mg Pentafluorphenol (0.49 mmol), in wenig Dichlormethan gelöst, 1.1 mg 4-Dimethylaminopyridin (10 µM) und 19.4 mg (0.10 mmol) EDC hinzugegeben. Nach 15 h Rühren wird das Reaktionsgemisch eingeengt. Das Rohprodukt wird ohne weitere Reinigung umgesetzt.
LC-MS (ESI, Methode 11): m/z = 1317 (M+Na)⁺, 1295 (M+H)⁺.
LC-HR-FT-ICR-MS: ber. für C₇₁H₆₈F₅N₄O₁₄ (M+H)⁺ 1295.46467 gef. 1295.46430.

### Methode B:

691 mg (Rohgemisch, ca. 0.6 mmol) 2(*S*)-Benzyloxycarbonylamino-3-{4,4'-bis-benzyloxy-3'-[2(*S*)-benzyloxycarbonyl-2-(5-benzyloxycarbonylamino-2(*S*)-*tert-*butoxycarbonylamino-4(*R*)-hydroxy-pentanoylamino)-ethyl]-biphenyl-3-yl}-propionsäure (Beispiel 17A) werden in 25 ml Dichlormethan vorgelegt und mit 547.6 mg (2.98 mmol) Pentafluorphenol, gelöst in 6 ml Dichlormethan, versetzt. Man fügt 7.3 mg (0.06 mmol) DMAP hinzu und kühlt auf -25°C (Ethanol/Kohlendioxid-Bad). Bei -25°C werden 148 mg (0.774 mmol) EDC hinzugefügt. Die Mischung erwärmt sich über Nacht langsam auf RT. Die Reaktionsmischung wird im Vakuum eingeengt und im Hochvakuum kurz getrocknet. Das Rohprodukt wird ohne weitere Reinigung umgesetzt.

### Beispiel 19A

### 5,17-Bis-benzyloxy-14(S)-benzyloxycarbonylamino-11(S)-(3-benzyloxycarbonylamino-2(R)-hydroxy-propyl)-10,13-dioxo-9,12-diaza-tricyclo[14.3.1.1^{2,6}]-henicosa-1(19),2,4,6(21),16(20),17-hexaen-8(S)-carbonsäurebenzylester

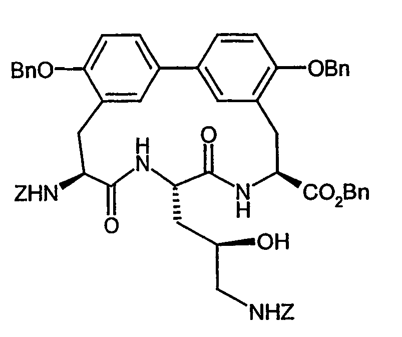

### Methode A:

Zu einer Lösung von 119.3 mg 2(*S*)-(5-Benzyloxycarbonylamino-2(*S*)-tert-butoxycarbonylamino-4(*R*)-hydroxy-pentanoylamino)-3-[4,4'-bis-benzyloxy-3'-(2(*S*)-benzyloxycarbonylamino-2-pentafluorphenyloxycarbonyl-ethyl)-biphenyl-3-yl]-propionsäurebenzylester (Beispiel 18A) in 2.7 ml 1,4-Dioxan werden 4 ml einer 4 M Salzsäure-Lösung in 1,4-Dioxan hinzugegeben. Bis zum Reaktionsende werden weitere 1.5 ml 4 M Salzsäure-Lösung in 1,4-Dioxan zugegeben. Die Reaktionslösung wird eingedampft und zweimal mit Chloroform codestilliert. Das Rohprodukt (LC-HR-FT-ICR-MS, Methode 13: ber. für C₆₆H₆₀F₅N₄O₁₂ (M+H)⁺ 1195.41224, gef. 1195.41419) wird in 100 ml Chloroform gelöst und über 3 h zu einer sehr gut gerührten Suspension von 200 ml Chloroform und 100 ml gesättigter wässriger Natriumhydrogencarbonat-Lösung hinzugetropft. Die Reaktionsmischung wird 2 h kräftig gerührt. Nach Trennung der zwei Phasen wird die wässrige Phase mit Chloroform extrahiert. Die vereinigten organischen Phasen werden mit 5%-iger wässriger Zitronensäure-Lösung gewaschen, über Magnesiumsulfat getrocknet und zur Trockne eingedampft. Das Rohprodukt wird mit Acetonitril gewaschen und im Hochvakuum getrocknet.
Ausbeute: 60.5 mg (65% d. Th.)
LC-MS (ESI, Methode11): m/z = 1011 (M+H)⁺.

### Methode B:

Circa 0.595 mmol 2(*S*)-(5-Benzyloxycarbonylamino-2(*S*)-tert-butoxycarbonylamino-4(*R*)-hydroxy-pentanoylamino)-3-[4,4'-bis-benzyloxy-3'-(2(*S*)-benzyloxycarbonyl-amino-2-pentafluorphenyloxycarbonyl-ethyl)-biphenyl-3-yl)-propionsäurebenzylester (Beispiel 18A) werden in 8 ml Dioxan gelöst und dann bei 0°C mit 16 ml 4 N Salzsäure-Lösung in Dioxan tropfenweise versetzt. Nach 45 min erfolgt erneute Zugabe von 6 ml 4 N Salzsäure-Lösung in Dioxan und nach 15 min nochmals 8 ml. Die Mischung wird 30 min bei 0°C gerührt, bevor die Reaktionslösung schonend eingeengt, mit Chloroform codestilliert (zweimal) und kurz im Hochvakuum getrocknet wird. Das Rohprodukt (732 mg, 0.59 mmol) wird in 1000 ml Chloroform gelöst und tropfenweise mit einer Lösung von 6 ml Triethylamin in 50 ml Chloroform versetzt. Es wird über Nacht bei RT gerührt. Zur Aufarbeitung wird das Gemisch schonend im Vakuum einrotiert und der Rückstand in Acetonitril verrührt. Die entstandenen Kristalle werden abgesaugt, mit Acetonitril gewaschen und im Hochvakuum getrocknet.
Ausbeute: 360 mg (60 % d. Th.).
MS (EI): m/z = 1011 (M+H)⁺.
HPLC (Methode 3): Rₜ = 5.59 min.
¹H-NMR (400 MHz, d₆-DMSO): δ = 1.52-1.65 (m, 1H), 1.73-1.84 (m, 1H), 2.82-3.01 (m, 3H), 3.02-3.11 (m, 1H), 3.46 (s, 1H), 3.57-3.68 (m, 1H), 4.47-4.56 (m, 1H), 4.64-4.71 (m, 1H), 4.73-4.85 (m, 2H), 4.88-5.00 (m, 4H), 5.09 (s, 2H), 5.14-5.20 (m, 4H), 6.29 (d, 1H), 7.00-7.11 (m, 4H), 7.21-7.40 (m, 20H), 7.41-7.48 (m, 9H), 8.77 (d, 1H), 8.87 (d, 1H).

### Beispiel 20A

### 14(S)-Amino-11(S)-(3-amino-2(R)-hydroxy-propyl)-5,17-dihydroxy-10,13-dioxo-9,12-diaza-tricyclo[14.3.1.1^{2,6}]henicosa-1(19),2,4,6(21),16(20),17-hexaen-8(S)-carbonsäure Dihydrochlorid

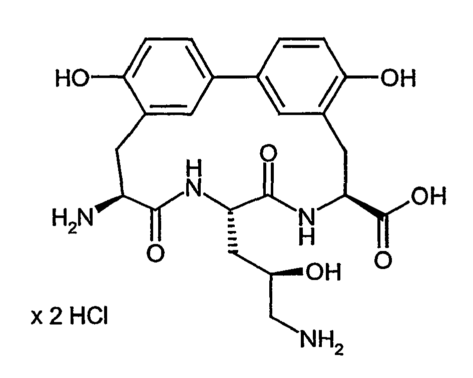

### Methode A:

Eine Lösung von 10 mg (9.9 µM) 5,17-Bis-benzyloxy-14(*S*)-benzyloxycarbonylamino-11(*S*)-(3-benzyloxycarbonylamino-2(*R*)-hydroxy-propyl)-10,13-dioxo-9,12-diaza-tricyclo[14.3.1.1^{2,6}]henicosa-1(19),2,4,6(21),16(20),17-hexaen-8(*S*)-carbonsäurebenzylester (Beispiel 19A) und 50 µl Ameisensäure in 10 ml Ethanol wird in Gegenwart von 10 mg Pd/C über 16 h unter Wasserstoff bei Normaldruck kräftig gerührt. Die Reaktionslösung wird eingedampft, der Rückstand in 1 N Salzsäure-Lösung aufgenommen und filtriert. Das Rohprodukt wird über eine RP 18 Kartusche mit Acetonitril/Wasser gereinigt. Man erhält 2 mg (42.8% d. Th.) des Produktes.

### Methode B:

Es werden 200 mg (0.20 mmol) 5,17-Bis-benzyloxy-14(*S*)-benzyloxycarbonylamino-11 (*S*)-(3-benzyloxycarbonylamino-2(*R*)-hydroxy-propyl)-10,13-dioxo-9,12-diaza-tri-cyclo[14.3.1.1^{2,6}]henicosa-1(19),2,4,6(21),16(20),17-hexaen-8(*S*)-carbonsäure-benzylester (Beispiel 19A) in einem Gemisch aus 220 ml Essigsäure/Wasser/Ethanol 4:1:1 gegeben (Ethanol kann durch THF substituiert werden). Dazu gibt man 73 mg 10 %ige Palladium/Kohle (10 % Pd/C) und hydriert anschließend 15 h bei Normaldruck. Das Reaktionsgemisch wird über vorgewaschenem Kieselgur filtriert und das Filtrat im Vakuum einrotiert. Der Rückstand wird mit 4.95 ml 0.1 N wässriger Salzsäure versetzt und eingeengt. Man verrührt den Rückstand mit 10 ml Diethylether und dekantiert ab. Der zurückgebliebene Feststoff wird im Hochvakuum getrocknet.
Ausbeute: 103 mg (95 % d. Th.).
HPLC (Methode 3): Rₜ = 3.04 min;
LC-MS (Methode 6): Rₜ = 0.38 min
MS (EI): m/z = 473 (M+H)⁺.
¹H-NMR (400 MHz, D₂O): δ = 2.06-2.20 (m, 1H), 2.74-2.89 (m, 1H), 2.94-3.05 (m, 1H), 3.12-3.25 (m, 2H), 3.53 (d, 1H), 3.61-3.72 (m, 1H), 3.97-4.07 (m, 1H), 4.53 (s, 1H), 4.61 (d, 1H), 4.76-4.91 (m, 12H), 7.01-7.05 (m, 2H), 7.07 (s, 1H), 7.40-7.45 (m, 2H), 7.51 (d, 1H).

### Beispiel 21A

### (8S,11S,14S)-14-[(Tert-butoxycarbonyl)amino]-11-{(2R)-3-[(tert-butoxycarbonyl)amino]-2-hydroxypropyl}-5,17-dihydroxy-10,13-dioxo-9,12-diazatricyclo[14.3.1.1^{2,6}]henicosa-1(20),2(21),3,5,16,18-hexaen-8-carbonsäure

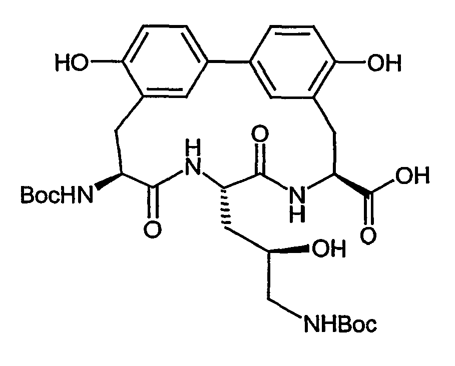

### Methode A:

5.2 mg (9.5 µmol) 14(*S*)-Amino-11(*S*)-(3-amino-2(*R*)-hydroxy-propyl)-5,17-dihydroxy-10,13-dioxo-9,12-diaza-tricyclo[14.3.1.1^{2,6}]henicosa-1(19),2,4,6(21),16(20),17-hexaen-8(*S*)-carbonsäure Dihydrochlorid (Beispiel 20A) werden unter Argon in trockenem Methanol (p.a., 0.5 ml) gelöst. Unter starkem Rühren werden bei Raumtemperatur zunächst eine wässrige Natriumhydrogencarbonat-Lösung (1 M, 100 µl) und dann eine methanolische Lösung von Di-*tert*.-butylcarbonat (0.1 M, 570 µl, 57 µmol) zugetropft. Nach etwa 1-2 Tagen ist vollständiger Umsatz erreicht. Das Reaktionsgemisch wird im Vakuum eingedampft und im Hochvakuum getrocknet. Das erhaltene Rohprodukt wird durch Gelchromatographie [Sephadex LH-20; Methanol/1 M Natriumhydrogencarbonat-Lösung (1:0.0001)] aufgereinigt. Man erhält 5.3 mg (83% d. Th.). Produkt.
HPLC/UV-Vis (Methode 14) Rₜ = 7.4 min.
λₘₐₓ (qualitativ) = ~193 nm(s), 206 (sh), 269 (m), ~284 (sh) (H₂O/Acetonitril + 0.01% TFA [4:6]).
LC-HR-FT-ICR-MS: ber. für C₃₃H₄₄N₄O₁₁ [M+H]⁺ 673.3079 gef. 673.3082.

### Methode B:

50 mg (0.09 mmol) 14(*S*)-Amino-11(*S*)-(3-amino-2(*R*)-hydroxy-propyl)-5,17-di-hydroxy-10,13-dioxo-9,12-diaza-tricyclo[14.3.1.1^{2,6}]henicosa-1(19),2,4,6(21),16(20),17-hexaen-8(*S*)-carbonsäure Dihydrochlorid (Beispiel 20A) werden in einem Gemisch von 8 ml Methanol/Wasser (9:1) vorgelegt. Dazu gibt man 1 ml 1 N Natriumhydrogencarbonatlösung und anschließend 80 mg (0.37 mmol) Di-*tert*-butyldicarbonat in 2 ml Methanol/Wasser (9:1). Es wird über Nacht bei RT gerührt. Die Lösung wird zur Aufarbeitung mit 60 ml Ethylacetat und 30 ml Wasser versetzt. Die organische Phase wird einmal mit 0.1 normaler Salzsäure gewaschen, getrocknet und im Vakuum einrotiert.
Ausbeute: 49 mg (79 % d. Th.).
LC-MS (Methode 9): Rₜ = 2.56 min.
MS (EI): m/z = 673 (M+H)⁺.

### Beispiel 22A

### tert-Butyl-(2R)-3-[(8S,11S,14S)-8-(aminocarbonyl)-14-[(tert-butoxyearbonyl)-amino]-5,17-dibydroxy-10,13-dioxo-9,12-diazatricyclo[14.3.1.1^{2,6}]henicosa-1(20),2(21),3,5,16,18-hexaen-11-yl]-2-hydroxypropylcarbamat

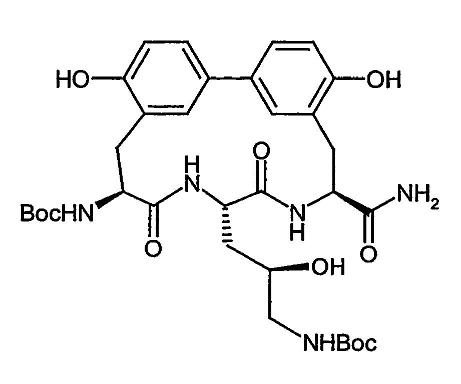

### Methode A:

4.1 mg (6.1 µmol) (8*S*,11*S*,14*S*)-14-[(*tert*-Butoxycarbonyl)amino]-11-{(2*R*)-3-[(*tert-*butoxycarbonyl)amino]-2-hydroxypropyl}-5,17-dihydroxy-10,13-dioxo-9,12-diazatricyclo[14.3.1.1^{2,6}]henicosa-1(20),2(21),3,5,16,18-hexaen-8-carbonsäure (Beispiel 21A) werden unter Argonschutzgasatmosphäre in trockenem *N,N*-Dimethylformamid (p.a., 0.5 ml) gelöst. Nach Zugabe von festem Natriumdisulfit (6.1 µmol) wird bei RT eine frisch zubereitete Lösung von Diisopropylethylamin (7.9 mg, 61 µmol), Ammoniumchlorid (1.6 mg, 30 µmol) und HATU (4.6 mg, 12.2 µmol) in Dimethylformamid (0.5 ml, Lösung A) zugetropft. Bis zum vollständigen Eduktumsatz muss Lösung A noch zweifach zugegeben werden (nach 1.5 h Reaktionszeit und nach 2 h Reaktionszeit). Man rührt weitere 20 min und bricht die Reaktion dann durch Zugabe von Wasser (0.5 ml) ab. Das Reaktionsgemisch wird eingefroren und anschließend gefriergetrocknet. Das erhaltene Rohprodukt wird durch Gelchromatographie [Sephadex LH-20; Methanol/Essigsäure (1:0.0001) dotiert mit Natriumdisulfit] aufgereinigt. Ausbeute: 2.2 mg ( 52% d. Th.).
HPLC-UV-Vis (Methode 14): Rₜ = 7.06 min.
λₘₐₓ (qualitativ) = ~202 nm (s), 268 (m), ~285 (sh). (H₂O/Acetonitril + 0.01% TFA [4:6]).
LC-HR-FT-ICR-MS (Methode 13): ber. für C₃₃H₄₆N₅O₁₀ [M+H]⁺ 672.3239 gef. 672.3239.

### Methode B:

Unter Argon werden 49 mg (0.07 mmol) (8*S*,11*S*,14*S*)-14-[(*Tert*-butoxycarbonyl)amino]-11-{(2*R*)-3-[(*tert*-butoxycarbonyl)amino]-2-hydroxypropyl}-5,17-dihydroxy-10,13-dioxo-9,12-diazatricyclo[14.3.1.1^{2,6}]henicosa-1(20),2(21),3,5,16,18-hexaen-8-carbonsäure (Beispiel 21A) in 1 ml DMF gelöst und auf 0°C abgekühlt. Dazu gibt man 42 mg (0.11 mmol) HATU hinzu und lässt 10 min bei 0°C nachrühren. 1.46 ml (0.73 mmol) einer 0.5 molaren Ammoniaklösung in Dioxan werden zugetropft und über Nacht bei RT gerührt. Nach ca. 18 h werden die gleichen Mengen an Reagenzien noch einmal dazu gegeben. Nach 3 Tagen wird das Gemisch im Vakuum einrotiert und mittels RP-HPLC präparativ gereinigt.
Ausbeute: 16 mg (33 % d. Th.).
HPLC (Methode 3): Rₜ = 3.83 min.

### Beispiel 23A

### tert-Butyl-(2R)-3-[(8S,11S,14S)-8-[(benzylamino)carbonyl]-14-[(tert-butoxycarbonyl)amino]-5,17-dihydroxy-10,13-dioxo-9,12-diazatricyclo[14.3.1.1^{2,6}]-henicosa-1(20),2(21),3,5,16,18-hexaen-11-yl]-2-hydroxypropylcarbamat

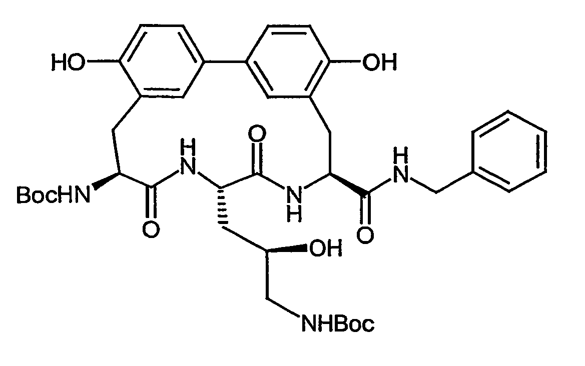

Zu einer auf 0°C gekühlten Lösung von 7 mg (0.01 mmol) ((8*S*,11*S*,14*S*)-14-[(*tert-*butoxycarbonyl)amino]-11-{(2*R*)-3-[(*tert*-butoxycarbonyl)amino]-2-hydroxypropyl}-5,17-dihydroxy-10,13-dioxo-9,12-diazatricyclo[14.3.1.1^{2,6}]henicosa-1(20),2(21),3,5,16,18-hexaen-8-carbonsäure (Beispiel 21A) in 0.5 ml absolutem DMF werden unter Argon 7.9 mg (0.021 mmol) HATU zugegeben. Nach 10 min bei 0°C werden 2.3 mg (0.021 mmol) Benzylamin zugeben, und es wird über Nacht bei RT gerührt. Die Reaktionsmischung wird im Vakuum eingeengt und der Rückstand mittels präparativer RP-HPLC getrennt.
Ausbeute: 1.5 mg (18.9 % d. Th.).
LC-MS (Methode 6): Rₜ = 4.4 min.
MS (ESI-pos): m/z = 785 (M+Na)⁺, 762 (M+H)⁺.

### Beispiel 24A

### tert-Butyl-(2R)-3-[(8S,11S,14S)-14-[(tert-butoxycarbonyl)amino]-5,17-dihydroxy-8-{[(2-hydroxyethyl)(methyl)amino]carbonyl}-10,13-dioxo-9,12-di-aza-tricyclo[14.3.1.1^{2,6}]henicosa-1(20),2(21),3,5,16,18-hexaen-11-yl]-2-hydroxypropylcarbamat

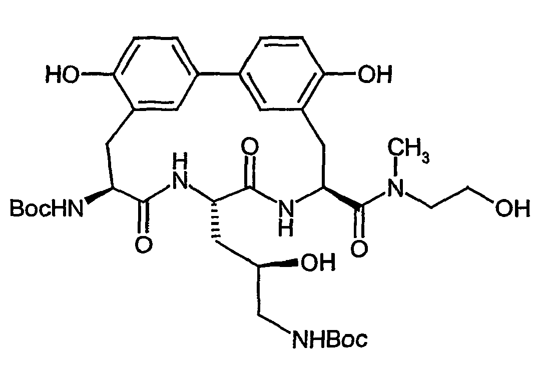

Unter Argon werden 15 mg (0.022 mmol) (8S,11S,14S)-14-[(tert-Butoxycarbonyl)amino]-11-{(2R)-3-[(tert-butoxycarbonyl)amino]-2-hydroxypropyl}-5,17-dihydroxy-10,13-dioxo-9,12-diazatricyclo[14.3.1.1^{2,6}]henicosa-1(20),2(21),3,5,16,18-hexaen-8-carbonsäure (Beispiel 21A) in 0.5 ml DMF gelöst und auf 0°C abgekühlt. Dazu gibt man 10.2 mg (0.027 mmol) HATU und 8.64 mg (0.067 mmol) N,N-Diisopropylethylamin und lässt 10 min bei 0°C nachrühren. 3.34 mg (0.045 mmol) 2-Methylaminoethanol werden hinzugegeben und über Nacht bei RT gerührt. Das Reaktionsgemisch wird eingeengt und mittels Gilson-HPLC gereinigt.
Ausbeute: 3.8 mg (23% d. Th.).
LC-MS (Methode 21): Rₜ= 3.90 min.

In Analogie zu Beispiel 24A können die in der folgenden Tabelle aufgeführten Beispiele 25A bis 32A hergestellt werden.

| **Beispiel-Nr.** | **Struktur** | **Analytische Daten** |
|---|---|---|
| **25A** | 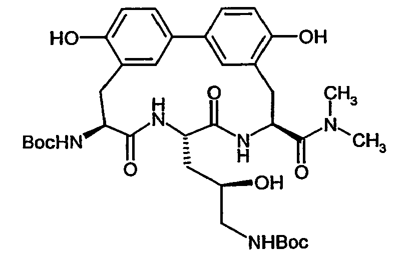 | HPLC (Methode 3): |
| | | Rₜ = 3.15 min. |
| **26A** | 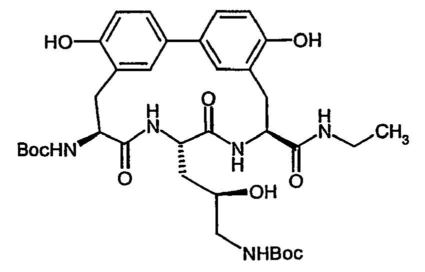 | HPLC (Methode 3): |
| | | Rₜ = 3.18 min. |
| **27A** | 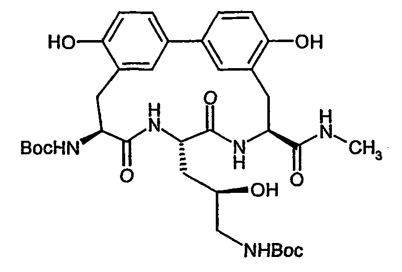 | HPLC (Methode 3): |
| | | Rₜ= 3.10 min. |
| **28A** | 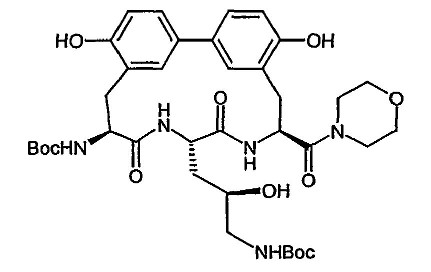 | LC-MS (Methode 21): |
| | | Rₜ = 3.97 min. |
| **29A** | 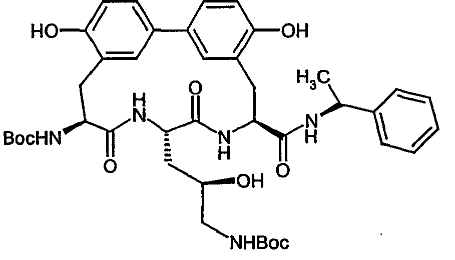 | HPLC (Methode 4): |
| | | Rₜ = 4.15 min. |
| **30A** | 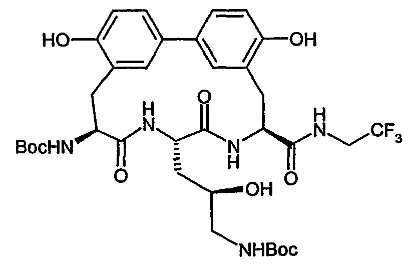 | HPLC (Methode 3): |
| | | Rₜ = 3.42 min. |
| **31A** | 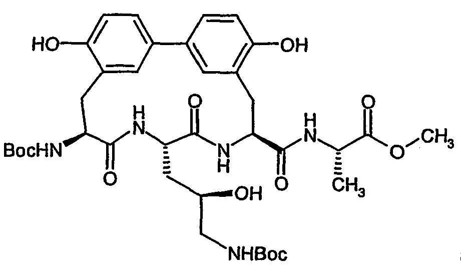 | LC-MS (Methode 15): |
| | | Rₜ = 2.18 min |
| | | MS (EI): m/z = 834 |
| | | (M+H)⁺ |
| **32A** | 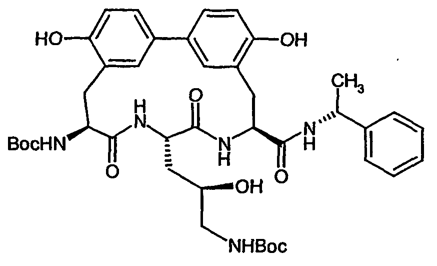 | HPLC (Methode 4): |
| | | Rₜ = 4.16 min. |

In Analogie zu Beispiel 24A können unter Verwendung von 2 Äquiv. HATU und 3 Äquiv. Amin die in der folgenden Tabelle aufgeführten Beispiele 33A und 34A hergestellt werden.

| **Beispiel-Nr.** | **Struktur** | **Analytische Daten** |
|---|---|---|
| **33A** | 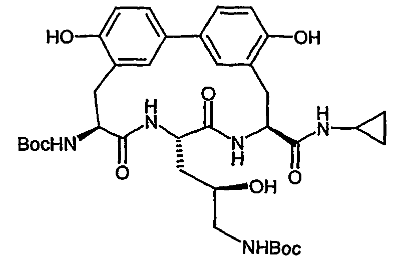 | HPLC (Methode 3): |
| | | Rₜ = 3.18 min. |
| **34A** | 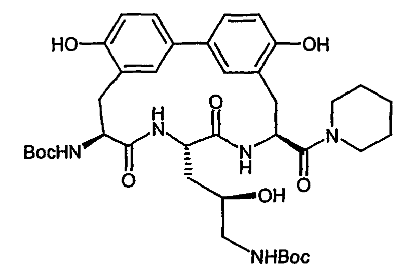 | HPLC (Methode 3): |
| | | Rₜ = 3.37 min. |

In Analogie zu Beispiel 24A können unter Verwendung von 2 Äquiv. HATU, 2 Äquiv. Amin und ohne Zugabe von DIPEA die in der folgenden Tabelle aufgeführten Beispiele 35A und 36A hergestellt werden.

| **Beispiel-Nr.** | **Struktur** | **Analytische Daten** |
|---|---|---|
| **35A** | 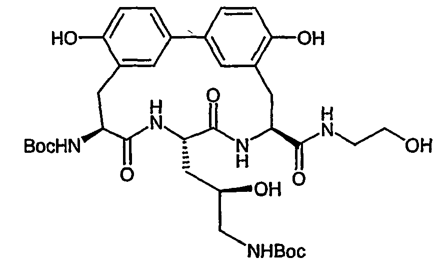 | HPLC (Methode 3): |
| | | Rₜ = 3.04 min |
| **36A** | 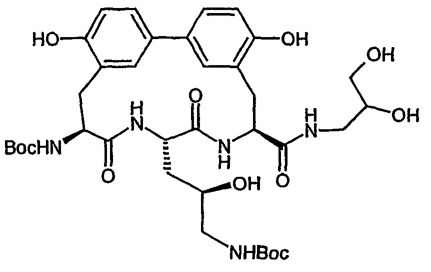 | HPLC (Methode 1): |
| | | Rₜ = 1.75 min. |

### Beispiel 37A

### Benzyl-2-(benzyloxy)-N-(tert-butoxycarbonyl)-5-iod-L-phenylalanyl-L-phenylalaninat

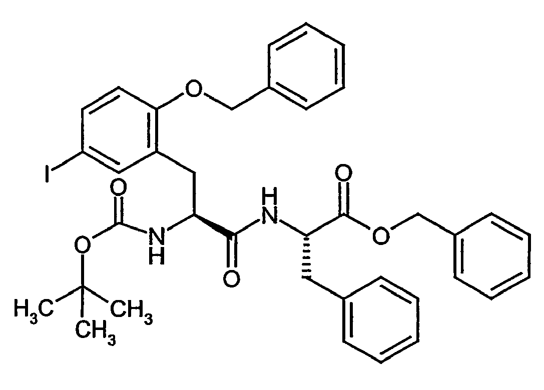

0.4 g (0.8 mmol) 2-(Benzyloxy)-N-(tert-butoxycarbonyl)-5-iod-L-phenylalanin (Beispiel 6A) und 0.282 g (0.970 mmol, 1.2 Äquiv.) L-Phenylalaninbenzylester Hydrochlorid werden unter Argon in 6 ml DMF vorgelegt und nacheinander mit 0.382 g (1.01 mmol, 1.25 Äquiv.) HATU und 0.49 ml (0.36 mg, 2.8 mmol, 3.5 Äquiv.) Diisopropylethylamin bei RT versetzt. Die Mischung wird 12 Stunden bei RT nachgerührt. Nach Versetzen mit 150 ml Wasser fällt das Produkt in Form weißer Kristalle aus. Die Kristalle werden abgesaugt, mit Wasser gewaschen und im Vakuum getrocknet.
Ausbeute: 0.669 g (quant.)
LC-MS (Methode 15): Rₜ = 3.11 min.
MS (EI): m/z = 735 (M+H)⁺

In Analogie zu Beispiel 37A können die in der folgenden Tabelle aufgeführten Beispiele 38A bis 41A hergestellt werden.

| **Beispiel-Nr.** | **Struktur** | **Analytische Daten** |
|---|---|---|
| **38A** | 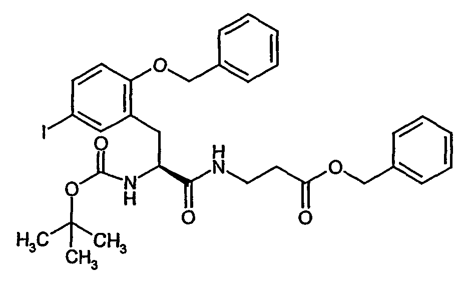 | LC-MS (Methode 15): |
| | | Rₜ = 2.86 min. |
| | | MS (EI): m/z = 659 |
| | | (M+H)⁺ |
| **39A** | 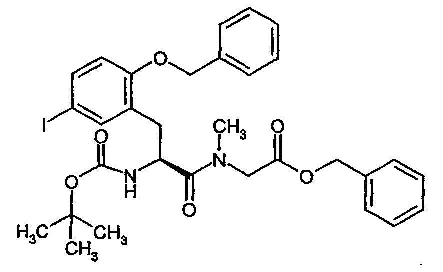 | LC-MS (Methode 15): |
| | | Rₜ = 2.96 min. |
| | | MS (EI): m/z = 659 |
| | | (M+H)⁺ |
| **40A** | 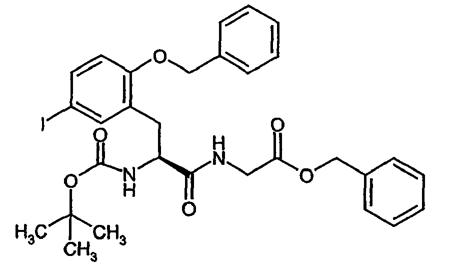 | LC-MS (Methode 15): |
| | | Rₜ = 2.85 min. |
| | | MS (EI): m/z = 644 |
| | | (M+H)⁺ |
| **41A** | 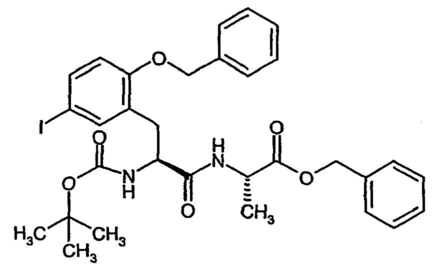 | LC-MS (Methode 15): |
| | | Rₜ = 2.93 min. |
| | | MS (EI): m/z = 659 |
| | | (M+H)⁺ |

### Beispiel 42A

### 2-(Trimethylsilyl)ethyl-2-(S)-benzyloxycarbonylamino-3-[3'[-2-[tert-butoxycarbonylamino(3-amino-[1-(S)-benzyloxy-1-oxo-2-phenyl-ethyl]-3-oxopropyl)]-4,4'-bis(benzyloxy)-1,1'-biphenyl-3-yl]]propanoat

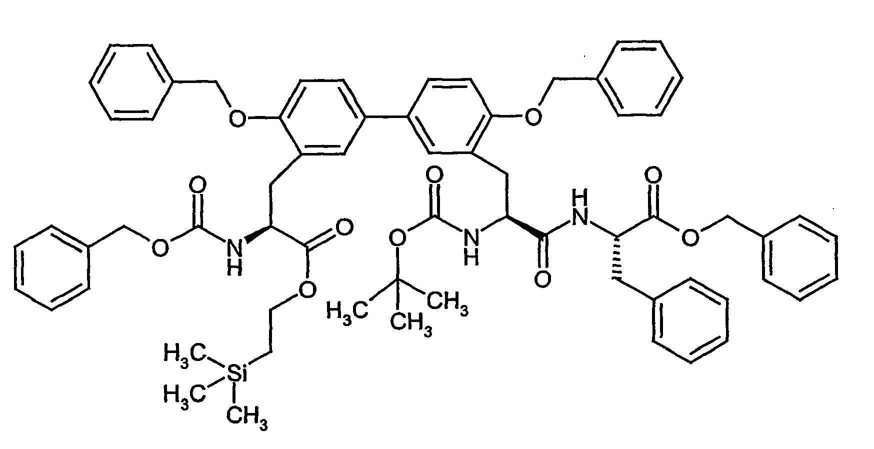

0.593 g (0.939 mmol) 2-(Trimethylsilyl)ethyl-2-(benzyloxy)-N-[(benzyloxy)-carbonyl]-5-(4,4,5,5-tetramethyl-1,3,2-dioxaborolan-2-yl)-L-phenylalaninat (Beispiel 84A) und 0.734 g (0.939 mmol) Benzyl-2-(benzyloxy)-N-(tert-butoxycarbonyl)-5-iod-L-phenylalanyl-L-phenylalaninat (Beispiel 37A) werden unter Argon in 6 ml DMSO gelöst. Die entstehende Lösung wird 30 min mit Argon gespült. Anschließend versetzt man mit 0.069 g (0.094 mmol, 0.1 Äquiv.) Bis(diphenylphosphino)ferrocen-palladium(II)chlorid und 0.612 g (1.88 mmol, 2.0 Äquiv.) Cäsiumcarbonat. Nach 10-minütigem Spülen mit Argon erhitzt man für 3 Tage bei 80°C, wobei weiterhin mit Argon gespült wird. Nach Abkühlen auf RT reinigt man die Rohlösung durch Chromatographie an Kieselgel (Cyclohexan/Essigsäureethylester 2:1). Anschließend werden die eingeengten produkthaltigen Fraktionen über eine präparative RP-HPLC gereinigt.
Ausbeute: 0.367 g (29 % d. Th.)
LC-MS (Methode 15): Rₜ = 3.50 min.

In Analogie zu Beispiel 42A können die in der folgenden Tabelle aufgeführten Beispiele 43A bis 46A hergestellt werden.

| **Beispiel-Nr.** | **Struktur** | **Analytische Daten** |
|---|---|---|
| **43A** | 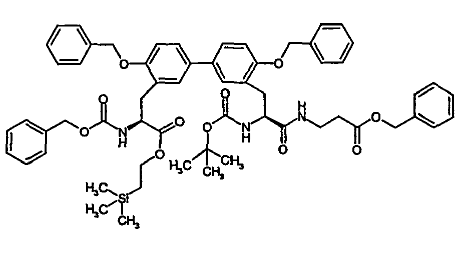 | LC-MS (Methode 15): |
| | | Rₜ = 3.39 min. |
| | | MS (EI): m/z = 1036 |
| | | (M+H)⁺ |
| **44A** | 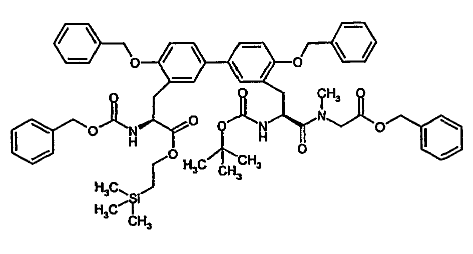 | LC-MS (Methode 15): |
| | | Rₜ = 3.42 min. |
| | | MS (EI): m/z = 1036 |
| | | (M+H)⁺ |
| **45A** | 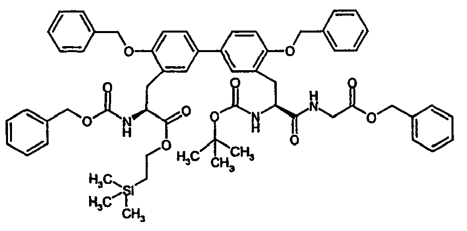 | LC-MS (Methode 15): |
| | | Rₜ = 3.38 min. |
| | | MS (EI): m/z = 1022 |
| | | (M+H)⁺ |
| **46A** | 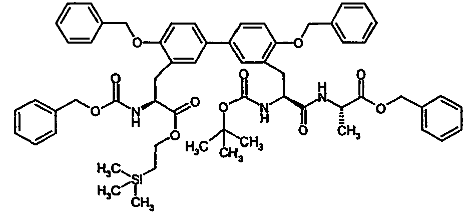 | LC-MS (Methode 15): |
| | | Rₜ = 3.40 min. |
| | | MS (EI): m/z = 1036 |
| | | (M+H)⁺ |

### Beispiel 47A

### 2-(Trimethylsilyl)ethyl-2-(S)-benzyloxycarbonylamino-3-[3'[-2-[amino(3-amino-[1-(S)-benzyloxy-1-oxo-2-phenyl-ethyl]-3-oxopropyl)]-4,4'-bis(benzyloxy)-1,1'-biphenyl-3-yl]]propanoat

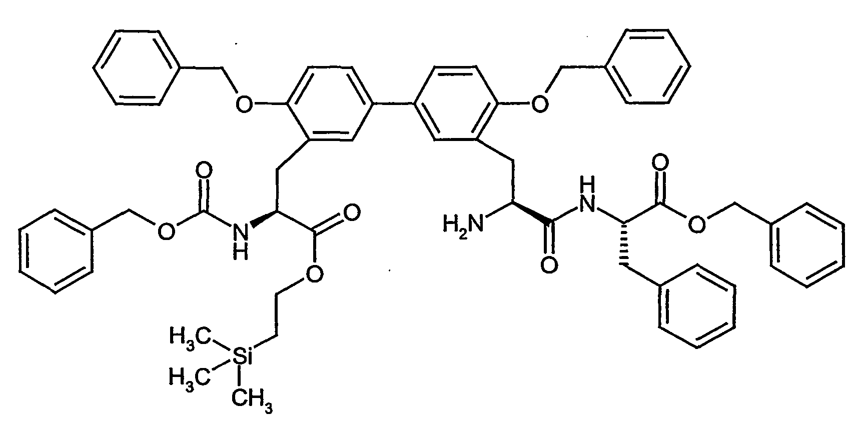

0.37 g (0.27 mmol) 2-(Trimethylsilyl)ethyl-2-(*S*)-benzyloxycarbonylamino-3-[3'[-2-[*tert*-butoxycarbonylamino(3-amino-[1-(*S*)-benzyloxy-1-oxo-2-phenyl-ethyl]-3-oxopropyl)]-4,4'-bis(benzyloxy)-1,1'-biphenyl-3-yl]]propanoat (Beispiel 42A) wird unter Argon in 10 ml einer 4 M Lösung von Chlorwasserstoff in Dioxan gelöst und 3 h bei RT gerührt. Man engt am Rotationsverdampfer ein und trocknet im Vakuum. Das Rohprodukt wird ohne weitere Charakterisierung weiter umgesetzt.

In Analogie zu Beispiel 47A können die in der folgenden Tabelle aufgeführten Beispiele 48A bis 51A hergestellt werden.

| **Beispiel-Nr.** | **Struktur** |
|---|---|
| **48A** | 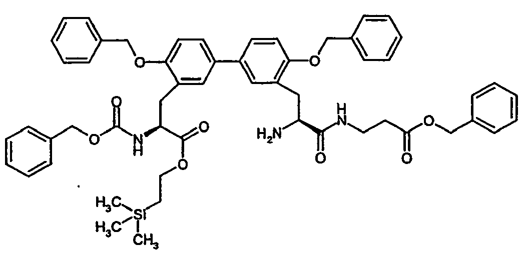 |
| **49A** | 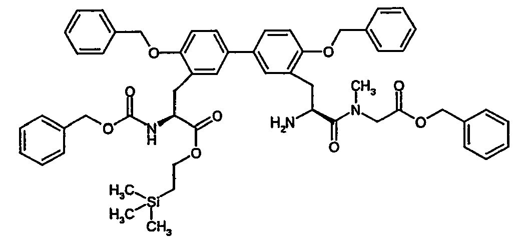 |
| **50A** | 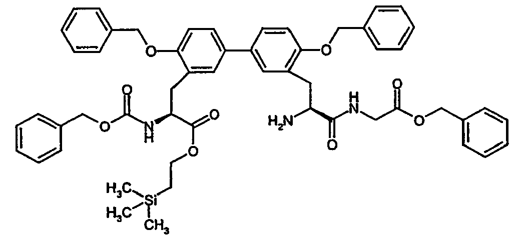 |
| **51A** | 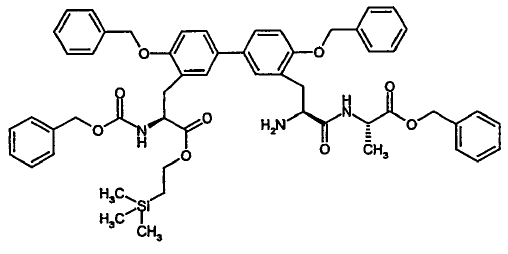 |

### Beispiel 52A

### 2-(Trimethylsilyl)ethyl-2-(S)-benzyloxycarbonylamino-3-[3'[-2-[5-benzyloxycarbonylamino-2(S)-tert-butoxycarbonylamino-4(R)-(tert-butyldimethylsilyloxy)-pentanoylamino(3-amino-[1-(S)-benzyloxy-1-oxo-2-phenyl-ethyl]-3-oxopropyl)]-4,4'-bis(benzyloxy)-1,1'-biphenyl-3-yl]]propanoat

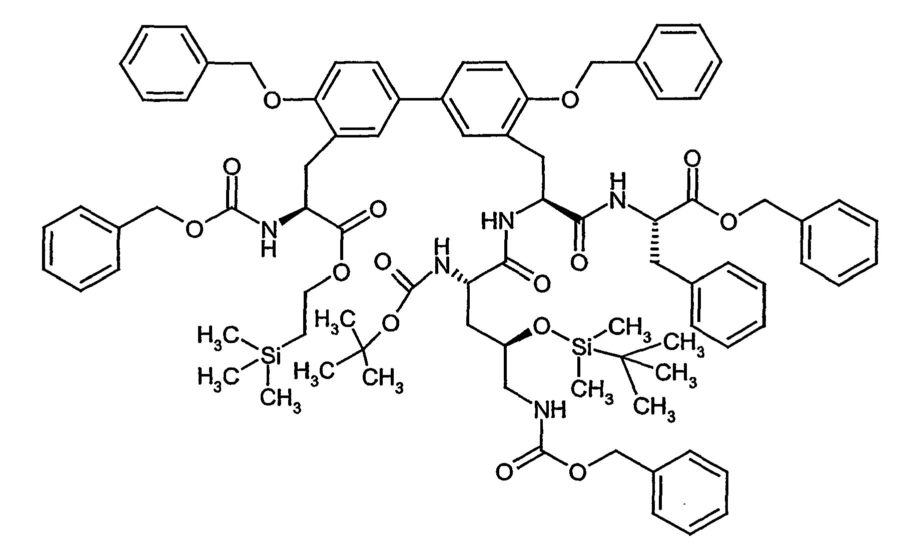

0.27 g (0.27 mmol) 2-(Trimethylsilyl)ethyl-2-(*S*)-benzyloxycarbonylamino-3-[3'[-2-[amino(3-amino-[1-(*S*)-benzyloxy-1-oxo-2-phenyl-ethyl]-3-oxopropyl)]-4,4'-bis(benzyloxy)-1,1'-biphenyl-3-yl]]propanoat (Beispiel 47A) und 0.16 g (0.32 mmol, 1.2 Äquiv.) 5-Benzyloxycarbonylamino-2(*S*)-*tert*-butoxycarbonylamino-4(*R*)-(*tert*-butyldimethylsilyloxy)-pentansäure werden unter Argon in 5 ml wasserfreiem DMF gelöst. Bei RT wird 0.13 g (0.34 mmol, 1.25 Äquiv.) HATU und 0.16 ml (0.12 g, 0.95 mmol, 3.5 Äquiv.) *N,N*-Diisopropylethylamin hinzugegeben. Das Reaktionsgemisch wird 12 h bei RT gerührt. Das Reaktionsgemisch wird direkt per präparativer RP-HPLC gereinigt und ohne weitere Charakterisierung umgesetzt. Ausbeute: 0.288 g (71 % d. Th.)

In Analogie zu Beispiel 52A können die in der folgenden Tabelle aufgeführten Beispiel 53A bis 56A hergestellt werden.

| **Beispiel-Nr.** | **Struktur** | **Analytische Daten** |
|---|---|---|
| **53A** | 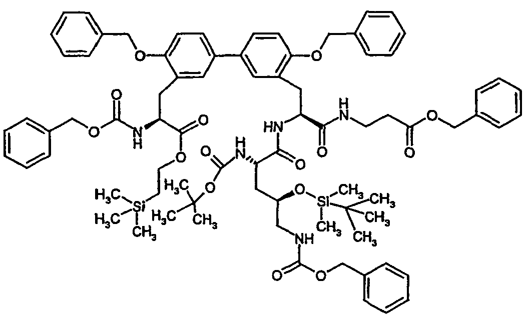 | LC-MS (Methode 15): |
| | | Rₜ = 3.84 min. |
| | | MS (EI): m/z = 1415 |
| | | (M+H)⁺ |
| **54A** | 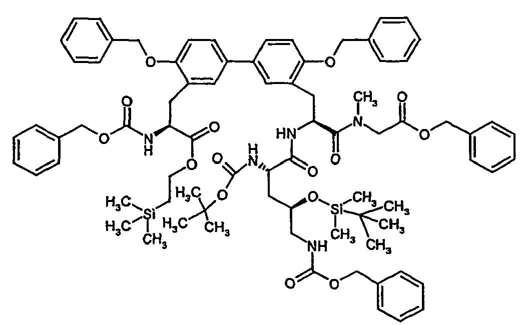 | LC-MS (Methode 15): |
| | | Rₜ = 3.92 min. |
| | | MS (EI): m/z = 1415 |
| | | (M+H)⁺ |
| **55A** | 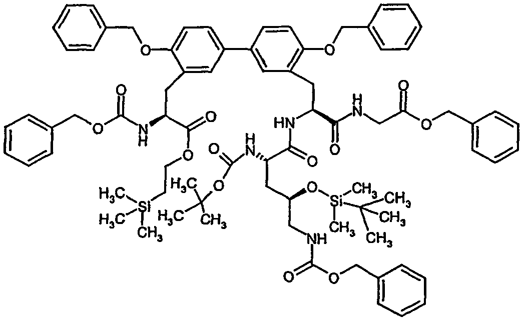 | LC/MS (Methode 15): |
| | | Rₜ = 3.97 min |
| | | MS (EI): m/z = 1401 |
| | | (M+H)⁺ |
| **56A** | 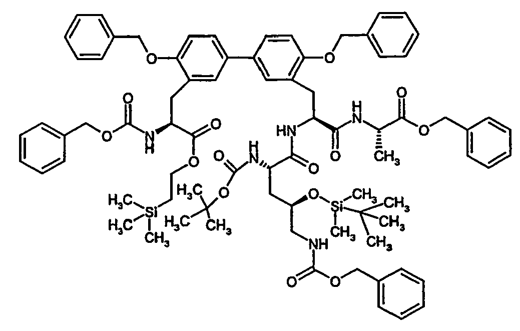 | LC-MS (Methode 16): |
| | | Rₜ = 2.98 min. |
| | | MS (EI): m/z = 1415 |
| | | (M+H)⁺ |

### Beispiel 57A

### 2-(S)-Benzyloxycarbonylamino-3-[3'[-2-[5-benzyloxycarbonylamino-2(S)-tert-butoxycarbonylamino-4(R)-(hydroxyoxy)-pentanoylamino(3-amino-[1-(S)-benzyloxy-1-oxo-2-phenyl-ethyl]-3-oxopropyl)]-4,4'-bis(benzyloxy)-1,1'-biphenyl-3-yl]]propionsäure

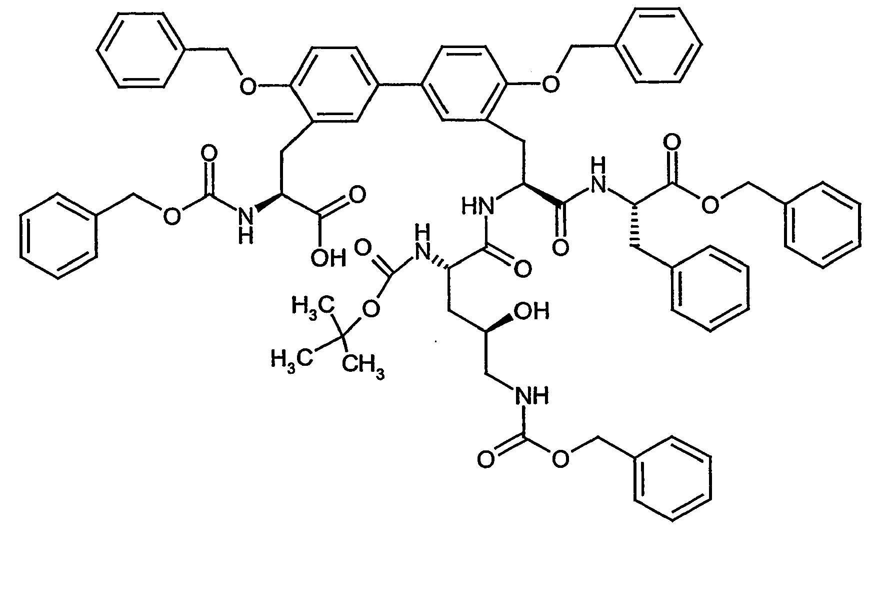

Zu einer Lösung von 0.29 g (0.19 mmol) 2-(Trimethylsilyl)ethyl-2-(*S*)-benzyloxycarbonylamino-3-[3'[-2-[5-benzyloxycarbonylamino-2(*S*)-*tert*-butoxycarbonylamino-4(*R*)-(*tert*-butyldimethylsilyloxy)-pentanoylamino(3-amino-[1-(*S*)-benzyloxy-1-oxo-2-phenyl-ethyl]-3-oxopropyl)]-4,4'-bis(benzyloxy)-1,1'-biphenyl-3-yl]]propanoat (Beispiel 52A) in 3 ml DMF werden 1.2 ml einer 1.0 M Lösung von Tetrabutylammoniumfluorid in THF (1.2 mmol, 6.3 Äquiv.) hinzugegeben. Nach 4 h Rühren bei RT wird das Reaktionsgemisch auf 0°C gekühlt und mit 50 ml Wasser versetzt. Nach Zugabe von 50 ml Essigsäureethylester und 1 ml 1 N wässriger Salzsäure werden die Phasen getrennt. Die wässrige Phase wird mehrfach mit Essigsäureethylester extrahiert. Nach Trocknen der organischen Phase über Magnesiumsulfat wird im Vakuum eingeengt und am Hochvakuum getrocknet. Das Rohprodukt wird ohne weitere Reinigung umgesetzt.

In Analogie zu Beispiel 57A können die in der folgenden Tabelle aufgeführten Beispiele 58A bis 61A hergestellt werden.

| **Beispiel-Nr.** | **Struktur** |
|---|---|
| **58A** | 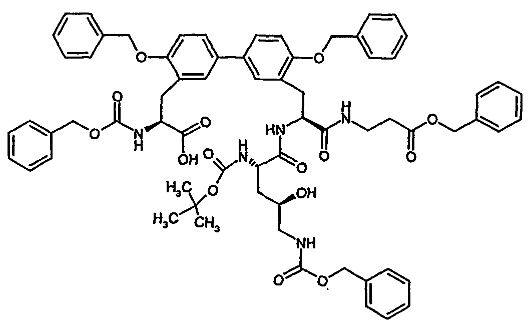 |
| **59A** | 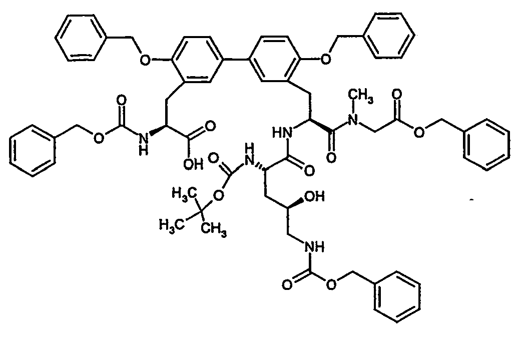 |
| **60A** | 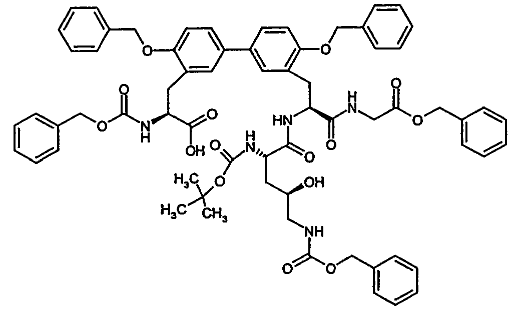 |
| **61A** | 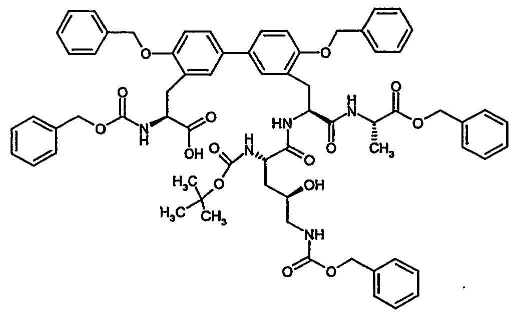 |

### Beispiel 62A

### Pentafluorphenyl-2-(S)-benzyloxycarbonylamino-3-[3'[-2-[5-benzyloxycarbonylamino-2(S)-tert-butoxycarbonylamino-4(R)-(hydroxyoxy)-pentanoylamino(3-amino-[1-(S)-benzyloxy-1-oxo-2-phenyl-ethyl]-3-oxopropyl)]-4,4'-bis-(benzyloxy)-1,1'-biphenyl-3-yl]]propionat

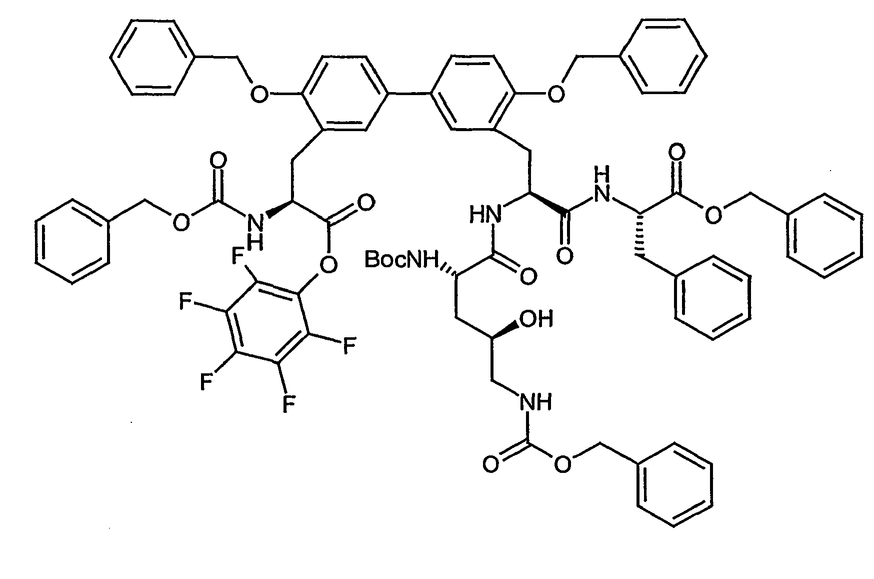

0.25 g (Rohgemisch, ca. 0.19 mmol) 2-(*S*)-Benzyloxycarbonylamino-3-[3'[-2-[5-benzyloxycarbonylamino-2(*S*)-*tert*-butoxycarbonylamino-4(*R*)-(hydroxyoxy)-pentanoylamino(3-amino-[1-(*S*)-benzyloxy-1-oxo-2-phenyl-ethyl]-3-oxopropyl)]-4,4'-bis-(benzyloxy)-1,1'-biphenyl-3-yl]]propionsäure (Beispiel 57A) werden in 4 ml DCM vorgelegt und mit 0.18 g (0.97 mmol, 5.0 Äquiv.) Pentafluorphenol und 0.02 g (0.02 mmol, 0.1 Äquiv.) DMAP versetzt. Man kühlt auf -25°C und gibt 0.048 g (0.25 mmol, 1.3 Äquiv.) EDC hinzu. Die Mischung erwärmt sich über Nacht langsam auf RT. Die Reaktionsmischung wird im Vakuum eingeengt und im Hochvakuum kurz getrocknet. Das Rohprodukt wird ohne weitere Reinigung umgesetzt.

In Analogie zu Beispiel 62A können die in der folgenden Tabelle aufgeführten Beispiele 63A bis 66A hergestellt werden.

| **Beispiel-Nr.** | **Struktur** |
|---|---|
| **63A** | 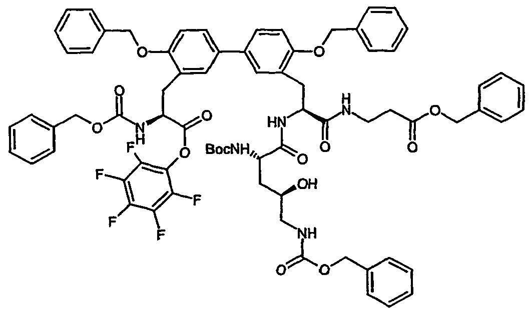 |
| **64A** | 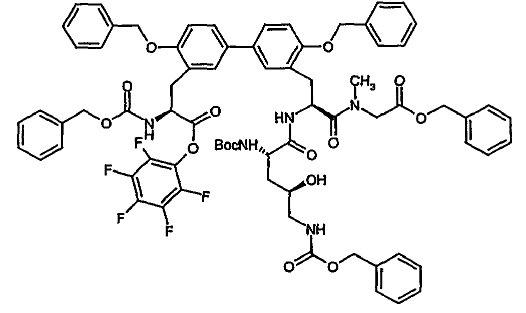 |
| **65A** | 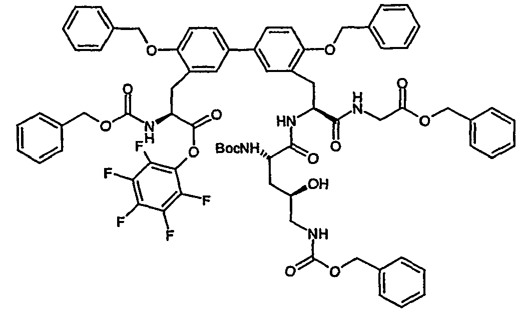 |
| **66A** | 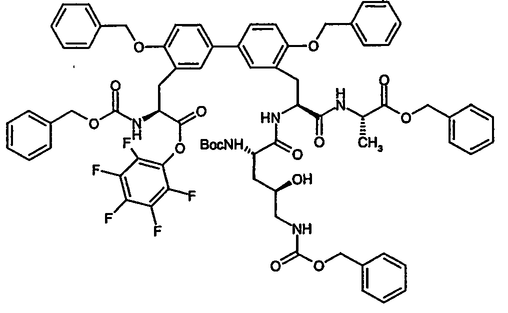 |

### Beispiel 67A

### Pentafluorphenyl-2-(S)-benzyloxycarbonylamino-3-[3'[-2-(5-benzyloxycarbonylamino-2(S)-amino-4(R)-(hydroxyoxy)-pentanoylamino(3-amino-[1-(S)-benzyloxy-1-oxo-2-phenyl-ethyl]-3-oxopropyl)]-4,4'-bis(benzyloxy)-1,1'-biphenyl-3-yl]]propionat

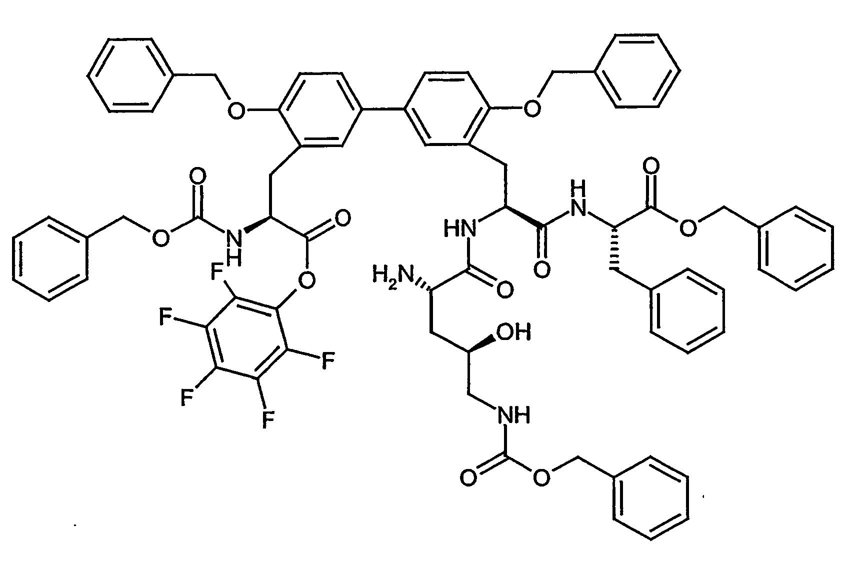

0.28 g (0.19 mmol) Pentafluorphenyl-2-(*S*)-benzyloxycarbonylamino-3-[3'[-2-[5-benzyloxycarbonylamino-2(*S*)-*tert*-butoxycarbonylamino-4(*R*)-(hydroxyoxy)-pentanoylamino(3-amino-[1-(*S*)-benzyloxy-1-oxo-2-phenyl-ethyl]-3-oxopropyl)]-4,4'-bis-(benzyloxy)-1,1'-biphenyl-3-yl]]propionat (Beispiel 62A) werden bei RT in 4 ml einer 4 M Chlorwasserstoff-Dioxan-Lösung gelöst. Nach 3 h bei RT wird die Reaktionslösung bei 30°C im Vakuum eingeengt und im Hochvakuum getrocknet. Das Rohprodukt wird ohne weitere Reinigung umgesetzt.

In Analogie zu Beispiel 67A können die in der folgenden Tabelle aufgeführten Beispiele 68A bis 71A hergestellt werden.

| | |
|---|---|
| **Beispiel-Nr.** | **Struktur** |
| **68A** | 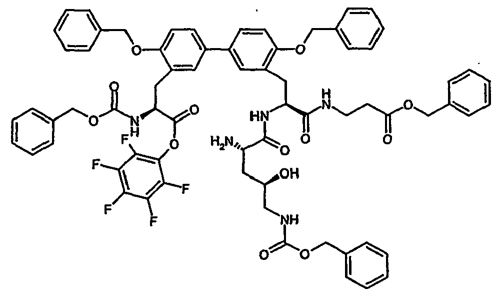 |
| **69A** | 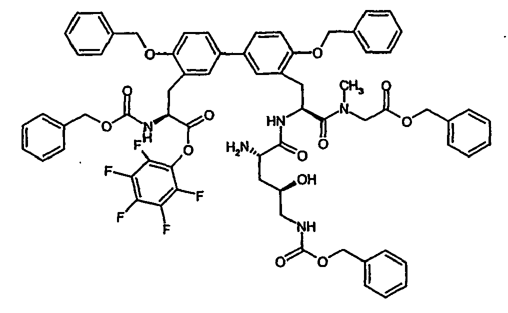 |
| **70A** | 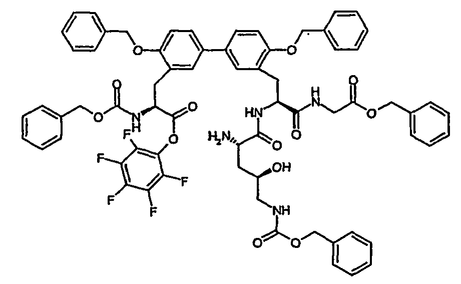 |
| **71A** | 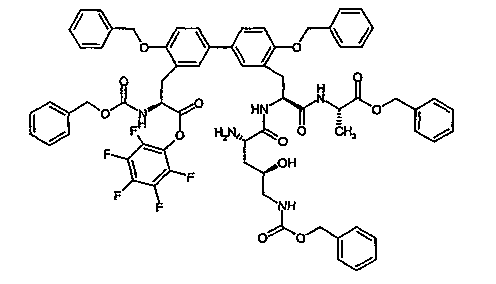 |

### Beispiel 72A

### Benzyl-N-{[(8S,11S,14S)-5,17-bis(benzyloxy)-14-{[(benzyloxy)carbonyl]amino}-11-((2R)-3-{[(benzyloxy)carbonyl]amino}-2-hydroxypropyl)-10,13-diozo-9,12-diazatricyclo[14.3.1.1^{2,6}]henicosa-1(20),2(21),3,5,16,18-hexaen-8-yl]carbonyl}-L-phenylalaninat

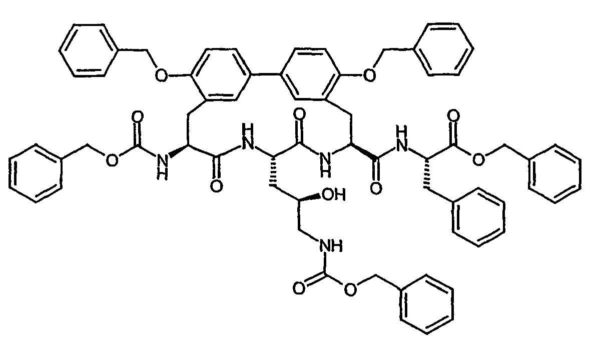

0.26 g (0.19 mmol) Pentafluorphenyl-2-(*S*)-benzyloxycarbonylamino-3-[3'[-2-[5-benzyloxycarbonylamino-2(*S*)-amino-4(*R*)-(hydroxyoxy)-pentanoylamino-(3-amino-[1-(*S*)-benzyloxy-1-oxo-2-phenyl-ethyl]-3-oxopropyl)]-4,4'-bis(benzyloxy)-1,1'-biphenyl-3-yl]]propionat (Beispiel 67A) werden in 200 ml Chloroform gelöst und bei RT innerhalb von 4 h zu einer Lösung aus 2000 ml Chloroform und gesättigter wässriger Natriumhydrogencarbonat-Lösung getropft. Nach beendeter Zugabe wird 1 h nachgerührt. Anschließend werden die Phasen getrennt. Die wässrige Phase wird zweimal mit 500 ml DCM gewaschen. Die vereinigten organischen Phasen mit 2000 ml 0.1 M wässriger Salzsäure gewaschen, über Natriumsulfat getrocknet und im Vakuum eingeengt. Der Rückstand wird in 15 ml Acetonitril : Methanol (2:1) suspendiert und 1 h bei RT gerührt. Der ungelöste Feststoff wird abfiltriert und im Vakuum getrocknet. Zur weiteren Reinigung wird der Feststoff 15 min in Methanol gekocht. Man filtriert erneut ab, trocknet im Vakuum und erhält so das Produkt.
Ausbeute: 0.022 g (10 % d. Th.)
LC-MS (Methode 15): Rₜ = 3.13 min.
MS (EI): m/z = 1158 (M+H)⁺

In Analogie zu Beispiel 72A können die in der folgenden Tabelle aufgeführten Beispiele 73A bis 76A hergestellt werden.

| **Beispiel-Nr.** | **Struktur** | **Analytische Daten** |
|---|---|---|
| **73A** | 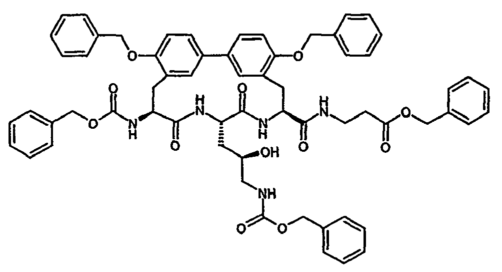 | LC-MS (Methode 15): |
| | | Rₜ = 2.97min. |
| | | MS (EI): m/z = 1082 |
| | | (M+H)⁺ |
| **74A** | 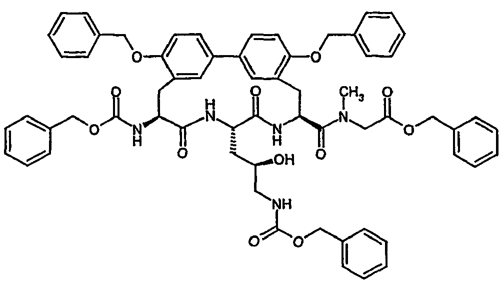 | LC-MS (Methode 15): |
| | | Rₜ = 3.00 min. |
| | | MS (EI): m/z = I082 |
| | | (M+H)⁺ |
| **75A** | 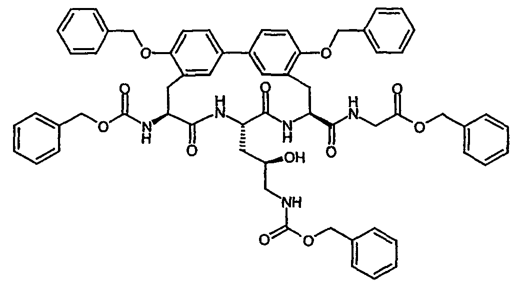 | LC/MS (Methode 15): |
| | | Rₜ = 2.94 min. |
| | | MS (EI): m/z = 1068 |
| | | (M+H)⁺ |
| **76A** | 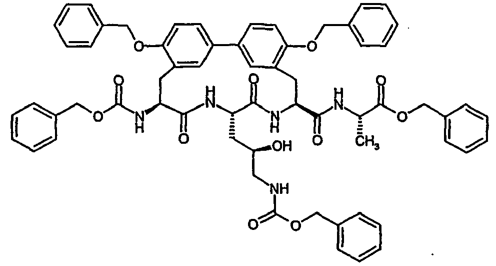 | LC/MS (Methode 15): |
| | | Rₜ = 2.95 min. |
| | | MS (EI): m/z = 1083 |
| | | (M+H)⁺ |

### Beispiel 77A

### 2(S)-[S-Benzyloxycarbonylamino-2(S)-tert-butoxycarbonylamino-pentanoylamino]-3-{4,4'-bis-benzyloxy-3'-[2(S)-benzyloxycarbonylamino-2-(2-trimethylsilyl-ethoxy-carbonyl)-ethyl]-biphenyl-3-yl}-propionsäurebenzylester

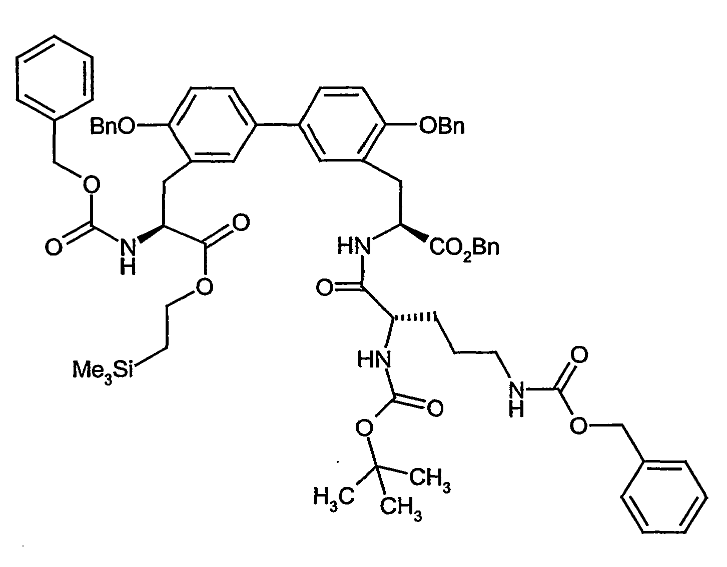

Die Herstellung erfolgt analog Beispiel 16A aus 0.47 g (0.51 mmol) der Verbindung aus Beispiel 15A und 0.19 g (0.51 mmol) N_{α}-Boc-N_{δ}-Z-L-Ornithin mit 0.19 g (0.51 mmol) HATU und 0.35 ml (1.65 mmol) N,N-Diisopropylethylamin in 5.55 ml trockenem DMF.
Ausbeute: 0.58 g (92% d.Th.)
LC-MS (Methode 18): Rₜ = 3.46 min
MS: m/z = 1212 (M+H)⁺

### Beispiel 78A

### 2(S)-Benzyloxycarbonylamino-3-{4,4'-bis-benzylozy-3'-[2(S)-benzyloxycarbonyl-2-(5-benzyloxycarbonylamino)-2(S)-tert-butoxycarbonylaminopentanoylamino)-ethyl]-biphenyl-3-yl}-propionsäure

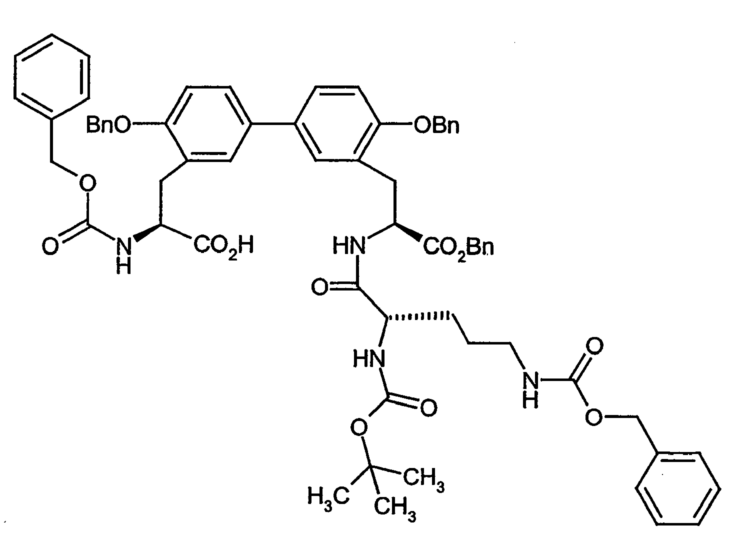

Die Herstellung erfolgt analog Beispiel 17A aus 0.82 g (0.68 mmol) der Verbindung aus Beispiel 77A mit 2 Äquiv. (1.3 ml) Tetrabutylammoniumfluorid (1 M in THF) in 30 ml getrocknetem DMF.
Ausbeute: 772 mg (94% d.Th.)
LC-MS (Methode 20): Rₜ= 1.62 min.
MS: m/z =1112 (M+H)⁺

### Beispiel 79A

### 2(S)-(5-Benzyloxycarbonylamino-2(S)-tert.-butoxycarbonylamino-pentanoylamino)-3-[4,4'-bis-benzyloxy-3'-(2(S)-benzyloxycarbonylamino-2-pentafluorphenyloxycarbonylethyl)-biphenyl-3-yl]-propionsäurebenzylester

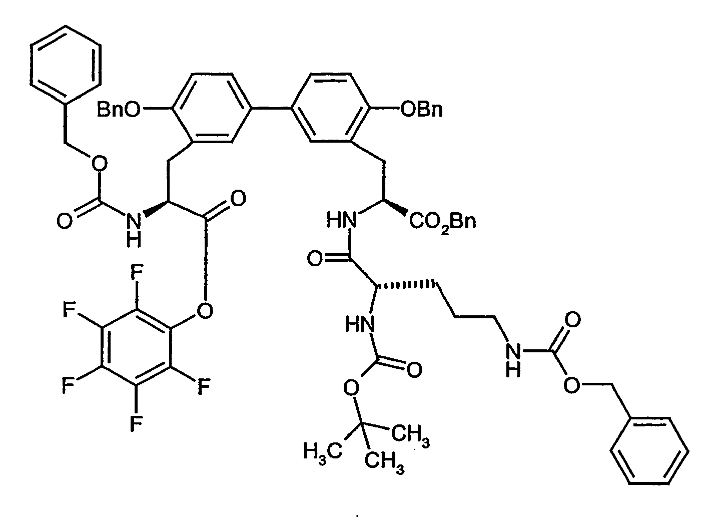

Die Herstellung erfolgt analog Beispiel 18A (Methode A) aus 422 mg (0.38 mmol) der Verbindung aus Beispiel 78A und 349 mg (1.9 mmol) Pentafluorphenol mit 80 mg (0.42 mmol) EDCI und 4.63 mg (0.04 mmol) DMAP in 4 ml Dichlormethan.
Ausbeute: 502 mg (95% d.Th.)
LC-MS (Methode 20): Rₜ = 3.13 min.
MS: m/z = 1278 (M+H)⁺

### Beispiel 80A

### 2(S)-(5-Benzyloxycarbonylamino-2(S)-amino-pentanoylamino)-3-[4,4'-bis-benzyloxy-3'-(2-(S)-benzyloxycarbonylamino-2-pentafluorphenyloxycarbonyl ethyl)-biphenyl-3-yl]-propionsäurebenzylester-Hydrochlorid

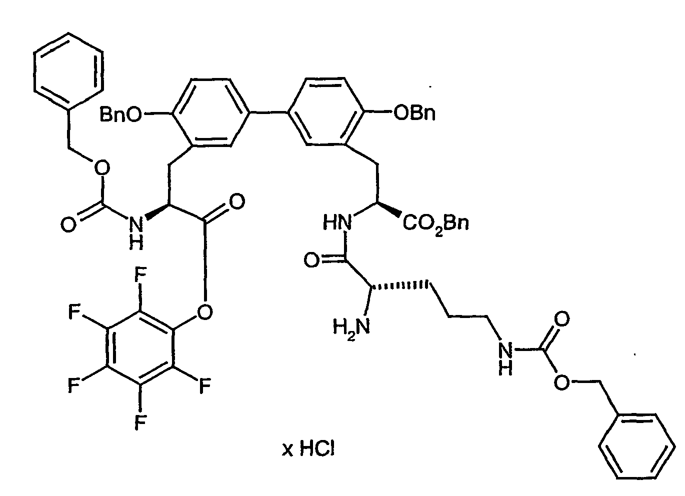

215 mg (0.17 mmol) der Verbindung aus Beispiel 79A werden in einem Eisbad unter Rühren mit 5 ml 4 M Dioxan / Chlorwasserstoff-Lösung versetzt. Man lässt eine Stunde rühren und dampft alles im Vakuum bis zur Gewichtskonstanz ein.
Ausbeute: 200 mg (92% d.Th.)
LC-MS (Methode 20): Rₜ = 4.25 min.
MS: m/z = 1178 (M+H)⁺

### Beispiel 81A

### 5,17-Bis-benzyloxy-14(S)-benzyloxycarbonyl-amino-11(S)-(3-benzyloxycarbonylamino-propyl)-10,13-dioxo-9,12-diaza-tricyclo[14.3.1.1^{2,6}]-henicosa-1(19),2,4,6(21),16(20),17-hexaen-8(S)-carbonsäurebenzylester

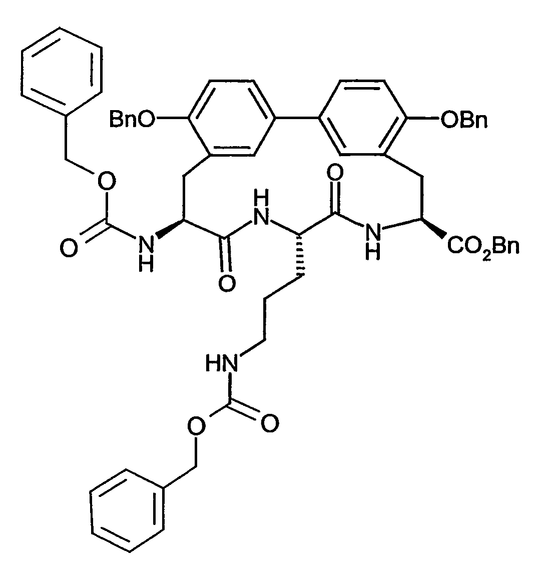

1.35 g (0.91 mmol) der Verbindung aus Beispiel 80A werden in 3 1 Chloroform vorgelegt und unter kräftigem Rühren innerhalb von 20 min bei RT mit 2.54 ml (18.2 mmol) Triethylamin in 50 ml Chloroform versetzt. Man lässt über Nacht nachrühren und dampft alles im Vakuum zur Trockne ein. Den Rückstand verrührt man mit 5 ml Acetonitril, filtriert und trocknet den Rückstand bis zur Gewichtskonstanz.
Ausbeute: 890 mg (93% d.Th.)
LC-MS (Methode 20): Rₜ = 5.10 min.
MS: m/z = 994 (M+H)⁺

### Beispiel 82A

### (8S,11S,14S)-14-Amino-11-(3-aminopropyl)-5,17-dihydroxy-10,13-dioxo-9.12-diazatricyclo[14.3.1.1.^{2,6}]-henicosa-1(20),2(21),3,5,6,18-hexaen-8-carbonsäure-Dihydrochlorid

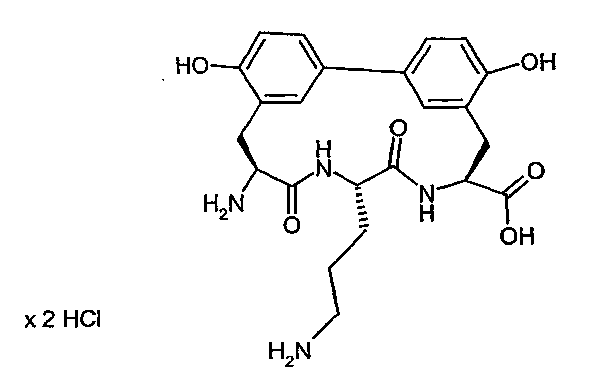

50 mg (0.05 mmol) der Verbindung aus Beispiel 81A werden in 50 ml Eisessig /Wasser / Ethanol (4 / 1 / 1) suspendiert, mit 30 mg Pd/C (10%-ig)-Katalysator versetzt und 20 Stunden bei RT hydriert. Nach Abfiltrieren des Katalysators über Kieselgur dampft man das Filtrat im Vakuum zur Trockne ein und versetzt unter Rühren mit 2.5 ml 0.1 N Salzsäure. Man dampft im Vakuum zur Trockne ein und trocknet bis zur Gewichtskonstanz.
Ausbeute: 17 mg (63% d. Th.)
DC (Methanol / Dichlormethan / 25%-iger Ammoniak = 5 / 3 / 2): R_{f} = 0.6
LC-MS (Methode 9) Rₜ = 0.28 min.
MS: m/z = 457 (M+H)⁺

### Beispiel 83A

### (8S,11S,14S)-14-[(tert-Butoxycarbonyl)-amino-11-[3-[(tert-butoxycarbonyl)-amino]propyl}-5,17-dihydroxy-10,13-dioxo-9,12-diazatricyclo[14.3.1.1^{2,6}]-henicosa-1(20),2(21),3,5,16,18-hexaen-8-carbonsäure

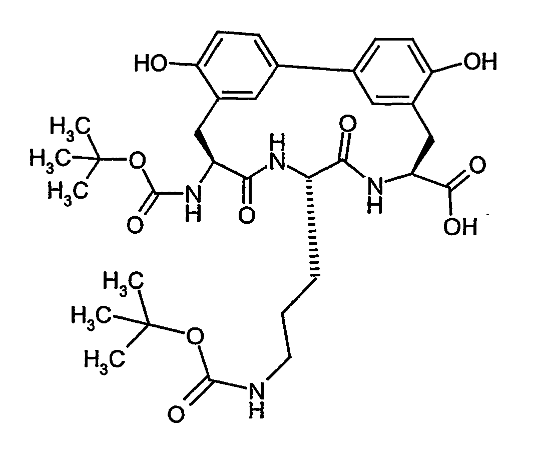

225 mg (0.42 mmol) der Verbindung aus Beispiel 82A werden in 2.25 ml Wasser und 2.25 ml 1 N Natronlauge gelöst, im Eisbad gekühlt und unter Rühren mit 278 mg (1.27 mmol) Di-tert-butyl-dicarbonat versetzt. Man erwärmt nach der Zugabe kurz auf 30°C und lässt über Nacht bei RT weiterreagieren. Man säuert mit 0.1 N Salzsäure bis etwa pH = 5 an und dampft alles vorsichtig im Vakuum bei RT zur Trockne ein. Den Rückstand rührt man mit Diethylether aus, filtriert und trocknet ihn bis zur Gewichtskonstanz.
Ausbeute: 259 mg (93% d.Th.)
LC-MS (Methode 18): Rₜ = 1.96 min.
MS: m/z = 656 (M+H)⁺

### Beispiel 84A

### 2-(Trimethylsilyl)ethyl-2-(benzyloxy)-N-[(benzyloxy)carbonyl]-4-(4,4,5,5-tetramethyl-1,3,2-dioxaborolan-2-yl)-L-phenylalaninat

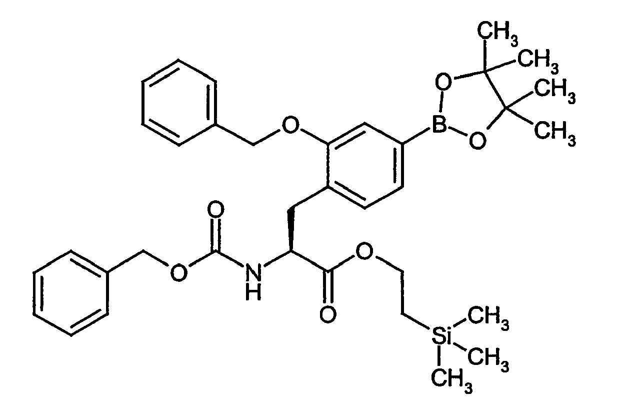

0.924 g (3.64 mmol, 1.15 Äquiv.) 4,4,4',4',5,5,5',5'-Octamethyl-2,2'-bi-1,3,2-dioxaborolan, 0.932 g (9.50 mmol, 3 Äquiv.) Kaliumacetat und 0.116 g (0.160 mmol, 0.05 Äquiv.) Bis-(diphenylphosphino)ferrocenpalladium(II)chlorid werden bei RT zu einer entgasten Lösung von 2.00 g (3.17 mmol) 2(*S*)-Benzyloxycarbonylamino-3-(2-benzyloxy-5-iod-phenyl)-propionsäure-(2-trimethylsilyl)-ethylester (Beispiel 11A) in 20 ml DMF gegeben. Die Mischung wird 6 Stunden bei 80°C nachgerührt. Man nimmt in Wasser und Essigsäureethylester auf, trennt die Phasen und wäscht die wässrige Phase mehrfach mit Essigsäureethylester. Die vereinigten organischen Phasen werden über Natriumsulfat getrocknet und im Vakuum eingeengt. Das Rohprodukt wird durch Chromatographie an Kieselgel (Cyclohexan/ Essigsäureethylester 10:1) gereinigt.
Ausbeute: 1.12 g (56% d. Th.)
LC-MS (Methode 22): Rₜ = 4.50 min
MS (EI): m/z = 632 (M+H)⁺
¹H-NMR (200 MHz, CDCl₃): δ = 0.92 (dd, 2H), 1.31 (s, 12H), 2.95-3.95 (m, 2H), 4.11 (m_{c}, 2H), 4.55 (11 (m_{c}, 1H), 4.99 (s, 2H), 5.08 (s, 2H), 5.53 (d, 1H), 6.90 (d, 1H), 7.15-7.47 (m, 10 H), 7.58 (d, 1H), 7.67 (dd, 1H).

In Analogie zu Beispiel 37A können die in der folgenden Tabelle aufgeführten Beispiele 85A bis 87A hergestellt werden.

| **Beispiel-Nr.** | **Struktur** | **Analytische Daten** |
|---|---|---|
| **85A** | 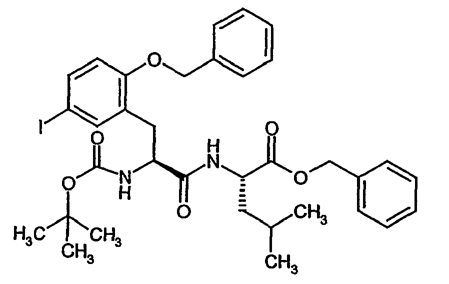 | LC-MS (Methode 15): |
| | | Rₜ = 3.12 min. |
| | | MS (EI): m/z = 701 |
| | | (M+H)⁺ |
| **86A** | 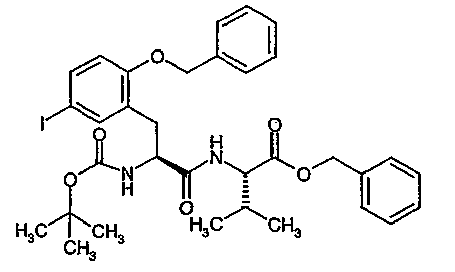 | LC-MS (Methode 15): |
| | | Rₜ = 3.08 min. |
| | | MS (EI): m/z = 687 |
| | | (M+H)⁺ |
| **87A** | 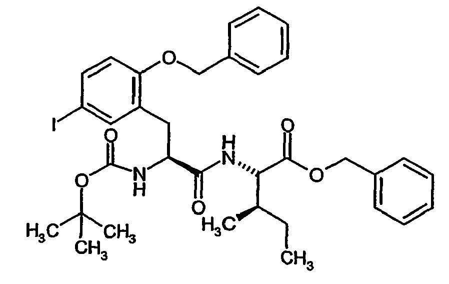 | LC-MS (Methode 15): |
| | | Rₜ = 3.14 min. |
| | | MS (EI): m/z = 701 |
| | | (M+H)⁺ |

In Analogie zu Beispiel 42A können die in der folgenden Tabelle aufgeführten Beispiele 88A bis 90A hergestellt werden.

| **Beispiel- Nr.** | **Struktur** | **Analytische Daten** |
|---|---|---|
| **88A** | 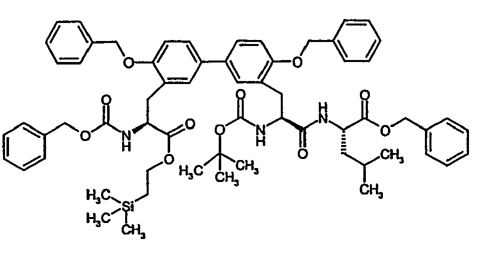 | LC-MS (Methode 16): |
| | | Rₜ = 2.59 min. |
| | | MS (EI): m/z = 1078 |
| | | (M+H)⁺ |
| **89A** | 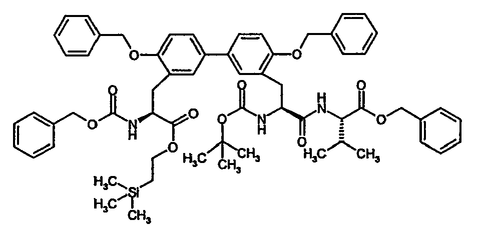 | LC-MS (Methode 15): |
| | | Rₜ = 3.49 min. |
| | | MS (EI): m/z = 1064 |
| | | (M+H)⁺ |
| **90A** | 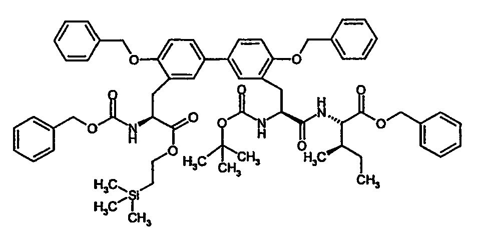 | LC-MS (Methode 15): |
| | | Rₜ = 3.55 min. |
| | | MS (EI): m/z = 1078 |
| | | (M+H)⁺ |

In Analogie zu Beispiel 47A können die in der folgenden Tabelle aufgeführten Beispiele 91A bis 93A hergestellt werden.

| **Beispiel-Nr.** | **Struktur** | **Analytische Daten** |
|---|---|---|
| **91A** | 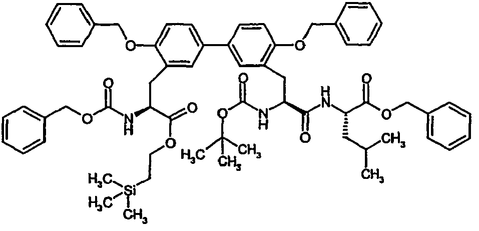 | LC-MS (Methode 16): |
| | | Rₜ = 2.59 min. |
| | | MS (EI): m/z = 1078 |
| | | (M+H)⁺ |
| **92A** | 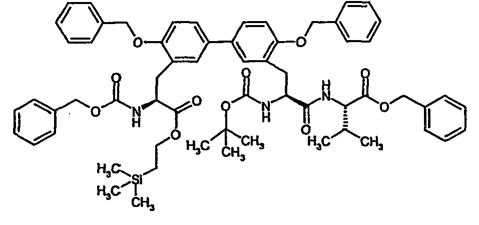 | -- |
| **93A** | 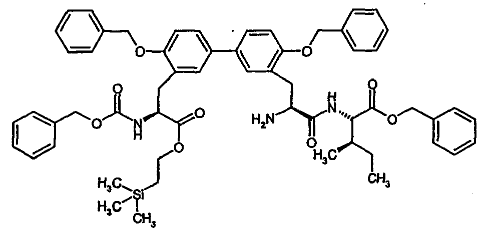 | -- |

In Analogie zu Beispiel 52A können die in der folgenden Tabelle aufgeführten Beispiel 94A bis 96A hergestellt werden.

| **Beispiel-Nr.** | **Struktur** | **Analytische Daten** |
|---|---|---|
| **94A** | 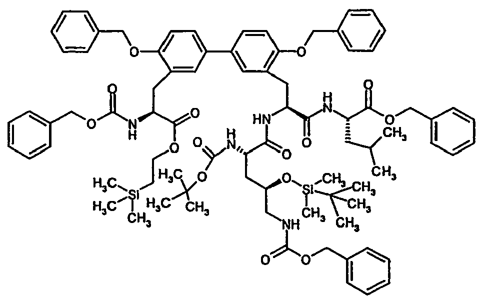 | LC-MS (Methode 16): |
| | | Rₜ = 3.40 min. |
| | | MS (EI): m/z = 1457 |
| | | (M+H)⁺ |
| **95A** | 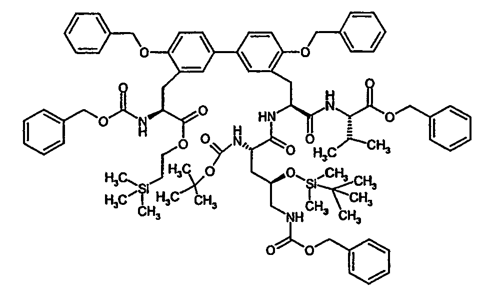 | LC-MS (Methode 16): |
| | | Rₜ = 3.17 min |
| | | MS (EI): m/z = 1442 |
| | | (M+H)⁺ |
| **96A** | 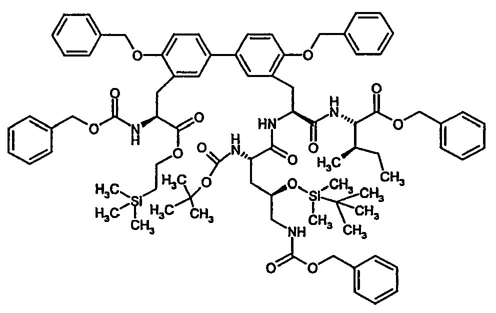 | LC-MS (Methode 16): |
| | | Rₜ = 3.33 min |
| | | MS (EI): m/z = 1457 |
| | | (M+H)⁺ |

In Analogie zu Beispiel 57A können die in der folgenden Tabelle aufgeführten Beispiele 97A bis 99A hergestellt werden.

| **Beispiel-Nr.** | **Struktur** |
|---|---|
| **97A** | 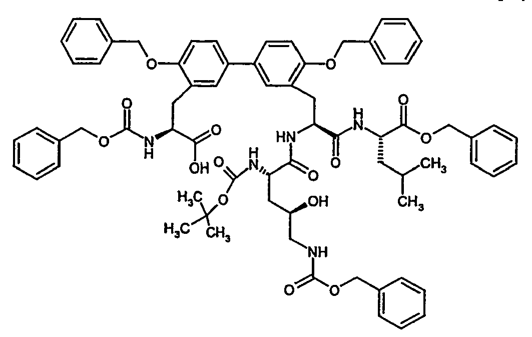 |
| **98A** | 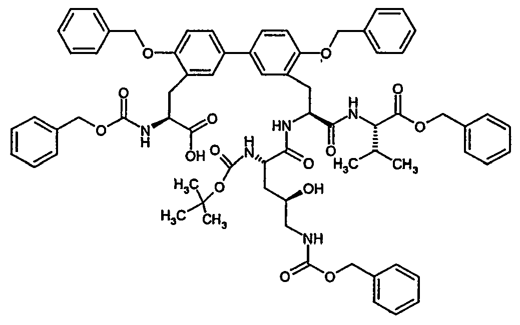 |
| **99A** | 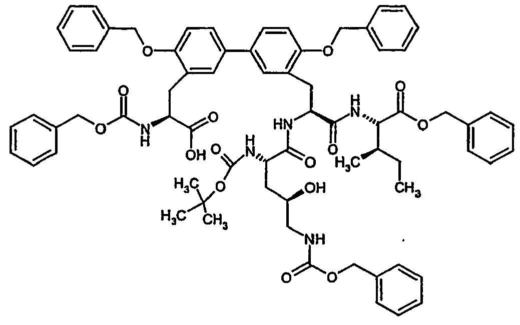 |

In Analogie zu Beispiel 62A können die in der folgenden Tabelle aufgeführten Beispiele 100A bis 102A hergestellt werden.

| **Beispiel-Nr.** | **Struktur** |
|---|---|
| **100A** | 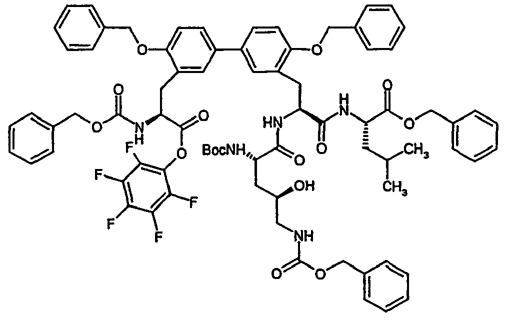 |
| **101A** | 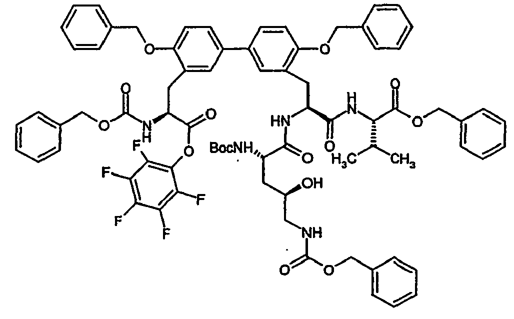 |
| **102A** | 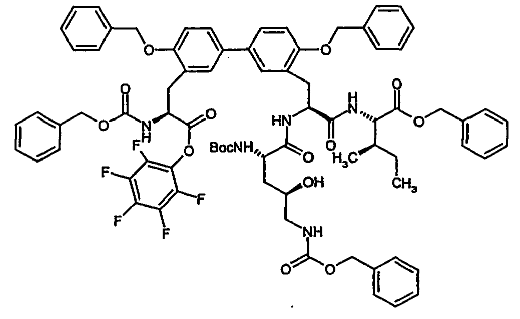 |

In Analogie zu Beispiel 67A können die in der folgenden Tabelle aufgeführten Beispiele 103A bis 105A hergestellt werden.

| **Beispiel-Nr.** | **Struktur** |
|---|---|
| **103A** | 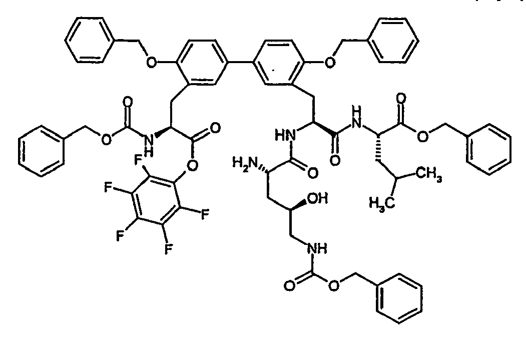 |
| **104A** | 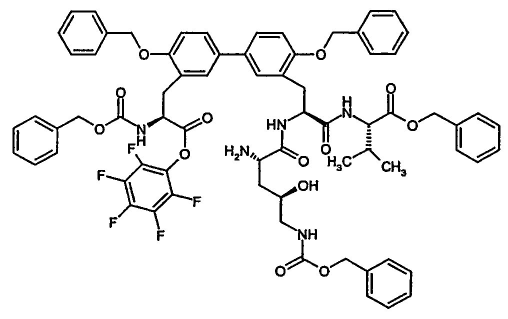 |
| **105A** | 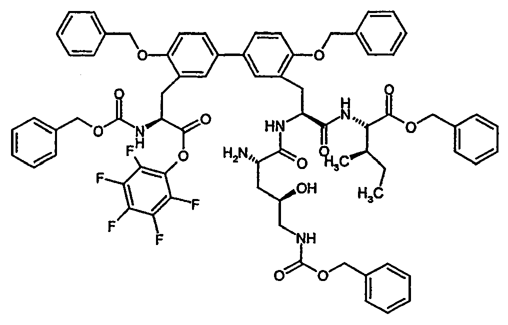 |

In Analogie zu Beispiel 72A können die in der folgenden Tabelle aufgeführten Beispiele 106A bis 108A hergestellt werden.

| **Beispiel-Nr.** | **Struktur** | **Analytische Daten** |
|---|---|---|
| **106A** | 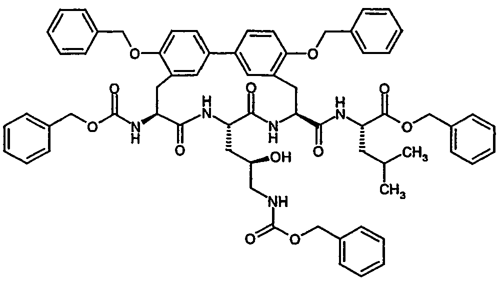 | LC-MS (Methode 15): |
| | | Rₜ = 3.10 min. |
| | | MS (EI): m/z = 1124 |
| | | (M+H)⁺ |
| **107A** | 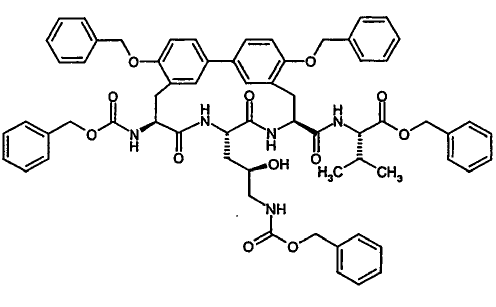 | LC-MS (Methode 24): |
| | | Rₜ = 3.31 min. |
| | | MS (EI): m/z = 1110 |
| | | (M+H)⁺ |
| **108A** | 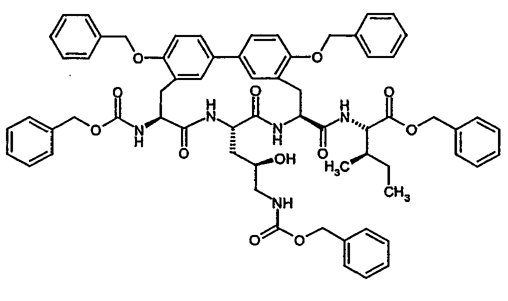 | LC-MS (Methode 24): |
| | | Rₜ = 3.32 min. |
| | | MS (EI): m/z = 1124 |
| | | (M+H)⁺ |

In Analogie zu Beispiel 24A kann das in der folgenden Tabelle aufgeführte Beispiel 109A hergestellt werden.

| **Beispiel-Nr.** | **Struktur** | **Analytische Daten** |
|---|---|---|
| **109A** | 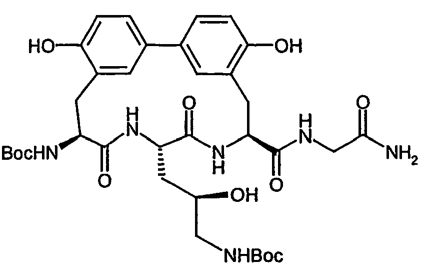 | LC-MS (Methode 24): |
| | | Rₜ = 1.94 min |
| | | MS (EI): m/z = 729 |
| | | (M+H)⁺ |

### Beispiel 110A

### 2-(Benzyloxy)-N-(tert-butoxycarbonyl)-iod-N-methyl-L-phenylalanin

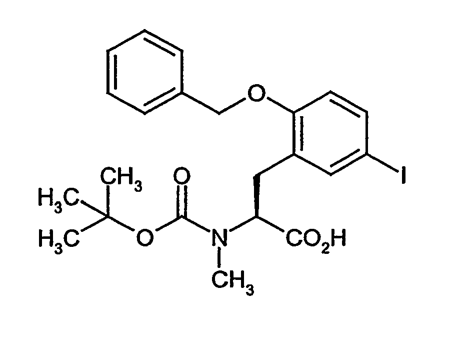

Unter Argonatmosphäre werden 500 mg (1 mmol) der Verbindung aus Beispiel 6A in 20 ml THF gelöst, mit 90.5 mg (3.02 mmol) Natriumhydrid und 0.51 ml (1141.6 mg; 8.04 mmol) Methyliodid (80%-ig) versetzt und über Nacht bei Raumtemperatur gerührt. Man verdünnt mit 25 ml Essigsäureethylester und 25 ml Wasser und stellt mit 0.1 N Salzsäure auf pH = 9 ein. Man engt im Vakuum auf ein kleines Volumen ein. Man versetzt mit 10 ml Essigsäureethylester und 10 ml Wasser, schüttelt alles heftig und trennt die organische Phase ab. Nach Trocknen mit Natriumsulfat und Einengen im Vakuum erhält man 140 mg Produkt (19% d. Th.).

Die wässrige Phase säuert man an (pH = 3) und schüttelt sie dreimal mit 20 ml Essigsäureethylester aus. Nach Einengen im Vakuum und Trocknen im Vakuum erhält man 351 mg Produkt (68% d. Th.).
LC-MS (Methode 17): Rₜ = 3.9 min.
MS (EI): m/z = 511 (M+H)⁺

### Beispiel 111A

### Benzyl-2-(benzyloxy)-N-(tert-butoxycarbonyl)-5-iod-N-methyl-L-phenylalaninat

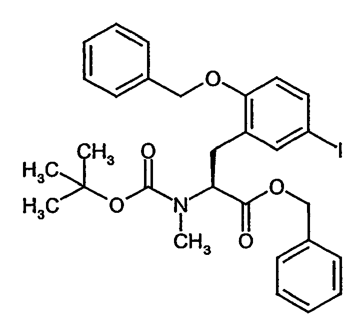

Die Herstellung erfolgt analog zu Beispiel 7A aus 350 mg (0.68 mmol) der Verbindung aus Beispiel 110A, 8.29 mg (0.07 mmol) DMAP, 148 mg (1.37 mmol) Benzylalkohol und 157.46 mg (0.82 mmol) EDC in 3 ml Acetonitril.
Ausbeute: 382 mg (93% d. Th.)
LC-MS (Methode 17): Rₜ = 4.8 min.
MS (EI): m/z = 601 (M+H)⁺

### Beispiel 112A

### Benzyl-2-(benzyloxy)-N-(tert-butoxycarbonyl)-N-methyl-5-(4,4,5,5-tetramethyl-1,3,2-dioxaborolan-2-yl)-L-phenylalaninat

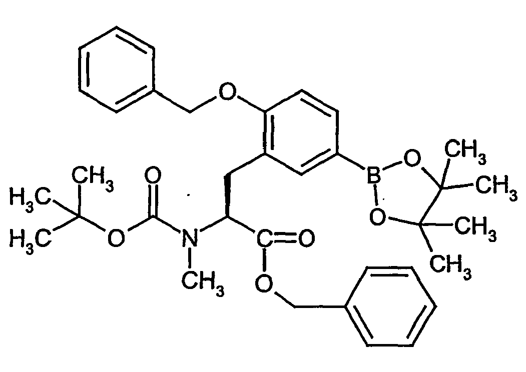

Analog zu Beispiel 8A werden 380 mg (0.63 mmol) der Verbindung aus Beispiel 111A in einem ausgeheizten Kolben in 4 ml DMF vorgelegt und unter Rühren bei Raumtemperatur mit 184.5 mg (0.73 mmol) 4,4,4',4',5,5,5',5'-Octamethyl-2,2'-bi-1,3,2-dioxaborolan, 186 mg (1.9 mmol) Kaliumacetat und 23.15 mg (0.03 mmol) Bis(diphenylphosphino)-ferrocen-palladium(II)chlorid versetzt. Man lässt 4 h bei 80°C reagieren. Nach der Aufarbeitung und Chromatographie (Kieselgel 60, Laufmittel: Cyclohexan/Essigsäureethylester = 4/1) erhält man das Produkt.
Ausbeute: 196 mg
LC-MS (Methode 17): Rₜ = 4.9 min.
MS (EI): m/z = 601 (M+H)⁺

### Beispiel 113A

### 2(S)-Benzyloxycarbonylamino-3-[4,4'-bis-benzyloxy-3'-(2(S)-benzyloxycarbonyl-(2-tert-butoxycarbonyl-2-methyl)amino-ethyl)-biphenyl-3-yl]-propionsäure-2-(trimethylsilyl)-ethylester

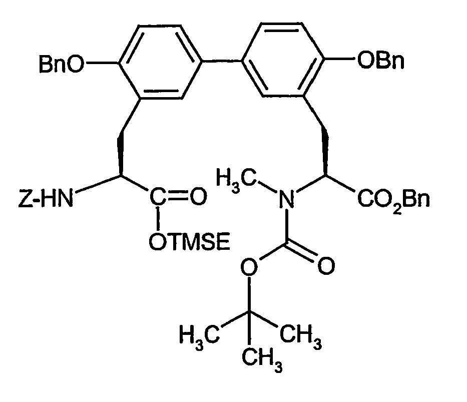

Die Herstellung erfolgt analog Beispiel 12A (Methode B) aus 190 mg (0.32 mmol) der Verbindung aus Beispiel 112A, 199.5 mg (0.32 mmol) der Verbindung aus Beispiel 11A, 195.5 mg (0.63 mmol) Cäsiumcarbonat und 23.15 mg (0.03 mmol) Bis(diphenylphosphino)ferrocen-palladium(II)chlorid in 1.5 ml DMF unter Argonatmosphäre.
Ausbeute: 212 mg (66% d. Th.)
LC-MS (Methode 25): Rₜ= 4.86 min.
MS (EI): m/z = 978 (M+H)⁺

### Beispiel 114A

### 2(S)-Benzyloxycarbonylamino-3-[4,4'-bis-benzyloxy-3'-(2(S)-benzyloxycarbonyl-2-methylaminoethyl-biphenyl-3-yl]-propionsäure-2-(trimethylsilyl)-ethylester-Hydrochlorid

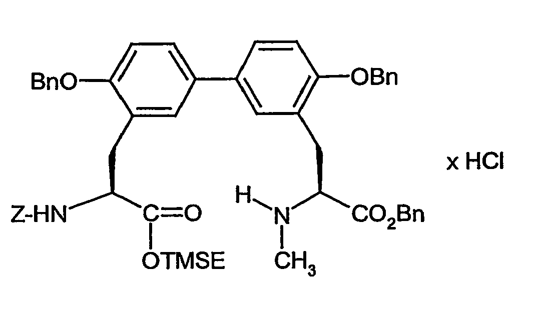

Die Herstellung erfolgt analog Beispiel 15A aus 930 mg (0.95 mmol) der Verbindung aus Beispiel 113A und 22.14 ml einer 4 M Dioxan/-Chlorwasserstofflösung in 15 ml Dioxan.
Ausbeute: 915 mg (78% d. Th.)
LC-MS (Methode 25): Rₜ = 2.53 min.
MS (EI): m/z = 878 (M+H)⁺

### Beispiel 115A

### 2(S)-{Methyl-[5-benzyloxycarbonylamino-2(S)-tert-butoxycarbonylamino-4(R)-(tert-butyldimethylsilyloxy)-pentanoyl]amino}-3-{4,4'-bis-benzyloxy-3'-[2(S)-benzyloxycarbonylamino-2-(2-trimethylsilyl-ethoxycarbonyl)ethyl]-biphenyl-3-yl}-propionsäurebenzylester

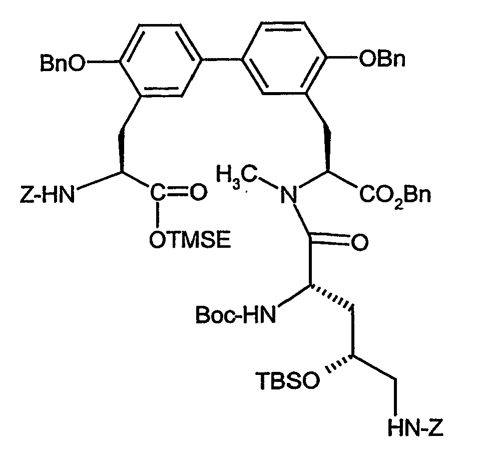

Die Herstellung erfolgt analog Beispiel 16A aus 922 mg (1.01 mmol) der Verbindung aus Beispiel 114A, 0.5 g (1.01 mmol) der Verbindung aus Beispiel 14A, 421 mg (1.11 mmol) HATU und 0.7 ml (518 mg; 3.27 mmol) DIPEA in 4.2 ml DMF.
Ausbeute: 703 mg (51% d. Th.)
LC-MS (Methode 16): Rₜ = 3.17 min.
MS (EI): m/z = 1356 (M+H)⁺

### Beispiel 116A

### 2(S)-Benzyloxycarbonylamino-3-{4,4'-bis-benzyloxy-3'-[2(S)-benzyloxycarbonyl-2-{methyl-(5-benzyloxycarbonylamino-2(S)-tert-butoxycarbonylamino-4(R)-hydroxy-pentanoyl)amino}-ethyl]-biphenyl-3-yl}-propionsäure

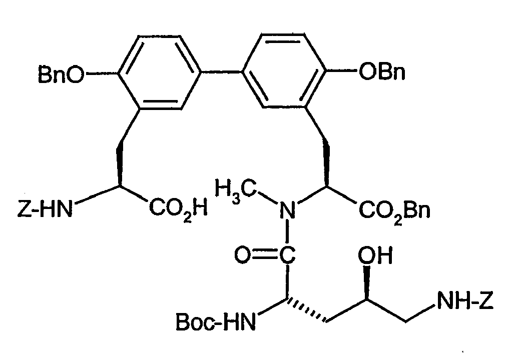

Die Herstellung erfolgt analog Beispiel 17A aus 360 mg (0.27 mmol) der Verbindung aus Beispiel 115A und 0.8 ml (3 Äquiv.) 1 M Tetrabutylammoniumfluorid-Lösung (THF) in 20 ml DMF.
Ausbeute: 159 mg (53% d. Th.)
LC-MS (Methode 23): Rₜ = 3.19 min.
MS (EI): m/z = 1142 (M+H)⁺

### Beispiel 117A

### 2(S)-[Methyl-(5-benzyloxycarbonylamino)-2(S)-tert-butoxycarbonylamino-4(R)-hydroxy-pentanoyl]amino-3-[4,4'-bis-benzyloxy-3'-(2(S)-benzyloxycarbonylamino-2-pentafluorphenyloxycarbonyl-ethyl)-biphenyl-3-yl]-propionsäurebenzylester

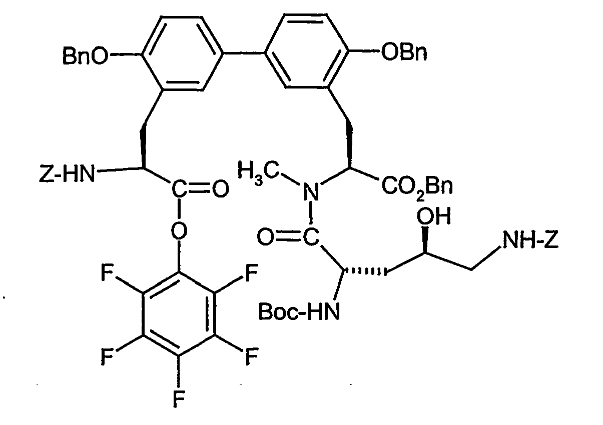

Die Herstellung erfolgt analog Beispiel 18A (Methode A) aus 330 mg (0.29 mmol) der Verbindung aus Beispiel 116A, 265.6 mg (1.44 mmol) Pentafluorphenol, 3.53 mg (0.03 mmol) DMAP und 60.87 mg (0.32 mmol) EDC in 10 ml Dichlormethan.
Ausbeute: 271 mg (69% d. Th.)
LC-MS (Methode 23): Rₜ = 3.38 min.
MS (EI): m/z =1308 (M+H)⁺

### Beispiel 118A

### 2(S)-[Methyl-(5-benzyloxycarbonylamino)-2(S)-amino-4(R)-hydroxy-pentanoyl]amino-3-[4,4'-bis-benzyloxy-3'-(2(S)-benzyloxycarbonylamino-2-pentafluor-phenyloxycarbonyl-ethyl)-biphenyl-3-yl]-propionsäurebenzylester-Hydrochlorid

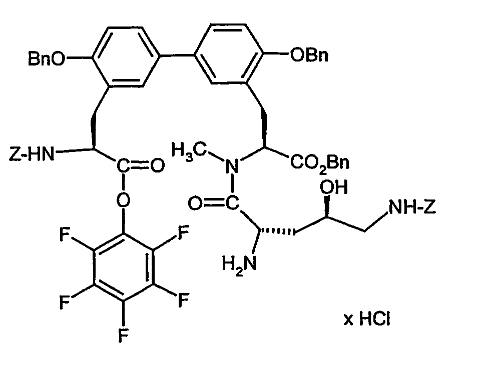

130 mg (0.1 mmol) der Verbindung aus Beispiel 117A werden in 0.5 ml Dioxan gelöst und vorsichtig mit 5 ml 4 M Dioxan-Chlorwasserstoff-Lösung versetzt (Eisbad). Nach 30 Minuten lässt man bei Raumtemperatur noch 2 h weiterreagieren. Man dampft alles im Vakuum zur Trockne ein und trocknet im Hochvakuum bis zur Gewichtskonstanz.
Ausbeute: 130 mg (70% d. Th.)
LC-MS (Methode 15): Rₜ = 2.68 min.
MS (EI): m/z = 1208 (M+H)⁺

### Beispiel 119A

### Benzyl-(8S, 11S, 14S)-5,17-bis(benzyloxy)-14-{[(benzyloxy)carbonyl]amino}-11-((2R)-3-{[(benzyloxy)carbonyl]amino}-2-hydroxypropyl-9-methyl-10,13-dioxo-9,12-diazatricyclo[14.3.1.1^{2.6}]henicosa-1(20),2(21),3,5,16,18-hexaen-8-carboxylat

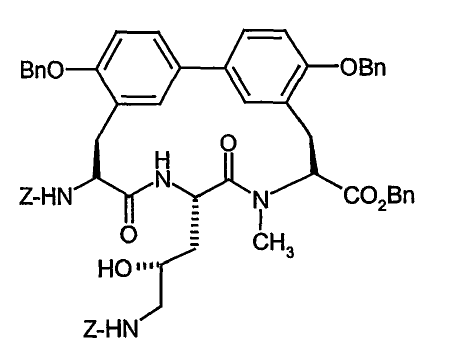

130 mg (0.1 mmol) der Verbindung aus Beispiel 118A werden in 220 ml trockenem Chloroform vorgelegt. Bei Raumtemperatur versetzt man unter Rühren innerhalb von 20 Minuten mit 23 ml (20 Äquiv.) Triethylamin in 5 ml Dichlormethan. Man lässt über Nacht nachrühren. Anschließend wird alles im Vakuum zur Trockne eingedampft. Der Rückstand wird mit Acetonitril ausgerührt. Nach dem Trocknen des Rückstandes gewinnt man 44 mg Produkt. Aus der Mutterlauge wird durch RP-HPLC noch weiteres Produkt gewonnen (30 mg).
Ausbeute: 74 mg (69% d. Th.)
LC-MS (Methode 15): Rₜ = 3.13 min.
MS (EI): m/z = 1024 (M+H)⁺

### Beispiel 120A

### (8S, 11S, 14S)-14-Amino-11-[(2R)-3-amino-2-hydroxypropyl]-5,17-dihydroxy-9-methyl-10,13-dioxo-9,12-diazatricyclo[14.3.1.1^{2,6}]henicosa-1(20),2(21),3,5,16,18-hexaen-carbonsäure-Di-trifluoracetat

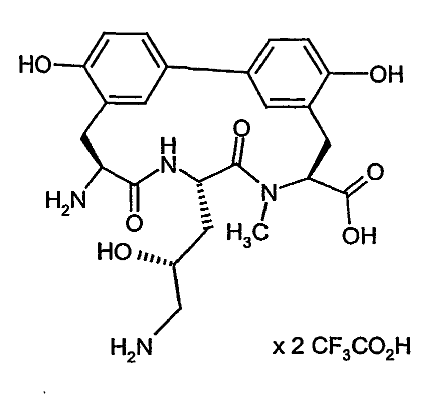

33 mg (0.032 mmol) der Verbindung aus Beispiel 119A werden mit verdünnter Trifluoressigsäure vorsichtig behandelt. Die entstandene klare Lösung wird anschließend lyophilisiert.
Ausbeute: 23 mg (quantitativ)
LC-MS (Methode 15): Rₜ = 0.92 min.
MS (EI): m/z = 486 (M+H)⁺

### Beispiel 121A

### (8S, 11S, 14S)-5,17-Bis(benzyloxy)-14-{[benzyloxycarbonyl]amino}-11-(2R)-3-{[benzyloxycarbonyl]-amino}-2-hydroxypropyl-9-methyl-10,13-dioxo-9,12-diazatricyclo[14.3.1.1^{2,6}]henicosa-1(20),2(21),3,5,16,18-hexaen-8-carbonsäure

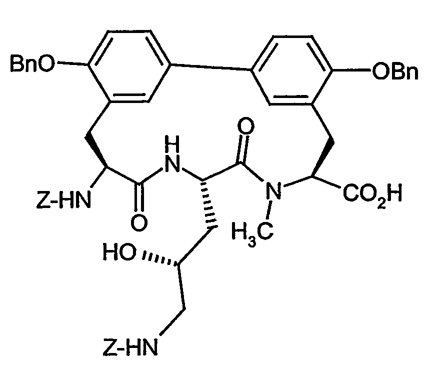

37 mg (0.04 mmol) der Verbindung aus Beispiel 119A werden in 2 ml THF gelöst, mit 0.14 ml 1 N Lithiumhydroxid-Lösung versetzt und 3 h bei Raumtemperatur gerührt. Anschließend säuert man mit 1 N Salzsäure an und dampft alles im Hochvakuum zur Trockne ein.
Ausbeute: 33 mg (71% d. Th.)
LC-MS (Methode 23): Rₜ = 2.90 min.
MS (EI): m/z = 934 (M+H)⁺

### Beispiel 122A

### (8S, 11S, 14S)-5,17-Bis(benzyloxy)-14-{[benzyloxycarbonyl]amino}-11-(2R)-3-{[benzyloxycarbonyl]amino}-2-hydroxypropyl-9-methyl-10,13-dioxo-9,12-diazatricyclo[14.3.1.1^{2,6}]henicosa-1(20),2(21),3,5,16,18-hexaen-8-carbonsäureamid

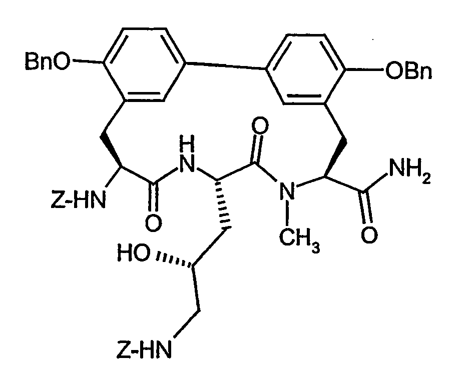

30 mg (0.03 mmol) der Verbindung aus Beispiel 121A werden in 1 ml DMF gelöst und mit 0.01 ml (3 Äquiv.) Triethylamin versetzt. Nach Abkühlen der Reaktionslösung im Eisbad versetzt man mit 8.76 mg (2 Äquiv.) Chlorameisensäureisobutylester und lässt 30 Minuten reagieren. Nach einer weiteren Stunde Rühren bei Raumtemperatur versetzt man mit 0.64 ml (10 Äquiv.) 0.5 N Dioxan-Ammoniak-Lösung und rührt über Nacht. Nach Einengen im Vakuum wird der Rückstand mittels RP-HPLC gereinigt.
Ausbeute: 11 mg (37% d. Th.)
LC-MS (Methode 23): Rₜ = 2.91 min.
MS (EI): m/z = 934 (M+H)⁺

Analog zu den oben aufgeführten Vorschriften der Beispiele 115A bis 122A werden die in der folgenden Tabelle aufgeführten Beispiele 123A bis 129A aus den entsprechenden Edukten hergestellt:

| **Beispiel-Nr.** | **Struktur** | **hergestellt analog** | **Analytische Daten** |
|---|---|---|---|
| **123A** | 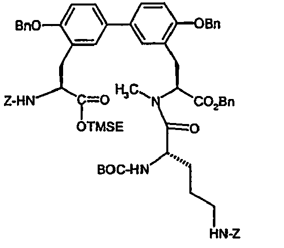 | 115A | LC-MS (Methode 25): Rₜ = |
| | | | 4.85 min. |
| | | | MS (EI): m/z = 1226 |
| | | | (M+H)⁺ |
| **124A** | 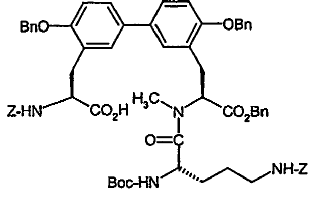 | 116A | LC-MS (Methode 25): Rₜ = |
| | | | 2.04 min. |
| | | | MS (EI): m/z = 1126 |
| | | | (M+H)⁺ |
| **125A** | 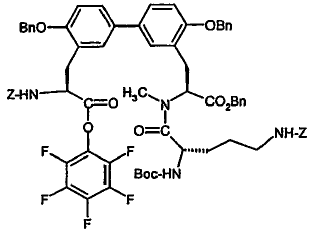 | 117A | LC-MS (Methode 25): Rₜ = |
| | | | 3.79 min. |
| | | | MS (EI): m/z = 1292 |
| | | | (M+H)⁺ |
| **126A** | 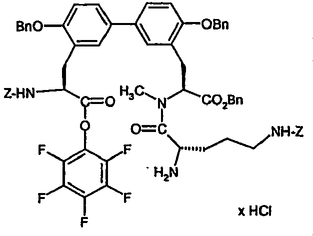 | 118A | LC-MS (Methode 25): Rₜ = |
| | | | 3.72 min. |
| | | | MS (EI): m/z = 1192 |
| | | | (M+H)⁺ |
| **127A** | 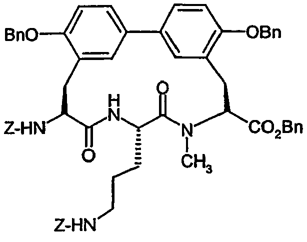 | 119A | LC-MS (Methode 25): Rₜ = |
| | | | 4.39 min. |
| | | | MS (EI): m/z = 1008 |
| | | | (M+H)⁺ |
| **128A** | 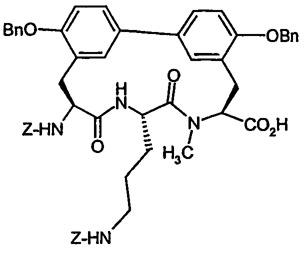 | 121A | LC-MS (Methode 26): Rₜ = |
| | | | 3.64 min. |
| | | | MS (EI): m/z = 918 |
| | | | (M+H)⁺ |
| **129A** | 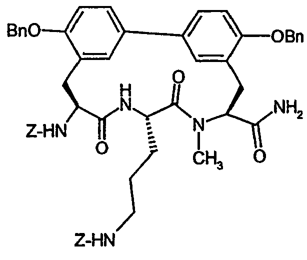 | 122A | LC-MS (Methode 25): Rₜ = |
| | | | 3.8 min. |
| | | | MS (EI): m/z = 917 |
| | | | (M+H)⁺ |

### Beispiel 130A

### 2(S)-tert-Butoxycarbonylamino-5-nitro-4-oxo-pentansäure-benzylester

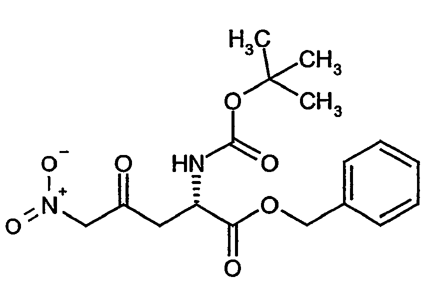

Eine Lösung A von 10 g (30.9 mmol) 2(*S*)-tert-Butoxycarbonylamino-bernsteinsäure-1-benzylester und 5.27 g (32.5 mmol) 1,1'-Carbonyldiimidazol in 100 ml Tetrahydrofuran wird 5 h bei RT gerührt. Zu einer Lösung B von 3.2 g (34.2 mmol) Kalium-tert-butylat in 100 ml Tetrahydrofuran werden bei 0°C 18.8 g (30.9 mmol) Nitromethan zugetropft. Die Lösung B wird unter Erwärmen auf RT nachgerührt, und anschließend wird bei RT Lösung A zugetropft. Die resultierende Mischung wird 16 h bei RT gerührt und mit 20%iger Salzsäure auf pH 2 eingestellt. Das Lösungsmittel wird eingedampft. Das zurückbleibende Rohprodukt wird in Essigsäureethylester/Wasser aufgenommen. Nach Trennung der Phasen wird die organische Phase zweimal mit Wasser ausgeschüttelt, über Natriumsulfat getrocknet und eingeengt. Man erhält 13 g (99% d. Th.) des Produkts.
MS (ESI): m/z = 334 (M+H)⁺
¹H-NMR (300 MHz, d₆-DMSO): δ = 1.37 (s, 9H), 2.91 (m, 1H), 3.13 (m, 1H), 4.44 (m, 1H), 5.12 (s, 2H), 5.81 (m, 2H), 7.2-7.5 (m, 5H).

### Beispiel 131A

### 2(S)-tert-Butoxycarbonylamino-4(R)-hydroxy-5-nitro-pentansäure-benzylester

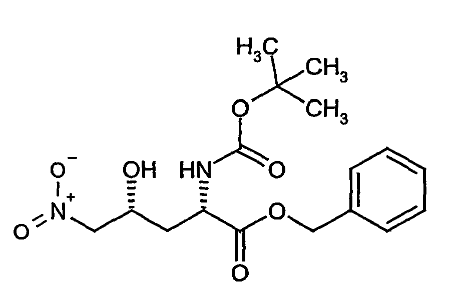

Eine Lösung von 11.3 g (30.8 mmol) 2(*S*)-tert-Butoxycarbonylamino-5-nitro-4-oxopentansäure-benzylester in 300 ml Tetrahydrofuran wird auf -78°C gekühlt, mit 30.8 ml einer 1M Lösung von L-Selectrid® in Tetrahydrofuran tropfenweise versetzt und 1 h bei -78°C nachgerührt. Nach Erwärmen auf RT wird die Lösung vorsichtig mit gesättigter Ammoniumchlorid-Lösung versetzt. Die Reaktionslösung wird eingeengt und der Rückstand in Wasser und Essigsäureethylester aufgenommen. Die wässrige Phase wird dreimal mit Essigsäureethylester extrahiert. Die vereinigten organischen Phasen werden über Natriumsulfat getrocknet und eingedampft. Das Rohprodukt wird an Kieselgel 60 vorgereinigt (Laufmittel: Cyclohexan/Essigsäureethylester 10/1), die gesammelten Fraktionen werden eingeengt und mit Cyclohexan/Essigsäureethylester 5/1 ausgerührt. Die zurückbleibenden Kristalle werden abgesaugt und getrocknet. Man erhält 2.34 g (21% d. Th.) des gewünschten Diastereomers. Aus der Mutterlauge erhält man durch chromatographische Trennung an Lichrospher Diol 10 µM (Laufmittel: Ethanol/*iso*-Hexan 5/95) weitere 0.8 g (6.7%) des Produkts.
MS (ESI): m/z = 369 (M+H)⁺.
¹H-NMR (300 MHz, d₆-DMSO): δ = 1.38 (s, 9H), 1.77 (m, 1H), 1.97 (m, 1H), 4.10-4.44 (m, 3H), 4.67 (m, 1H), 5.12 (m, 2H), 5.49 (d, 1H), 7.25-7.45 (m, 5H).

### Ausführungsbeispiele

Die Synthese von Ausführungsbeispielen kann ausgehend von partiell geschützten Biphenomycin-Derivaten (wie z.B. 21A) erfolgen.

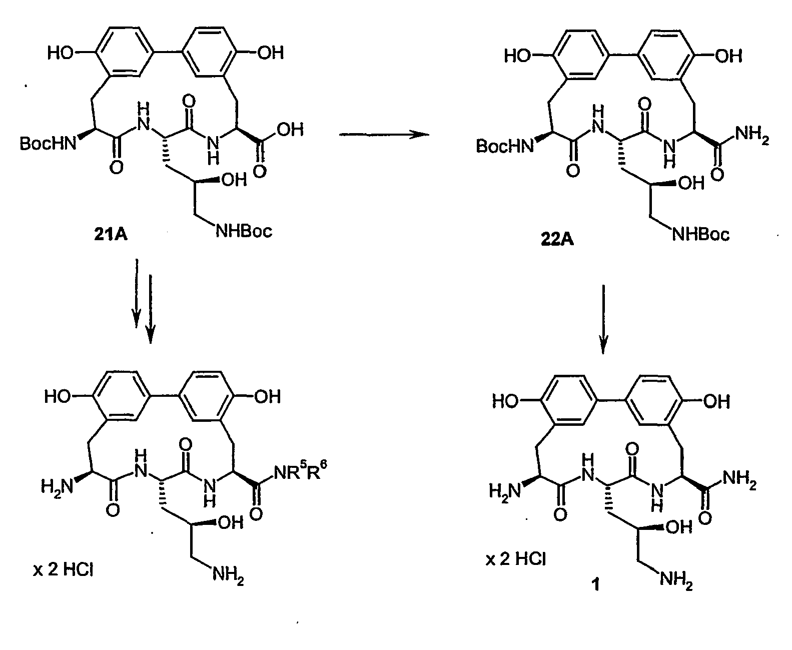

### Beispiel 1

### (8S,11S,14S)-14-Amino-11-[(2R)-3-amino-2-hydroxypropyl]-5,17-dihydroxy-10,13-dioxo-9,12-diazatricyclo[14.3.1.1^{2,6}]henicosa-1(20),2(21),3,5,16,18-hexaen-8-carbonsäureamid Dihydrochlorid

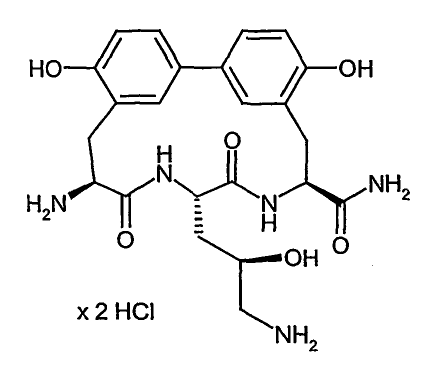

### Methode A:

Zu einer Lösung von 2.15 mg (3.2 µmol) *tert*-Butyl-(2*R*)-3-[(8*S*,11*S*,14*S*)-8-(aminocarbonyl)-14-[(*tert*-butoxycarbonyl)amino]-5,17-dihydroxy-10,13-dioxo-9,12-diazatricyclo[14.3.1.1^{2,6}]hericosa-1(20),2(21),3,5,16,18-hexaen-11-yl]-2-hydroxy-propylcarbamat (Beispiel 22A) in trockenem Dioxan (p.a., 1.0 ml) wird unter Argon eine 4 M Lösung von Salzsäuregas in Dioxan (1.0 ml) zugetropft. Nach ca. 30 min ist vollständiger Umsatz erreicht. Das Reaktionsgemisch wird eingefroren und mittels Gefriertrocknung von Lösungsmitteln befreit. Die Aufreinigung erfolgt durch Gelchromatographie [Sephadex LH-20; Methanol / konzentrierte Salzsäure (1:0.0001) dotiert mit Natriumdisulfit], wobei 1.4 mg (80% d. Th.) Produkt erhalten werden. HPLC-UV-Vis (Methode 14): Rₜ = 3.09 min.
λₘₐₓ (qualitativ) = ~204 nm (s), 269 (m), ~285 (sh) (H₂O/Acetonitril + 0.01% TFA [7:3]).
¹H-NMR (500 MHz, CD₃OD): δ = 1.79 (ddd, 1H, J = 13.6, 9.2, 5.9Hz), 1.99 (ddd, 1H, J = 13.6, 9.6, 4.0Hz), 2.82 (dd, 1H, J = 12.8, 9.6Hz), 2.87 (dd, 1H, J = 17.1, 12.1Hz), 3.04 (dd, 1H, J =12.8, 2.9Hz), 3.11 (dd, 1H, J =14.8, 3.0Hz), 3.38 (dd, 1H, J = 16.9, 1.9Hz), 3.57 (dd, 1H, J = 11.7, 5.4Hz), 3.92 (tt, 1H, J = 9.4, 3.5Hz), 4.23 (dd, 1H, J = 4.9, 3.0Hz), 4.90 (m, 1H), 4.91 (m, 1H), 6.79 (d, 1H, J = 8.3Hz), 6.85 (d, 1H, J = 8.4Hz), 7.10 (d, 1H, J = 2.3Hz), 7.25 (dd, 1H, J = 8.3, 2.3Hz), 7.36 (dd, 1H, J = 8.5, 2.4Hz), 7.44 (d, 1H, J = 2.1Hz).
¹³C NMR (125.5 MHz, CD₃OD): δ = 30.3, 30.8, 39.5, 45.4, 50.6, 53.8, 55.3, 65.3, 115.6, 116.3, 120.8, 125.3, 126.2, 126.8, 127.0, 130.9, 132.7, 133.5, 155.0, 155.7, 168.4, 172.8, 177.0.
LC-HR-FT-ICR-MS (Methode 13): ber. für C₂₃H₃₀N₅O₆ [M+H]⁺ 472.2191 gef. 472.2191.

### Methode B:

Unter Argon werden 14.8 mg (0.02 mmol) *tert*-Butyl-(2*R*)-3-[(8*S*,11*S*,14*S*)-8-(aminocarbonyl)-14-[(*tert*-butoxycarbonyl)amino]-5,17-dihydroxy-10,13-dioxo-9,12-diazatricyclo[14.3.1.1^{2,6}]henicosa-1(20),2(21),3,5,16,18-hexaen-11-yl]-2-hydroxypropylcarbamat (Beispiel 22A) in 0.5 ml Dioxan vorgelegt. Die Mischung wird auf 0°C abgekühlt und mit 0.8 ml 4 M Salzsäurelösung in Dioxan tropfenweise versetzt. Nach 45 min wird das Gemisch im Vakuum einrotiert und der Rückstand noch zweimal mit Dioxan aufgenommen und erneut im Vakuum einrotiert. Das Produkt wird am Hochvakuum getrocknet.
Ausbeute: 12 mg (100 % d. Th.).
HPLC (Methode 8): Rₜ = 4.87 min.
MS (EI): m/z = 472 (M+H-2HCl)⁺.
¹H-NMR (400 MHz, D₂O): δ = 0.58-0.67 (m, 2H), 1.65-1.86 (m, 3H), 1.88-1.98 (m, 1H), 2.03-2.13 (m, 1H), 2.87-3.02 (m, 4H), 3.09-3.19 (m, 2H), 3.38 (d, 1H), 3.59-3.69 (m, 2H), 3.88-3.96 (m, 1H), 4.46-4.51 (m, 1 H), 4.85-5.01 (m, 5H), 6.98 (dd, 2H), 7.05 (dd, 1H), 7.36 (s, 1H), 7.43 (dd, 1H), 7.50 (dd, 1H).

### Beispiel 2

### (8S,11S,14S)-14-Amino-11-[(2R)-3-amino-2-hydroxypropyl]-N-benzyl-5,17-dihydroxy-10,13-dioxo-9,12-diazatricyclo[14.3.1.1^{2,6}]henicosa-1(20),2(21),3,5,16,18-hexaen-8-carboxamid Dihydrochlorid

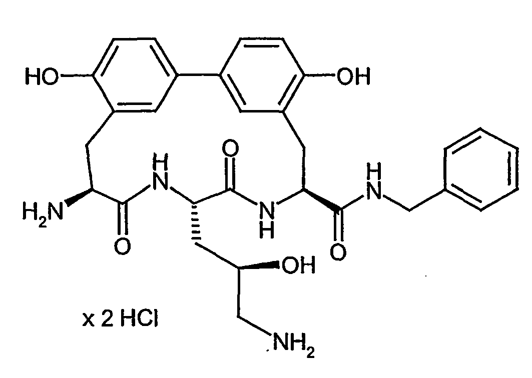

Zu einer Lösung von *tert*-Butyl-(2*R*)-3-[(8*S*,11*S*,14*S*)-8-[(benzylamino)carbonyl]-14-[(*tert*-butoxycarbonyl)amino]-5,17-dihydroxy-10,13-dioxo-9,12-diazatricyclo-[14.3.1.1^{2,6}]henicosa-1(20),2(21),3,5,16,18-hexaen-11-yl]-2-hydroxypropylcarbamat (Beispiel 23A) in 0.5 ml 1,4-Dioxan werden unter Eiskühlung 0.5 ml 4 N Salzsäure-Lösung in Dioxan getropft. Die Eiskühlung wird entfernt und die Mischung wird 2 h bei RT gerührt, bevor im Vakuum eingeengt und im Hochvakuum getrocknet wird. Der Rückstand wird in einem Gemisch von Dichlormethan und Methanol aufgenommen und die Lösungsmittel über Nacht abgedampft.
LC-MS (Methode 7): Rₜ = 2.02 min.
MS (ESI-pos): m/z = 562 (M+H-2HCl)⁺.
¹H-NMR (400MHz, D₂O): δ = 1.70-1.81 (m, 1H), 1.82-1.91 (m, 1H), 2.71-2.84 (m, 2H), 2.89-2.97 (m, 2H), 3.18 (d, 1H), 3.42-3.53 (m, 1H), 3.67-3.73 (m, 1H), 4.21-4.26 (m, 1H), 4.29 (d, 1H), 4.27-4.33 (m, 1H), 4.34 (d, 1H), 6.80-6.83 (m, 2H), 6.89 (s, 1H), 7.19-7.24 (m, 4H), 7.26-7.31 (m, 3H), 7.35 (d, 1H).

Analog zu Beispiel 1 können die in der folgenden Tabelle aufgeführten Beispiele 3 bis 14 hergestellt werden.

| **Beispiel-Nr.** | **Struktur** | **Analytische Daten** |
|---|---|---|
| **3** | 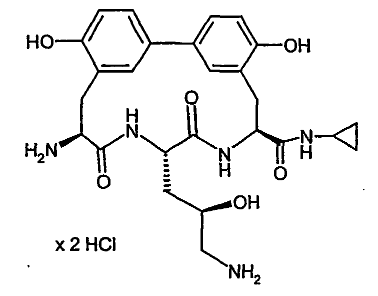 | LC-MS (Methode 20): |
| | | Rₜ = 1.13 min. |
| | | MS (ESIpos): m/z = |
| | | 512 (M+H)⁺ |
| **4** | 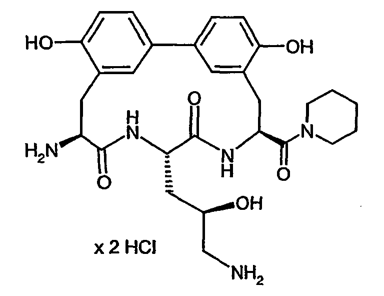 | LC-MS (Methode 20): |
| | | Rₜ = 2.09 min. |
| | | MS (ESIpos): m/z = |
| | | 540 (M+H)⁺ |
| **5** | 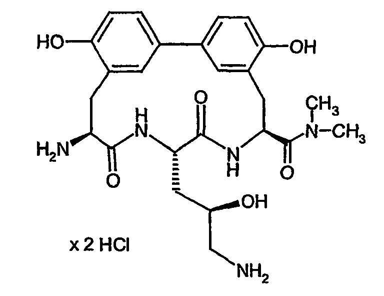 | LC-MS (Methode 20): |
| | | Rₜ = 1.44 min. |
| | | MS (ESIpos): m/z = |
| | | 500 (M+H)⁺ |
| **6** | 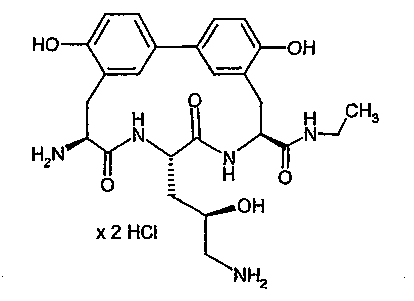 | LC-MS (Methode 20): |
| | | Rₜ = 0.35 min. |
| | | MS (ESIpos): m/z = |
| | | 500 (M+H)⁺ |
| **7** | 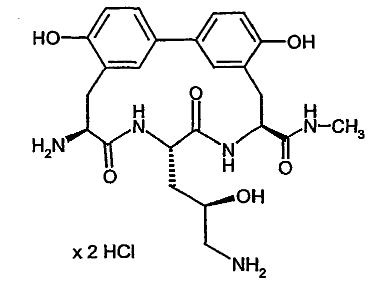 | LC-MS (Methode 20): |
| | | Rₜ = 0.32 min. |
| | | MS (ESIpos): m/z = |
| | | 486 (M+H)⁺ |
| **8** | 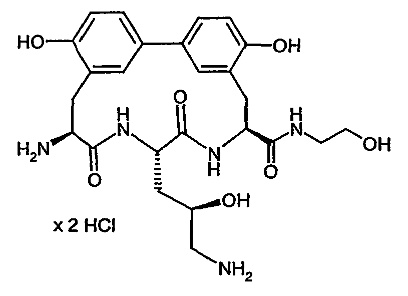 | LC-MS (Methode 20): |
| | | Rₜ = 0.35 min. |
| | | MS (ESIpos): m/z = |
| | | 516 (M+H)⁺ |
| **9** | 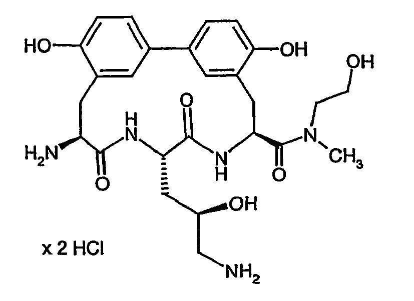 | LC-MS (Methode 21): |
| | | Rₜ = 2.79 min. |
| | | MS (ESIpos): m/z = |
| | | 530 (M+H)⁺ |
| **10** | 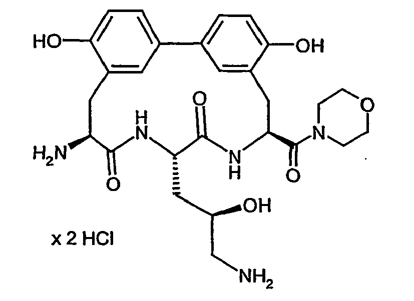 | LC-MS (Methode 21): |
| | | Rₜ = 2.85 min. |
| | | MS (ESIpos): m/z = |
| | | 542 (M+H)⁺ |
| **11** | 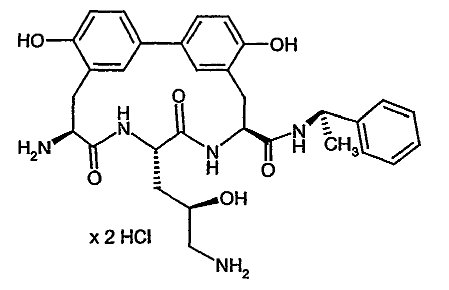 | LC-MS (Methode 21): |
| | | Rₜ = 3.09 min. |
| | | MS (ESIpos): m/z = |
| | | 576 (M+H)⁺ |
| **12** | 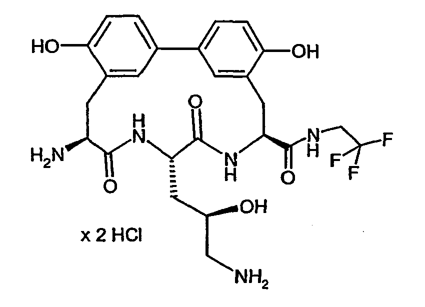 | LC-MS (Methode 21): |
| | | Rₜ = 2.88 min. |
| | | MS (ESIpos): m/z = |
| | | 554 (M+H)⁺ |
| **13** | 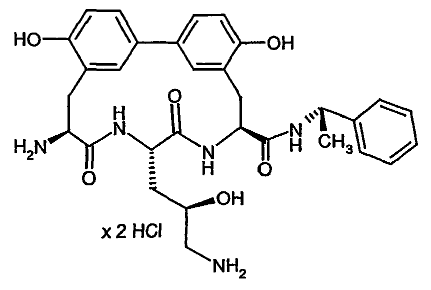 | LC-MS (Methode 21): |
| | | Rₜ = 3.10 min. |
| | | MS (ESIpos): m/z = |
| | | 576 (M+H)⁺ |
| **14** | 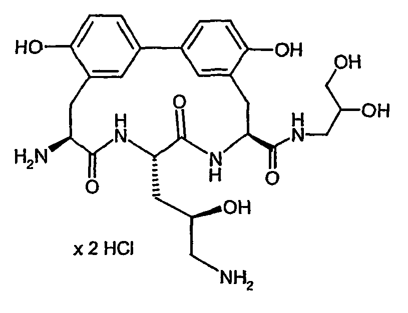 | ¹H-NMR (400MHz, |
| | | D₂O): |
| | | δ = 1.78-1.88 (m, 1H), |
| | | 1.93-2.00 (m, 1H), |
| | | 2.78-2.88 (m, 2H), |
| | | 2.98-3.06 (m, 2H), |
| | | 3.17-3.30 (m, 2H), |
| | | 3.33 (d, 1H), 3.42- |
| | | 3.57 (m, 3H), 3.73- |
| | | 3.84 (m, 1H), 4.68- |
| | | 4.82 (m, 2H), 6.86 (d, |
| | | 1H), 6.87 (d, 1H), 7.24 |
| | | (m, 1H), 7.32 (d, 1H), |
| | | 7.40 (d, 1H). |
| | | MS (EI): m/z = 546 |
| | | (M+H)⁺, 568 (M+Na)⁺ |

### Beispiel 15

### N-{[(8S,11S,14S)-14-amino-11-[(2R)-3-amino-2-hydroxrypropyl]-5,17-dihydroxy-10,13-dioxo-9,12-diazatricyclo[14.3.1.1^{2,6}]henicosa-1(20),2(21),3,5,16,18-hexaen-8-yl]carbonyl}-L-phenylalanin Dihydrochlorid

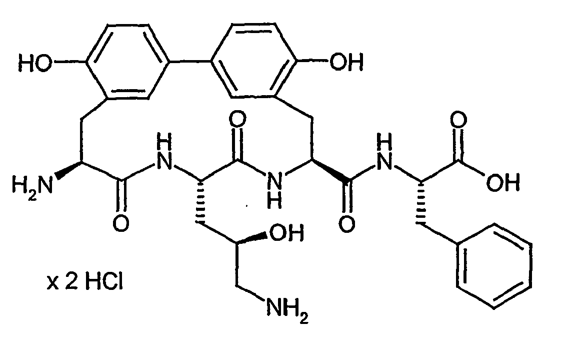

0.02 g (0.02 mmol) Benzyl-N-{[(*8S,11S,14S*)-5,17-bis(benzyloxy)-14-{[(benzyloxy)-carbonyl]amino}-11-((2*R*)-3-{[(benzyloxy)carbonyl]amino}-2-hydroxypropyl)-10,13-dioxo-9,12-diazatricyclo[14.3.1.1^{2.6}]henicosa-1(20),2(21),3,5,16,18-hexaen-8-yl]carbonyl}-L-phenylalaninat werden in 6 ml Essigsäure:Wasser:Ethanol (4:1:1) suspendiert und mit 0.01 g Pd/C versetzt. Man hydriert bei Normaldruck unter kräftigem Rühren für 48 h. Die Reaktionslösung wird filtriert. Der Rückstand wird mit 0.25 ml 0.1 N Salzsäure versetzt. Nach Einengen am Rotationsverdampfer trocknet man im Vakuum. Zur weiteren Reinigung wird in Isopropanol:Diethylether (1:1) ausgerührt.
Ausbeute: 0.0037 g (28 % d. Th.)
LC-MS (Methode 15): Rₜ = 1.27 min.
MS (EI): m/z = 620 (M+H)⁺

In Analogie zu Beispiel 15 können die in der folgenden Tabelle aufgeführten Beispiele 16 und 17 hergestellt werden.

| **Beispiel-Nr.** | **Struktur** | **Analytische Daten** |
|---|---|---|
| **16** | 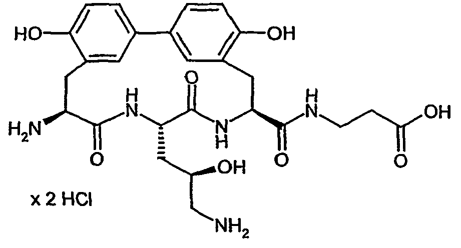 | LC-MS (Methode 15): |
| | | Rₜ = 0.701 min. |
| | | MS (EI): m/z = 544 |
| | | (M+H)⁺ |
| **17** | 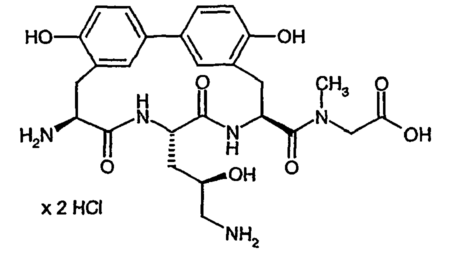 | LC-MS (Methode 17): |
| | | Rₜ = 1.55 min. |
| | | MS (EI): m/z = 544 |
| | | (M+H)⁺ |

Ausgehend von (8S,11S,14S)-14-[(tert-Butoxycarbonyl)-amino-11-[3-[(tert-butoxycarbonyl)amino]propyl}-5,17-dihydroxy-10,13-dioxo-9,12-diazatricyclo[14.3.1.1^{2,6}]-henicosa-1(20),2(21),3,5,16,18-hexaen-8-carbonsäure (Beispiel 83A) können die in der folgenden Tabelle aufgeführten L-Ornithin-haltigen Amide hergestellt werden (Beispiele 18 bis 24).

| **Beispiel-Nr.** | **Struktur** | **Analytische Daten** |
|---|---|---|
| **18** | 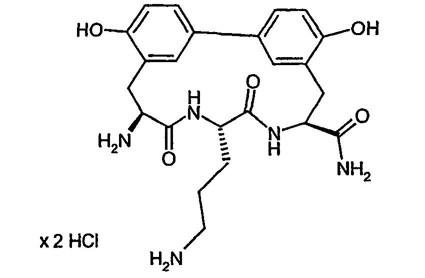 | LC-MS (Methode 20): |
| | | Rₜ = 0.33 min |
| | | MS (EI): m/z = 456 |
| | | (M+H)⁺ |
| **19** | 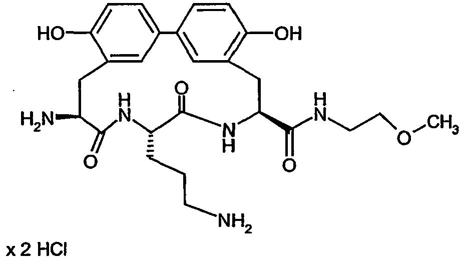 | LC-MS (Methode 19): |
| | | Rₜ =1.54 min. |
| | | MS (EI): m/z = 514 |
| | | (M+H)⁺ |
| **20** | 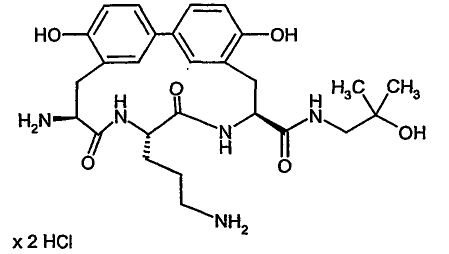 | LC-MS (Methode 18): |
| | | Rₜ = 0.66 min. |
| | | MS (EI): m/z = 528 |
| | | (M+H)⁺ |
| **21** | 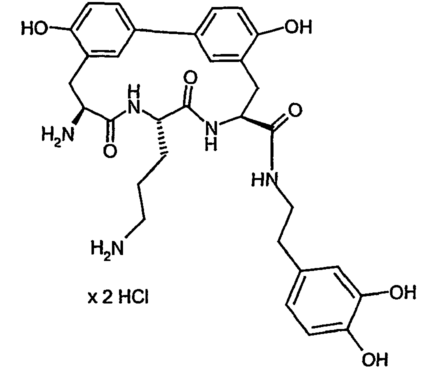 | LC-MS (Methode 19): |
| | | Rₜ =1.6 min. |
| | | MS (EI): m/z = 592 |
| | | (M+H)⁺ |
| **22** | 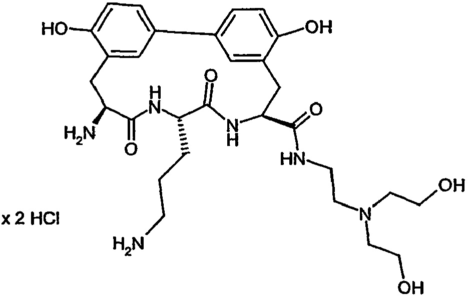 | MS (EI): m/z = 587 |
| | | (M+H)⁺ |
| **23** | 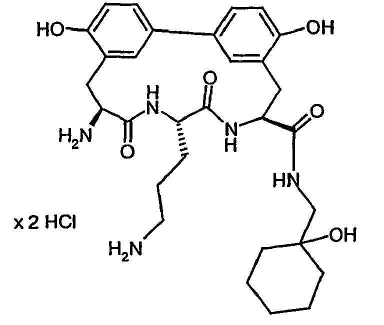 | LC-MS (Methode 18): |
| | | Rₜ = 1.21 min. |
| | | MS (EI): m/z = 568 |
| | | (M+H)⁺ |
| **24** | 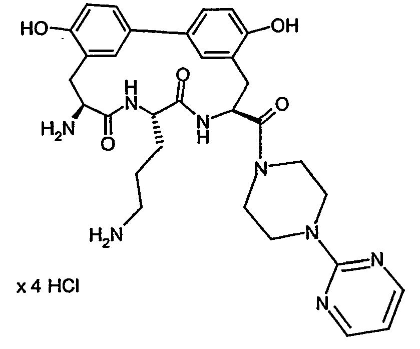 | LC-MS (Methode 18): |
| | | Rₜ =1.27 min. |
| | | MS (EI): m/z = 603 |
| | | (M+H)⁺ |

In Analogie zu Beispiel 15 können die in der folgenden Tabelle aufgeführten Beispiele 25 und 26 hergestellt werden.

| **Beispiel-Nr.** | **Struktur** | **Analytische Daten** |
|---|---|---|
| **25** | 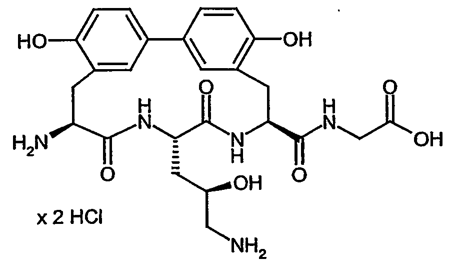 | LC-MS (Methode 22): |
| | | Rₜ = 0.30 min |
| | | MS (EI): m/z = 530 |
| | | (M+H)⁺ |
| **26** | 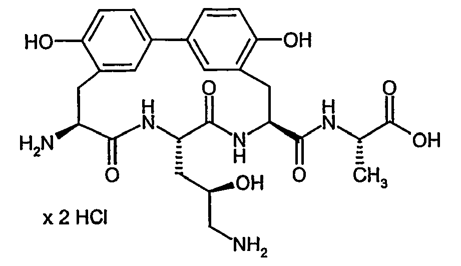 | LC-MS (Methode 15): |
| | | Rₜ = 0.88 min |
| | | MS (EI): m/z = 544 |
| | | (M+H)⁺ |

Ausgehend von (8S,11S,14S)-14-[(tert-Butoxycarbonyl)-amino-11-[3-[(tert-butoxycarbonyl)amino]propyl}-5,17-dihydroxy-10,13-dioxo-9,12-diazatricyclo[14.3.1.1^{2,6}]-henicosa-1(20),2(21),3,5,16,18-hexaen-8-carbonsäure (Beispiel 83A) können die in der folgenden Tabelle aufgeführten L-Ornithin-haltigen Amide hergestellt werden (Beispiele 27 bis 33).

| **Beispiel-Nr.** | **Struktur** | **Analytische Daten** |
|---|---|---|
| **27** | 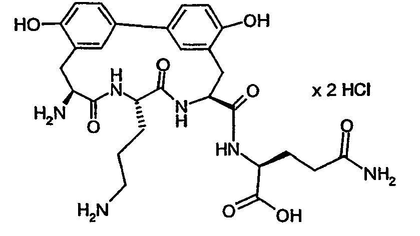 | LC-MS (Methode 15): |
| | | Rₜ = 0.72 min. |
| | | MS (EI): m/z = 584 |
| | | (M+H)⁺ |
| **28** | 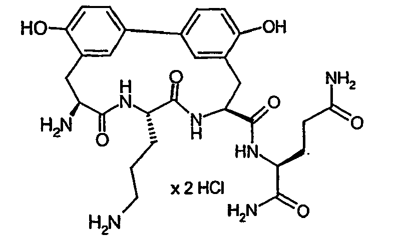 | LC-MS (Methode 15): |
| | | Rₜ = 0.69 min |
| | | MS (EI): m/z = 583 |
| | | (M+H)⁺ |
| **29** | 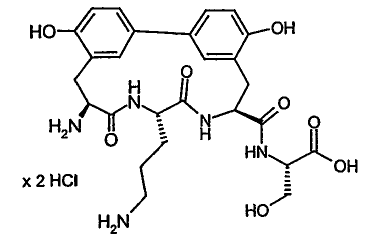 | LC-MS (Methode 15): |
| | | Rₜ = 0.72 min. |
| | | MS (EI): m/z = 543 |
| | | (M+H)⁺ |
| **30** | 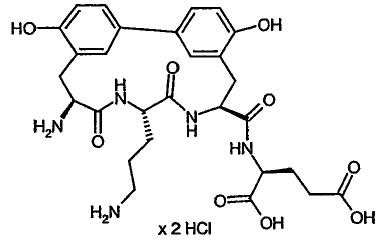 | LC-MS (Methode 15): |
| | | Rₜ = 0.83 min. |
| | | MS (EI): m/z = 585 |
| | | (M+H)⁺ |
| **31** | 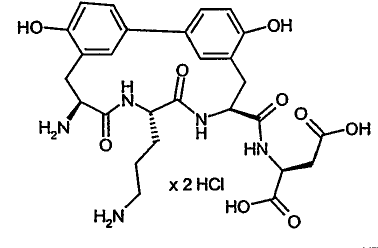 | LC-MS (Methode 23): |
| | | Rₜ = 1.04 min. |
| | | MS (EI): m/z = 571 |
| | | (M+H)⁺ |
| **32** | 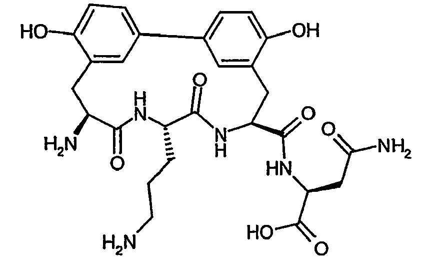 | LC-MS (Methode 23): |
| | | Rₜ = 1.00 min. |
| | | MS (EI): m/z = 570 |
| | | (M+H)⁺ |
| **33** | 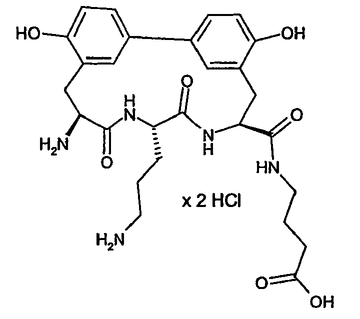 | LC-MS (Methode 24): |
| | | Rₜ = 0.27 min. |
| | | MS (EI): m/z = 541 |
| | | (M+H)⁺ |

### Beispiel 34

### (8S, 11S, 14S)-14-Amino-11-[(2R)-3-amino-2-hydroxypropyl]-5,17-dihydroxy-9-methyl-10,13-dioxo-9,12-diazatricyclo[14.3.1.1^{2,6}]henicosa-1(20),2(21),3,15,16,18-hexaen-carbonsäureamid-Dihydrochlorid

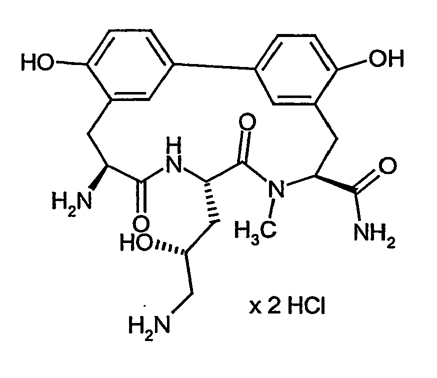

11 mg (0.01 mmol) der Verbindung aus Beispiel 122A werden in 10 ml Eisessig/Ethanol/Wasser (4/1/1) gelöst, mit 6 mg Pd-C (10%-ig)-Katalysator versetzt und bei Raumtemperatur über Nacht hydriert. Nach Abfiltrieren des Katalysators dampft man den Rückstand im Vakuum zur Trockne ein, versetzt mit 0.1 N Salzsäure und dampft erneut zur Trockne ein.
Ausbeute: 7 mg (96% d. Th.)
MS (EI): m/z = 485 (M+H)⁺

Analog der Vorschrift des Beispiels 34 kann das in der folgenden Tabelle aufgeführte Beispiel 35 hergestellt werden:

| **Beispiel-Nr.** | **Struktur** | **Analytische Daten** |
|---|---|---|
| **35** | 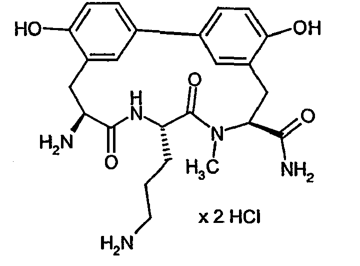 | LC-MS (Methode 22): |
| | | Rₜ = 1.46 min. |
| | | MS (EI): m/z = 469 |
| | | (M+H)⁺ |

In Analogie zu Beispiel 1 können die in der folgenden Tabelle aufgeführten Beispiele 36 und 37 hergestellt werden.

| **Beispiel-Nr.** | **Struktur** | **Analytische Daten** |
|---|---|---|
| **36** | 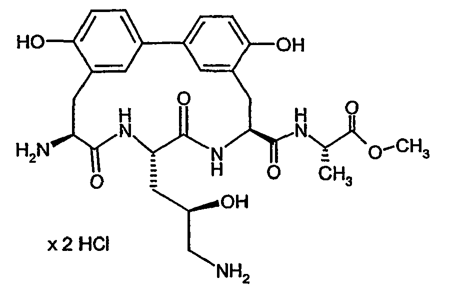 | LC-MS(Methode 15): |
| | | Rₜ=1.52 min |
| | | MS (EI): m/z = 558 |
| | | (M+H)⁺ |
| **37** | 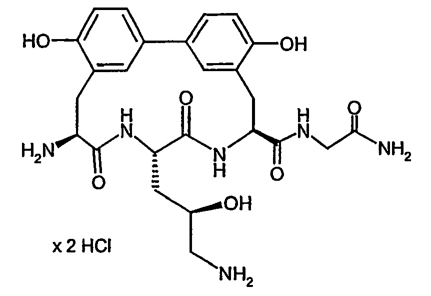 | LC-MS(Methode 24): |
| | | Rₜ= 0.42 min |
| | | MS (EI): m/z = 529 |
| | | (M+H)⁺ |

In Analogie zu Beispiel 15 können die in der folgenden Tabelle aufgeführten Beispiele 38 bis 40 hergestellt werden.

| **Beispiel-Nr.** | **Struktur** | **Analytische Daten** |
|---|---|---|
| **38** | 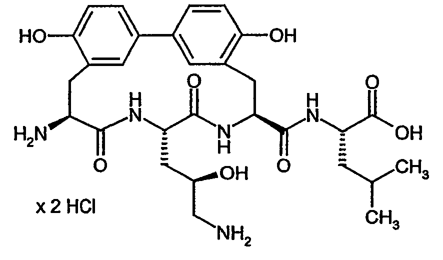 | LC-MS (Methode 23): |
| | | Rₜ = 0.95 min. |
| | | MS (EI): m/z = 586 |
| | | (M+H)⁺ |
| **39** | 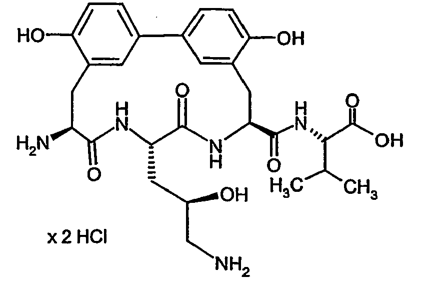 | LC-MS (Methode 24): |
| | | Rₜ= 0.80 min. |
| | | MS (EI): m/z = 572 |
| | | (M+H)⁺ |
| **40** | 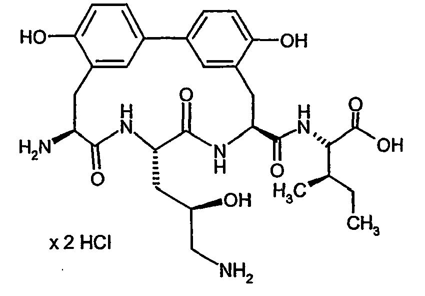 | LC-MS (Methode 24): |
| | | Rₜ = 0.94 min. |
| | | MS (EI): m/z = 586 |
| | | (M+H)⁺ |

### A. Bewertung der physiologischen Wirksamkeit

Die *in vitro*-Wirkung der erfindungsgemäßen Verbindungen kann in folgenden Assays gezeigt werden:

### In vitro Transkription-Translation mit E. coli Extrakten

Zur Herstellung eines S30-Extraktes werden logarithmisch wachsende *Escherichia coli* MRE 600 (M. Müller; University Freiburg) geerntet, gewaschen und wie beschrieben für den *in vitro* Transkriptions-Translations-Test eingesetzt (Müller, M. and Blobel, G. Proc Natl Acad Sci U S A (1984) 81, pp.7421-7425).

Dem Reaktionsmix des *in vitro* Transkriptions-Translations-Tests werden zusätzlich 1 µl cAMP (11,25 mg/ml) je 50 µl Reaktionsmix zugegeben. Der Testansatz beträgt 105 µl, wobei 5 µl der zu testenden Substanz in 5%igem DMSO vorgelegt werden. Als Transkriptionsmatrize werden 1 µg/100µl Ansatz des Plasmides pBESTLuc (Promega, Deutschland) verwendet. Nach Inkubation für 60 min bei 30°C werden 50 µl Luziferinlösung (20 mM Tricine, 2,67 mM MgSO4, 0,1 mM EDTA, 33,3 mM DTT pH 7,8, 270 µM CoA, 470 µM Luziferin, 530 µM ATP) zugegeben und die entstehende Biolumineszenz für 1 Minute in einem Luminometer gemessen. Als IC₅₀ wird die Konzentration eines Inhibitors angegeben, die zu einer 50%igen Inhibition der Translation von Firefly Luziferase führt.

### In vitro Transkription-Translation mit S. aureus Extrakten

### Konstruktion eines S. aureus Luziferase Reporterplasmids

Zur Konstruktion eines Reporterplasmids, welches in einem *in vitro* Transkriptions-Translations-Assay aus *S. aureus* verwendet werden kann, wird das Plasmid pBESTluc (Promega Corporation, USA) verwendet. Der in diesem Plasmid vor der Firefly Luziferase vorhandene *E. coli tac* Promoter wird gegen den *capA1* Promoter mit entsprechender Shine-Dalgamo Sequence aus *S*. *aureus* ausgetauscht. Dazu werden die Primer CAPFor 5'-CGGCC-AAGCTTACTCGGATCCAGAGTTTGCAAAATATACAGGGGATTATATATAATGGAAAACAAGAAAGGAAAATAGGAGGTTTATATGGAAGACGCCA-3' und CAPRev 5'-GTCATCGTCGGGAAGACCTG-3' verwendet. Der Primer CAPFor enthält den *capA1* Promotor, die Ribosomenbindestelle und die 5'-Region des Luziferase Gens. Nach PCR unter Verwendung von pBESTluc als Template kann ein PCR-Produkt isoliert werden, welches das Firefly Luziferase Gen mit dem fusionierten *capA1* Promotor enthält. Dieses wird nach einer Restriktion mit ClaI und HindIII in den ebenfalls mit ClaI und HindIII verdauten Vektor pBESTluc ligiert. Das entstandene Plasmid pla kann in *E. coli* repliziert werden und als Template im *S. aureus in vitro* Transkriptions-Translations-Test verwendet werden.

### Herstellung von S30 Extrakten aus S. aureus

Sechs Liter BHI Medium werden mit einer 250 ml Übernachtkultur eines *S. aureus* Stammes inokuliert und bei 37°C bis zu einer OD600nm von 2-4 wachsen gelassen. Die Zellen werden durch Zentrifugation geerntet und in 500 ml kaltem Puffer A (10 mM Tris-acetat, pH 8,0, 14 mM Mg-acetat, 1 mM DTT, 1 M KCl) gewaschen. Nach erneutem Abzentrifugieren werden die Zellen in 250 ml kaltem Puffer A mit 50 mM KCl gewaschen und die erhaltenen Pellets bei -20°C für 60 min eingefroren.

Die Pellets werden in 30 bis 60 min auf Eis aufgetaut und bis zu einem Gesamtvolumen von 99 ml in Puffer B (10 mM Tris-acetat, pH 8,0, 20 mM Mg-acetat, 1 mM DTT, 50 mM KCl) aufgenommen. Je 1,5 ml Lysostaphin (0,8 mg/ml) in Puffer B werden in 3 vorgekühlte Zentrifugenbecher vorgelegt und mit je 33 ml der Zellsuspension vermischt. Die Proben werden für 45 bis 60 min bei 37°C unter gelegentlichem Schütteln inkubiert, bevor 150 µl einer 0,5 M DTT Lösung zugesetzt werden. Die lysierten Zellen werden bei 30.000 x g 30 min bei 4°C abzentrifugiert. Das Zellpellet wird nach Aufnahme in Puffer B unter den gleichen Bedingungen nochmals zentrifugiert und die gesammelten Überstände werden vereinigt. Die Überstände werden nochmals unter gleichen Bedingungen zentrifugiert und zu den oberen 2/3 des Überstandes werden 0,25 Volumen Puffer C (670 mM Tris-acetat, pH 8,0, 20 mM Mg-acetat, 7 mM Na₃-Phosphoenolpyruvat, 7 mM DTT, 5,5 mM ATP, 70 µM Aminosäuren (complete von Promega), 75 µg Pyruvatkinase (Sigma, Deutschland)/ml gegeben. Die Proben werden für 30 min bei 37°C inkubiert. Die Überstände werden über Nacht bei 4°C gegen 2 1 Dialysepuffer (10 mM Tris-acetat, pH 8,0, 14 mM Mg-acetat, 1 mM DTT, 60 mM K-acetat) mit einem Pufferwechsel in einem Dialyseschlauch mit 3500 Da Ausschluss dialysiert. Das Dialysat wird auf eine Proteinkonzentration von etwa 10 mg/ml konzentriert, indem der Dialyseschlauch mit kaltem PEG 8000 Pulver (Sigma, Deutschland) bei 4°C bedeckt wird. Die S30 Extrakte können aliquotiert bei -70°C gelagert werden.

### Bestimmung der IC₅₀ im S. aureus in vitro Transcriptions-Translations-Assay

Die Inhibition der Proteinbiosynthese der Verbindungen kann in einem *in vitro* Transkriptions-Translations-Assay gezeigt werden. Der Assay beruht auf der zellfreien Transkription und Translation von Firefly Luziferase unter Verwendung des Reporterplasmids pla als Template und aus *S. aureus* gewonnenen zellfreien S30 Extrakten. Die Aktivität der entstandenen Luziferase kann durch Lumineszenzmessung nachgewiesen werden.

Die Menge an einzusetzenden S30 Extrakt bzw. Plasmid pla muss für jede Präparation erneut ausgetestet werden, um eine optimale Konzentration im Test zu gewährleisten. 3 µl der zu testenden Substanz gelöst in 5% DMSO werden in eine MTP vorgelegt. Anschließend werden 10 µl einer geeignet konzentrierten Plasmidlösung p1a zugegeben. Anschließend werden 46 µl eines Gemisches aus 23 µl Premix (500 mM K-acetat, 87,5 mM Tris-acetat, pH 8,0, 67,5 mM Ammoniumacetat, 5 mM DTT, 50 µg Folsäure/ml, 87,5 mg PEG 8000/ml, 5 mM ATP, 1,25 mM je NTP, 20 µM je Aminosäure, 50 mM PEP (Na₃-Salz), 2,5 mM cAMP, 250 µg je *E. coli* tRNA/ml) und 23 µl einer geeigneten Menge *S. aureus* S30 Extrakt zugegeben und vermischt. Nach Inkubation für 60 min bei 30°C werden 50 µl Luziferinlösung (20 mM Tricine, 2,67 mM MgSO₄, 0,1 mM EDTA, 33,3 mM DTT pH 7,8, 270 µM CoA, 470 µM Luziferin, 530 µM ATP) und die entstehende Biolumineszenz für 1 min in einem Luminometer gemessen. Als IC₅₀ wird die Konzentration eines Inhibitors angegeben, die zu einer 50%igen Inhibition der Translation von Firefly Luziferase führt.

### Bestimmung der Minimalen Hemmkonzentration (MHK):

Die minimale Hemmkonzentration (MHK) ist die minimale Konzentration eines Antibiotikums, mit der ein Testkeim in seinem Wachstum über 18-24 h inhibiert wird. Die Hemmstoffkonzentration kann dabei nach mikrobiologischen Standardverfahren bestimmt werden (siehe z.B. The National Committee for Clinical Laboratory Standards. Methods for dilution antimicrobial susceptibility tests for bacteria that grow aerobically; approved standard-fifth edition. NCCLS document M7-A5 [ISBN 1-56238-394-9]. NCCLS, 940 West Valley Road, Suite 1400, Wayne, Pennsylvania 19087-1898 USA, 2000). Die MHK der erfindungsgemäßen Verbindungen wird im Flüssigdilutionstest im 96er-Mikrotiter-Platten-Maßstab bestimmt. Die Bakterienkeime wurden in einem Minimalmedium (18,5 mM Na₂HPO₄, 5,7 mM KH₂PO₄, 9,3 mM NH₄Cl, 2,8 mM MgSO₄, 17,1 mM NaCl, 0,033 µg/ml Thiaminhydrochlorid, 1,2 µg/ml Nicotinsäure, 0,003 µg/ml Biotin, 1% Glucose, 25 µg/ml von jeder proteinogenen Aminosäure mit Ausnahme von Phenylalanin; [H.-P. Kroll; unveröffentlicht]) unter Zusatz von 0,4% BH Bouillon kultiviert (Testmedium). Im Fall von *Enterococcus faecalis* ICB 27159 wird dem Testmedium hitzeinaktiviertes fötales Kälberserum (FCS; GibcoBRL, Deutschland) in einer Endkonzentration von 10% zugesetzt. Übernachtkulturen der Testkeime werden auf eine OD₅₇₈ von 0,001 (im Falle der Enterokokken auf 0,01) in frisches Testmedium verdünnt und 1:1 mit Verdünnungen der Testsubstanzen (Verdünnungsstufen 1:2) in Testmedium inkubiert (150 µl Endvolumen). Die Kulturen werden bei 37°C für 18-24 Stunden inkubiert; Enterokokken in Gegenwart von 5% CO₂.

Die jeweils niedrigste Substanzkonzentration, bei der kein sichtbares Bakterienwachstum mehr auftrat, wird als MHK definiert. Die MHK-Werte in µM einiger erfindungsgemäßer Verbindungen gegenüber einer Reihe von Testkeimen sind in der nachstehenden Tabelle beispielhaft aufgeführt. Die Verbindungen zeigen eine abgestufte antibakterielle Wirkung gegen die meisten der Testkeime.

**Tabelle A**

| Bsp. Nr. | MHK *S. aureus* 133 | MHK *S. aureus* RN4220 | MHK *S. aureus* 25701 | MHK *E. faecalis.* ICB 27159 | MHK *B. catarrhalis* M3 | IC50 *E. coli* MRE600 Translation | IC50 *S. aureus* 133 Translation | IC50 *S. aureus* RN4220 Translation |
|---|---|---|---|---|---|---|---|---|
| 1 | 0,2 | 0,1 | 6,25 | 6,25 | 1,56 | 0,15 | 0,9 | 0,5 |
| 2 | 25 | 12,5 | 50 | 25 | 25 | 0,55 | 1,3-4,5 | 3,4 |
| 37 | 0,8 | ---- | ---- | ---- | ---- | ---- | 0,5 | ---- |

| | | | | | | | | |
|---|---|---|---|---|---|---|---|---|
| Alle Konzentrationsangaben in µM. | | | | | | | | |

### Systemische Infektion mit S. aureus 133

Die Eignung der erfindungsgemäßen Verbindungen zur Behandlung von bakteriellen Infektionen kann in verschiedenen Tiermodellen gezeigt werden. Dazu werden die Tiere im allgemeinen mit einem geeigneten virulenten Keim infiziert und anschließend mit der zu testenden Verbindung, die in einer an das jeweilige Therapiemodell angepassten Formulierung vorliegt, behandelt. Speziell kann die Eignung der erfindungsgemäßen Verbindungen zur Behandlung von bakteriellen Infektionen in einem Sepsismodell an Mäusen nach Infektion mit *S. aureus* demonstriert werden.

Dazu werden *S. aureus* 133 Zellen über Nacht in BH-Bouillon (Oxoid, Deutschland) angezüchtet. Die Übemachtkultur wurde 1:100 in frische BH-Bouillon verdünnt und für 3 Stunden hochgedreht. Die in der logarithmischen Wachstumsphase befindlichen Bakterien werden abzentrifugiert und 2 x mit gepufferter, physiologischer Kochsalzlösung gewaschen. Danach wird am Photometer (Dr. Lange LP 2W) eine Zellsuspension in Kochsalzlösung mit einer Extinktion von 50 Einheiten eingestellt. Nach einem Verdünnungsschritt (1:15) wird diese Suspension 1:1 mit einer 10 %-igen Mucinsuspension gemischt. Von dieser Infektionslösung wird 0,2 ml/20 g Maus i.p. appliziert. Dies entspricht einer Zellzahl von etwa 1-2 x 10E6 Keimen/Maus. Die i.v.-Therapie erfolgt 30 Minuten nach der Infektion. Für den Infektionsversuch werden weibliche CFW1-Mäuse verwendet. Das Überleben der Tiere wird über 6 Tage protokolliert. Das Tiermodell ist so eingestellt, daß unbehandelte Tiere innerhalb von 24 h nach der Infektion versterben. Für die Beispielverbindung 2 konnte in diesem Modell eine therapeutische Wirkung von ED100 = 1.25 mg/kg demonstriert werden.

### B. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.

### Herstellung:

Die Mischung aus Wirkstoff, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Pesskraft von 15 kN verwendet.

### Oral applizierbare Suspension:

### Zusammensetzung:

1000 mg der Verbindung von Beispiel 1, 1000 mg Ethanol (96%), 400 mg Rhodigel (Xanthan gum der Fa. FMC, Pennsylvania, USA) und 99 g Wasser.
Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers: Bis zum Abschluss der Quellung des Rhodigels wird ca. 6h gerührt.

## Patentansprüche

1. Verbindung der Formel 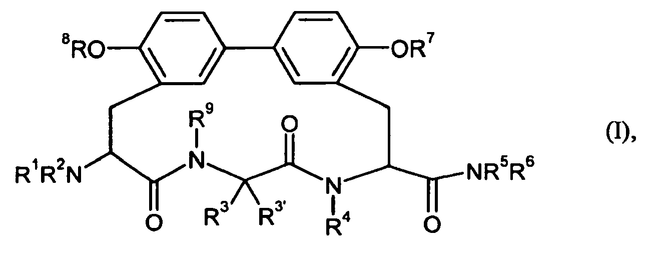 worin
R¹ gleich Wasserstoff, C₁-C₆-Alkyl, C₆-C₁₀-Aryl, 5- bis 10-gliedriges Heteroaryl, 4- bis 10-gliedriges Heterocyclyl, C₁-C₆-Alkylcarbonyl, C₆-C₁₀-Arylcarbonyl, 4- bis 10-gliedriges Heterocyclylcarbonyl, 5- bis 10-gliedriges Heteroarylcarbonyl, C₁-C₆-Alkoxycarbonyl, Aminocarbonyl, C₁-C₆-Alkylaminocarbonyl, C₁-C₆-Dialkylaminocarbonyl, C₁-C₆-Alkylsulfonyl, C₆-C₁₀-Arylsulfonyl, 4- bis 10-gliedriges Heterocyclylsulfonyl, 5- bis 10-gliedriges Heteroarylsulfonyl oder ein über die Carbonylgruppe der Aminosäure-Säurefunktion gebundener α-Aminosäurerest in der L- oder in der D-Konfiguration ist,
wobei R¹ außer Wasserstoff substituiert sein kann mit 0, 1, 2 oder 3 Substituenten R¹⁻¹, wobei die Substituenten R¹⁻¹ unabhängig voneinander ausgewählt werden aus der Gruppe bestehend aus Halogen, C₁-C₆-Alkyl, Trifluormethyl, Trifluormethoxy, Nitro, Cyano, Amino, C₁-C₆-Alkylamino, C₁-C₆-Dialkylamino, C₃-C₈-Cycloalkyl, C₆-C₁₀-Aryl, 5- bis 10-gliedriges Heteroaryl, 4- bis 10-gliedriges Heterocyclyl, Hydroxy, C₁-C₆-Alkoxy und Carboxyl,
R² gleich Wasserstoff oder C₁-C₆-Alkyl ist,
wobei R² außer Wasserstoff substituiert sein kann mit 0, 1, 2 oder 3 Substituenten R²⁻¹, wobei die Substituenten R²⁻¹ unabhängig voneinander ausgewählt werden aus der Gruppe bestehend aus Halogen, Amino, C₁-C₆-Alkylamino und C₁-C₆-Dialkylamino,
oder
R¹ und R² zusammen mit dem Stickstoffatom, an das sie gebunden sind, einen 4- bis 10-gliedrigen Heterocyclus bilden, der substituiert sein kann mit 0, 1 oder 2 Substituenten R¹⁻², wobei die Substituenten R¹⁻² unabhängig voneinander ausgewählt werden aus der Gruppe bestehend aus Halogen, Trifluormethyl, Amino, C₁-C₆-Alkylamino, C₁-C₆-Dialkylamino, C₃-C₈-Cycloalkyl, C₆-C₁₀-Aryl, 5- bis 10-gliedriges Heteroaryl, 4- bis 10-gliedriges Heterocyclyl, Hydroxy, C₁-C₆-Alkoxy, Carboxyl, C₁-C₆-Alkoxycarbonyl und Aminocarbonyl,
R³ gleich Wasserstoff, C₁-C₆-Alkyl oder die Seitengruppe einer natürlich vorkommenden α-Aminosäure in der L- oder in der D-Konfiguration ist, worin Alkyl substituiert sein kann mit 0, 1, 2 oder 3 Substituenten R³⁻¹, wobei die Substituenten R³⁻¹ unabhängig voneinander ausgewählt werden aus der Gruppe bestehend aus Trifluormethyl, Nitro, Amino, C₁-C₆-Alkylamino, C₁-C₆-Dialkylamino, C₃-C₈-Cycloalkyl, C₆-C₁₀-Aryl, 5- bis 10-gliedriges Heteroaryl, 4- bis 10-gliedriges Heterocyclyl, Hydroxy, C₁-C₆-Alkoxy, Carboxyl, C₁-C₆-Alkoxycarbonyl, Aminocarbonyl, C₁-C₆-Alkylaminocarbonyl, C₁-C₆-Dialkylaminocarbonyl, Guanidino und Amidino,
worin Cycloalkyl, Aryl, Heteroaryl und Heterocyclyl substituiert sein können mit 0, 1 oder 2 Substituenten R³⁻², wobei die Substituenten R³² unabhängig voneinander ausgewählt werden aus der Gruppe bestehend aus Halogen, C₁-C₆-Alkyl, Trifluormethyl und Amino,
und worin freie Aminogruppen in der Seitengruppe der Aminosäure mit C₁-C₆-Alkyl, C₂-C₆-Alkenyl, C₂-C₆-Alkinyl, C₃-C₈-Cycloalkyl, C₆-C₁₀-Aryl, 5- bis 10-gliedriges Heteroaryl, 4- bis 10-gliedriges Heterocyclyl, C₁-C₆-Alkylcarbonyl, C₆-C₁₀-Arylcarbonyl, 5- bis 10-gliedriges Heteroarylcarbonyl, 4- bis 10-gliedriges Hetero-cyclylcarbonyl, C₁-C₆-Alkoxycarbonyl, Aminocarbonyl, C₁-C₆-Alkyl-aminocarbonyl, C₁-C₆-Dialkylaminocarbonyl, C₆-C₁₀-Arylaminocarbonyl, C₁-C₆-Alkylsulfonyl, C₆-C₁₀-Arylsulfonyl, 4- bis 10-gliedriges Heterocyclylsulfonyl oder 5- bis 10-gliedriges Heteroarylsulfonyl substituiert sein können,
R^{3'} gleich Wasserstoff, C₁-C₆-Alkyl oder C₃-C₈-Cycloalkyl ist,
R⁴ gleich Wasserstoff, C₁-C₆-Alkyl oder C₃-C₈-Cycloalkyl ist,
R⁵ gleich Wasserstoff, C₁-C₆-Alkyl, C₂-C₆-Alkenyl, C₃-C₈-Cycloalkyl, C₆-C₁₀-Aryl, 5- bis 10-gliedriges Heteroaryl, 4- bis 10-gliedriges Heterocyclyl oder ein über die Aminogruppe der Aminosäure gebundener α-Aminosäurerest in der L- oder in der D-Konfiguration ist,
wobei R⁵ substituiert sein kann mit 0, 1, 2 oder 3 Substituenten R⁵⁻¹, wobei die Substituenten R⁵⁻¹ unabhängig voneinander ausgewählt werden aus der Gruppe bestehend aus Halogen, C₁-C₆-Alkyl, Trifluormethyl, Trifluormethoxy, Nitro, Cyano, Amino, C₁-C₆-Alkylamino, C₁-C₆-Dialkylamino, C₃-C₈-Cycloalkyl, C₆-C₁₀-Aryl, 5- bis 10-glied-riges Heteroaryl, 4- bis 10-gliedriges Heterocyclyl, Hydroxy, C₁-C₆-Alkoxy, Carboxyl, C₁-C₆-Alkoxycarbonyl, Aminocarbonyl, C₁-C₆-Alkylaminocarbonyl, C₁-C₆-Dialkylaminocarbonyl, Aminosulfonyl, C₁-C₆-Alkylaminosulfonyl, C₁-C₆-Dialkylaminosulfonyl, C₆-C₁₀-Arylaminosulfonyl, 4- bis 10-gliedriges Heterocyclylaminosulfonyl, 5-bis 10-gliedriges Heteroarylaminosulfonyl, Aminocarbonylamino, Hydroxycarbonylamino und C₁-C₆-Alkoxycarbonylamino,
worin Alkyl, Alkylamino, Dialkylamino, Cycloalkyl, Aryl, Heteroaryl und Heterocyclyl substituiert sein können mit 0, 1, 2 oder 3 Substituenten R⁵⁻², wobei die Substituenten R⁵⁻² unabhängig voneinander ausgewählt werden aus der Gruppe bestehend aus Hydroxy, Amino, Carboxyl und Aminocarbonyl,
R⁶ gleich Wasserstoff, C₁-C₆-Alkyl oder C₃-C₈-Cycloalkyl ist,
oder
R⁵ und R⁶ zusammen mit dem Stickstoffatom, an das sie gebunden sind, einen 4- bis 10-gliedrigen Heterocyclus bilden, der substituiert sein kann mit 0, 1, 2 oder 3 Substituenten R⁵⁻⁶, wobei die Substituenten R⁵⁻⁶ unabhängig voneinander ausgewählt werden aus der Gruppe bestehend aus Halogen, C₁-C₆-Alkyl, Trifluormethyl, Nitro, Amino, C₁-C₆-Alkylamino, C₁-C₆-Dialkylamino, C₃-C₈-Cycloalkyl, C₆-C₁₀-Aryl, halogeniertes C₆-C₁₀-Aryl, 5- bis 10-gliedriges Heteroaryl, 4- bis 10-gliedriges Heterocyclyl, Hydroxy, C₁-C₆-Alkoxy, Carboxyl, C₁-C₆-Alkylcarbonyl, C₁-C₆-Alkoxycarbonyl, Aminocarbonyl, C₁-C₆-Alkylaminocarbonyl und C₁-C₆-Dialkylaminocarbonyl,
R⁷ gleich Wasserstoff, C₁-C₆-Alkyl, C₁-C₆-Alkylcarbonyl oder C₃-C₈-Cycloalkyl ist,
R⁸ gleich Wasserstoff oder C₁-C₆-Alkyl ist und
R⁹ gleich Wasserstoff oder C₁-C₆-Alkyl ist
oder eines ihrer Salze, ihrer Solvate oder der Solvate ihrer Salze.

2. Verbindung nach Anspruch 1, **dadurch gekennzeichnet, dass** sie der Formel 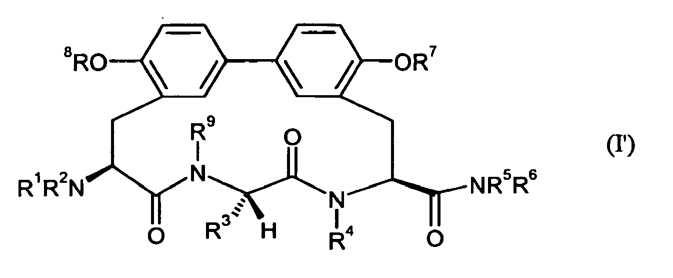 entspricht, worin R¹ bis R⁹ die gleiche Bedeutung wie in Formel (I) haben.

3. Verbindung nach Anspruch 1 oder 2, **dadurch gekennzeichnet, dass**
R¹ gleich Wasserstoff, C₁-C₆-Alkyl oder C₁-C₆-Alkylcarbonyl ist,
R² gleich Wasserstoff ist,
R³ gleich C₁-C₆-Alkyl oder die Seitengruppe einer natürlich vorkommenden α-Aminosäure in der L- oder in der D-Konfiguration ist, worin Alkyl substituiert sein kann mit 0, 1, 2 oder 3 Substituenten R³⁻¹, wobei die Substituenten R³⁻¹ unabhängig voneinander ausgewählt werden aus der Gruppe bestehend aus Trifluormethyl, Nitro, Amino, C₁-C₆-Alkylamino, C₁-C₆-Dialkylamino, C₃-C₈-Cycloalkyl, C₆-C₁₀-Aryl, 5- bis 10-gliedriges Heteroaryl, 4- bis 10-gliedriges Heterocyclyl, Hydroxy, C₁-C₆-Alkoxy, Carboxyl, C₁-C₆-Alkoxycarbonyl, Aminocarbonyl, C₁-C₆-Alkylaminocarbonyl, C₁-C₆-Dialkylaminocarbonyl, Guanidino und Amidino,
worin Cycloalkyl, Aryl, Heteroaryl und Heterocyclyl substituiert sein können mit 0, 1 oder 2 Substituenten R³⁻², wobei die Substituenten R³² unabhängig voneinander ausgewählt werden aus der Gruppe bestehend aus Halogen, C₁-C₆-Alkyl, Trifluormethyl und Amino,
und worin freie Aminogruppen in der Seitengruppe der Aminosäure mit C₁-C₆-Alkyl substituiert sein können,
R^{3'} gleich Wasserstoff, C₁-C₆-Alkyl oder C₃-C₈-Cycloalkyl ist,
R⁴ gleich Wasserstoff, C₁-C₆-Alkyl oder C₃-C₈-Cycloalkyl ist,
R⁵ gleich Wasserstoff, C₁-C₆-Alkyl, C₂-C₆-Alkenyl, C₃-C₈-Cycloalkyl, C₆-C₁₀-Aryl, 5- bis 10-gliedriges Heteroaryl, 4- bis 10-gliedriges Heterocyclyl oder ein über die Aminogruppe der Aminosäure gebundener α-Aminosäurerest in der L- oder in der D-Konfiguration ist,
wobei Alkyl, Alkenyl, Cycloalkyl, Aryl, Heteroaryl und Heterocyclyl substituiert sein können mit 0, 1, 2 oder 3 Substituenten R⁵⁻¹, wobei die Substituenten R⁵⁻¹ unabhängig voneinander ausgewählt werden aus der Gruppe bestehend aus Halogen, C₁-C₆-Alkyl, Trifluormethyl, Trifluormethoxy, Nitro, Cyano, Amino, C₁-C₆-Alkylamino, C₁-C₆-Dialkylamino, C₃-C₈-Cycloalkyl, C₆-C₁₀-Aryl, 5- bis 10-gliedriges Heteroaryl, 4- bis 10-gliedriges Heterocyclyl, Hydroxy, C₁-C₆-Alkoxy, Carboxyl, C₁-C₆-Alkoxycarbonyl, Aminocarbonyl, C₁-C₆-Alkylaminocarbonyl und C₁-C₆-Dialkylaminocarbonyl,
worin Alkyl, Alkylamino, Dialkylamino, Cycloalkyl, Aryl, Heteroaryl und Heterocyclyl substituiert sein können mit 0, 1, 2 oder 3 Substituenten R⁵⁻², wobei die Substituenten R⁵⁻² unabhängig voneinander ausgewählt werden aus der Gruppe bestehend aus Hydroxy, Amino, Carboxyl und Aminocarbonyl,
R⁶ gleich Wasserstoff, C₁-C₆-Alkyl oder C₃-C₈-Cycloalkyl ist,
oder
R⁵ und R⁶ zusammen mit dem Stickstoffatom, an das sie gebunden sind, einen 4- bis 10-gliedrigen Heterocyclus bilden, der substituiert sein kann mit 0, 1, 2 oder 3 Substituenten R⁵⁻⁶, wobei die Substituenten R⁵⁻⁶ unabhängig voneinander ausgewählt werden aus der Gruppe bestehend aus Halogen, C₁-C₆-Alkyl, Amino, C₁-C₆-Alkylamino, C₁-C₆-Dialkylamino, Hydroxy, C₁-C₆-Alkoxy, Carboxyl, C₁-C₆-Alkoxycarbonyl, Aminocarbonyl, C₁-C₆-Alkylaminocarbonyl und C₁-C₆-Dialkylaminocarbonyl,
R⁷ gleich Wasserstoff, C₁-C₆-Alkyl, C₁-C₆-Alkylcarbonyl oder C₃-C₈-Cycloalkyl ist,
R⁸ gleich Wasserstoff ist,
und
R⁹ gleich Wasserstoff ist.

4. Verbindung nach einem der Ansprüche 1 bis 3, **dadurch gekennzeichnet, dass**
R¹ gleich Wasserstoff ist,
R² gleich Wasserstoff ist,
R³ gleich Aminocarbonylmethyl, 3-Aminoprop-1-yl, 2-Hydroxy-3-aminoprop-1-yl, 1-Hydroxy-3-aminoprop-1-yl, 3-Guanidinoprop-1-yl, 2-Aminocarbonyl-ethyl, 2-Hydroxycarbonylethyl, 4-Aminobut-1-yl, Hydroxymethyl, 2-Hydroxyethyl, 2-Aminoethyl, 4-Amino-3-hydroxybut-1-yl oder (1-Piperidin-3-yl)-methyl ist,
R^{3'} gleich Wasserstoff ist,
R⁴ gleich Wasserstoff, Methyl, Ethyl, iso-Propyl oder Cyclopropyl ist,
R⁵ gleich Wasserstoff, C₁-C₆-Alkyl oder C₃-C₈-Cycloalkyl ist,
wobei Alkyl und Cycloalkyl substituiert sein können mit 0, 1, 2 oder 3 Substituenten R⁵⁻¹, wobei die Substituenten R⁵⁻¹ unabhängig voneinander ausgewählt werden aus der Gruppe bestehend aus Halogen, C₁-C₆-Alkyl, Trifluormethyl, Trifluormethoxy, Amino, C₁-C₆-Alkylamino, C₁-C₆-Dialkylamino, C₃-C₈-Cycloalkyl, C₆-C₁₀-Aryl, 5- bis 10-gliedriges Heteroaryl, 5- bis 7-gliedriges Heterocyclyl, Hydroxy, C₁-C₆-Alkoxy, Carboxyl, C₁-C₆-Alkoxycarbonyl, Aminocarbonyl, C₁-C₆-Alkylaminocarbonyl und C₁-C₆-Dialkylaminocarbonyl,
R⁶ gleich Wasserstoff oder Methyl ist,
oder
R⁵ und R⁶ zusammen mit dem Stickstoffatom, an das sie gebunden sind, ein Piperidinyl oder Morpholinyl bilden,
R⁷ gleich Wasserstoff ist,
R⁸ gleich Wasserstoff ist,
und
R⁹ gleich Wasserstoff ist.

5. Verbindung nach einem der Ansprüche 1 bis 4, **dadurch gekennzeichnet, dass**
R¹ gleich Wasserstoff ist,
R² gleich Wasserstoff ist,
R³ gleich 3-Aminoprop-1-yl oder 2-Hydroxy-3-aminoprop-1-yl ist,
R^{3'} gleich Wasserstoff ist,
R⁴ gleich Wasserstoff oder Methyl ist,
R⁵ gleich Wasserstoff, C₁-C₆-Alkyl oder Cyclopropyl ist,
wobei Alkyl substituiert sein kann mit 0, 1, 2 oder 3 Substituenten R⁵⁻¹, wobei die Substituenten R⁵⁻¹ unabhängig voneinander ausgewählt werden aus der Gruppe bestehend aus Trifluormethyl, Amino, Hydroxy, Carboxyl, Aminocarbonyl und Phenyl,
R⁶ gleich Wasserstoff oder Methyl ist,
R⁷ gleich Wasserstoff ist,
R⁸ gleich Wasserstoff ist,
und
R⁹ gleich Wasserstoff ist.

6. Verbindung nach einem der Ansprüche 1 bis 3, **dadurch gekennzeichnet, dass** R¹ gleich Wasserstoff ist.

7. Verbindung nach einem der Ansprüche 1, 2 und 6, **dadurch gekennzeichnet, dass** R² gleich Wasserstoff ist.

8. Verbindung nach einem der Ansprüche 1 bis 4, 6 und 7, **dadurch gekennzeichnet, dass** R³ gleich 3-Aminoprop-1-yl oder 2-Hydroxy-3-aminoprop-1-yl ist.

9. Verbindung nach einem der Ansprüche 1 bis 3 oder 6 bis 8, **dadurch gekennzeichnet, dass** R^{3'} gleich Wasserstoff ist.

10. Verbindung nach einem der Ansprüche 1 bis 4 oder 6 bis 9, **dadurch gekennzeichnet, dass** R⁴ gleich Wasserstoff oder Methyl.

11. Verbindung nach einem der Ansprüche 1 bis 4 oder 6 bis 10, **dadurch gekennzeichnet, dass**
R⁵ gleich Wasserstoff, C₁-C₆-Alkyl oder Cyclopropyl ist,
wobei Alkyl substituiert sein kann mit 0, 1, 2 oder 3 Substituenten R⁵⁻¹, wobei die Substituenten R⁵⁻¹ unabhängig voneinander ausgewählt werden aus der Gruppe bestehend aus Trifluormethyl, Amino, Hydroxy, Carboxyl, Aminocarbonyl und Phenyl.

12. Verbindung nach einem der Ansprüche 1 bis 3 oder 6 bis 11, **dadurch gekennzeichnet, dass** R⁶ gleich Wasserstoff oder Methyl ist.

13. Verbindung nach einem der Ansprüche 1 bis 4 oder 6 bis 12, **dadurch gekennzeichnet, dass** R⁵ und R⁶ zusammen mit dem Stickstoffatom, an das sie gebunden sind, ein Piperidinyl oder Morpholinyl bilden.

14. Verbindung nach einem der Ansprüche 1 bis 3 oder 6 bis 13, **dadurch gekennzeichnet, dass** R⁷ gleich Wasserstoff ist.

15. Verbindung nach einem der Ansprüche 1, 2, 6 bis 14, **dadurch gekennzeichnet, dass** R⁸ gleich Wasserstoff ist.

16. Verbindung nach einem der Ansprüche 1, 2, 6 bis 15, **dadurch gekennzeichnet, dass** R⁹ gleich Wasserstoff ist.

17. Verfahren zur Herstellung einer Verbindung der Formel (I) nach Anspruch 1, **dadurch gekennzeichnet, dass** eine Verbindung der Formel 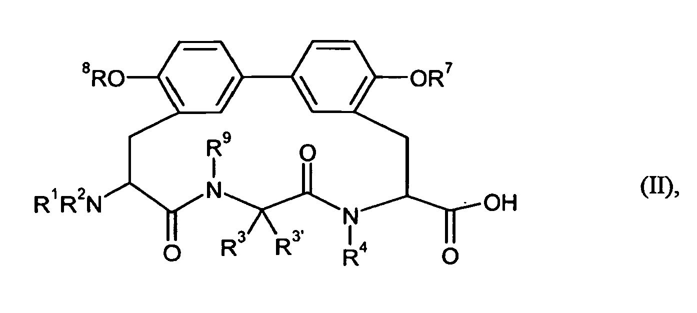 worin R¹ bis R⁴ und R⁷ bis R⁹ die in Anspruch 1 angegebene Bedeutung haben,
mit einer Verbindung der Formel
H-NR⁵R⁶ (III),
worin R⁵ und R⁶ die in Anspruch 1 angegebene Bedeutung haben, umgesetzt wird.

18. Verbindung nach einem der Ansprüche 1 bis 16 zur Behandlung und/oder Prophylaxe von Krankheiten.

19. Arzneimittel enthaltend mindestens eine Verbindung nach einem der Ansprüche 1 bis 16 in Kombination mit mindestens einem pharmazeutisch verträglichen, pharmazeutisch unbedenklichen Träger oder sonstigen Exzipienten.

20. Verwendung einer Verbindung nach einem der Ansprüche 1 bis 16 zur Herstellung eines Arzneimittels zur Behandlung und/oder Prophylaxe von bakteriellen Erkrankungen.

21. Arzneimittel nach Anspruch 19 zur Behandlung und/oder Prophylaxe von bakteriellen Infektionen.

## Claims

1. Compound of formula 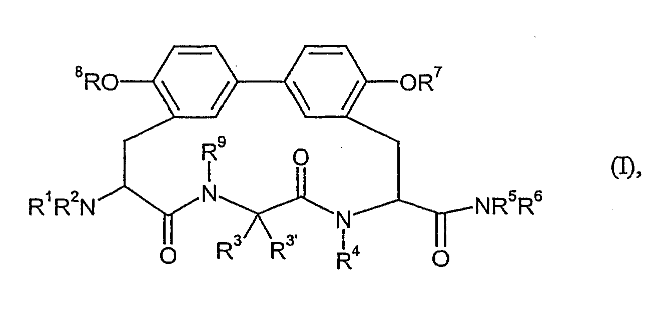 wherein
R¹ is hydrogen, C₁-C₆-alkyl, C₆-C₁₀-aryl, 5- to 10-membered heteroaryl, 4-to 10-membered heterocyclyl, C₁-C₆-alkylcarbonyl, C₆-C₁₀-arylcarbonyl, 4- to 10-membered heterocyclylcarbonyl, 5- to 10-membered heteroarylcarbonyl, C₁-C₆-alkoxycarbonyl, aminocarbonyl, C₁-C₆-alkylaminocarbonyl, C₁-C₆-dialkylaminocarbonyl, C₁-C₆-alkylsulfonyl, C₆-C₁₀-arylsulfonyl, 4- to 10-membered heterocyclylsulfonyl, 5- to 10-membered heteroarylsulfonyl or an α-amino acid residue in L- or D-configuration bonded via the carbonyl group of the amino acid acid functionality,
whereby R¹ apart from hydrogen may be substituted with 0, 1, 2 or 3 substituents R¹⁻¹, whereby the substituents R¹⁻¹ are selected independently of one another from the group consisting of halogen, C₁-C₆-alkyl, trifluoromethyl, trifluoromethoxy, nitro, cyano, amino, C₁-C₆-alkylamino, C₁-C₆-dialkylamino, C₃-C₈-cycloalkyl, C₆-C₁₀-aryl, 5- to 10-membered heteroaryl, 4- to 10-membered heterocyclyl, hydroxy, C₁-C₆-alkoxy and carboxy,
R² is hydrogen or C₁-C₆-alkyl,
whereby R² apart from hydrogen may be substituted with 0, 1, 2 or 3 substituents R²⁻¹, whereby the substituents R²⁻¹ are selected independently of one another from the group consisting of halogen, amino, C₁-C₆-alkylamino and C₁-C₆-dialkylamino,
or
R¹ and R² together with the nitrogen atom to which they are bonded form a 4-to 10-membered heterocycle which may be substituted with 0, 1 or 2 substituents R¹⁻², whereby the substituents R¹⁻² are selected independently of one another from the group consisting of halogen, trifluoromethyl, amino, C₁-C₆-alkylamino, C₁-C₆-dialkylamino, C₃-C₈-cycloalkyl, C₆-C₁₀-aryl, 5- to 10-membered heteroaryl, 4- to 10-membered heterocyclyl, hydroxy, C₁-C₆-alkoxy, carboxy, C₁-C₆-alkoxycarbonyl and aminocarbonyl,
R³ is hydrogen, C₁-C₆-alkyl or the side group of a naturally occurring α-amino acid in L- or D-configuration, wherein alkyl may be substituted with 0, 1, 2 or 3 substituents R³⁻¹, whereby the substituents R³⁻¹ are selected independently of one another from the group consisting of trifluoromethyl, nitro, amino, C₁-C₆-alkylamino, C₁-C₆-dialkylamino, C₃-C₈-cycloalkyl, C₆-C₁₀-aryl, 5- to 10-membered heteroaryl, 4- to 10-membered heterocyclyl, hydroxy, C₁-C₆-alkoxy, carboxy, C₁-C₆-alkoxycarbonyl, aminocarbonyl, C₁-C₆-alkylaminocarbonyl, C₁-C₆-dialkylaminocarbonyl, guanidino and amidino,
wherein cycloalkyl, aryl, heteroaryl and heterocyclyl may be substituted with 0, 1 or 2 substituents R³⁻², whereby the substituents R³⁻² are selected independently of one another from the group consisting of halogen, C₁-C₆-alkyl, trifluoromethyl and amino,
and wherein free amino groups in the side group of the amino acid may be substituted with C₁-C₆-alkyl, C₂-C₆-alkenyl, C₂-C₆-alkynyl, C₃-C₈-cycloalkyl, C₆-C₁₀-aryl, 5- to 10-membered heteroaryl, 4- to 10-membered heterocyclyl, C₁-C₆-alkylcarbonyl, C₆-C₁₀-arylcarbonyl, 5- to 10-membered heteroarylcarbonyl, 4- to 10-membered heterocyclylcarbonyl, C₁-C₆-alkoxycarbonyl, aminocarbonyl, C₁-C₆-alkylaminocarbonyl, C₁-C₆-dialkylaminocarbonyl, C₆-C₁₀- arylaminocarbonyl, C₁-C₆-alkylsulfonyl, C₆-C₁₀-arylsulfonyl, 4- to 10-membered heterocyclylsulfonyl or 5- to 10-membered heteroarylsulfonyl,
R^{3'} is hydrogen, C₁-C₆-alkyl or C₃-C₈-cycloalkyl,
R⁴ is hydrogen, C₁-C₆-alkyl or C₃-C₈-cycloalkyl,
R⁵ is hydrogen, C₁-C₆-alkyl, C₂-C₆-alkenyl, C₃-C₈-cycloalkyl, C₆-C₁₀-aryl, 5-to 10-membered heteroaryl, 4- to 10-membered heterocyclyl or an α-amino acid residue in L- or D-configuration bonded via the amino group of the amino acid,
whereby R⁵ may be substituted with 0, 1, 2 or 3 substituents R⁵⁻¹, whereby the substituents R⁵⁻¹ are selected independently of one another from the group consisting of halogen, C₁-C₆-alkyl, trifluoromethyl, trifluoromethoxy, nitro, cyano, amino, C₁-C₆-alkylamino, C₁-C₆-dialkylamino, C₃-C₈-cycloalkyl, C₆-C₁₀-aryl, 5- to 10-membered heteroaryl, 4- to 10-membered heterocyclyl, hydroxy, C₁-C₆-alkoxy, carboxy, C₁-C₆-alkoxycarbonyl, aminocarbonyl, C₁-C₆-alkylaminocarbonyl, C₁-C₆-dialkylaminocarbonyl, aminosulfonyl, C₁-C₆-alkylaminosulfonyl, C₁-C₆-dialkylaminosulfonyl, C₆-C₁₀-arylaminosulfonyl, 4- to 10-membered heterocyclylaminosulfonyl, 5- to 10-membered heteroarylaminosulfonyl, aminocarbonylamino, hydroxycarbonylamino and C₁-C₆-alkoxycarbonylamino,
wherein alkyl, alkylamino, dialkylamino, cycloalkyl, aryl, heteroaryl and heterocyclyl may be substituted with 0, 1, 2 or 3 substituents R⁵⁻², whereby the substituents R⁵⁻² are selected independently of one another from the group consisting of hydroxy, amino, carboxy and aminocarbonyl,
R⁶ is hydrogen, C₁-C₆-alkyl or C₃-C₈-cycloalkyl,
or
R⁵ and R⁶ together with the nitrogen atom to which they are bonded form a 4-to 10-membered heterocycle which may be substituted with 0, 1, 2 or 3 substituents R⁵⁻⁶, whereby the substituents R⁵⁻⁶ are selected independently of one another from the group consisting of halogen, C₁-C₆-alkyl, trifluoromethyl, nitro, amino, C₁-C₆-alkylamino, C₁-C₆-dialkylamino, C₃-C₈-cycloalkyl, C₆-C₁₀-aryl, halogenated C₆-C₁₀-aryl, 5- to 10-membered heteroaryl, 4- to 10-membered heterocyclyl, hydroxy, C₁-C₆-alkoxy, carboxy, C₁-C₆-alkylcarbonyl, C₁-C₆-alkoxycarbonyl, aminocarbonyl, C₁-C₆-alkylaminocarbonyl and C₁-C₆-dialkylaminocarbonyl,
R⁷ is hydrogen, C₁-C₆-alkyl, C₁-C₆-alkylcarbonyl or C₃-C₈-cycloalkyl,
R⁸ is hydrogen or C₁-C₆-alkyl, and
R⁹ is hydrogen or C₁-C₆-alkyl,
or one of the salts, the solvates or the solvates of the salts thereof.

2. Compound according to claim 1, **characterized in that** it corresponds to formula 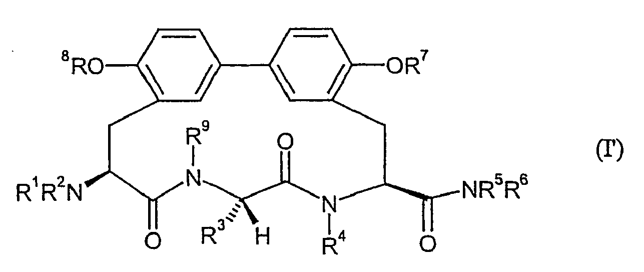 wherein R¹ to R⁹ have the same meaning as in formula (I).

3. Compound according to claim 1 or 2, **characterized in that**
R¹ is hydrogen, C₁-C₆-alkyl or C₁-C₆-alkylcarbonyl,
R² is hydrogen,
R³ is C₁-C₆-alkyl or the side group of a naturally occurring α-amino acid in L- or D-configuration, wherein alkyl may be substituted with 0, 1, 2 or 3 substituents R³⁻¹, whereby the substituents R³⁻¹ are selected independently of one another from the group consisting of trifluoromethyl, nitro, amino, C₁-C₆-alkylamino, C₁-C₆-dialkylamino, C₃-C₈-cycloalkyl, C₆-C₁₀-aryl, 5- to 10-membered heteroaryl, 4- to 10-membered heterocyclyl, hydroxy, C₁-C₆-alkoxy, carboxy, C₁-C₆-alkoxycarbonyl, aminocarbonyl, C₁-C₆-alkylaminocarbonyl, C₁-C₆-dialkylaminocarbonyl, guanidino and amidino,
wherein cycloalkyl, aryl, heteroaryl and heterocyclyl may be substituted with 0, 1 or 2 substituents R³⁻², whereby the substituents R³⁻² are selected independently of one another from the group consisting of halogen, C₁-C₆-alkyl, trifluoromethyl and amino,
and wherein free amino groups in the side group of the amino acid may be substituted with C₁-C₆-alkyl,
R^{3'} is hydrogen, C₁-C₆-alkyl or C₃-C₈-cycloalkyl,
R⁴ is hydrogen, C₁-C₆-alkyl or C₃-C₈-cycloalkyl,
R⁵ is hydrogen, C₁-C₆-alkyl, C₂-C₆-alkenyl, C₃-C₈-cycloalkyl, C₆-C₁₀-aryl, 5-to 10-membered heteroaryl, 4- to 10-membered heterocyclyl or an α-amino acid residue in L- or D-configuration bonded via the amino group of the amino acid,
whereby alkyl, alkenyl, cycloalkyl, aryl, heteroaryl and heterocyclyl may be substituted with 0, 1, 2 or 3 substituents R⁵⁻¹, whereby the substituents R⁵⁻¹ are selected independently of one another from the group consisting of halogen, C₁-C₆-alkyl, trifluoromethyl, trifluoromethoxy, nitro, cyano, amino, C₁-C₆-alkylamino, C₁-C₆-dialkylamino, C₃-C₈-cycloalkyl, C₆-C₁₀-aryl, 5- to 10-membered heteroaryl, 4- to 10-membered heterocyclyl, hydroxy, C₁-C₆-alkoxy, carboxy, C₁-C₆-alkoxycarbonyl, aminocarbonyl, C₁-C₆-alkylaminocarbonyl and C₁-C₆-dialkylaminocarbonyl,
wherein alkyl, alkylamino, dialkylamino, cycloalkyl, aryl, heteroaryl and heterocyclyl may be substituted with 0, 1, 2 or 3 substituents R⁵⁻², whereby the substituents R⁵⁻² are selected independently of one another from the group consisting of hydroxy, amino, carboxy and aminocarbonyl,
R⁶ is hydrogen, C₁-C₆-alkyl or C₃-C₈-cycloalkyl,
or
R⁵ and R⁶ together with the nitrogen atom to which they are bonded form a 4-to 10-membered heterocycle which may be substituted with 0, 1, 2 or 3 substituents R⁵⁻⁶, whereby the substituents R⁵⁻⁶ are selected independently of one another from the group consisting of halogen, C₁-C₆-alkyl, amino, C₁-C₆-alkylamino, C₁-C₆-dialkylamino, hydroxy, C₁-C₆-alkoxy, carboxy, C₁-C₆-alkoxycarbonyl, aminocarbonyl, C₁-C₆-alkylaminocarbonyl and C₁-C₆-dialkylaminocarbonyl,
R⁷ is hydrogen, C₁-C₆-alkyl, C₁-C₆-alkylcarbonyl or C₃-C₈-cycloalkyl,
R⁸ is hydrogen,
and
R⁹ is hydrogen.

4. Compound according to any one of claims 1 to 3, **characterized in that**
R¹ is hydrogen,
R² is hydrogen,
R³ is aminocarbonylmethyl, 3-aminoprop-1-yl, 2-hydroxy-3-aminoprop-1-yl, 1-hydroxy-3-aminoprop-1-yl, 3-guanidinoprop-1-yl, 2-aminocarbonlyethyl, 2-hydroxycarbonylethyl, 4-aminobut-1-yl, hydroxymethyl, 2-hydroxyethyl, 2-aminoethyl, 4-amino-3-hydroxybut-1-yl or (1-piperidin-3-yl)methyl,
R^{3'} is hydrogen,
R⁴ is hydrogen, methyl, ethyl, isopropyl or cyclopropyl,
R⁵ is hydrogen, C₁-C₆-alkyl or C₃-C₈-cycloalkyl,
whereby alkyl and cycloalkyl may be substituted with 0, 1, 2 or 3 substituents R⁵⁻¹, whereby the substituents R⁵⁻¹ are selected independently of one another from the group consisting of halogen, C₁-C₆-alkyl, trifluoromethyl, trifluoromethoxy, amino, C₁-C₆-alkylamino, C₁-C₆-dialkylamino, C₃-C₈-cycloalkyl, C₆-C₁₀-aryl, 5- to 10-membered heteroaryl, 5- to 7-membered heterocyclyl, hydroxy, C₁-C₆-alkoxy, carboxy, C₁-C₆-alkoxycarbonyl, aminocarbonyl, C₁-C₆-alkylaminocarbonyl and C₁-C₆-dialkylaminocarbonyl,
R⁶ is hydrogen or methyl,
or
R⁵ and R⁶ together with the nitrogen atom to which they are bonded form a piperidinyl or morpholinyl,
R⁷ is hydrogen,
R⁸ is hydrogen,
and
R⁹ is hydrogen.

5. Compound according to any one of claims 1 to 4, **characterized in that**
R¹ is hydrogen,
R² is hydrogen,
R³ is 3-aminoprop-1-yl or 2-hydroxy-3-aminoprop-1-yl,
R^{3'} is hydrogen,
R⁴ is hydrogen or methyl,
R⁵ is hydrogen, C₁-C₆-alkyl or cyclopropyl,
whereby alkyl may be substituted with 0, 1, 2 or 3 substituents R⁵⁻¹, whereby the substituents R⁵⁻¹ are selected independently of one another from the group consisting of trifluoromethyl, amino, hydroxy, carboxy, aminocarbonyl and phenyl,
R⁶ is hydrogen or methyl,
R⁷ is hydrogen,
R⁸ is hydrogen
and
R⁹ is hydrogen.

6. Compound according to any one of claims 1 to 3, **characterized in that** R¹ is hydrogen.

7. Compound according to any one of claims 1, 2 and 6, **characterized in that** R² is hydrogen.

8. Compound according to any one of claims 1 to 4, 6 and 7, **characterized in that** R³ is 3-aminoprop-1-yl or 2-hydroxy-3-aminoprop-1-yl.

9. Compound according to any one of claims 1 to 3 or 6 to 8, **characterized in that** R^{3'} is hydrogen.

10. Compound according to any one of claims 1 to 4 or 6 to 9, **characterized in that** R⁴ is hydrogen or methyl.

11. Compound according to any one of claims 1 to 4 or 6 to 10, **characterized in that**
R⁵ is hydrogen, C₁-C₆-alkyl or cyclopropyl,
whereby alkyl may be substituted with 0, 1, 2 or 3 substituents R⁵⁻¹, whereby the substituents R⁵⁻¹ are selected independently of one another from the group consisting of trifluoromethyl, amino, hydroxy, carboxy, aminocarbonyl and phenyl.

12. Compound according to any one of claims 1 to 3 or 6 to 11, **characterized in that** R⁶ is hydrogen or methyl.

13. Compound according to any one of claims 1 to 4 or 6 to 12, **characterized in that** R⁵ and R⁶ together with the nitrogen atom to which they are bonded form a piperidinyl or morpholinyl.

14. Compound according to any one of claims 1 to 3 or 6 to 13, **characterized in that** R⁷ is hydrogen.

15. Compound according to any one of claims 1, 2, 6 to 14, **characterized in that** R⁸ is hydrogen.

16. Compound according to any one of claims 1, 2, 6 to 15, **characterized in that** R⁹ is hydrogen.

17. Process for preparing a compound of the formula (I) according to claim 1, **characterized in that** a compound of formula 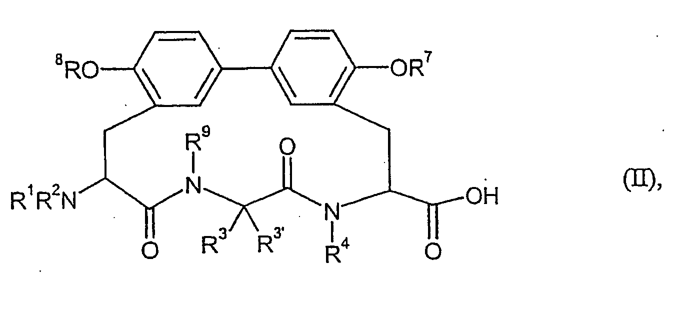 wherein R¹ to R⁴ and R⁷ to R⁹ have the meaning indicated in claim 1,
is reacted with a compound of formula
H-NR⁵R⁶ (III),
wherein R⁵ and R⁶ have the meaning indicated in claim 1.

18. Compound according to any one of claims 1 to 16 for the treatment and/or prophylaxis of diseases.

19. Medicament comprising at least one compound according to any one of claims 1 to 16 in combination with at least one pharmaceutically suitable, pharmaceutically acceptable carrier or other excipients.

20. Use of a compound according to any one of claims 1 to 16 for the production of a medicament for the treatment and/or prophylaxis of bacterial diseases.

21. Medicament according to claim 19 for the treatment and/or prophylaxis of bacterial infections.

## Revendications

1. Composé de formule 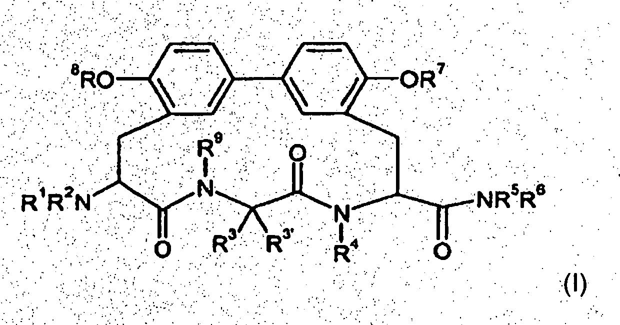 dans laquelle
R¹ représente un hydrogène, un alkyle en C₁-C₆, un aryle en C₆-C₁₀, un hétéroaryle de 5 à 10 atomes, un hétérocyclyle de 4 à 10 atomes, un alkylcarbonyle en C₁-C₆, un arylcarbonyle en C₆-C₁₀, un héterocyclylcarbonyle de 4 à 10 atomes, un hétéroarylcarbonyle de 5 à 10 atomes, un alcoxycarbonyle en C₁-C₆, un aminocarbonyle, un alkylaminocarbonyle en C₁-C₆, un dialkylaminocarbonyle en C₁-C₆, un alkylsulfonyle en C₁-C₆, un arylsulfonyle en C₆-C₁₀, un hétérocyclylsulfonyle de 4 à 10 atomes, un hétéroarylsulfonyle de 5 à 10 atomes ou le reste d'un α-acide aminé en configuration L ou D lié par le biais du groupe carbonyle de la fonction acide de l'acide aminé,
où R¹ peut être substitué, outre l'hydrogène, par 0, 1, 2 ou 3 substituants R¹⁻¹, où les substituants R¹⁻¹ sont choisis indépendamment les uns des autres dans le groupe constitué par les halogènes, les alkyles en C₁-C₆, le trifluorométhyle, le trifluorométhoxy, les nitros, les cyanos, les aminos, les alkylaminos en C₁-C₆, les dialkylaminos en C₁-C₆, les cycloalkyles en C₃-C₈, les aryles en C₆-C₁₀, les hétéroaryles de 5 à 10 atomes, les hétérocyclyles de 4 à 10 atomes, les hydroxy, les alcoxy en C₁-C₆ et les carboxyles,
R² représente un hydrogène ou un alkyle en C₁-C₆,
où R² peut être substitué, outre l'hydrogène, par 0, 1, 2 ou 3 substituants R²⁻¹, où les substituants R²⁻¹ sont choisis indépendamment les uns des autres dans le groupe constitué par les halogènes, les aminos, les alkylaminos en C₁-C₆ et les dialkylaminos en C₁-C₆,
ou
R¹ et R², ensemble avec l'atome d'azote, auquel ils sont liés, forment un hétérocycle de 4 à 10 atomes, qui peut être substitué par 0, 1 ou 2 substituants R¹⁻², où les substituants R¹⁻² sont choisis indépendamment les uns des autres dans le groupe constitué par les halogènes, le trifluorométhyle, les aminos, les alkylaminos en C₁-C₆, les dialkylaminos en C₁-C₆, les cycloalkyles en C₃-C₈, les aryles en C₆-C₁₀, les hétéroaryles de 5 à 10 atomes, les hétérocyclyles de 4 à 10 atomes, les hydroxy, les alcoxy en C₁-C₆, les carboxyles, les alcoxycarboniles en C₁-C₆ et les aminocarbonyles,
R³ représente un hydrogène, un alkyle en C₁-C₆ ou la chaîne latérale d'un α-acide aminé naturel en configuration L ou D, où l'alkyle peut être substitué par 0, 1, 2 ou 3 substituants R³⁻¹, où les substituants R³⁻¹ sont choisis indépendamment les uns des autres dans le groupe constitué par le trifluorométhyle, les nitros, les aminos, les alkylaminos en C₁-C₆, les dialkylaminos en C₁-C₆, les cycloalkyles en C₃-C₈, les aryles en C₆-C₁₀, les hétéroaryles de 5 à 10 atomes, les hétérocyclyles de 4 à 10 atomes, les hydroxy, les alcoxy en C₁-C₆, les carboxyles, les alcoxycarbonyles en C₁-C₆, les aminocarbonyles, les alkylaminocarbonyles en C₁-C₆, les dialkylaminocarbonyles en C₁-C₆, les guanidinos et les amidinos,
où les cycloalkyles, les aryles, les hétéroaryles et les hétérocyclyles peuvent être substitués par 0, 1 ou 2 substituants R³⁻², où les substituants R³⁻² sont choisis indépendamment les uns des autres dans le groupe constitué par les halogènes, les alkyles en C₁-C₆, le trifluorométhyle et les aminos,
et où les groupes amino libres dans la chaîne latérale de l'acide aminé peuvent être substitués par un alkyle en C₁-C₆, un alcényle en C₂-C₆, un alcinyle en C₂-C₆, un cycloalkyle en C₃-C₈, un aryle en C₆-C₁₀, un hétéroaryle de 5 à 10 atomes, un hétérocyclyle de 4 à 10 atomes, un alkylcarbonyle en C₁-C₆, un arylcarbonyle en C₆-C₁₀, un hétéroarylcarbonyle de 5 à 10 atomes, un hétérocyclylcarbonyle de 4 à 10 atomes, un alcoxycarbonyle en C₁-C₆, un aminocarbonyle, un alkylaminocarbonyle en C₁-C₆, un dialkylaminocarbonyle en C₁-C₆, un aryleaminocarbonyle en C₆-C₁₀, un alkylsulfonyle en C₁-C₆, un arylesulfonyle en C₆-C₁₀, un hétérocyclylsulfonyle de 4 à 10 atomes ou un hétéroarylsulfonyle de 5 à 10 atomess,
R^{3'} représente un hydrogène, un alkyle en C₁-C₆ ou un cycloalkyle en C₃-C₈,
R⁴ représente un hydrogène, un alkyle en C₁-C₆ ou un cycloalkyle en C₃-C₈,
R⁵ représente un hydrogène, un alkyle en C₁-C₆, un alcényle en C₂-C₆, un cycloalkyle en C₃-C₈, un aryle en C₆-C₁₀, un hétéroaryle de 5 à 10 atomes, un héterocyclyle de 4 à 10 atomes, ou le reste d'un α-acide aminé en configuration L ou D lié par le biais du groupe amino de l'acide aminé,
où R⁵ peut être substitué par 0, 1, 2 ou 3 substituants R⁵⁻¹, où les substituants R⁵⁻¹ sont choisis indépendamment les uns des autres dans le groupe constitué par les halogènes, les alkyles en C₁-C₆, le trifluorométhyle, le trifluorométhoxy, les nitros, les cyanos, les aminos, les alkylaminos en C₁-C₆, les dialkylaminos en C₁-C₆, les cycloalkyles en C₃-C₈, les aryles en C₆-C₁₀, les hétéroaryles de 5 à 10 atomes, les hétérocyclyles de 4 à 10 atomes, les hydroxy, les alcoxy en C₁-C₆, les carboxyles, les alcoxycarbonyles en C₁-C₆, les aminocarbonyles, les alkylaminocarbonyles en C₁-C₆, les dialkylaminocarbonyles en C₁-C₆, les aminosulfonyles, les alkylaminosulfonyles en C₁-C₆, les dialkylaminosulfonyles en C₁-C₆, les arylaminosulfonyles en C₆-C₁₀, les hétérocyclylaminosulfonyles de 4 à 10 atomes, les hétéroarylaminosulfonyles de 5 à 10 atomes, les aminocarbonylaminos, les hydroxycarbonylaminos et les alcoxycarbonylaminos en C₁-C₆,
où les alkyle, les alkylaminos, les dialkylaminos, les cycloalkyle, les aryles, les hétéroaryles et les hétérocyclyles peuvent être substitués par 0, 1, 2 ou 3 substituants R⁵⁻², où les substituants R⁵⁻² sont choisis indépendamment les uns des autres dans le groupe constitué par les hydroxy, les aminos, les carboxyles et les aminocarbonyles,
R⁶ représente un hydrogène, un alkyle en C₁-C₆ ou un cycloalkyle en C₃-C₈,
ou
R⁵ et R⁶, ensemble avec l'atome d'azote, auquel ils sont liés, forment un hétérocycle de 4 à 10 atomes, qui peut être substitué par 0, 1, 2 ou 3 substituants R⁵⁻⁶, où les substituants R⁵⁻⁶ sont choisis indépendamment les uns des autres dans le groupe constitué par les halogènes, les alkyle en C₁-C₆, le trifluorométhyle, les nitros, les aminos, les alkylaminos en C₁-C₆, les dialkylaminos en C₁-C₆, les cycloalkyles en C₃-C₈, les aryles en C₆-C₁₀, les aryles halogénés en C₆-C₁₀, les hétéroaryles de 5 à 10 atomes, les hétérocyclyles de 4 à 10 atomes, les hydroxy, les alcoxy en C₁-C₆, les carboxyles, les alkylcarbonyles en C₁-C₆, les alcoxycarbonyles en C₁-C₆, les aminocarbonyles, les alkylaminocarbonyles en C₁-C₆ et les dialkylaminocarbonyle en C₁-C₆,
R⁷ représente un hydrogène, un alkyle en C₁-C₆, un alkylcarbonyle en C₁-C₆ ou un cycloalkyle en C₃-C₈,
R⁸ représente un hydrogène ou un alkyle en C₁-C₆, et
R⁹ représente un hydrogène ou un alkyle en C₁-C₆,
ou un de ses sels, de ses solvates ou les solvates de ses sels.

2. Composé selon la revendication 1, **caractérisé en ce qu'**il correspond à la formule 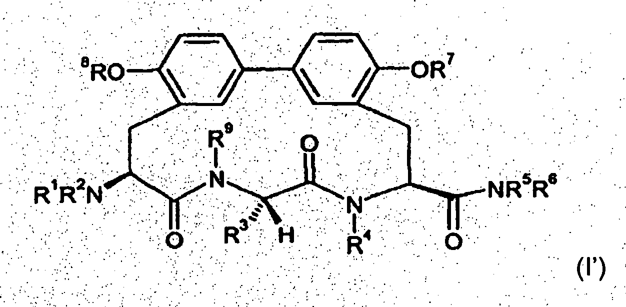 dans laquelle R¹ à R⁹ ont la même signification que dans la formule (I).

3. Composé selon la revendication 1 ou 2, **caractérisé en ce que**
R¹ représente un hydrogène, un alkyle en C₁-C₆, ou un alkylcarbonyle en C₁-C₆,
R² représente un hydrogène,
R³ représente un alkyle en C₁-C₆ ou la chaîne latérale d'un α-acide aminé naturel en configuration L ou D, où l'alkyle peut être substitué par 0, 1, 2 ou 3 substituants R³⁻¹, où les substituants R³⁻¹ sont choisis indépendamment les uns des autres dans le groupe constitué par le trifluorométhyle, les nitros, les aminos, les alkylaminos en C₁-C₆, les dialkylaminos en C₁-C₆, les cycloalkyles en C₃-C₈, les aryles en C₆-C₁₀, les hétéroaryles de 5 à 10 atomes, les hétérocyclyles de 4 à 10 atomes, les hydroxy, les alcoxy en C₁-C₆, les carboxyles, les alcoxycarbonyles en C₁-C₆, les aminocarbonyles, les alkylaminocarbohyles en C₁-C₆, les dialkylaminocarbonyles en C₁-C₆, les guanidinos et les amidinos,
où les cycloalkyles, les aryles, les hétéroaryles et les hétérocyclyles peuvent être substitués par 0, 1 ou 2 substituants R³⁻², où les substituants R³⁻² sont choisis indépendamment les uns des autres dans le groupe constitué par les halogènes, les alkyles en C₁-C₆, le trifluorométhyle et les aminos,
et où les groupes amino libres dans la chaîne latérale de l'acide aminé peuvent être substitués par un alkyle en C₁-C₆,
R³ représente un hydrogène, un alkyle en C₁-C₆ ou un cycloalkyle en C₃-C₈,
R⁴ représente un hydrogène, un alkyle en C₁-C₆ ou un cycloalkyle en C₃-C₈,
R⁵ représente un hydrogène, un alkyle en C₁-C₆, un alcényle en C₂-C₆, un cycloalkyle en C₃-C₈, un aryle en C₆-C₁₀, un hétéroaryle de 5 à 10 atomes, un hétérocyclyle de 4 à 10 atomes, ou le reste d'un α-acide aminé en configuration L ou D lié par le biais du groupe amino de l'acide aminé,
où les alkyles, les alcényles, les cycloalkyles, les aryles, les hétéroaryles et les hétérocyclyles peuvent être substitués par 0, 1, 2 ou 3 substituants R⁵⁻¹, où les substituants R⁵⁻¹ sont choisis indépendamment les uns des autres dans le groupe constitué par les halogènes, les alkyles en C₁-C₆, le trifluorométhyle, le trifluorométhoxy, les nitros, les cyanos, les aminos, les alkylaminos en C₁-C₆, les dialkylaminos en C₁-C₆, les cycloalkyles en C₃-C₈, les aryles en C₆-C₁₀, les hétéroaryles de 5 à 10 atomes, les hétérocyclyles de 4 à 10 atomes, les hydroxy, les alcoxy en C₁-C₆, les carboxyles, les alcoxycarbonyles en C₁-C₆, les aminocarbonyles, les alkylaminocarbonyles en C₁-C₆ et les dialkylaminocarbonyles en C₁-C₆,
où les alkyles, les alkylaminos, les dialkylaminos, les cycloalkyles, les aryles, les hétéroaryles et les hétérocyclyles peuvent être substitués par 0, 1, 2 ou 3 substituants R⁵⁻², où les substituants R⁵⁻² sont choisis indépendamment les uns des autres dans le groupe constitué par les hydroxy, les aminos, les carboxyles et les aminocarbonyles,
R⁶ représente un hydrogène, un alkyle en C₁-C₆ ou un cycloalkyle en C₃-C₈,
ou
R⁵ et R⁶, ensemble avec l'atome d'azote, auquel ils sont liés, forment un hétérocycle de 4 à 10 atomes, qui peut être substitué par 0, 1, 2 ou 3 substituants R⁵⁻⁶, où les substituants R⁵⁻⁶ sont choisis indépendamment les uns des autres dans le groupe constitué par les halogènes, les alkyles en C₁-C₆, les aminos, les alkylaminos en C₁-C₆, les dialkylaminos en C₁-C₆, les hydroxy, les alcoxy en C₁-C₆, les carboxyles, les alcoxycarbonyles en C₁-C₆, les aminocarbonyles, les alkylaminocarbonyles en C₁-C₆ et les dialkylaminocarbonyles en C₁-C₆,
R⁷ représente un hydrogène, un alkyle en C₁-C₆, un alkylcarbohyle en C₁-C₆ ou un cycloalkyle en C₃-C₈,
R⁸ représente un hydrogène,
et
R⁹ représente un hydrogène.

4. Composé selon l'une des revendications 1 à 3, **caractérisé en ce que**
R¹ représente un hydrogène,
R² représente un hydrogène,
R³ représente un aminocarbonylméthyle, un 3-aminoprop-1-yle, un 2-hydroxy-3-aminoprop-1-yle, un 1-hydroxy-3-aminoprop-1-yle, un 3-guanidinoprop-1-yle, un 2-aminocarbonyléthyle, un 2-hydroxycarbonyléthyle, un 4-aminobut-1-yle, un hyoroxyméthyle, un 2-hydroxyéthyle, un 2-aminoéthyle, un 4-amino-3-hydroxy-but-1-yle ou un (1-pipéridin-3-yl)-méthyle,
R^{3'} représente un hydrogène,
R⁴ représente un hydrogène, un méthyle, un éthyle, un isopropyle ou un cyclopropyle,
R⁵ représente un hydrogène, un alkyle en C₁-C₆, ou un cycloalkyle en C₃-C₈,
où l'alkyle et le cycloalkyle peuvent être substitués par 0, 1, 2 ou 3 substituants R⁵⁻¹, où les substituants R⁵⁻¹ sont choisis indépendamment les uns des autres dans le groupe constitué par les halogènes, les alkyles en C₁-C₆, le trifluorométhyle, le trifluorométhoxy, les aminos, les alkylaminos en C₁-C₆, les dialkylaminos en C₁-C₆, les cycloalkyles en C₃-C₈, les aryles en C₆-C₁₀, les hétéroaryles de 5 à 10 atomes, les hétérocyclyles de 5 à 7 atomes, les hydroxy, les alcoxy en C₁-C₆, les carboxyles, les alcoxycabonyles en C₁-C₆, les aminocarbonyles, les alkylaminocarbonyles en C₁-C₆ et les dialkylaminocarbonyles en C₁-C₆,
R⁶ représente un hydrogène ou un méthyle,
ou
R⁵ et R⁶, ensemble avec l'atome d'azote, auquel ils sont liés, forment un pipéridinyle ou un morpholinyle,
R⁷ représente un hydrogène,
R⁸ représente un hydrogène,
et
R⁹ représente un hydrogène.

5. Composé selon l'une des revendications 1 à 4, **caractérisé en ce que**
R¹ représente un hydrogène,
R² représente un hydrogène,
R³ représente un 3-aminoprop-1-yle ou un 2-hydroxy-3-aminoprop-1-yle,
R^{3'} représente un hydrogène,
R⁴ représente un hydrogène ou un méthyle,
R⁵ représente un hydrogène, un alkyle en C₁-C₆ ou un cyclopropyle,
où l'alkyle peut être substitué par 0, 1, 2 ou 3 substituants R⁵⁻¹, où les substituants R⁵⁻¹ sont choisis indépendamment les uns des autres dans le groupe constitué par le trifluorométhyle, les aminos, les hydroxy, les carboxyles, les aminocarbonyles et les phényles,
R⁶ représente un hydrogène ou un méthyle,
R⁷ représente un hydrogène,
R⁸ représente un hydrogène,
et
R⁹ représente un hydrogène.

6. Composé selon l'une des revendications 1 à 3, **caractérisé en ce que** R¹ représente un hydrogène.

7. Composé selon l'une des revendications 1, 2 et 6, **caractérisé en ce que** R² représente un hydrogène.

8. Composé selon l'une des revendications 1 à 4, 6 et 7, **caractérisé en ce que** R³ représente un 3-ammoprop-1-yle ou un 2-hydrox-3-aminoprop-1-yle.

9. Composé selon l'une des revendications 1 à 3 ou 6 à 8, **caractérisé en ce que** R^{3'} représente un hydrogène.

10. Composé selon l'une des revendications 1 à 4 ou 6 à 9, **caractérisé en ce que** R⁴ représente un hydrogène ou un méthyle.

11. Composé selon l'une des revendications 1 à 4 ou 6 à 10, **caractérisé en ce que**
R⁵ représente un hydrogène, un alkyle en C₁-C₆ ou un cyclopropyle,
où l'alkyle peut être substitué par 0, 1, 2 ou 3 substituants R⁵⁻¹, où les substituants R⁵⁻¹ sont choisis indépendamment les uns des autres dans le groupe constitué par le trifluorométhyle, les aminos, les hydroxy, les carboxyles, les aminocarbonyles et les phényles.

12. Composé selon l'une des revendications 1 à 3 ou 6 à 11, **caractérisé en ce que** R⁶ représente hydrogène ou méthyle.

13. Composé selon l'une des revendications 1 à 4 ou 6 à 12, **caractérisé en ce que** R⁵ et R⁶, ensemble avec l'atome d'azote, auquel ils sont liés, forment un pipéridinyle ou un morpholinyle.

14. Composé selon l'une des revendications 1 à 3 ou 6 à 13, **caractérisé en ce que** R⁷ représente un hydrogène.

15. Composé selon l'une des revendications 1, 2, 6 à 14, **caractérisé en ce que** R⁸ représente un hydrogène

16. Composé selon l'une des revendications 1, 2, 6 à 15, **caractérisé en ce que** R⁹ représente un hydrogène.

17. Procéde de préparation d'un composé de formule (I) selon la revendication 1, **caractérisé en ce que** l'on fait réagir un composé de formule 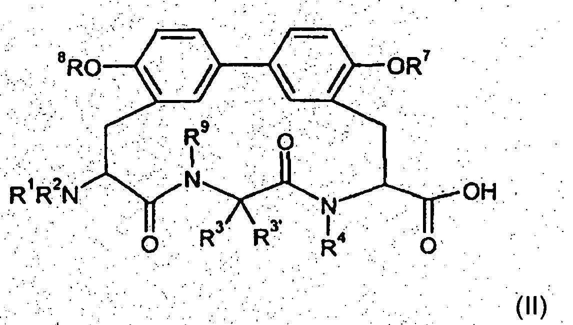 dans laquelle R¹ à R⁴ et R⁷ à R⁹ ont la signification indiquée dans la revendication 1,
avec un composé de formule
H-NR⁵R⁶ (III),
dans laquelle R⁵ et R⁶ ont la signification indiquée dans la revendication 1.

18. Composé selon l'une des revendications 1 à 16, pour le traitement et/ou la prophylaxie de maladies.

19. Médicament contenant au moins un composé selon l'une des revendications 1 à 16 en combinaison avec au moins un support pharmaceutiquement acceptable, pharmaceutiquement non toxique ou autres excipients.

20. Utilisation d'un composé selon l'une des revendications 1 à 16 pour la fabrication d'un médicament pour le traitement et/ou la prophylaxie de maladies bactériennes.

21. Médicament selon la revendication 19 pour le traitement et/ou la prophylaxie des infections bactériennes.
